(19) Europäisches Patentamt
European Patent Office
Office européen des brevets

(11) Publication number: **0 665 224 A1**

(12) **EUROPEAN PATENT APPLICATION published in accordance with Art. 158(3) EPC**

(21) Application number: **93922632.0**

(22) Date of filing: **14.10.93**

(86) International application number: **PCT/JP93/01478**

(87) International publication number: **WO 94/08975 (28.04.94 94/10)**

(51) Int. Cl.[6]: **C07D 239/26**, C07D 239/34, C07D 239/38, C07D 239/42, C07D 401/04, C07D 491/044, C07D 495/04, C07D 239/30, C07D 239/36, C07D 239/28, C07D 471/04, A01N 43/54

(30) Priority: **16.10.92 JP 304622/92**
**01.06.93 JP 154303/93**

(43) Date of publication of application: **02.08.95 Bulletin 95/31**

(84) Designated Contracting States: **AT BE CH DE DK ES FR GB GR IE IT LI LU MC NL PT SE**

(71) Applicant: **NIPPON SODA CO., LTD.**
**2-1, Ohtemachi 2-chome**
**Chiyoda-ku,**
**Tokyo 100 (JP)**

(72) Inventor: **YAMADA, Hirokazu, Odawara Research Center**
**Nippon Soda Co., Ltd.**
**345, Aza Yanagimachi**
**Takada, Odawara-shi**
**Kanagawa 250-02 (JP)**
Inventor: **TANAKA, Katsunori, Takaoka Plant**
**Nippon Soda Co., Ltd.**
**300, Mukainohonmachi**
**Takaoka-shi**
**Toyama 933 (JP)**
Inventor: **ADACHI, Hiroyuki, Odawara Research Center**
**Nippon Soda Co., Ltd.**
**345, Aza Yanagimachi**
**Takada, Odawara-shi**
**Kanagawa 250-02 (JP)**
Inventor: **YAMADA, Shigeo, Odawara Research Center**
**Nippon Soda Co., Ltd.**
**345, Aza Yanagimachi**
**Takada, Odawara-shi**
**Kanagawa 250-02 (JP)**
Inventor: **SHIMODA, Susumu, Odawara Research Center**
**Nippon Soda Co., Ltd.**
**345, Aza Yanagimachi**
**Takada, Odawara-shi**
**Kanagawa 250-02 (JP)**
Inventor: **HASHIMOTO, Sho Nippon Soda Co., Ltd.**
**2-1, Ohtemachi 2-chome**
**Chiyoda-ku**
**Tokyo 100 (JP)**
Inventor: **UMEDA, Nobuhiro Odawara Research Center**
**Nippon Soda Co. Ltd.**
**345, Aza Yanagimachi**
**Takada, Odawara-shi**
**Kanagawa 250-02 (JP)**

(74) Representative: **de Bruijn, Leendert C. et al**
**Nederlandsch Octrooibureau**
**Scheveningseweg 82**
**P.O. Box 29720**
**NL-2502 LS Den Haag (NL)**

(54) **PYRIMIDINE DERIVATIVE.**

(57) A compound represented by general formula (I) and excellent in herbicidal and agrohorticultural antibacterial activities, wherein $R^1$ and $R^2$ represent each hydrogen, alkyl, etc., or alternatively they may be combined



Rank Xerox (UK) Business Services
(3.10/3.09/3.3.4)

together to represent oxo, thioxo, etc., or a spiro ring selected from among (a), (b), etc.; $R^3$ and $R^5$ represent each hydrogen, halogen, alkyl, etc., and $R^4$ represents hydrogen, halogen, amino, etc., provided that $R^3$ and $R^5$ are different from each other when $R^4$ is hydrogen and that $R^4$ may be combined with $R^3$ or $R^5$ and the pyrimidine ring to represent a condensed ring represented by (c) or (d); $R^6$ and $R^7$ represent each hydrogen, halogen, alkyl, etc., provided that the case wherein both $R^6$ and $R^7$ represent hydrogen is excluded; $R^8$ represents halogen, alkyl, etc.; and n represents 0, 1, 2 or 3; provided that when both $R^1$ and $R^2$ represent hydrogen, then $R^4$ is combined with $R^3$ or $R^5$ and the pyrimidine group to represent the above condensed ring.

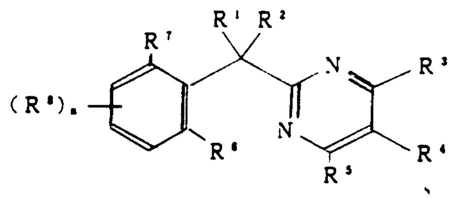

(I)

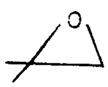

(a)

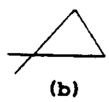

(b)

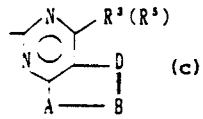

(c)

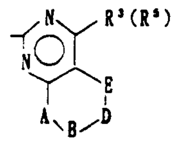

(d)

Background Arts

In cultivation of agricultural and horticultural crops, a number of herbicides have been used for weed control for which great amount of labour has been required, while many chemical agents have been also used for the control of plant diseases and pest insects on various crops. However, it is still urgently required to develop novel compounds which can provide firm controlling effectiveness on weeds, diseases and pest insects with less dosages and can be used safely, because of insufficiency of presently available agricultural chemicals in controlling effectiveness, restriction to those chemicals in their use arising from the appearance of the strains of pathogenic organisms and pest insects which are resistant to such chemicals, phytotoxicity of the chemicals, and residues and contamination thereof in and into the environment.

In some publications such as European Patent No. 461, 079, WO 91/10653, and J. Chem. Res. (S), 1977, 186, some compounds similar to the compounds specified in the present invention are disclosed.

It is an object of the present invention to provide novel compounds which can be synthesized advantageously in an industrial scale and can be used as selective herbicides or fungicides for agricultural and horticultural use, which are highly safe and can provide firm effectiveness against weeds or pathogenic organisms at less dosages.

Disclosure of the Invention

The present invention is directed to pyrimidine derivatives represented by a general formula (I), and herbicides and fungicides for agricultural and horticultural use, which comprise the said pyrimidine derivatives:

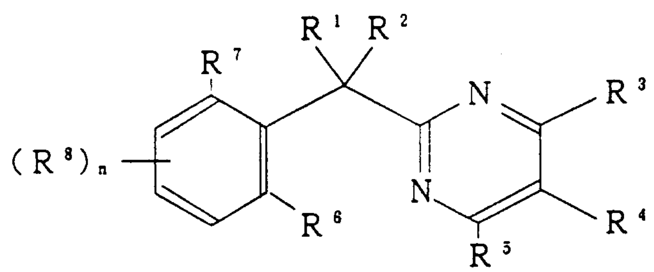

(I)

wherein $R^1$ and $R^2$ are each independently hydrogen; alkyl; alkenyl; alkynyl; phenyl; cyano; $COOR^9$ wherein $R^9$ is hydrogen or alkyl; halogen; $OR^{10}$ where in $R^{10}$ is hydrogen, alkyl, alkenyl, alkynyl, phenyl or cycloalkyl; $SR^{11}$ wherein $R^{11}$ is hydrogen, alkyl, alkenyl, alkynyl, phenyl or cycloalkyl; $OCOR^{12}$ wherein $R^{12}$ is hydrogen, alkyl, alkenyl, alkynyl, phenyl or cycloalkyl; amino, $Z^1P(=Z^2)(Z^3R^{13})(Z^4R^{14})$ wherein $R^{13}$ and $R^{14}$ are each independently hydrogen, alkyl, alkenyl, alkynyl, phenyl or cycloalkyl, $Z^1$, $Z^2$, $Z^3$, and $Z^4$ are each independently O or S, or they form in together oxo, thioxo, cyclic ketal, cyclic thioketal, $=CR^{15}R^{16}$ - (wherein $R^{15}$ and $R^{1}$11are each independently hydrogen, nitro, cyano, alkyl, alkenyl, alkynyl, alkylcarbonyl, carbamoyl, carboxy, alkoxycarbonyl, alkenyloxycarbonyl, alkynyloxycarbonyl, phenyl, alkylsulfonyl or phenylsulfonyl, $=NR^{17}$ wherein $R^{17}$ is hydrogen, alkyl, alkenyl, alkynyl, phenyl or cycloalkyl, $=NNR^{18}R^{19}$ wherein $R^{18}$ and $R^{19}$ are each independently hydrogen, alkyl, alkenyl, alkynyl, phenyl, cycloalkyl, alkoxycarbonyl or carboxy, $=NOR^{20}$ wherein $R^{20}$ is hydrogen, alkyl, alkenyl, alkynyl, phenyl, or cycloalkyl; or any spiro ring selected from the group consisting of:

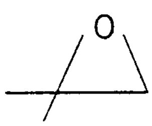 , 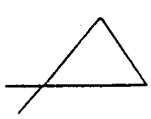 , 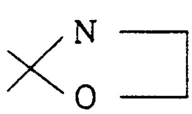 , 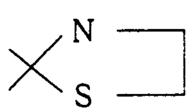 , and

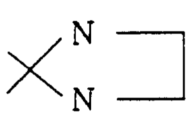

$R^3$ and $R^5$ are each independently hydrogen; halogen; alkyl; alkenyl; alkynyl; phenyl; cycloalkyl; $S(O)_m R^{21}$ wherein $R^{21}$ is hydrogen, alkyl, alkenyl, alkynyl, phenyl or cycloalkyl and m denotes an integar 0, 1 or 2; $NR^{22}R^{23}$ wherein $R^{22}$ and $R^{23}$ are each independently hydrogen, alkyl, alkenyl, alkynyl, phenyl or cycloalkyl, or may form in together a ring; $NR^{24}NR^{25}R^{26}$ wherein $R^{24}$, $R^{25}$ and $R^{26}$ are each independently hydrogen, alkyl, alkoxycarbonyl or phenyl; $OR^{27}$ wherein $R^{27}$ is hydrogen, alkyl, alkenyl, alkynyl, phenyl or cycloalkyl; cyano; $COOR^{28}$ wherein $R^{28}$ is hydrogen, alkyl, alkenyl, alkynyl, phenyl or cycloalkyl; $CONR^{29}R^{30}$ wherein $R^{29}$ and $R^{30}$ are each independently hydrogen, alkyl, alkenyl, alkynyl, phenyl or cycloalkyl; $PZ^5(OR^{31})(OR^{32})$ wherein $Z^5$ is O or S, $R^{31}$ and $R^{32}$ are each independently alkyl or phenyl;

$R^4$ is hydrogen; halogen; amino; cyano; alkyl; alkenyl; alkynyl; phenyl; cycloalkyl; $COOR^{33}$ wherein $R^{33}$ is hydrogen, alkyl, alkenyl, alkynyl, phenyl or cycloalkyl; $COR^{34}$ wherein $R^{34}$ is hydrogen, alkyl, alkenyl, alkynyl, phenyl or cycloalkyl; $CONR^{35}R^{36}$ wherein $R^{35}$ and $R^{36}$ are each independently hydrogen, alkyl, alkenyl, alkynyl, phenyl or cycloalkyl; $OR^{37}$ wherein $R^{37}$ is hydrogen, alkyl, alkenyl, alkynyl, phenyl or cycloalkyl; $S(O)pR^{38}$ wherein $R^{38}$ is hydrogen, alkyl, alkenyl, alkynyl, phenyl or cycloalkyl and p denotes an integar 0, 1 or 2. However, when $R^4$ is hydrogen, $R^3$ and $R^5$ are different and $R^4$ combines with $R^3$ or $R^5$ and a pyrimidine ring to form a condensed ring represented by the following general formulas:

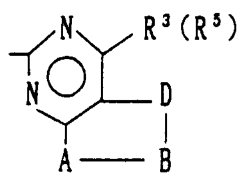

or

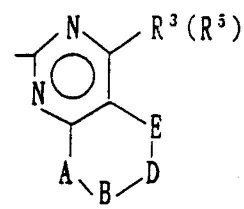

wherein any one of A, B, D and E represents

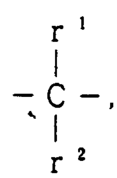

O, $S(O)_q$ or

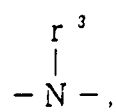

and the rest represents

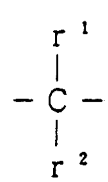

wherein $r^1$, $r^2$ and $r^3$ are each independently hydrogen, halogen, alkyl, phenyl, alkylcarbonyl, phenylcarbonyl or alkoxycarbonyl, or they form double bonds in combining with adjacent $r^1$ or $r^3$, and q denotes an integar 0, 1 or 2;

$R^6$ and $R^7$ are each independently hydrogen; halogen; alkyl; alkenyl; alkynyl; phenyl; cycloalkyl; $S(O)_r R^{39}$ wherein $R^{39}$ is alkyl, alkenyl, alkynyl, phenyl or cycloalkyl and r denotes an integar 0, 1 or 2; $OR^{40}$ wherein $R^{40}$ is hydrogen, alkyl, alkenyl, alkynyl, phenyl or cycloalkyl; $COOR^{41}$ wherein $R^{41}$ is hydrogen,

alkyl, alkenyl, alkynyl, phenyl or cycloalkyl; $CONR^{42}R^{43}$ wherein $R^{42}$ and $R^{43}$ are each independently hydrogen, alkyl, alkenyl, alkynyl, phenyl or cycloalkyl; amino; nitro or cyano, with the proviso that both $R^6$ and $R^7$ never be hydrogen at the same time,

Each of $R^8$ may be same or different, representing halogen; alkyl; alkenyl; alkynyl; phenyl; cycloalkyl; $S(O)tR^{44}$ wherein $R^{44}$ is alkyl, alkenyl, alkynyl, phenyl or cycloalkyl and t denotes an integer 0, 1 or 2; $OR^{45}$ wherein $R^{45}$ is hydrogen, alkyl, alkenyl, alkynyl, phenyl or cycloalkyl; $COOR^{46}$ wherein $R^{46}$ is hydrogen, alkyl, alkenyl, alkynyl, phenyl or cycloalkyl; $CONR^{47}R^{48}$ wherein $R^{47}$ and $R^{48}$ are each independently hydrogen, alkyl, alkenyl, alkynyl, phenyl or cycloalkyl; amino; nitro or cyano;

n denotes an integer 0, 1, 2 or 3, with the proviso that $R^4$ is the condensed ring described above in combining with $R^3$ or $R^5$ and a pyrimidine ring when both $R^1$ and $R^2$ are hydrogen.

In the definition for the substituents used in the compounds of the present invention, each substituent may be further substituted by the following substituents.

1. Any of alkyl, alkenyl and alkynyl:
   can be substituted by a halogen, hydroxy, an alkoxy, phenyl, nitro, cyano, an alkoxycarbonyl, an alkylthio and an alkylcarbonyloxy.
2. Phenyl:
   can be substituted by a halogen, an alkyl, an alkoxy and nitro.
3. Cycloalkyl:
   can be substituted by an alkyl, an alkoxy, carboxy and an alkoxycarbonyl.
4. Amino:
   can be substituted by an alkyl, an alkenyl, an alkynyl, an alkoxycarbonyl and an alkylcarbonyl.
5. Carboxy:
   can be substituted by an alkali metal salt.
6. Carbamoyl:
   can be substituted by an alkyl.

In addition to the substituents as described above. $P(=O)(OH)_2$, $P(=O)(O\text{-alkyl})_2$ and $P(=S)(O\text{-alkyl})_2$ can be the substituents of alkyl in respect of $R^{17}$, and trialkylsilyl can be the substituent of alkynyl in respect of $R^4$.

In the whole definitions described above, the number of carbons in alkyl is normally in a range of from 1 to 6, the number of carbons in alkenyl and alkynyl are in a range of from 2 to 6, respectively and the number of carbons in cycloalkyl is in a range of from 3 to 6.

For the ring formed by $R^{22}$ and $R^{23}$ of $NR^{22}R^{23}$, the ones represented by the following formulas can be exemplified.

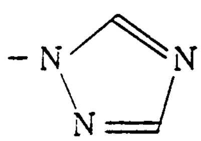 , 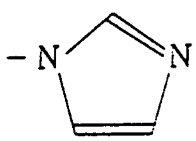 , 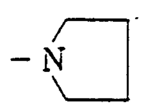

In the general formula (I), the optical isomers can be also obtained when $R^1$ and $R^2$ are different, and such isomers are included in the pyridine derivative compounds specified in the present invention.

The pyridine derivative compounds specified in the present invention can be manufactured according to the process described hereinbelow or by further employing known similar reactions properly thereto.

i )

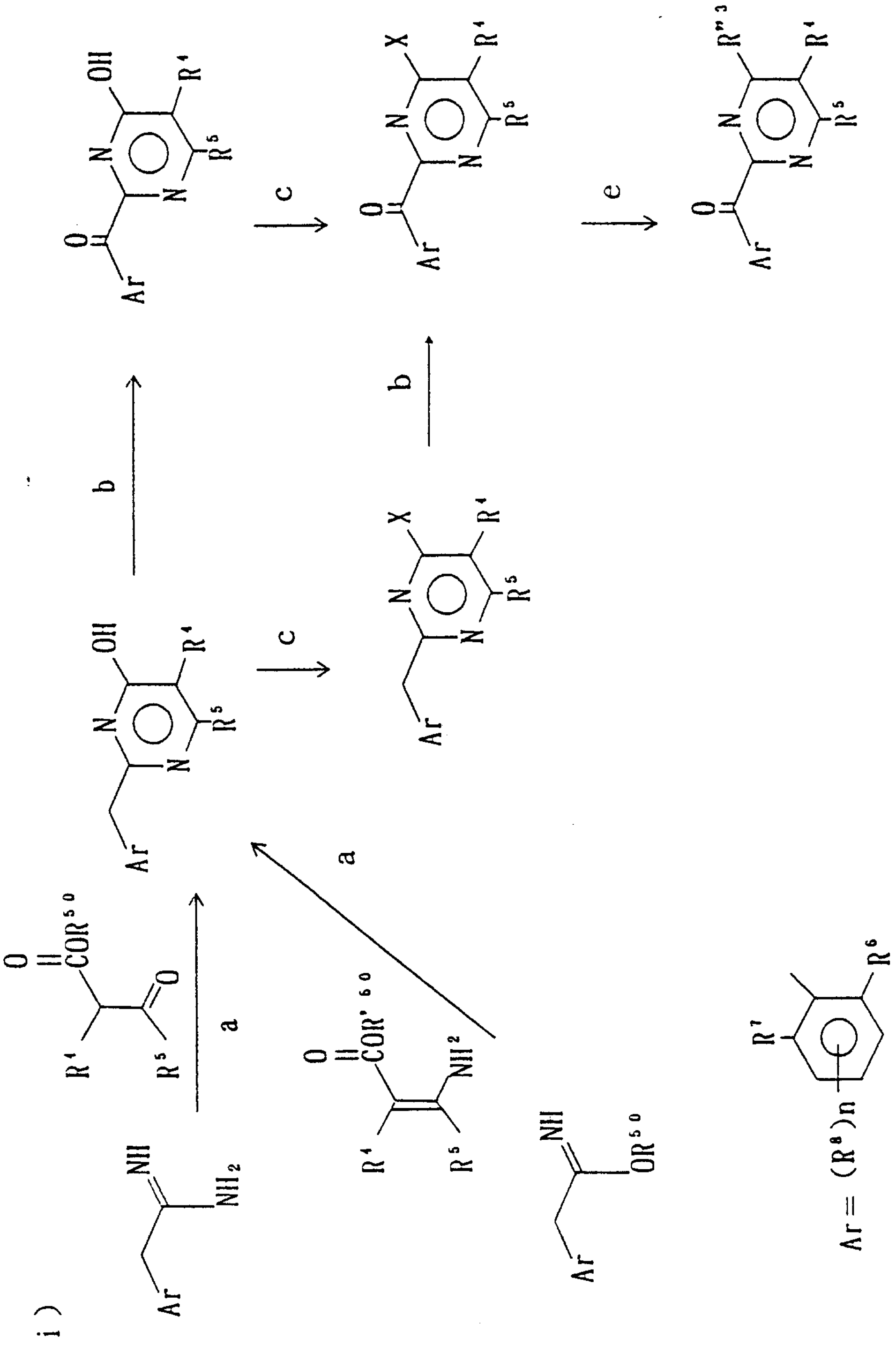

ii)

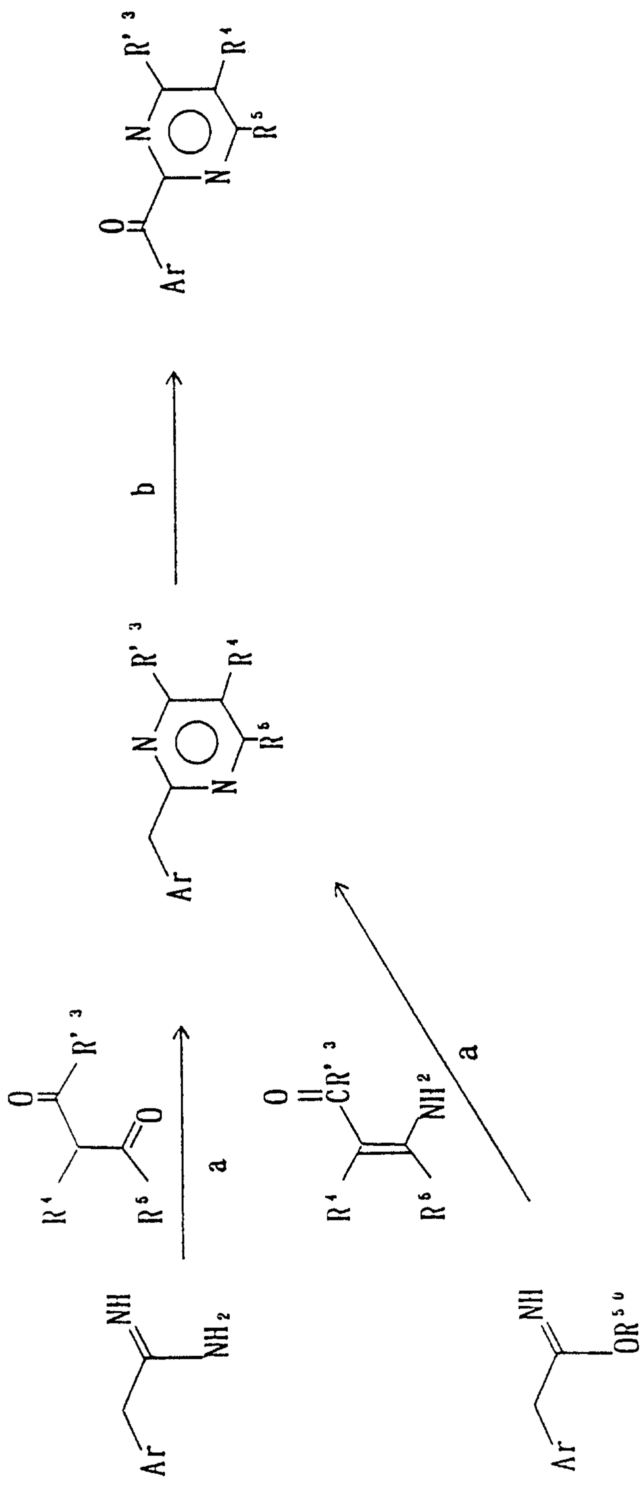

iii)

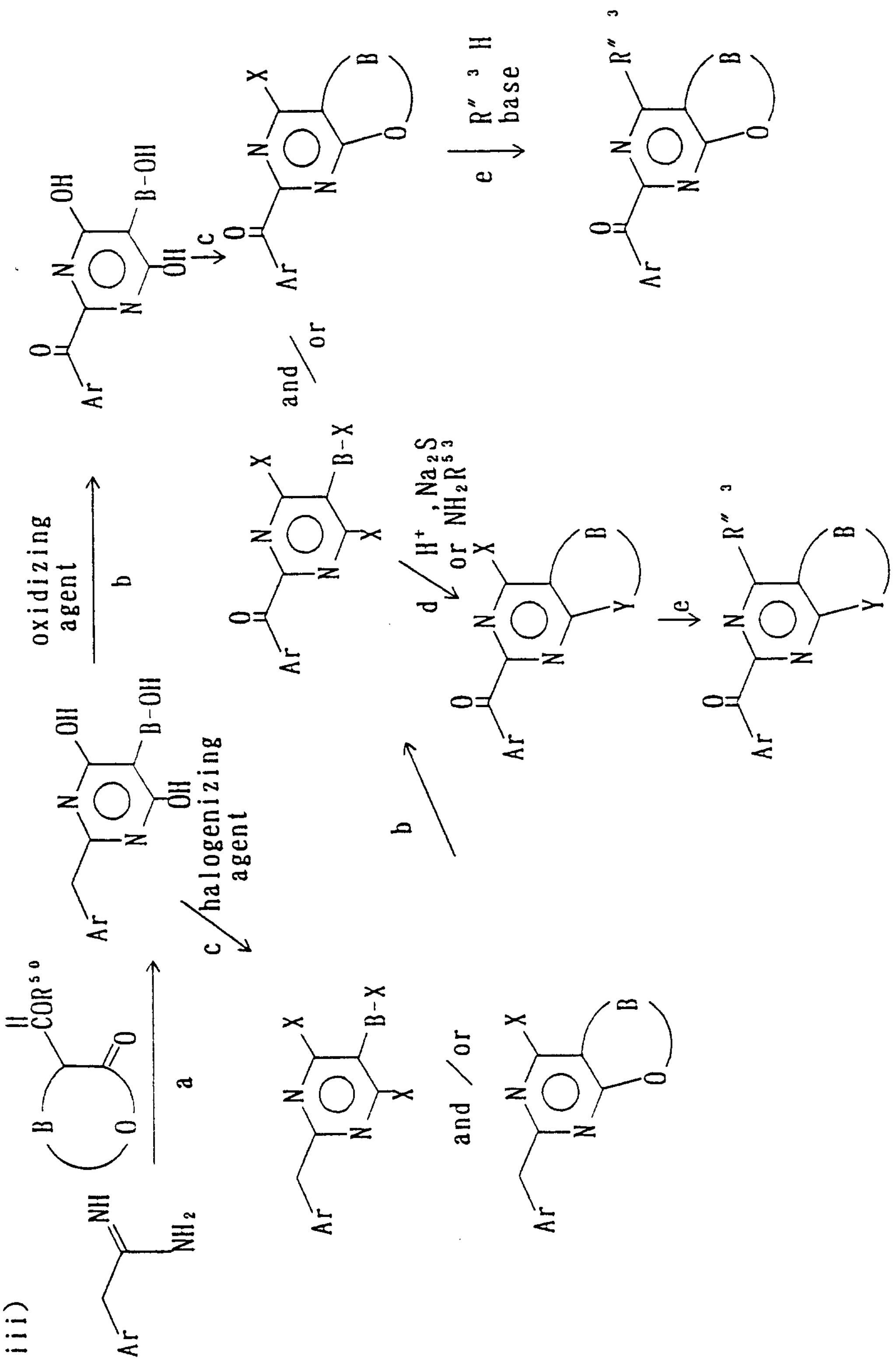
a
oxidizing agent
b
c halogenizing agent
c
b
and/or
and/or
d H+, $Na_2S$ or $NH_2R^{53}$
e
e $R''^3H$ base

iv)

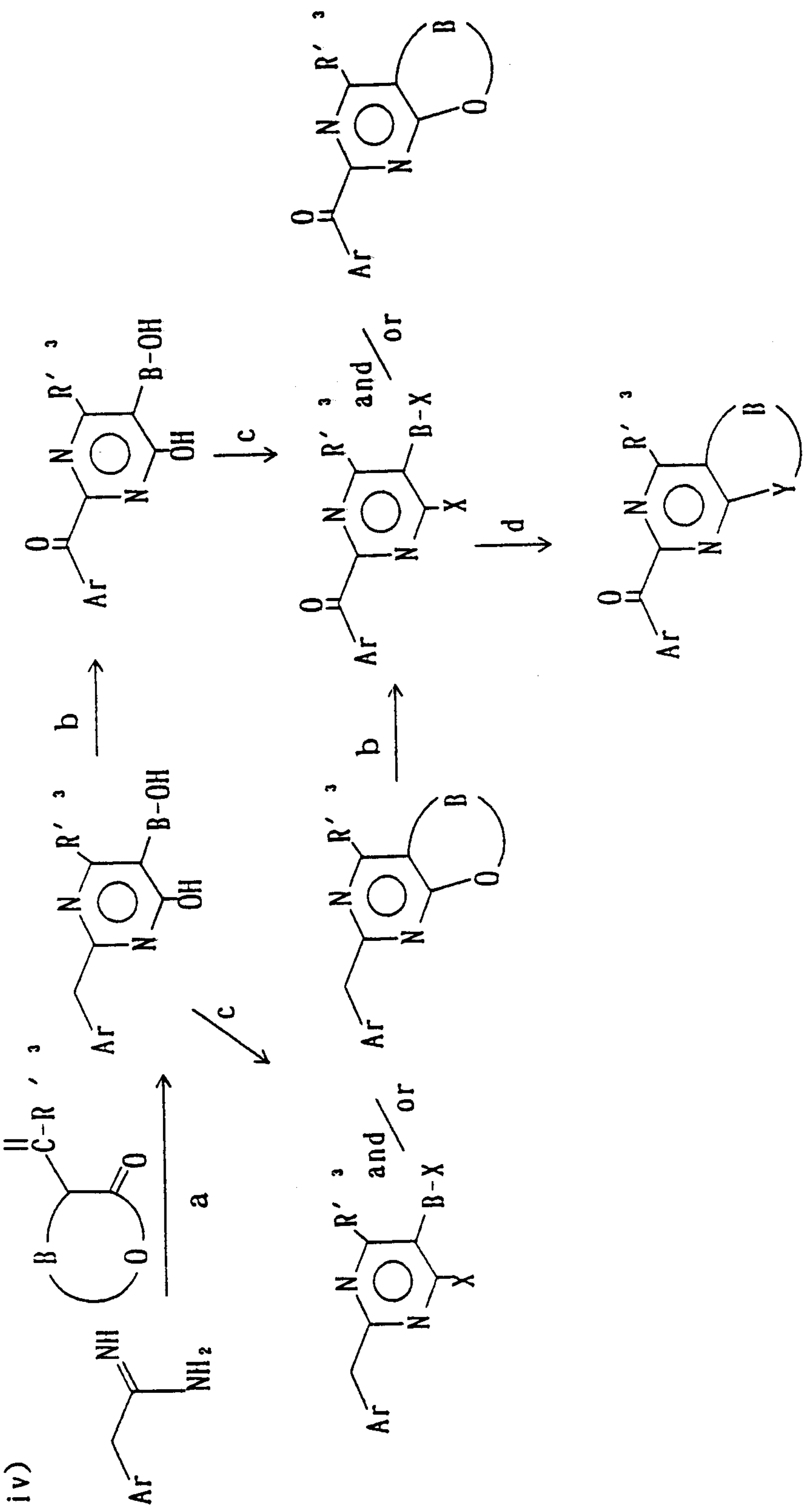

v)

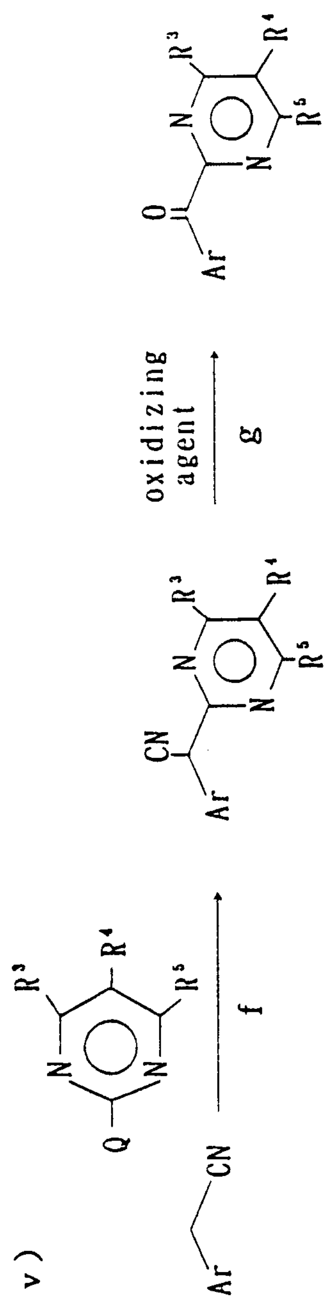


vi)

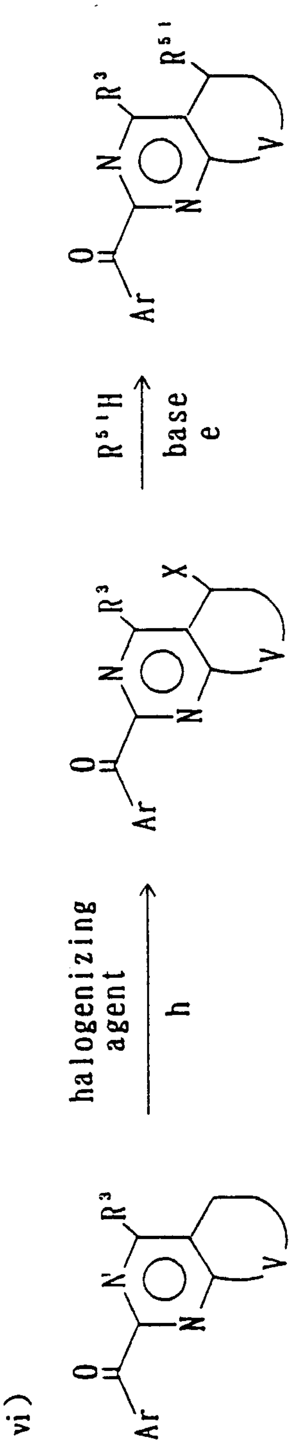

vii)

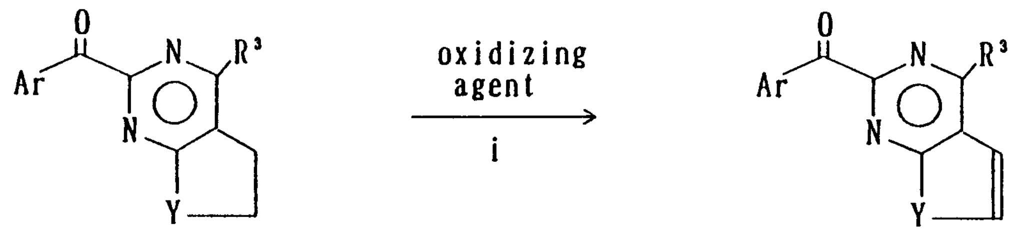


viii)

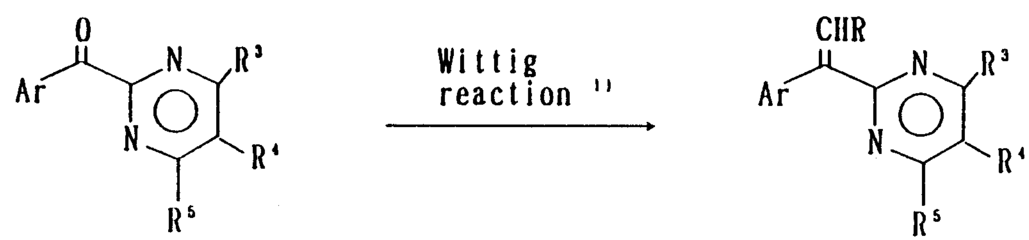


1) J. Org. Chem., 27, 998 (1961)

ix)

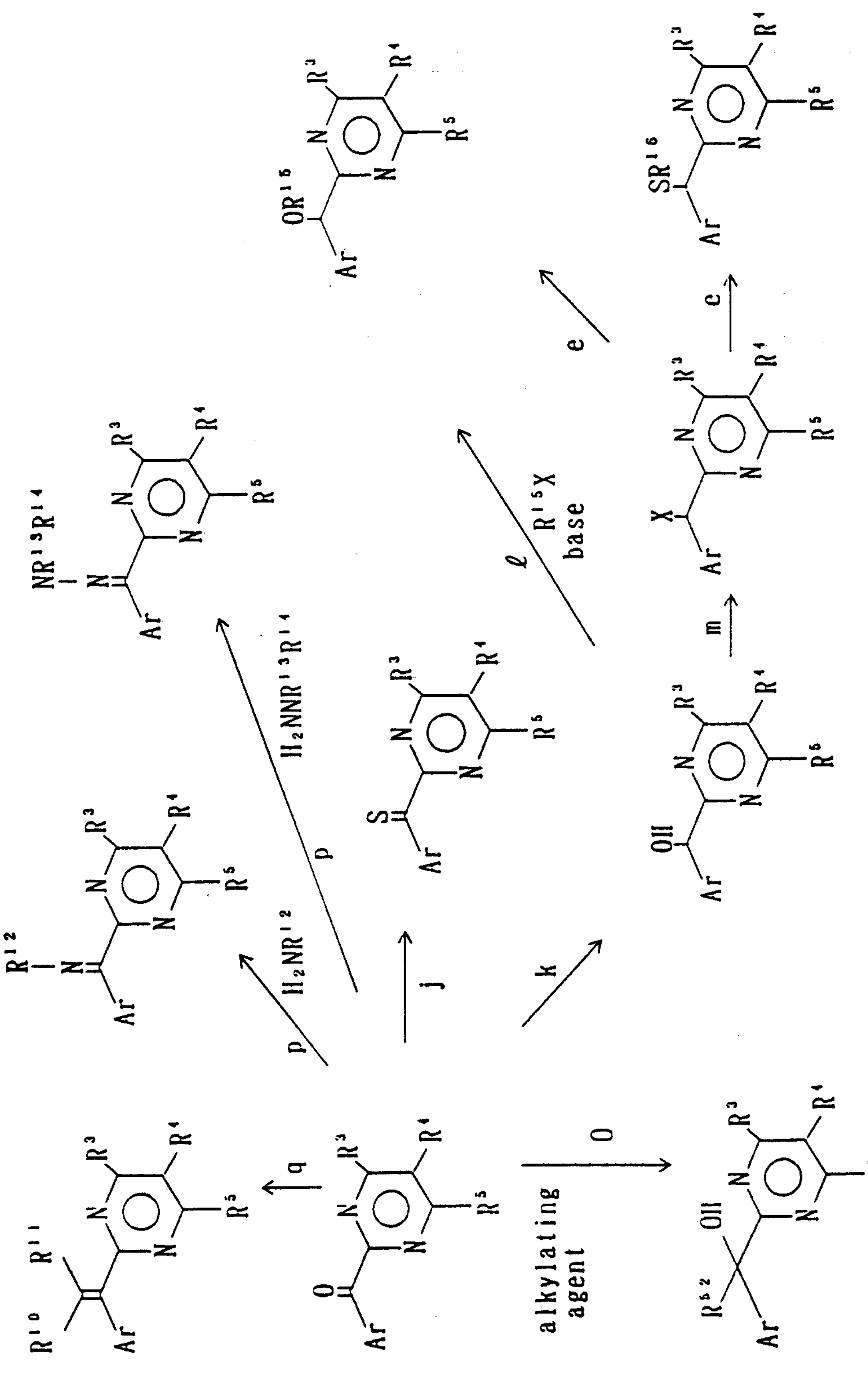


wherein $R^3$, $R^4$, $R^5$, $R^6$, A and n are as defined above; $R'^3$ is hydrogen, alkyl, alkenyl, alkynyl, phenyl or cycloalkyl; $R''^3$ is S(O)m$R^{21}$ wherein $R^{21}$ and m are as defined above, $NR^{22}R^{23}$ where in $R^{22}$ and $R^{23}$ are as defined above, $NR^{24}NR^{25}R^{26}$ wherein $R^{24}$, $R^{25}$ and $R^{26}$ are as defined above, $OR^{27}$ wherein $R^{27}$ is as defined above, $COOR^{28}$ wherein $R^{28}$ is as defined above, $CONR^{29}R^{30}$ wherein $R^{29}$ and $R^{30}$ are as defined above or cyano; $R^{50}$ and $R'^{50}$ are alkyl; $R^{51}$ is alkoxy; $R^{52}$ is alkyl, alkenyl or alkynyl; X is halogen; Y is O, S or $NR^{53}$ wherein $R^{53}$ is hydrogen, alkyl, phenyl or alkoxy; B is $C_2$-$C_4$ alkylene which may be substituted and may have 1 or 2 unsaturated bond(s); V is $C_1$-$C_3$ alkylene wherein one of the carbons may be

substituted with O, S or Nr'3 wherein r'3 is hydrogen, alkyl or phenyl. Yet, the alkylene may be substituted with alkylcarbonyl, phenylcarbonyl or alkoxycarbonyl, Q is an leaving group such as halogen, alkylsulfonyl and benzylsulfonyl.

The reaction a described above is carried out by subjecting any of raw material amidine, mineral acid thereof or organic acid salt thereof such as carboxylic acid salt to a reaction with $\beta$-dicarbonyl compound in a solvent in the presence of a base at a temperature of from room temperature to the boiling point of the solvent for a period of from 1 to 48 hours. For the solvent, alcohol such as ethanol, ethers such as THF, hydrocarbons such as benzene and toluene, DMF or the like, or mixtures thereof can be used. For the base, metal alcoholates comprising lower alcohols such as sodium methylate and potassium methylate, inorganic bases such as potassium carbonate and sodium hydroxide, and organic bases such as DBU can be used.

The reaction b described above is carried out in a solvent in the presence of a oxidizing agent at a temperature of from room temperature to the boiling point of said solvent. For the solvent, water, ethers such as dioxane, hydrocarbons such as benzene and toluene, acetic acid, acetic anhydride, pyridine, halogenized hydrocarbons such as methylene chloride or their homogenized or bilayer mixtures can be used. For the oxidizing agent such as $KMnO_4$, $K_2Cr_2O_7$ and $SeO_2$ can be used.

The reaction c described above is carried out without solvent or in a solvent, in the presence of a halogenizing agent at a temperature of from room temperature to the boiling point of said solvent. For the solvent, halogenized hydrocarbons such as methylene chloride and chloroform, hydrocarbons such as benzene and toluene or mixtures thereof can be used. For the halogenizing agent such as $POX_3$, $PX_3$ and $PCl_5$, wherein X is halogen, can be used.

The reaction d shown above is carried out for a period of from 1 hour up to 48 hours in a solvent in the presence of an acid or a base at a temperature of from room temperature to the boiling point of the solvent. If Y is O, water, dioxane or the like, or mixtures thereof can be used as the solvent. For the acid to be used in said reaction, sulfuric acid, sodium dihydrogenphosphate, mixture of sodium dihydrogenphosphate and phosphoric acid or the like can be used, and the reaction is carried out for a period of from 1 to 10 hours at a temperature of from 90 °C to the boiling point of the solvent. If Y is S, ethers such as dioxane, hydrocarbons such as benzene, and DMF or the like can be used as the solvent. For the base, sodium sulfide or the like can be used, and the reaction is carried out for a period of from 1 to 48 hours at a temperature of from room temperature to the boiling point of the solvent. If Y is $NR^{53}$, water, alcohols such as methanol, ethers such as dioxane, DMF or the like, or mixtures thereof, can be used as the solvent. For the base, potassium carbonate, sodium hydroxide or the like can be used together with $NH_2R^{53}$ when appropriate, then the reaction is carried out for a period of from 1 to 48 hours at a temperature of from room temperature to the boiling point of the solvent under pressure, if necessary.

The reaction e shown above is carried out for a period of from several minutes up to 24 hours in a solvent in the presence of a base at a temperature of from room temperature to the boiling point of the solvent. For the solvent, alcohols such as ethanol, ethers such as THF and dioxane, hydrocarbons such as benzene and toluene, DMF or the like, or mixtures thereof, can be used. For the base, metal alcoholate of lower alcohols such as sodium methylate and potassium ethylate, inorganic bases such as potassium carbonate and sodium hydroxide, organic bases such as DBU, etc., can be used.

The reaction f shown above is carried out for a period of from several minutes up to 24 hours in a solvent in the presence of a base at a temperature of from -78 °C to the boiling point of the solvent. For the solvent, ethers such as THF, DMF or the like can be used. For the base, sodium hydride, potassium tert-butoxide, n-butyl lithium, lithium diisopropylamide or the like, can be used.

The reaction g is carried out for a period of from 1 hour to 24 hours in an halogenized hydrocarbon such as chloroform in the presence of a peroxy acid such as m-chloroperbenzoic acid at a temperature of from -10 °C to the boiling point of the solvent. As alternatives, the reaction is carried out for a period of from 1 hour up to 48 hours in an ether such as THF or in DMF or the like, in the presence of oxygen at a temperature of from 0 °C up to the boiling point of the solvent, or the reaction is carried out in bilayer solvent comprising a hydrocarbon such as toluene and water in the presence of a quaternary ammonium salt and by subjecting the reacting solution to air oxidation by using an inorganic base such as sodium hydroxide .

The reaction h shown above is carried out for a period of from 1 to 48 hours in a solvent in the presence of halogenizing agent at a temperature of from -10 °C to the boiling point of the solvent. For the solvent, halogenized hydrocarbons such as carbon tetrachloride and chloroform, hydrocarbons such as benzene and toluene, mixtures thereof or the like can be used. For the halogenizing agent, N-chlorosuccinimide, N-bromosuccinimide or the like can be used. In addition, light, benzoyl peroxide or the like can be also used as a catalyst, if necessary.

The react ion i shown above is carried out for a period of from 1 to 48 hours in a solvent in the presence of oxidizing agent at a temperature of from 0 °C to the boiling point of the solvent. For the solvent, water, ethers such as dioxane, hydrocarbons such as benzene and toluene, halogenized hydrocarbons such as methylene chloride, or the homogeneous or bilayer mixtures thereof can be used. For the oxidizing agent, $KMnO_4$, $K_2Cr_2O_7$, $NiO_2$, chloranil, N-chlorosuccinimide, N-bromosuccinimide or the like can be used.

The reaction j shown above is carried out for a period of from 1 to 24 hours in a solvent such as ether like dimethoxyethane or hydrocarbon like toluene in the presence of Lawesson's reagent at a temperature of from 0 °C to the boiling point of the solvent.

The reaction k shown above is carried out for a period of from 1 to 48 hours in a solvent such as water, alcohol like methanol and a mixture thereof in the presence of $NaBH_4$ at a temperature of from 0 °C to the boiling point of the solvent.

The reaction ℓ shown above is carried out for a period of from several minutes to 24 hours in a solvent in the presence of a base at a temperature of from 0 °C to the boiling point of the solvent. For the solvent, alcohols such as ethanol, ketones such as acetone, ethers such as THF and dioxane, hydrocarbons such as benzene and toluene, DMF or the like, or mixtures thereof can be used. For the base, inorganic bases such as sodium hydride, potassium tert-butoxide, potassium carbonate and sodium hydroxide, and organic bases such as DBU can be used.

The reaction m shown above is carried out either without solvent or in a solvent, in the presence of an halogenizing agent at a temperature of from 0 °C to the boiling point of the solvent. For the solvent, water, halogenized hydrocarbons such as methylene chloride and chloroform, hydrocarbons such as benzene and toluene, or mixtures thereof can be used. For the halogenizing agent, HX, $PX_3$, $SOCl_2$ wherein X is halogen, or the like can be used.

The reaction o shown above is carried out for a period of from several minutes to 10 hours in a solvent in the presence of an alkylating agent at a temperature of from -78 °C to the boiling point of the solvent. For the solvent, ethers such as diethylether or the like can be used, and for the alkylating agent, alkyl metals such as alkyl lithium, Grignard reagents such as alkyl magnesium bromide or the like can be used.

The reaction p shown above is carried out in a solvent either in the presence of an acidic catalyst or without the catalyst at a temperature of from 0 °C to the boiling point of the solvent. For the solvent, alcohols such as ethanol, hydrocarbons such as toluene, halogenized hydrocarbons such as chloroform, ethers, DMF or the like, or mixtures thereof can be used. For the catalyst, inorganic acids such as hydrochloric acid and sulfuric acid, and organic acids such as acetic acid can be used.

The reaction q shown above is carried out in a solvent either in the presence of a base or without base at a temperature of from -78 °C to the boiling point of the solvent. In the reaction, a catalyst may be added, when appropriate. For the solvent, ethers such as THF, hydrocarbons such as benzene and toluene, halogenized hydrocarbons such as chloroform and methylene chloride, and mixtures thereof can be used.

For the base, inorganic bases such as sodium hydride and potassium tert-butoxide, organic metal bases such as butyl lithium, and organic bases such as DBU and pyridine, can be used. For the catalyst, lewis acids such as $TiCl_4$ and $SnCl_4$ can be used.

The afore-mentioned compounds of i) and ii) represented by the following general formulas :

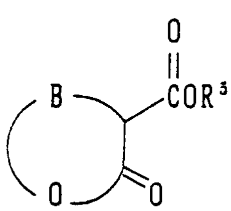

and

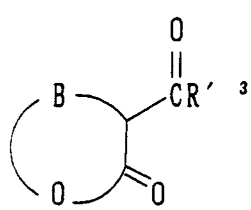

are known or can be manufactured according to the customary methods in the art (See J. Org. Chem., 43, 346 (1978) or J. Med. Chem., 28, 934 (1985)).

## Best Mode for Carrying Out the Invention

The present invention is further described in detail with referring to the following Examples, wherein the chemical structures of each compounds are determined based on the results obtained from chemical analysis by using IR, NMR, MS, etc.

Example 1:

2-(2-chlorobenzoyl)-4-trifluoromethyl-5,6-dihydrofuro[2,3-d]pyrimidine (Compound No. III-13 ) .

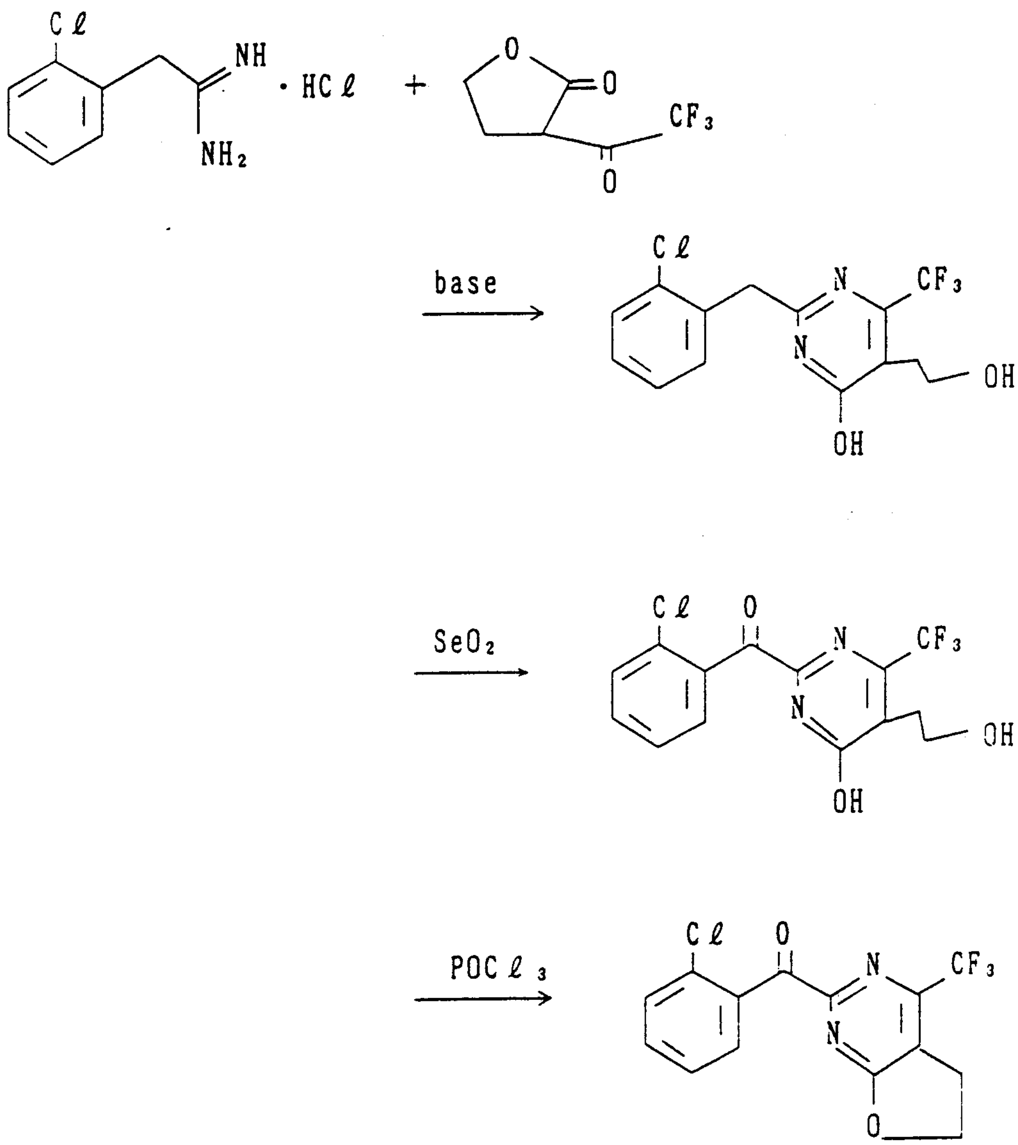


To 50 ml of ethanol was dissolved 0.7g of metallic sodium, and 5g of 2-(2-chloropheny)acetoamidine hydrochloride and 5.3g of α-trifluoroacetyl-γ-butylolactone were further added to the resulting solution at room temperature, then the solution was stirred and continuously refluxed under heating for 18 hours. The solution reacted was then condensed under reduced pressure and acidified with 10% hydrochloric acid. The crystals precipitated were separated by filtration, washed, and further washed with ether, then dried, affording 5.2g of 2-(2-chlorobenzyl)-4-trifluoromethyl-5-(2-hydroxyethyl)-6-hydroxypyrimidine.

To this product were added 50 ml dioxane, 10 ml water and 3.4g of $SeO_2$, then the resulting solution was refluxed under heating with continuous stirring for 3 days. Then an element Se was separated by filtration from the reacted solution, and the filtrate was washed with saturated brine and condensed under reduced pressure following to drying over magnesium sulfate to precipitate crystals. The crystals precipitated were then washed with ether, then 4g of 2-(2-chlorobenzoyl)-4-trifluoromethyl-5-(2-hydroxyethyl)-6-hydroxypyrimidine was obtained.

Furthermore, to this reaction product were added 200 ml toluene and 2g of $POCl_3$, then the resulting solution was stirred, refluxed under heating for 2 hours. The solution reacted was then condensed, added with water, extracted with methylene chloride, washed with saturated brine, dried over magnesium sulfate

and then condensed under reduced pressure to obtain crystals. The crystals obtained was purified with ether, affording 3g of the objective compound.

Example 2:

2-(2-chlorobenzoyl)-4-trifluoromethyl-furo[2,3-d]pyrimidine (Compound No. III-16)

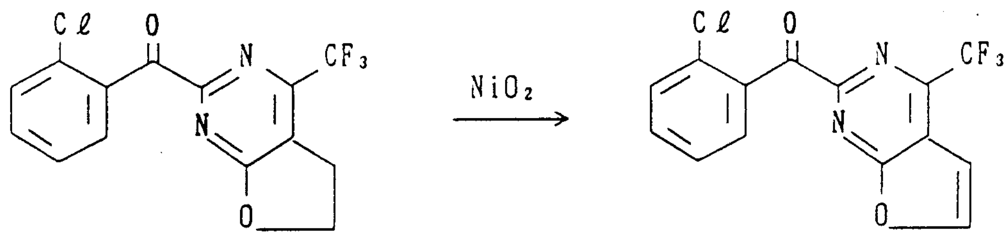


To 20ml toluene was dissolved 0.5g 2-(2-chlorobenzoyl)-4-trifluoromethyl-5,6-dihydrofuro[2,3-d]-pyrimidine, and 2g of $NiO_2$ was added there to, then the mixture was refluxed under heating for 5 hours.

An insoluble material was separated by filtration from the reaction solution, and the filtrate was washed with saturated brine, dried over $MgSO_4$, and condensed under reduced pressure, then purified by silica gel column chromatography to obtain 250mg of the objective compound No. III-16.

Example 3

2-(2-chlorobenzoyl)-4-trifluoromethyl-5,6-dihydropyrolo[2,3-d]pyrimidine (Compound No. III-17)

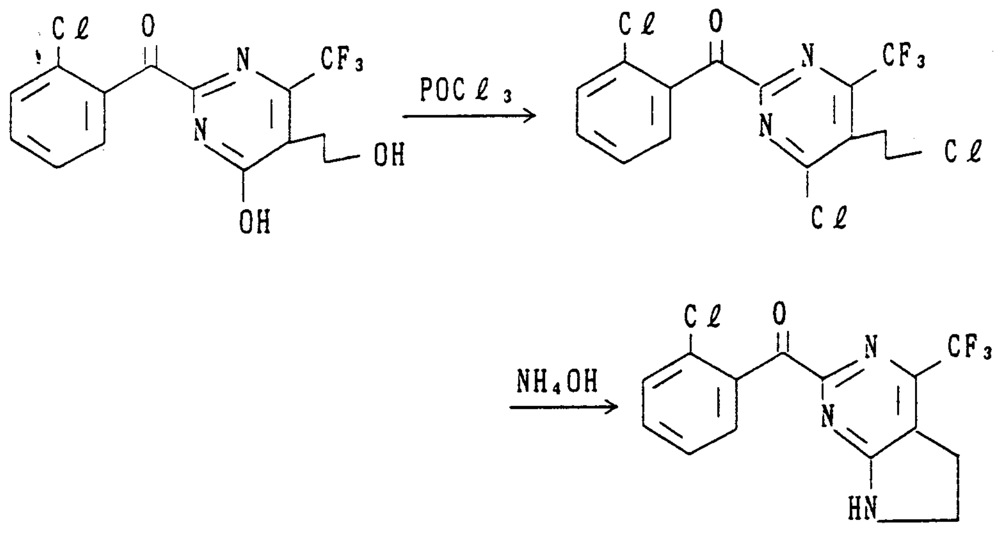


To 30 ml $POCl_3$ was dissolved 5g of 2-(2-chlorobenzoyl)-4-trifluoromethyl-5-(2-hydroxyethyl)-6-hydroxypyrimidine, then the solution was refluxed under heating for 2 hours. The reaction solution was condensed under reduced pressure, poured into water and followed by an extraction with methylene chloride. Then the extract obtained was washed with saturated brine, dried with $MgSO_4$, and condensed under reduced pressure, then purified by using silica gel column chromatography to obtain 3g of 2-(2-chlorobenzoyl)-4-trifluoromethyl-5-(2-chloroethyl)-6-chloropyrimidine.

To 10 ml MeOH was dissolved 1g of 2-(2-chlorobenzoyl)-4-trifluoromethyl-5-(2-chloroethyl)-6-chloropyrimidine, then the solution was added with 3 ml aqueous ammonia, sealed in a tube, then stirred at 100 °C for 18 hours. The resulting reaction solution was added with ethyl acetate, washed with saturated brine, dried with $MgSO_4$, condensed under reduced pressure, and purified by using silica gel column

chromatography to obtain 300mg of the objective compound No. III-17.

Example 4

2-(2-chlorobenzyl)-4-hydroxy-5,6,7,8-tetrahydroquinazoline (Compound No. III-1)

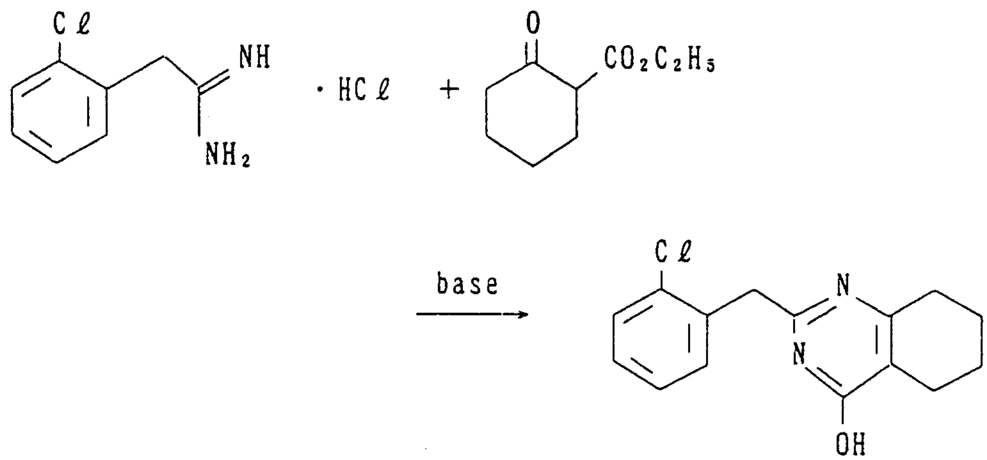


To 50 ml ethanol was dissolved 0.95g of metallic sodium, and 7g of 2-(2-chlorophenylacetoamidine hydrochloride and 6.3g of 2-ethoxycarbonylcyclohexanone were further added thereto, then the solution was refluxed under heating for 18 hours. The solution reacted was condensed and then acidified with 10% hydrochloric acid to precipitate crystals, then the crystals obtained were washed with water and ether, respectively, and dried to obtain 7.2g of 2-(2-chlorobenzyl)-4-hydroxy-5,6,7,8-tetrahydroquinazoline.

Example 5

2-(2-chlorobenzoyl)-4-chloro-5,6,7,8-tetrahydroquinazoline (Compound No. III-3)

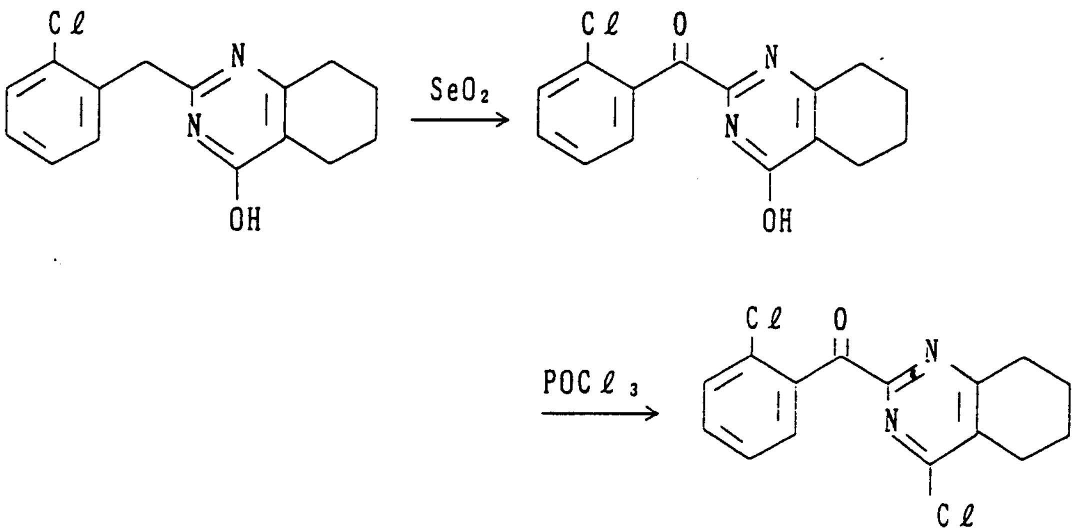


To 6g of 2-(2-chlorobenzyl)-4-hydroxy-5,6,7,8-tetrahydroquinazolinewere added each of 60 ml dioxane, 12 ml water and 3g of $SeO_2$, then the solution was refluxed under heating for 18 hours. The solution reacted was then condensed under reduced pressure, added with 20 ml $POCl_3$, and refluxed under heating for 3 hours. The element Se was separated from the reacted-solution by filtration, the filtrate was condensed under reduced pressure, then added with water to proceed to an extraction with ethyl acetate. The extract

was then washed with saturated brine, dried, condensed under reduced pressure, and purified by using silica gel column chromatography to obtain 4.2g of 2-(2-chlorobenzoyl)-4-chloro-5,6,7,8-tetrahydroquinazoline.

Example 6

2-(2-chlorobenzoyl)-4-methoxy-5,6,7,8-tetrahydroquinazoline (Compound No. III-4)

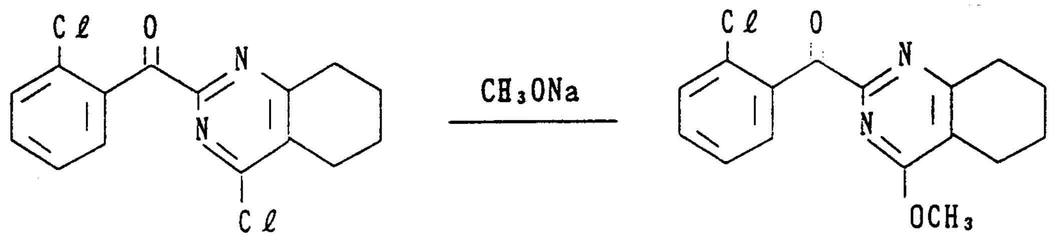


To 10 ml methanol was dissolved 0.7g of 2-(2-chlorobenzoyl)-4-chloro-5,6,7,8-tetrahydroquinazoline, then 0.6 ml sodium methylate methanol solution (28%) was further added thereto. The resulting solution was then stirred at room temperature for 2 hours. The solution reacted was poured into water to proceed to an extraction with ethyl acetate, then the extract was washed with saturated brine, dried with $MgSO_4$, and condensed under reduced pressure to obtain 0.7g of the objective compound No. III-4.

Example 7

2-(2-chlorobenzyl)-4-hydroxyquinazoline (Compound No. III-6)

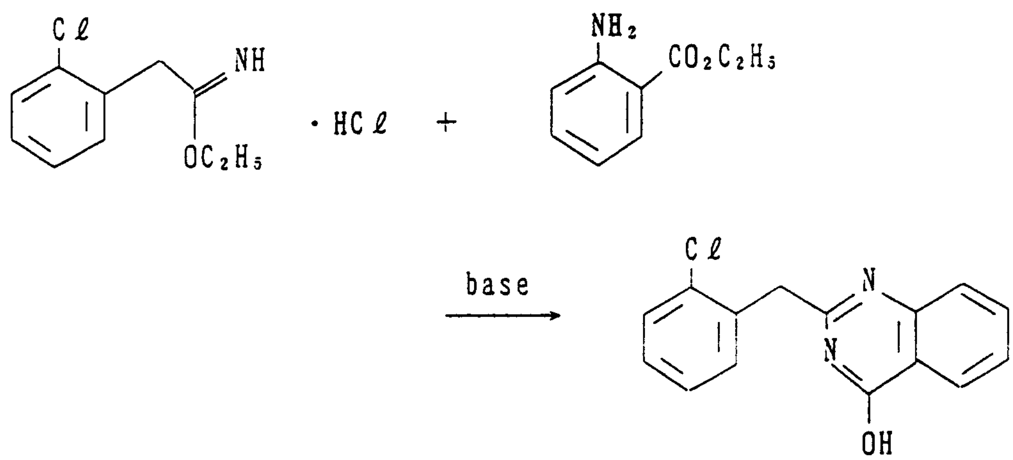


To 50 ml ethanol was dissolved 0.6g of metallic sodium, and 5g of 2-(2-chlorophenyl)-acetoimidic acid ethyl hydrochloride and 3.9g of ethyl 2-aminobenzoate were further added there to at room temperature. Then the solution was stirred, and refluxed under heating for 18 hours. The solution reacted was condensed under reduced pressure, then acidified with 10% hydrochloric acid to precipitate crystals. The crystals obtained was then separated by filtration, washed with water and ether, and dried to obtain 4g of 2-(2-chlorobenzyl)-4-hydroxyquinazoline.

Example 8

2-(2-chlorobenzoyl)-4-hydroxyquinazoline (Compound No. III-7)

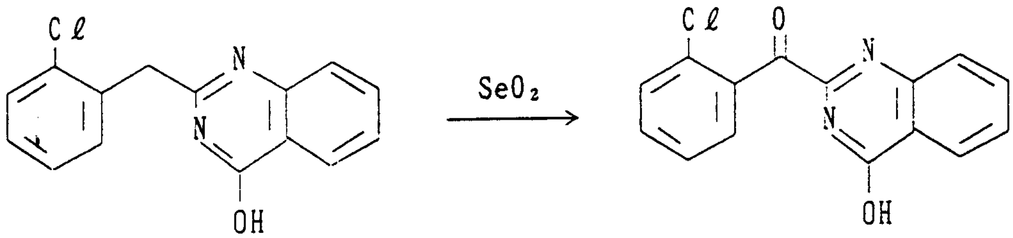


A mixture comprising 1.2g of 2-(2-chlorobenzyl)-4-hydroxyquinazolin e, 20 ml dioxane, 4 ml water and 1.0g of $SeO_2$ was refluxed under heating for 18 hours. The element Se was separated from the mixture by filtration, then the filtrate was washed with saturated brine, dried with $MgSO_4$, and condensed under reduced pressure to precipitate crystals. The crystals obtained was then washed with ether to obtain 1.0g of 2-(2-chlorobenzoyl)-4-hydroxyquinazoline.

Example 9

2 -(2-benzoyl)-4-methylthioquinazoline (Compound No. III-9)

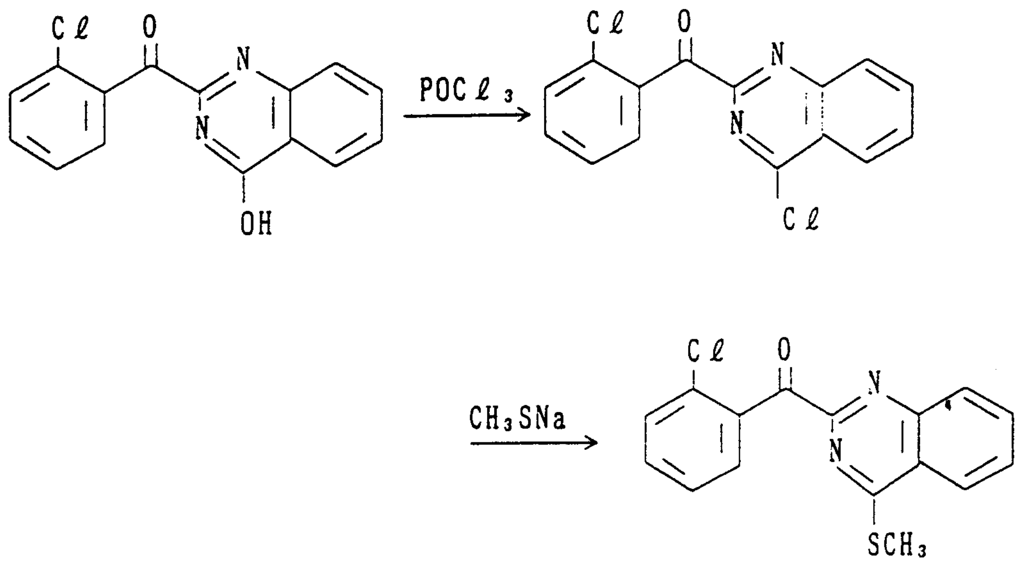


To 1.0g of 2-(2-chlorobenzoyl)-4-hydroxyquinazoline were added 20 ml toluene and 2g of $POCl_3$, then the solution was refluxed under heating for 2 hours. The solution reacted was condensed under reduced pressure, then 10 ml dioxane and 4 ml of 15% aqueous solution of sodium methylmercaptan were added thereto for stirring at room temperature for 2 hours. The solution reacted was poured into water to proceed to the extraction with ethyl acetate, then the extract obtained was washed with saturated brine, dried with $MgSO_4$, and condensed under reduced pressure to precipitate crystals. The crystals obtained were washed with ether, then affording 0.7g of the objective compound No. III-9.

Example 10

2-(2-chlorobenzoyl)-4-methylamino-6-trifluoromethylpyrimidine (Compound No. I-14)

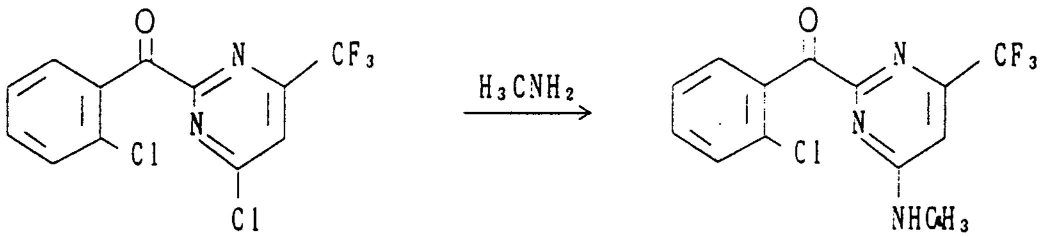


To 10 ml methanol was dissolved 0.6g of 4-chloro-2-(2-chlorobenzoyl)-6-trifluoromethylpyrimidine, then 0.43g of 40% methanol solution of methylamine was fed dropwise there to at room temperature. The solution was subjected to reaction for 1 hour at room temperature, then the reacted-solution was added with water, neutralized with dilute hydrochloric acid and extracted with ethyl acetate. The organic layer of the extract was washed with saturated brine, dried with Magnesium chloride, then concentrated. The resulting residue was purified by using silica gel column chromatography to obtain 0.30g of the objective compound No. I-14 in crystals. Melting point was from 111 to 112 °C.

Example 11

2-(2-chloro-6-fluorobenzyl)-4-trifluoromethyl-dihydropyrano[4,3d]pyrimid ine (Compound No. III-1442)

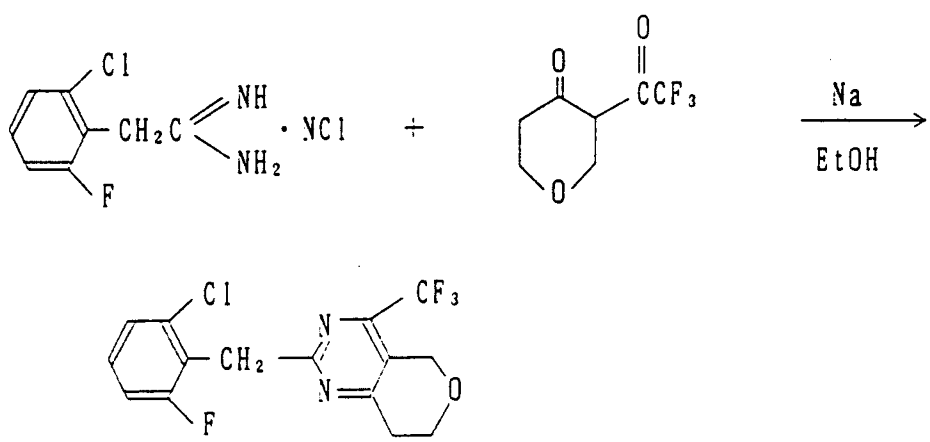


To 20 ml ethanol was dissolved 0.14g of metallic sodium, and 1.9g of 2-(2-chloro-6-fluorophenyl)-acetoamidine hydrochloride and 1.0g of 3-trifluoroacetyl-4-tetrahydropyranone were further added thereto, then the solution was stirred and refluxed under heating continuously for 3 hours. The solution reacted was concentrated under reduced pressure, acidified with 10% hydrochloric acid, extracted with ethyl acetate, dried with $MgSO_4$, then concentrated under reduced pressure. The concentrate was further purified by using silica gel column chromatography to obtain 0.6g of the objective compound No. III-1442.

Example 12

Ethyl-3-(2-chlorophenyl)-3-(4-trifluoromethyl-5,6-dihydrofuro[2,3d]pyrim idine-2-yl)propenate (Compound No. III-98)

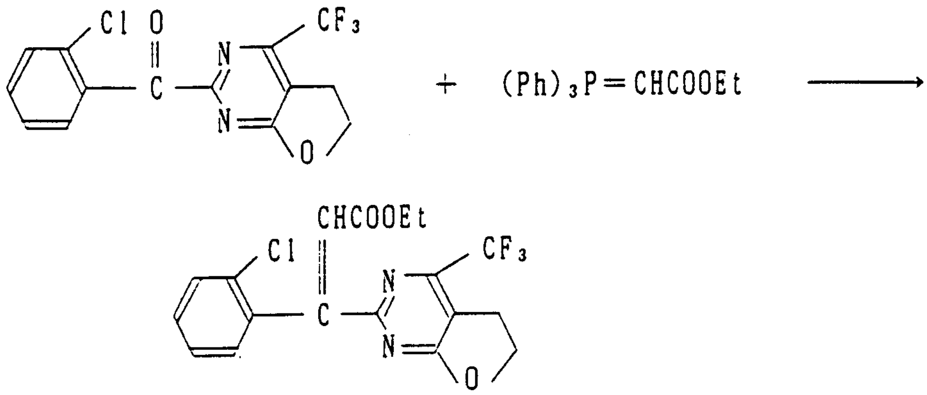


To 15 ml benzene were dissolved 1.0g of 2-(2-chlorobenzoyl)-4-trifluoromethyl-5,6-dihydrofuro[2,3d]-pyrimidine and 1.06g of triphenylcarbethoxymethylenephosphorane, then the solution was stirred and refluxed under heating continuously for 24 hours. The solution reacted was concentrated under reduced pressure, then purified by using silica gel column chromatography to obtain 0.78g of the objective compound No. III-98.

The representative examples of the compounds according to the present invention are shown in Tables 1 and 2, including the compounds recited in the Examples described above. In the tables, the numbers in brackets in the columns of physical data are indicated in °C.

Table. 1

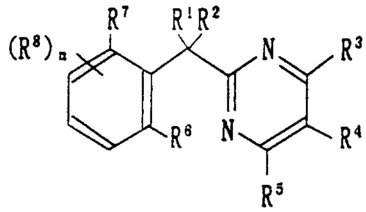

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-1 | =0 | | $CH_3$ | H | Cl | Cl | H | | 0 | [ 67- 69 ] |
| I-2 | 〃 | | 〃 | 〃 | $OCH_3$ | 〃 | 〃 | | 〃 | $n_D^{25.5}$ =1.5745 |
| I-3 | 〃 | | 〃 | 〃 | $SCH_3$ | 〃 | 〃 | | 〃 | $n_D^{25.5}$ =1.6078 |
| I-4 | 〃 | | 〃 | 〃 | $SOCH_3$ | 〃 | 〃 | | 〃 | $n_D^{26.0}$ =1.5734 |
| I-5 | 〃 | | 〃 | 〃 | $SO_2CH_3$ | 〃 | 〃 | | 〃 | |
| I-6 | 〃 | | 〃 | 〃 | $NHCH_3$ | 〃 | 〃 | | 〃 | |
| I-7 | 〃 | | 〃 | 〃 | OH | 〃 | 〃 | | 〃 | [191-193 ] |
| I-8 | 〃 | | 〃 | 〃 | $N(CH_3)_2$ | 〃 | 〃 | | 〃 | $n_D^{25.5}$ =1.5812 |
| I-9 | 〃 | | $CF_3$ | 〃 | Cl | 〃 | 〃 | | 〃 | [ 67- 68 ] |
| I-10 | 〃 | | 〃 | 〃 | $OCH_3$ | 〃 | 〃 | | 〃 | $n_D^{25.5}$ =1.5271 |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-11 | =O | | $CF_3$ | H | $SCH_3$ | Cl | H | | 0 | $n_D^{22.9}$=1.5677 |
| I-12 | 〃 | | 〃 | 〃 | $SOCH_3$ | 〃 | 〃 | | 〃 | |
| I-13 | 〃 | | 〃 | 〃 | $SO_2CH_3$ | 〃 | 〃 | | 0 | [135-139 ] |
| I-14 | 〃 | | 〃 | 〃 | $NHCH_3$ | 〃 | 〃 | | 〃 | [111-112 ] |
| I-15 | 〃 | | 〃 | 〃 | $OC_2H_5$ | 〃 | 〃 | | 〃 | $n_D^{22.9}$=1.5212 |
| I-16 | 〃 | | 〃 | 〃 | O 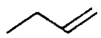 | 〃 | 〃 | | 〃 | $n_D^{25.4}$=1.5230 |
| I-17 | 〃 | | 〃 | 〃 | $SC_2H_5$ | 〃 | 〃 | | 〃 | $n_D^{23.8}$=1.5566 |
| I-18 | 〃 | | nPr | 〃 | Cl | 〃 | 〃 | | 〃 | $n_D^{23.8}$=1.5759 |
| I-19 | 〃 | | 〃 | 〃 | $OCH_3$ | 〃 | 〃 | | 〃 | $n_D^{23.1}$=1.5596 |
| I-20 | 〃 | | 〃 | 〃 | $SCH_3$ | 〃 | 〃 | | 〃 | $n_D^{23.9}$=1.5975 |
| I-21 | 〃 | | isoPr | 〃 | Cl | 〃 | 〃 | | 〃 | $n_D^{24.3}$=1.5708 |
| I-22 | 〃 | | 〃 | 〃 | $OCH_3$ | 〃 | 〃 | | 〃 | $n_D^{24.5}$=1.5555 |
| I-23 | 〃 | | 〃 | 〃 | $SCH_3$ | 〃 | 〃 | | 〃 | [ 71- 73 ] |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-24 | =O | | tBu | H | Cl | Cl | H | | 0 | [59-62 ] |
| I-25 | 〃 | | 〃 | 〃 | $OCH_3$ | 〃 | 〃 | | 〃 | $n_D^{23.8}$ =1.5421 |
| I-26 | 〃 | | tBu | H | $SCH_3$ | 〃 | 〃 | | 0 | $n_D^{23.7}$ =1.5828 |
| I-27 | 〃 | | 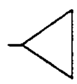 | 〃 | Cl | 〃 | 〃 | | 〃 | |
| I-28 | 〃 | | 〃 | 〃 | $OCH_3$ | 〃 | 〃 | | 〃 | $n_D^{26.6}$ =1.5768 |
| I-29 | 〃 | | 〃 | 〃 | $SCH_3$ | 〃 | 〃 | | 〃 | $n_D^{27.1}$ =1.6060 |
| I-30 | 〃 | | 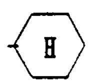 | 〃 | $OCH_3$ | 〃 | 〃 | | 〃 | $n_D^{24.1}$ =1.5690 |
| I-31 | 〃 | | 〃 | 〃 | $SCH_3$ | 〃 | 〃 | | 〃 | |
| I-32 | 〃 | | 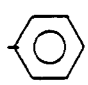 | 〃 | $OCH_3$ | 〃 | 〃 | | 〃 | [107-8 ] |
| I-33 | 〃 | | 〃 | 〃 | $SCH_3$ | 〃 | 〃 | | 〃 | [114-5 ] |
| I-34 | 〃 | | H | $CO_2CH_3$ | $OCH_3$ | 〃 | 〃 | | 〃 | [110-111 ] |
| I-35 | 〃 | | 〃 | $CO_2C_2H_5$ | $SCH_3$ | 〃 | 〃 | | 〃 | $n_D^{24.4}$ =1.6061 |
| I-36 | 〃 | | $CH_2OCH_3$ | H | Cl | 〃 | 〃 | | 〃 | $n_D^{24.2}$ =1.5799 |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-37 | =O | | $CH_2OCH_3$ | H | $OCH_3$ | Cl | H | | 0 | [54-6] |
| I-38 | 〃 | | 〃 | 〃 | $SCH_3$ | 〃 | 〃 | | 〃 | $n_D^{24.3}$ =1.6050 |
| I-39 | 〃 | | $CH_3$ | 〃 | Cl | F | H | | 0 | [75-6] |
| I-40 | 〃 | | 〃 | 〃 | $OCH_3$ | 〃 | 〃 | | 〃 | [111-3] |
| I-41 | 〃 | | 〃 | 〃 | $SCH_3$ | 〃 | 〃 | | 〃 | [98-100] |
| I-42 | 〃 | | 〃 | 〃 | $NHCH_3$ | 〃 | 〃 | | 〃 | [138-140] |
| I-43 | 〃 | | $CF_3$ | 〃 | $OCH_3$ | 〃 | 〃 | | 〃 | [89-91] |
| I-44 | 〃 | | 〃 | 〃 | $SCH_3$ | 〃 | 〃 | | 〃 | [49-50] |
| I-45 | 〃 | | $CH_3$ | 〃 | $OCH_3$ | Br | 〃 | | 〃 | $n_D^{25}$ =1.5919 |
| I-46 | 〃 | | 〃 | 〃 | 〃 | I | 〃 | | 〃 | $n_D^{25}$ =1.6180 |
| I-47 | 〃 | | $CF_3$ | $CH_3$ | Cl | Cl | 〃 | | 〃 | [77-9] |
| I-48 | 〃 | | 〃 | 〃 | $OCH_3$ | 〃 | 〃 | | 〃 | $n_D^{24.6}$ =1.5320 |
| I-49 | 〃 | | 〃 | 〃 | $SCH_3$ | 〃 | 〃 | | 〃 | $n_D^{24.5}$ =1.5637 |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-50 | =O | | $CH_3$ | nPr | Cl | Cl | H | | 0 | [70-4] |
| I-51 | 〃 | | 〃 | 〃 | $OCH_3$ | 〃 | 〃 | | 〃 | |
| I-52 | 〃 | | $CF_3$ | 〃 | $SCH_3$ | 〃 | 〃 | | 〃 | $n_D^{22.9}$ =1.5581 |
| I-53 | 〃 | | 〃 | isoPr | Cl | 〃 | 〃 | | 〃 | [83-5] |
| I-54 | 〃 | | 〃 | 〃 | SMe | 〃 | 〃 | | 〃 | $n_D^{22.9}$ =1.5587 |
| I-55 | 〃 | | $CH_3$ | H | Cl | $CH_3$ | 〃 | | 〃 | [82-4] |
| I-56 | 〃 | | 〃 | 〃 | $OCH_3$ | 〃 | 〃 | | 〃 | [82-4] |
| I-57 | 〃 | | 〃 | 〃 | $SCH_3$ | 〃 | 〃 | | 〃 | [82-4] |
| I-58 | 〃 | | 〃 | 〃 | $NHCH_3$ | 〃 | 〃 | | 〃 | [121-3 ] |
| I-59 | 〃 | | $CF_3$ | 〃 | Cl | 〃 | 〃 | | 〃 | [61-3] |
| I-60 | 〃 | | 〃 | 〃 | $OCH_3$ | 〃 | 〃 | | 〃 | [73-4] |
| I-61 | 〃 | | 〃 | 〃 | $SCH_3$ | 〃 | 〃 | | 〃 | [78-9] |
| I-62 | 〃 | | 〃 | 〃 | $NHCH_3$ | 〃 | 〃 | | 〃 | [117-9 ] |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-63 | =O | | $CH_3$ | H | Cl | $OCH_3$ | H | | 0 | $n_D^{23.1}$ =1.5831 |
| I-64 | 〃 | | 〃 | 〃 | $OCH_3$ | 〃 | 〃 | | 〃 | $n_D^{24.2}$ =1.5692 |
| I-65 | 〃 | | 〃 | 〃 | $SCH_3$ | 〃 | 〃 | | 〃 | [76-8] |
| I-66 | 〃 | | 〃 | 〃 | $NHCH_3$ | 〃 | 〃 | | 〃 | [151-3 ] |
| I-67 | 〃 | | $CF_3$ | 〃 | Cl | 〃 | 〃 | | 〃 | $n_D^{24.0}$ =1.5374 |
| I-68 | 〃 | | 〃 | 〃 | $OCH_3$ | 〃 | 〃 | | 〃 | $n_D^{24.3}$ =1.5246 |
| I-69 | 〃 | | 〃 | 〃 | $SCH_3$ | 〃 | 〃 | | 〃 | $n_D^{24.1}$ =1.5642 |
| I-70 | 〃 | | 〃 | 〃 | $NHCH_3$ | 〃 | 〃 | | 〃 | [115-7 ] |
| I-71 | 〃 | | $CH_3$ | 〃 | Cl | Cl | 〃 | 3-Cl | 1 | |
| I-72 | 〃 | | 〃 | 〃 | $OCH_3$ | 〃 | 〃 | 〃 | 〃 | [100-2 ] |
| I-73 | 〃 | | 〃 | 〃 | $SCH_3$ | 〃 | 〃 | 〃 | 〃 | [110-2 ] |
| I-74 | 〃 | | $CF_3$ | 〃 | Cl | 〃 | 〃 | 〃 | 〃 | [69-71 ] |
| I-75 | 〃 | | 〃 | 〃 | $OCH_3$ | 〃 | 〃 | 〃 | 〃 | [93-4] |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-76 | =O | | $CF_3$ | H | $SCH_3$ | Cl | H | 3-Cl | 1 | [138-140] |
| I-77 | 〃 | | $CH_3$ | 〃 | Cl | 〃 | 〃 | 4-Cl | 〃 | [105-7] |
| I-78 | 〃 | | 〃 | 〃 | $OCH_3$ | 〃 | 〃 | 〃 | 〃 | [95-7] |
| I-79 | 〃 | | 〃 | 〃 | $SCH_3$ | 〃 | 〃 | 〃 | 〃 | [85-7] |
| I-80 | 〃 | | 〃 | 〃 | $SO_2CH_3$ | 〃 | 〃 | 〃 | 〃 | [131-3] |
| I-81 | 〃 | | 〃 | 〃 | H<br>$NCH_2C\equiv CH$ | 〃 | 〃 | 〃 | 〃 | [131-5] |
| I-82 | 〃 | | $CF_3$ | 〃 | Cl | 〃 | 〃 | 〃 | 〃 | [73-5] |
| I-83 | 〃 | | 〃 | 〃 | $OCH_3$ | 〃 | 〃 | 〃 | 〃 | $n_D^{24.2}$=1.5373 |
| I-84 | 〃 | | 〃 | 〃 | $SCH_3$ | 〃 | 〃 | 〃 | 〃 | [71-3] |
| I-85 | 〃 | | 〃 | 〃 | $OC_2H_5$ | 〃 | 〃 | 〃 | 〃 | $n_D^{23.5}$=1.5347 |
| I-86 | 〃 | | 〃 | 〃 | 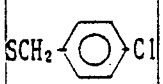 | 〃 | 〃 | 〃 | 〃 | $n_D^{22.8}$=1.6061 |
| I-87 | 〃 | | 〃 | 〃 | $SC_3H_7^n$ | 〃 | 〃 | 〃 | 〃 | $n_D^{23.9}$=1.5607 |
| I-88 | 〃 | | 〃 | 〃 | $NHCH_3$ | 〃 | 〃 | 〃 | 〃 | [124-6] |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-89 | =O | | $CF_3$ | H | $SO_2CH_3$ | Cl | H | 4-Cl | 1 | [120-2 ] |
| I-90 | 〃 | | $CH_3$ | 〃 | $SCH_3$ | 〃 | F | | 0 | [95-7] |
| I-91 | 〃 | | $CF_3$ | 〃 | Cl | 〃 | 〃 | | 〃 | [91-3] |
| I-92 | 〃 | | 〃 | 〃 | $OCH_3$ | 〃 | 〃 | | 〃 | [62-4] |
| I-93 | 〃 | | 〃 | 〃 | $SCH_3$ | 〃 | F | | 0 | $n_D^{23.2}$ =1.5531 |
| I-94 | 〃 | | isoPr | 〃 | Cl | 〃 | 〃 | | 〃 | $n_D^{23.9}$ =1.5570 |
| I-95 | 〃 | | 〃 | 〃 | $OCH_3$ | 〃 | 〃 | | 〃 | |
| I-96 | 〃 | | 〃 | 〃 | $SCH_3$ | 〃 | 〃 | | 〃 | $n_D^{22.9}$ =1.5811 |
| I-97 | 〃 | | 〃 | 〃 | $NHCH_3$ | 〃 | 〃 | | 〃 | |
| I-98 | 〃 | | 〃 | 〃 | $N(CH_3)_2$ | 〃 | 〃 | | 〃 | |
| I-99 | 〃 | | 〃 | 〃 | OH | 〃 | 〃 | | 〃 | |
| I-100 | 〃 | | $CH_3$ | 〃 | 〃 | 〃 | Cl | | 〃 | |
| I-101 | 〃 | | 〃 | 〃 | 〃 | Br | Br | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-102 | =0 | | $CH_3$ | H | OH | $CH_3$ | Cl | | 0 | |
| I-103 | 〃 | | 〃 | 〃 | 〃 | 〃 | $CH_3$ | | 〃 | |
| I-104 | 〃 | | 〃 | 〃 | 〃 | 〃 | H | 3-$CH_3$ | 1 | |
| I-105 | 〃 | | 〃 | 〃 | 〃 | 〃 | 〃 | 4-$CH_3$ | 〃 | |
| I-106 | 〃 | | 〃 | 〃 | 〃 | 〃 | 〃 | 5-$CH_3$ | 〃 | |
| I-107 | 〃 | | 〃 | 〃 | 〃 | $C_2H_5$ | $C_2H_5$ | | 0 | |
| I-108 | 〃 | | 〃 | 〃 | Cl | Cl | Cl | | 〃 | |
| I-109 | 〃 | | 〃 | 〃 | 〃 | Br | Br | | 〃 | |
| I-110 | 〃 | | 〃 | 〃 | 〃 | $CH_3$ | Cl | | 〃 | |
| I-111 | 〃 | | 〃 | 〃 | 〃 | $CH_3$ | $CH_3$ | | 〃 | |
| I-112 | 〃 | | 〃 | 〃 | 〃 | $CH_3$ | H | 3-$CH_3$ | 1 | |
| I-113 | 〃 | | 〃 | 〃 | 〃 | $CH_3$ | H | 4-$CH_3$ | 〃 | |
| I-114 | 〃 | | 〃 | 〃 | 〃 | $CH_3$ | H | 5-$CH_3$ | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-115 | =O | | $CH_3$ | H | Cl | $C_2H_5$ | $C_2H_5$ | | 0 | |
| I-116 | 〃 | | 〃 | 〃 | $OCH_3$ | Cl | Cl | | 〃 | |
| I-117 | 〃 | | 〃 | 〃 | 〃 | Br | Br | | 〃 | |
| I-118 | 〃 | | 〃 | 〃 | 〃 | $CH_3$ | Cl | | 〃 | |
| I-119 | 〃 | | 〃 | 〃 | 〃 | $CH_3$ | $CH_3$ | | 0 | |
| I-120 | 〃 | | 〃 | 〃 | 〃 | $CH_3$ | H | 3-$CH_3$ | 1 | |
| I-121 | 〃 | | 〃 | 〃 | 〃 | $CH_3$ | H | 4-$CH_3$ | 〃 | |
| I-122 | 〃 | | 〃 | 〃 | 〃 | $CH_3$ | H | 5-$CH_3$ | 〃 | |
| I-123 | 〃 | | 〃 | 〃 | 〃 | $C_2H_5$ | $C_2H_5$ | | 0 | |
| I-124 | 〃 | | 〃 | 〃 | $SCH_3$ | Cl | Cl | | 〃 | |
| I-125 | 〃 | | 〃 | 〃 | 〃 | Br | Br | | 〃 | |
| I-126 | 〃 | | 〃 | 〃 | 〃 | $CH_3$ | Cl | | 〃 | |
| I-127 | 〃 | | 〃 | 〃 | 〃 | $CH_3$ | $CH_3$ | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-128 | =0 | | $CH_3$ | H | $OCH_3$ | $CH_3$ | H | 3-$CH_3$ | 1 | |
| I-129 | 〃 | | 〃 | 〃 | 〃 | $CH_3$ | H | 4-$CH_3$ | 〃 | |
| I-130 | 〃 | | 〃 | 〃 | 〃 | $CH_3$ | H | 5-$CH_3$ | 〃 | |
| I-131 | 〃 | | 〃 | 〃 | 〃 | $C_2H_5$ | $C_2H_5$ | | 0 | |
| I-132 | 〃 | | 〃 | 〃 | $SO_2CH_3$ | Cl | Cl | | 〃 | |
| I-133 | 〃 | | 〃 | 〃 | 〃 | Br | Br | | 〃 | |
| I-134 | 〃 | | 〃 | 〃 | 〃 | $CH_3$ | Cl | | 〃 | |
| I-135 | 〃 | | 〃 | 〃 | 〃 | $CH_3$ | $CH_3$ | | 〃 | |
| I-136 | 〃 | | 〃 | 〃 | 〃 | $CH_3$ | H | 3-$CH_3$ | 1 | |
| I-137 | 〃 | | 〃 | 〃 | 〃 | $CH_3$ | H | 4-$CH_3$ | 〃 | |
| I-138 | 〃 | | 〃 | 〃 | 〃 | $CH_3$ | H | 5-$CH_3$ | 〃 | |
| I-139 | 〃 | | 〃 | 〃 | 〃 | $C_2H_5$ | $C_2H_5$ | | 0 | |
| I-140 | 〃 | | 〃 | 〃 | $NHCH_3$ | Cl | Cl | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-141 | =0 | | $CF_3$ | H | $NHCH_3$ | Br | Br | | 0 | |
| I-142 | 〃 | | 〃 | 〃 | 〃 | $CH_3$ | Cl | | 〃 | |
| I-144 | 〃 | | 〃 | 〃 | 〃 | $CH_3$ | $CH_3$ | | 〃 | |
| I-145 | 〃 | | $CH_3$ | 〃 | 〃 | $CH_3$ | H | 4-$CH_3$ | 1 | |
| I-146 | 〃 | | 〃 | 〃 | 〃 | $CH_3$ | H | 5-$CH_3$ | 〃 | |
| I-147 | 〃 | | 〃 | 〃 | 〃 | $C_2H_5$ | $C_2H_5$ | | 0 | |
| I-148 | 〃 | | 〃 | 〃 | $N(CH_3)_2$ | Cl | Cl | | 〃 | |
| I-149 | 〃 | | 〃 | 〃 | 〃 | Br | Br | | 〃 | |
| I-150 | 〃 | | 〃 | 〃 | 〃 | $CH_3$ | Cl | | 〃 | |
| I-151 | 〃 | | 〃 | 〃 | 〃 | $CH_3$ | $CH_3$ | | 〃 | |
| I-152 | 〃 | | 〃 | 〃 | 〃 | $CH_3$ | H | 3-$CH_3$ | 1 | |
| I-153 | 〃 | | 〃 | 〃 | 〃 | $CH_3$ | H | 4-$CH_3$ | 〃 | |
| I-154 | 〃 | | 〃 | 〃 | 〃 | $CH_3$ | H | 5-$CH_3$ | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-155 | =0 | | $CH_3$ | H | $N(CH_3)_2$ | $C_2H_5$ | $C_2H_5$ | | 0 | |
| I-156 | 〃 | | $CF_3$ | 〃 | OH | Cl | Cl | | 〃 | |
| I-157 | 〃 | | 〃 | 〃 | 〃 | Br | Br | | 〃 | |
| I-158 | 〃 | | 〃 | 〃 | 〃 | $CH_3$ | Cl | | 〃 | |
| I-159 | 〃 | | 〃 | 〃 | 〃 | $CH_3$ | $CH_3$ | | 〃 | |
| I-160 | 〃 | | 〃 | 〃 | 〃 | $CH_3$ | H | 3-$CH_3$ | 1 | |
| I-161 | 〃 | | 〃 | 〃 | 〃 | $CH_3$ | H | 4-$CH_3$ | 〃 | |
| I-162 | 〃 | | 〃 | 〃 | 〃 | $CH_3$ | H | 5-$CH_3$ | 〃 | |
| I-163 | 〃 | | 〃 | 〃 | 〃 | $C_2H_5$ | $C_2H_5$ | | 0 | |
| I-164 | 〃 | | 〃 | 〃 | Cl | Cl | Cl | | 〃 | |
| I-165 | 〃 | | 〃 | 〃 | 〃 | Br | Br | | 〃 | |
| I-166 | 〃 | | 〃 | 〃 | 〃 | $CH_3$ | Cl | | 0 | |
| I-167 | 〃 | | 〃 | 〃 | 〃 | $CH_3$ | $CH_3$ | | 〃 | [75-77] |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-168 | =O | | $CF_3$ | H | Cl | $CH_3$ | H | 3-$CH_3$ | 1 | [73-74 ] |
| I-169 | 〃 | | 〃 | 〃 | 〃 | $CH_3$ | H | 4-$CH_3$ | 〃 | [69-71 ] |
| I-170 | 〃 | | 〃 | 〃 | 〃 | $CH_3$ | H | 5-$CH_3$ | 〃 | |
| I-171 | 〃 | | 〃 | 〃 | 〃 | $C_2H_5$ | $C_2H_5$ | | 0 | |
| I-172 | 〃 | | 〃 | 〃 | $OCH_3$ | Cl | Cl | | 〃 | [82-83 ] |
| I-173 | 〃 | | 〃 | 〃 | 〃 | Br | Br | | 〃 | |
| I-174 | 〃 | | 〃 | 〃 | 〃 | $CH_3$ | Cl | | 〃 | |
| I-175 | 〃 | | 〃 | 〃 | 〃 | $CH_3$ | $CH_3$ | | 〃 | |
| I-176 | 〃 | | 〃 | 〃 | 〃 | $CH_3$ | H | 3-$CH_3$ | 1 | |
| I-177 | 〃 | | 〃 | 〃 | 〃 | $CH_3$ | H | 4-$CH_3$ | 〃 | |
| I-178 | 〃 | | 〃 | 〃 | 〃 | $CH_3$ | H | 5-$CH_3$ | 〃 | |
| I-179 | 〃 | | 〃 | 〃 | 〃 | $C_2H_5$ | $C_2H_5$ | | 0 | |
| I-180 | 〃 | | 〃 | 〃 | $SCH_3$ | Cl | Cl | | 〃 | $n_D^{22.4}$ =1.5693 |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-181 | =0 | | $CF_3$ | H | $SCH_3$ | Br | Br | | 0 | |
| I-182 | 〃 | | 〃 | 〃 | 〃 | $CH_3$ | Cl | | 〃 | |
| I-183 | 〃 | | 〃 | 〃 | 〃 | $CH_3$ | $CH_3$ | | 〃 | $n_D^{22.6}$ =1.5537 |
| I-184 | 〃 | | 〃 | 〃 | 〃 | $CH_3$ | H | 3-$CH_3$ | 1 | 〔71-72〕 |
| I-185 | 〃 | | 〃 | 〃 | 〃 | $CH_3$ | H | 4-$CH_3$ | 〃 | 〔96-97〕 |
| I-186 | 〃 | | 〃 | 〃 | 〃 | $CH_3$ | H | 5-$CH_3$ | 〃 | 〔83-85〕 |
| I-187 | 〃 | | 〃 | 〃 | 〃 | $C_2H_5$ | $C_2H_5$ | | 0 | |
| I-188 | 〃 | | 〃 | 〃 | $SO_2CH_3$ | Cl | Cl | | 〃 | |
| I-189 | 〃 | | 〃 | 〃 | 〃 | Br | Br | | 〃 | |
| I-190 | 〃 | | 〃 | 〃 | 〃 | $CH_3$ | Cl | | 〃 | |
| I-191 | 〃 | | 〃 | 〃 | 〃 | $CH_3$ | $CH_3$ | | 〃 | |
| I-192 | 〃 | | 〃 | 〃 | 〃 | $CH_3$ | H | 3-$CH_3$ | 1 | |
| I-193 | 〃 | | 〃 | 〃 | 〃 | $CH_3$ | H | 4-$CH_3$ | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-194 | =O | | $CF_3$ | H | $SO_2CH_3$ | $CH_3$ | H | 5-$CH_3$ | 1 | |
| I-195 | 〃 | | 〃 | 〃 | 〃 | $C_2H_5$ | $C_2H_5$ | | 0 | |
| I-196 | 〃 | | 〃 | 〃 | $NHCH_3$ | Cl | Cl | | 〃 | |
| I-197 | 〃 | | 〃 | 〃 | 〃 | Br | Br | | 〃 | |
| I-198 | 〃 | | 〃 | 〃 | 〃 | $CH_3$ | Cl | | 〃 | |
| I-199 | 〃 | | 〃 | 〃 | 〃 | $CH_3$ | $CH_3$ | | 〃 | |
| I-200 | 〃 | | 〃 | 〃 | 〃 | $CH_3$ | H | 3-$CH_3$ | 1 | |
| I-201 | 〃 | | 〃 | 〃 | 〃 | $CH_3$ | H | 4-$CH_3$ | 〃 | [134-136 ] |
| I-202 | 〃 | | 〃 | 〃 | 〃 | $CH_3$ | H | 5-$CH_3$ | 〃 | |
| I-203 | 〃 | | 〃 | 〃 | 〃 | $C_2H_5$ | $C_2H_5$ | | 0 | |
| I-204 | 〃 | | 〃 | 〃 | $N(CH_3)_2$ | Cl | Cl | | 〃 | |
| I-205 | 〃 | | 〃 | 〃 | 〃 | Br | Br | | 〃 | |
| I-206 | 〃 | | 〃 | 〃 | 〃 | $CH_3$ | Cl | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-207 | =0 | | $CF_3$ | H | $N(CH_3)_2$ | $CH_3$ | $CH_3$ | | 0 | |
| I-208 | 〃 | | 〃 | 〃 | 〃 | $CH_3$ | H | $3-CH_3$ | 1 | |
| I-209 | 〃 | | 〃 | 〃 | 〃 | $CH_3$ | H | $4-CH_3$ | 〃 | |
| I-210 | 〃 | | 〃 | 〃 | 〃 | $CH_3$ | H | $5-CH_3$ | 〃 | |
| I-211 | 〃 | | 〃 | 〃 | 〃 | $C_2H_5$ | $C_2H_5$ | | 0 | |
| I-212 | 〃 | | $C_2H_5$ | 〃 | OH | Cl | Cl | | 〃 | |
| I-213 | 〃 | | 〃 | 〃 | 〃 | Br | Br | | 〃 | |
| I-214 | 〃 | | 〃 | 〃 | 〃 | $CH_3$ | Cl | | 〃 | |
| I-215 | 〃 | | 〃 | 〃 | 〃 | $CH_3$ | $CH_3$ | | 〃 | |
| I-216 | 〃 | | 〃 | 〃 | 〃 | $CH_3$ | H | $3-CH_3$ | 1 | |
| I-217 | 〃 | | 〃 | 〃 | 〃 | $CH_3$ | H | $4-CH_3$ | 〃 | |
| I-218 | 〃 | | 〃 | 〃 | 〃 | $CH_3$ | H | $5-CH_3$ | 〃 | |
| I-219 | 〃 | | 〃 | 〃 | 〃 | $C_2H_5$ | $C_2H_5$ | | 0 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-220 | =O | | $CF_3$ | H | Cl | Cl | Cl | | 0 | |
| I-221 | 〃 | | 〃 | 〃 | 〃 | Br | Br | | 〃 | |
| I-222 | 〃 | | 〃 | 〃 | 〃 | $CH_3$ | Cl | | 〃 | |
| I-223 | 〃 | | $C_2H_5$ | 〃 | 〃 | $CH_3$ | $CH_3$ | | 〃 | |
| I-224 | 〃 | | 〃 | 〃 | 〃 | $CH_3$ | H | 3-$CH_3$ | 1 | |
| I-225 | 〃 | | 〃 | 〃 | 〃 | $CH_3$ | H | 4-$CH_3$ | 〃 | |
| I-226 | 〃 | | 〃 | 〃 | 〃 | $CH_3$ | H | 5-$CH_3$ | 〃 | |
| I-227 | 〃 | | 〃 | 〃 | 〃 | $C_2H_5$ | $C_2H_5$ | | 0 | |
| I-228 | 〃 | | 〃 | 〃 | $OCH_3$ | Cl | Cl | | 〃 | |
| I-229 | 〃 | | 〃 | 〃 | 〃 | Br | Br | | 〃 | |
| I-230 | 〃 | | 〃 | 〃 | 〃 | $CH_3$ | Cl | | 〃 | |
| I-231 | 〃 | | 〃 | 〃 | 〃 | $CH_3$ | $CH_3$ | | 〃 | |
| I-232 | 〃 | | 〃 | 〃 | 〃 | $CH_3$ | H | 3-$CH_3$ | 1 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-233 | =0 | | $C_2H_5$ | H | $OCH_3$ | $CH_3$ | H | 4-$CH_3$ | 1 | |
| I-234 | 〃 | | 〃 | 〃 | 〃 | $CH_3$ | H | 5-$CH_3$ | 〃 | |
| I-235 | 〃 | | 〃 | 〃 | 〃 | $C_2H_5$ | $C_2H_5$ | | 0 | |
| I-236 | 〃 | | 〃 | 〃 | $SCH_3$ | Cl | Cl | | 〃 | |
| I-237 | 〃 | | 〃 | 〃 | 〃 | Br | Br | | 〃 | |
| I-238 | 〃 | | 〃 | 〃 | 〃 | $CH_3$ | Cl | | 〃 | |
| I-239 | 〃 | | 〃 | 〃 | 〃 | $CH_3$ | $CH_3$ | | 〃 | |
| I-240 | 〃 | | 〃 | 〃 | 〃 | $CH_3$ | H | 3-$CH_3$ | 1 | |
| I-241 | 〃 | | 〃 | 〃 | 〃 | $CH_3$ | H | 4-$CH_3$ | 〃 | |
| I-242 | 〃 | | 〃 | 〃 | 〃 | $CH_3$ | H | 5-$CH_3$ | 〃 | |
| I-243 | 〃 | | 〃 | 〃 | 〃 | $C_2H_5$ | $C_2H_5$ | | 0 | |
| I-244 | 〃 | | 〃 | 〃 | $SO_2CH_3$ | Cl | Cl | | 〃 | |
| I-245 | 〃 | | 〃 | 〃 | 〃 | Br | Br | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-246 | =0 | | $C_2H_5$ | H | $SO_2CH_3$ | $CH_3$ | Cl | | 0 | |
| I-247 | 〃 | | 〃 | 〃 | 〃 | $CH_3$ | $CH_3$ | | 〃 | |
| I-248 | 〃 | | 〃 | 〃 | 〃 | $CH_3$ | H | 3-$CH_3$ | 1 | |
| I-249 | 〃 | | 〃 | 〃 | 〃 | $CH_3$ | H | 4-$CH_3$ | 〃 | |
| I-250 | 〃 | | 〃 | 〃 | 〃 | $CH_3$ | H | 5-$CH_3$ | 〃 | |
| I-251 | 〃 | | 〃 | 〃 | 〃 | $C_2H_5$ | $C_2H_5$ | | 0 | |
| I-252 | 〃 | | 〃 | 〃 | $NHCH_3$ | Cl | Cl | | 〃 | |
| I-253 | 〃 | | 〃 | 〃 | 〃 | Br | Br | | 〃 | |
| I-254 | 〃 | | 〃 | 〃 | 〃 | $CH_3$ | Cl | | 〃 | |
| I-255 | 〃 | | 〃 | 〃 | 〃 | $CH_3$ | $CH_3$ | | 〃 | |
| I-256 | 〃 | | 〃 | 〃 | 〃 | $CH_3$ | H | 3-$CH_3$ | 1 | |
| I-257 | 〃 | | 〃 | 〃 | 〃 | $CH_3$ | H | 4-$CH_3$ | 〃 | |
| I-258 | 〃 | | 〃 | 〃 | 〃 | $CH_3$ | H | 5-$CH_3$ | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-259 | =O | | $C_2H_5$ | H | $NHCH_3$ | $C_2H_5$ | $C_2H_5$ | | 0 | |
| I-260 | 〃 | | 〃 | 〃 | $N(CH_3)_2$ | Cl | Cl | | 〃 | |
| I-261 | 〃 | | 〃 | 〃 | 〃 | Br | Br | | 〃 | |
| I-262 | 〃 | | 〃 | 〃 | 〃 | $CH_3$ | Cl | | 〃 | |
| I-263 | 〃 | | 〃 | 〃 | 〃 | $CH_3$ | $CH_3$ | | 〃 | |
| I-264 | 〃 | | 〃 | 〃 | 〃 | $CH_3$ | H | 3-$CH_3$ | 1 | |
| I-265 | 〃 | | 〃 | 〃 | 〃 | $CH_3$ | H | 4-$CH_3$ | 〃 | |
| I-266 | 〃 | | 〃 | 〃 | 〃 | $CH_3$ | H | 5-$CH_3$ | 〃 | |
| I-267 | 〃 | | 〃 | 〃 | 〃 | $C_2H_5$ | $C_2H_5$ | | 0 | |
| I-268 | 〃 | | isoPr | 〃 | OH | Cl | Cl | | 〃 | |
| I-269 | 〃 | | 〃 | 〃 | 〃 | Br | Br | | 〃 | |
| I-270 | 〃 | | 〃 | 〃 | 〃 | $CH_3$ | Cl | | 0 | |
| I-271 | 〃 | | 〃 | 〃 | 〃 | $CH_3$ | $CH_3$ | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-272 | =0 | | isoPr | H | OH | $CH_3$ | H | 3-$CH_3$ | 1 | |
| I-273 | 〃 | | 〃 | 〃 | 〃 | $CH_3$ | H | 4-$CH_3$ | 〃 | |
| I-274 | 〃 | | 〃 | 〃 | 〃 | $CH_3$ | H | 5-$CH_3$ | 〃 | |
| I-275 | 〃 | | 〃 | 〃 | 〃 | $C_2H_5$ | $C_2H_5$ | | 0 | |
| I-276 | 〃 | | 〃 | 〃 | Cl | Cl | Cl | | 〃 | [85-88 ] |
| I-277 | 〃 | | 〃 | 〃 | 〃 | Br | Br | | 〃 | |
| I-278 | 〃 | | 〃 | 〃 | 〃 | $CH_3$ | Cl | | 〃 | |
| I-279 | 〃 | | 〃 | 〃 | 〃 | $CH_3$ | $CH_3$ | | 〃 | $n_D^{22.0}$ =1.5545 |
| I-280 | 〃 | | 〃 | 〃 | 〃 | $CH_3$ | H | 3-$CH_3$ | 1 | $n_D^{22.0}$ =1.5746 |
| I-281 | 〃 | | 〃 | 〃 | 〃 | $CH_3$ | H | 4-$CH_3$ | 〃 | $n_D^{22.8}$ =1.5704 |
| I-282 | 〃 | | 〃 | 〃 | 〃 | $CH_3$ | H | 5-$CH_3$ | 〃 | |
| I-283 | 〃 | | 〃 | 〃 | 〃 | $C_2H_5$ | $C_2H_5$ | | 0 | |
| I-284 | 〃 | | 〃 | 〃 | $OCH_3$ | Cl | Cl | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-285 | =0 | | isoPr | H | $OCH_3$ | Br | Br | | 0 | |
| I-286 | 〃 | | 〃 | 〃 | 〃 | $CH_3$ | Cl | | 〃 | |
| I-287 | 〃 | | 〃 | 〃 | 〃 | $CH_3$ | $CH_3$ | | 〃 | |
| I-288 | 〃 | | 〃 | 〃 | 〃 | $CH_3$ | H | 3-$CH_3$ | 1 | |
| I-289 | 〃 | | 〃 | 〃 | 〃 | $CH_3$ | H | 4-$CH_3$ | 〃 | |
| I-290 | 〃 | | 〃 | 〃 | 〃 | $CH_3$ | H | 5-$CH_3$ | 〃 | |
| I-291 | 〃 | | 〃 | 〃 | 〃 | $C_2H_5$ | $C_2H_5$ | | 0 | |
| I-292 | 〃 | | 〃 | 〃 | $SCH_3$ | Cl | Cl | | 〃 | [78-80 ] |
| I-293 | 〃 | | 〃 | 〃 | 〃 | Br | Br | | 〃 | |
| I-294 | 〃 | | 〃 | 〃 | 〃 | $CH_3$ | Cl | | 〃 | |
| I-295 | 〃 | | 〃 | 〃 | 〃 | $CH_3$ | $CH_3$ | | 〃 | $n_D^{22.2}$ =1.5622 |
| I-296 | 〃 | | 〃 | 〃 | 〃 | $CH_3$ | H | 3-$CH_3$ | 1 | $n_D^{22.9}$ =1.5862 |
| I-297 | 〃 | | 〃 | 〃 | 〃 | $CH_3$ | H | 4-$CH_3$ | 〃 | $n_D^{25.0}$ =1.5920 |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-298 | =0 | | isoPr | H | $SCH_3$ | $CH_3$ | H | 5-$CH_3$ | 1 | |
| I-299 | 〃 | | 〃 | 〃 | 〃 | $C_2H_5$ | $C_2H_5$ | | 0 | |
| I-300 | 〃 | | 〃 | 〃 | $SO_2CH_3$ | Cl | Cl | | 〃 | |
| I-301 | 〃 | | 〃 | 〃 | 〃 | Br | Br | | 〃 | |
| I-302 | 〃 | | 〃 | 〃 | 〃 | $CH_3$ | Cl | | 〃 | |
| I-303 | 〃 | | 〃 | 〃 | 〃 | $CH_3$ | $CH_3$ | | 〃 | |
| I-304 | 〃 | | 〃 | 〃 | 〃 | $CH_3$ | H | 3-$CH_3$ | 1 | |
| I-305 | 〃 | | 〃 | 〃 | 〃 | $CH_3$ | H | 4-$CH_3$ | 〃 | |
| I-306 | 〃 | | 〃 | 〃 | 〃 | $CH_3$ | H | 5-$CH_3$ | 〃 | |
| I-307 | 〃 | | 〃 | 〃 | 〃 | $C_2H_5$ | $C_2H_5$ | | 0 | |
| I-308 | 〃 | | 〃 | 〃 | $NHCH_3$ | Cl | Cl | | 〃 | |
| I-309 | 〃 | | 〃 | 〃 | 〃 | Br | Br | | 〃 | |
| I-310 | 〃 | | 〃 | 〃 | 〃 | $CH_3$ | Cl | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-311 | =0 | | isoPr | H | $NHCH_3$ | $CH_3$ | $CH_3$ | | 0 | |
| I-312 | 〃 | | 〃 | 〃 | 〃 | $CH_3$ | H | 3-$CH_3$ | 1 | |
| I-313 | 〃 | | 〃 | 〃 | 〃 | $CH_3$ | H | 4-$CH_3$ | 〃 | [124-126 ] |
| I-314 | 〃 | | 〃 | 〃 | 〃 | $CH_3$ | H | 5-$CH_3$ | 〃 | |
| I-315 | 〃 | | 〃 | 〃 | 〃 | $C_2H_5$ | $C_2H_5$ | | 0 | |
| I-316 | 〃 | | 〃 | 〃 | $N(CH_3)_2$ | Cl | Cl | | 〃 | |
| I-317 | 〃 | | 〃 | 〃 | 〃 | Br | Br | | 〃 | |
| I-318 | 〃 | | 〃 | 〃 | 〃 | $CH_3$ | Cl | | 〃 | |
| I-319 | 〃 | | 〃 | 〃 | 〃 | $CH_3$ | $CH_3$ | | 〃 | |
| I-320 | 〃 | | 〃 | 〃 | 〃 | $CH_3$ | H | 3-$CH_3$ | 1 | |
| I-321 | 〃 | | 〃 | 〃 | 〃 | $CH_3$ | H | 4-$CH_3$ | 〃 | |
| I-322 | 〃 | | 〃 | 〃 | 〃 | $CH_3$ | H | 5-$CH_3$ | 〃 | |
| I-323 | 〃 | | 〃 | 〃 | 〃 | $C_2H_5$ | $C_2H_5$ | | 0 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-324 | =0 | | tBu | H | OH | Cl | Cl | | 0 | |
| I-325 | 〃 | | 〃 | 〃 | 〃 | Br | Br | | 〃 | |
| I-326 | 〃 | | 〃 | 〃 | 〃 | $CH_3$ | Cl | | 〃 | |
| I-327 | 〃 | | 〃 | 〃 | 〃 | $CH_3$ | $CH_3$ | | 〃 | |
| I-328 | 〃 | | 〃 | 〃 | 〃 | $CH_3$ | H | 3-$CH_3$ | 1 | |
| I-329 | 〃 | | 〃 | 〃 | 〃 | $CH_3$ | H | 4-$CH_3$ | 〃 | |
| I-330 | 〃 | | 〃 | 〃 | 〃 | $CH_3$ | H | 5-$CH_3$ | 〃 | |
| I-331 | 〃 | | 〃 | 〃 | 〃 | $C_2H_5$ | $C_2H_5$ | | 0 | |
| I-332 | 〃 | | 〃 | 〃 | Cl | Cl | Cl | | 〃 | |
| I-333 | 〃 | | 〃 | 〃 | 〃 | Br | Br | | 〃 | |
| I-334 | 〃 | | 〃 | 〃 | 〃 | $CH_3$ | Cl | | 〃 | |
| I-335 | 〃 | | 〃 | 〃 | 〃 | $CH_3$ | $CH_3$ | | 〃 | |
| I-336 | 〃 | | 〃 | 〃 | 〃 | $CH_3$ | H | 3-$CH_3$ | 1 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-337 | =O | | tBu | H | Cl | $CH_3$ | H | 4-$CH_3$ | 1 | |
| I-338 | 〃 | | 〃 | 〃 | 〃 | $CH_3$ | H | 5-$CH_3$ | 〃 | |
| I-339 | 〃 | | 〃 | 〃 | 〃 | $C_2H_5$ | $C_2H_5$ | | 0 | |
| I-340 | 〃 | | 〃 | 〃 | $OCH_3$ | Cl | Cl | | 〃 | |
| I-341 | 〃 | | 〃 | 〃 | 〃 | Br | Br | | 〃 | |
| I-342 | 〃 | | 〃 | 〃 | 〃 | $CH_3$ | Cl | | 〃 | |
| I-343 | 〃 | | 〃 | 〃 | 〃 | $CH_3$ | $CH_3$ | | 〃 | |
| I-344 | 〃 | | 〃 | 〃 | 〃 | $CH_3$ | H | 3-$CH_3$ | 1 | |
| I-345 | 〃 | | 〃 | 〃 | 〃 | $CH_3$ | H | 4-$CH_3$ | 〃 | |
| I-346 | 〃 | | 〃 | 〃 | 〃 | $CH_3$ | H | 5-$CH_3$ | 〃 | |
| I-347 | 〃 | | 〃 | 〃 | 〃 | $C_2H_5$ | $C_2H_5$ | | 0 | |
| I-348 | 〃 | | 〃 | 〃 | $SCH_3$ | Cl | Cl | | 〃 | |
| I-349 | 〃 | | 〃 | 〃 | 〃 | Br | Br | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-350 | =O | | tBu | H | $SCH_3$ | $CH_3$ | Cl | | 0 | |
| I-351 | 〃 | | 〃 | 〃 | 〃 | $CH_3$ | $CH_3$ | | 〃 | |
| I-352 | 〃 | | 〃 | 〃 | 〃 | $CH_3$ | H | 3-$CH_3$ | 1 | |
| I-353 | 〃 | | 〃 | 〃 | 〃 | $CH_3$ | H | 4-$CH_3$ | 〃 | |
| I-354 | 〃 | | 〃 | 〃 | 〃 | $CH_3$ | H | 5-$CH_3$ | 〃 | |
| I-355 | 〃 | | 〃 | 〃 | 〃 | $C_2H_5$ | $C_2H_5$ | | 0 | |
| I-356 | 〃 | | 〃 | 〃 | $SO_2CH_3$ | Cl | Cl | | 〃 | |
| I-357 | 〃 | | 〃 | 〃 | 〃 | Br | Br | | 〃 | |
| I-358 | 〃 | | 〃 | 〃 | 〃 | $CH_3$ | Cl | | 〃 | |
| I-359 | 〃 | | 〃 | 〃 | 〃 | $CH_3$ | $CH_3$ | | 〃 | |
| I-360 | 〃 | | 〃 | 〃 | 〃 | $CH_3$ | H | 3-$CH_3$ | 1 | |
| I-361 | 〃 | | 〃 | 〃 | 〃 | $CH_3$ | H | 4-$CH_3$ | 〃 | |
| I-362 | 〃 | | 〃 | 〃 | 〃 | $CH_3$ | H | 5-$CH_3$ | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-363 | =0 | | tBu | H | $SO_2CH_3$ | $C_2H_5$ | $C_2H_5$ | | 0 | |
| I-364 | 〃 | | 〃 | 〃 | $NHCH_3$ | Cl | Cl | | 〃 | |
| I-365 | 〃 | | 〃 | 〃 | 〃 | Br | Br | | 〃 | |
| I-366 | 〃 | | 〃 | 〃 | 〃 | $CH_3$ | Cl | | 〃 | |
| I-367 | 〃 | | 〃 | 〃 | 〃 | $CH_3$ | $CH_3$ | | 〃 | |
| I-368 | 〃 | | 〃 | 〃 | 〃 | $CH_3$ | H | 3-$CH_3$ | 1 | |
| I-369 | 〃 | | 〃 | 〃 | 〃 | $CH_3$ | H | 4-$CH_3$ | 〃 | |
| I-370 | 〃 | | 〃 | 〃 | 〃 | $CH_3$ | H | 5-$CH_3$ | 〃 | |
| I-371 | 〃 | | 〃 | 〃 | 〃 | $C_2H_5$ | $C_2H_5$ | | 0 | |
| I-372 | 〃 | | 〃 | 〃 | $N(CH_3)_2$ | Cl | Cl | | 〃 | |
| I-373 | 〃 | | 〃 | 〃 | 〃 | Br | Br | | 〃 | |
| I-374 | 〃 | | 〃 | 〃 | 〃 | $CH_3$ | Cl | | 〃 | |
| I-375 | 〃 | | 〃 | 〃 | 〃 | $CH_3$ | $CH_3$ | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-376 | =0 | | tBu | H | $N(CH_3)_2$ | $CH_3$ | H | 3-$CH_3$ | 1 | |
| I-377 | 〃 | | 〃 | 〃 | 〃 | $CH_3$ | H | 4-$CH_3$ | 〃 | |
| I-378 | 〃 | | 〃 | 〃 | 〃 | $CH_3$ | H | 5-$CH_3$ | 〃 | |
| I-379 | 〃 | | 〃 | 〃 | 〃 | $C_2H_5$ | $C_2H_5$ | | 0 | |
| I-380 | 〃 | | 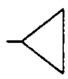 | 〃 | OH | Cl | Cl | | 〃 | |
| I-381 | 〃 | | 〃 | 〃 | 〃 | Br | Br | | 〃 | |
| I-382 | 〃 | | 〃 | 〃 | 〃 | $CH_3$ | Cl | | 〃 | |
| I-383 | 〃 | | 〃 | 〃 | 〃 | $CH_3$ | $CH_3$ | | 〃 | |
| I-384 | 〃 | | 〃 | 〃 | 〃 | $CH_3$ | H | 3-$CH_3$ | 1 | |
| I-385 | 〃 | | 〃 | 〃 | 〃 | $CH_3$ | H | 4-$CH_3$ | 〃 | |
| I-386 | 〃 | | 〃 | 〃 | 〃 | $CH_3$ | H | 5-$CH_3$ | 〃 | |
| I-387 | 〃 | | 〃 | 〃 | 〃 | $C_2H_5$ | $C_2H_5$ | | 0 | |
| I-388 | 〃 | | 〃 | 〃 | Cl | Cl | Cl | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-389 | =0 | | 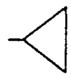 | H | Cl | Br | Br | | 0 | |
| I-390 | 〃 | | 〃 | 〃 | 〃 | $CH_3$ | Cl | | 〃 | |
| I-391 | 〃 | | 〃 | 〃 | 〃 | $CH_3$ | $CH_3$ | | 〃 | |
| I-392 | 〃 | | 〃 | 〃 | 〃 | $CH_3$ | H | 3-$CH_3$ | 1 | |
| I-393 | 〃 | | 〃 | 〃 | 〃 | $CH_3$ | H | 4-$CH_3$ | 〃 | |
| I-394 | 〃 | | 〃 | 〃 | 〃 | $CH_3$ | H | 5-$CH_3$ | 〃 | |
| I-395 | 〃 | | 〃 | 〃 | 〃 | $C_2H_5$ | $C_2H_5$ | | 0 | |
| I-396 | 〃 | | 〃 | 〃 | $OCH_3$ | Cl | Cl | | 〃 | |
| I-397 | 〃 | | 〃 | 〃 | 〃 | Br | Br | | 〃 | |
| I-398 | 〃 | | 〃 | 〃 | 〃 | $CH_3$ | Cl | | 〃 | |
| I-399 | 〃 | | 〃 | 〃 | 〃 | $CH_3$ | $CH_3$ | | 〃 | |
| I-400 | 〃 | | 〃 | 〃 | 〃 | $CH_3$ | H | 3-$CH_3$ | 1 | |
| I-401 | 〃 | | 〃 | 〃 | 〃 | $CH_3$ | H | 4-$CH_3$ | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-402 | =O | | 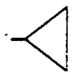 | H | $OCH_3$ | $CH_3$ | H | 5-$CH_3$ | 1 | |
| I-403 | 〃 | | 〃 | 〃 | 〃 | $C_2H_5$ | $C_2H_5$ | | 0 | |
| I-404 | 〃 | | 〃 | 〃 | $SCH_3$ | Cl | Cl | | 〃 | |
| I-405 | 〃 | | 〃 | 〃 | 〃 | Br | Br | | 〃 | |
| I-406 | 〃 | | 〃 | 〃 | 〃 | $CH_3$ | Cl | | 〃 | |
| I-407 | 〃 | | 〃 | 〃 | 〃 | $CH_3$ | $CH_3$ | | 〃 | |
| I-408 | 〃 | | 〃 | 〃 | 〃 | $CH_3$ | H | 3-$CH_3$ | 1 | |
| I-409 | 〃 | | 〃 | 〃 | 〃 | $CH_3$ | H | 4-$CH_3$ | 〃 | |
| I-410 | 〃 | | 〃 | 〃 | 〃 | $CH_3$ | H | 5-$CH_3$ | 〃 | |
| I-411 | 〃 | | 〃 | 〃 | 〃 | $C_2H_5$ | $C_2H_5$ | | 0 | |
| I-412 | 〃 | | 〃 | 〃 | $SO_2CH_3$ | Cl | Cl | | 〃 | |
| I-413 | 〃 | | 〃 | 〃 | 〃 | Br | Br | | 〃 | |
| I-414 | 〃 | | 〃 | 〃 | 〃 | $CH_3$ | Cl | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-415 | =O | | 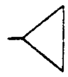 | H | $SO_2CH_3$ | $CH_3$ | $CH_3$ | | 0 | |
| I-416 | 〃 | | 〃 | 〃 | 〃 | $CH_3$ | H | 3-$CH_3$ | 1 | |
| I-417 | 〃 | | 〃 | 〃 | 〃 | $CH_3$ | H | 4-$CH_3$ | 〃 | |
| I-418 | 〃 | | 〃 | 〃 | 〃 | $CH_3$ | H | 5-$CH_3$ | 〃 | |
| I-419 | 〃 | | 〃 | 〃 | 〃 | $C_2H_5$ | $C_2H_5$ | | 0 | |
| I-420 | 〃 | | 〃 | 〃 | $NHCH_3$ | Cl | Cl | | 〃 | |
| I-421 | 〃 | | 〃 | 〃 | 〃 | Br | Br | | 〃 | |
| I-422 | 〃 | | 〃 | 〃 | 〃 | $CH_3$ | Cl | | 〃 | |
| I-423 | 〃 | | 〃 | 〃 | 〃 | $CH_3$ | $CH_3$ | | 〃 | |
| I-424 | 〃 | | 〃 | 〃 | 〃 | $CH_3$ | H | 3-$CH_3$ | 1 | |
| I-425 | 〃 | | 〃 | 〃 | 〃 | $CH_3$ | H | 4-$CH_3$ | 〃 | |
| I-426 | 〃 | | 〃 | 〃 | 〃 | $CH_3$ | H | 5-$CH_3$ | 〃 | |
| I-427 | 〃 | | 〃 | 〃 | 〃 | $C_2H_5$ | $C_2H_5$ | | 0 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-428 | =0 | | 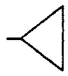 | H | $N(CH_3)_2$ | Cl | Cl | | 0 | |
| I-429 | 〃 | | 〃 | 〃 | 〃 | Br | Br | | 〃 | |
| I-430 | 〃 | | 〃 | 〃 | 〃 | $CH_3$ | Cl | | 〃 | |
| I-431 | 〃 | | 〃 | 〃 | 〃 | $CH_3$ | $CH_3$ | | 〃 | |
| I-432 | 〃 | | 〃 | 〃 | 〃 | $CH_3$ | H | 3-$CH_3$ | 1 | |
| I-433 | 〃 | | 〃 | 〃 | 〃 | $CH_3$ | H | 4-$CH_3$ | 〃 | |
| I-434 | 〃 | | 〃 | 〃 | 〃 | $CH_3$ | H | 5-$CH_3$ | 〃 | |
| I-435 | 〃 | | 〃 | 〃 | 〃 | $C_2H_5$ | $C_2H_5$ | | 0 | |
| I-436 | 〃 | | $CF_3$ | 〃 | Cl | $CF_3$ | H | | 〃 | [87-9°C] |
| I-437 | 〃 | | 〃 | 〃 | $OCH_3$ | 〃 | 〃 | | 〃 | [76-8°C] |
| I-438 | 〃 | | 〃 | 〃 | $SCH_3$ | 〃 | 〃 | | 〃 | $n_D^{24.0}$ =1.5196 |
| I-439 | 〃 | | 〃 | 〃 | $NHCH_3$ | 〃 | 〃 | | 〃 | |
| I-440 | 〃 | | 〃 | 〃 | $N(CH_3)_2$ | 〃 | 〃 | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-441 | =0 | | 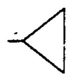 | H | OH | $CF_3$ | H | | 0 | |
| I-442 | 〃 | | 〃 | 〃 | Cl | $NO_2$ | 〃 | | 〃 | |
| I-443 | 〃 | | 〃 | 〃 | $OCH_3$ | 〃 | 〃 | | 〃 | [108-110℃] |
| I-444 | 〃 | | $CF_3$ | 〃 | $SCH_3$ | $NO_2$ | 〃 | | 0 | [116−8 ℃] |
| I-445 | 〃 | | 〃 | 〃 | $NHCH_3$ | 〃 | 〃 | | 〃 | |
| I-446 | 〃 | | 〃 | 〃 | $N(CH_3)_2$ | 〃 | 〃 | | 〃 | |
| I-447 | 〃 | | 〃 | 〃 | OH | 〃 | 〃 | | 〃 | |
| I-448 | 〃 | | 〃 | $CH_3$ | Cl | Cl | 〃 | | 〃 | [77-9℃] |
| I-449 | 〃 | | 〃 | 〃 | $OCH_3$ | 〃 | 〃 | | 〃 | $n_D^{24.6}$ =1.5320 |
| I-450 | 〃 | | 〃 | 〃 | $SCH_3$ | 〃 | 〃 | | 〃 | $n_D^{24.5}$ =1.5637 |
| I-451 | 〃 | | 〃 | 〃 | $NHCH_3$ | 〃 | 〃 | | 〃 | |
| I-452 | 〃 | | 〃 | 〃 | $N(CH_3)_2$ | 〃 | 〃 | | 〃 | |
| I-453 | 〃 | | 〃 | 〃 | OH | 〃 | 〃 | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-454 | =0 | | $CF_3$ | nPr | Cl | Cl | H | | 0 | [70-4℃] |
| I-455 | 〃 | | 〃 | 〃 | $OCH_3$ | 〃 | 〃 | | 〃 | |
| I-456 | 〃 | | 〃 | 〃 | $SCH_3$ | 〃 | 〃 | | 〃 | $n_D^{22.9}$ =1.5581 |
| I-457 | 〃 | | 〃 | 〃 | $NHCH_3$ | 〃 | 〃 | | 〃 | |
| I-458 | 〃 | | 〃 | 〃 | $N(CH_3)_2$ | 〃 | 〃 | | 〃 | |
| I-459 | 〃 | | 〃 | 〃 | OH | 〃 | 〃 | | 〃 | |
| I-460 | 〃 | | 〃 | isoPr | Cl | 〃 | 〃 | | 〃 | [83-5] |
| I-461 | 〃 | | 〃 | 〃 | $OCH_3$ | 〃 | 〃 | | 〃 | |
| I-462 | 〃 | | 〃 | 〃 | $SCH_3$ | 〃 | 〃 | | 〃 | $n_D^{22.9}$ =1.5587 |
| I-463 | 〃 | | 〃 | 〃 | $NHCH_3$ | 〃 | 〃 | | 〃 | |
| I-464 | 〃 | | 〃 | 〃 | $N(CH_3)_2$ | 〃 | 〃 | | 〃 | |
| I-465 | 〃 | | 〃 | 〃 | OH | 〃 | 〃 | | 〃 | |
| I-466 | 〃 | | 〃 | 〃 | $SCH_3$ | F | F | | 〃 | $n_D^{23.3}$ =1.5393 |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-467 | =0 | | $CF_3$ | nPr | $NHCH_3$ | F | F | | 0 | [123-124 ] |
| I-468 | ″ | | isoPr | ″ | $N(CH_3)_2$ | Cl | H | | ″ | [ 85-87 ] |
| I-469 | ″ | | ″ | ″ | S-isoPr | ″ | ″ | | ″ | $n_D^{23.2}$ =1.5783 |
| I-470 | ″ | | $CF_3$ | H | Cl | I | H | | ″ | $n_D^{22.9}$ =1.5880 |
| I-471 | ″ | | ″ | ″ | $SCH_3$ | ″ | ″ | | ″ | $n_D^{23.8}$ =1.6112 |
| I-472 | ″ | | ″ | ″ | $NHCH_3$ | ″ | ″ | | ″ | [95-97 ] |
| I-473 | ″ | | isoPr | ″ | $SCH_2COCH_3$ (C=O) | Cl | ″ | | ″ | $n_D^{25.5}$ =1.5811 |
| I-474 | ″ | | isoBu | ″ | Cl | ″ | ″ | | ″ | $n_D^{22.2}$ =1.5705 |
| I-475 | ″ | | ″ | ″ | $OCH_3$ | ″ | ″ | | ″ | $n_D^{22.2}$ =1.5549 |
| I-476 | ″ | | ″ | ″ | $SCH_3$ | ″ | ″ | | ″ | $n_D^{22.2}$ =1.5763 |
| I-477 | ″ | | isoPr | ″ | 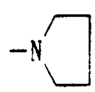 | ″ | ″ | | ″ | $n_D^{22.8}$ =1.5958 |
| I-478 | ″ | | ″ | ″ | N-isoPr | ″ | ″ | | ″ | $n_D^{23.7}$ =1.5733 |
| I-479 | ″ | | $CF_3$ | ″ | Cl | ″ | ″ | 4-$OCH_3$ | 1 | [77-79 ] |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-480 | =O | | $CF_3$ | H | $SCH_3$ | Cl | H | 4-$OCH_3$ | 1 | $n_D^{23.8}$ =1.5802 |
| I-481 | 〃 | | 〃 | 〃 | $NHCH_3$ | 〃 | 〃 | 〃 | 1 | [106-108] |
| I-482 | 〃 | | isoPr | 〃 | $NH_2$ | 〃 | 〃 | | 0 | [150-152] |
| I-483 | 〃 | | 〃 | 〃 | $NHC_2H_5$ | Cl | 〃 | | 〃 | |
| I-484 | 〃 | | secAm | 〃 | Cl | 〃 | 〃 | | 〃 | $n_D^{22.5}$ =1.5578 |
| I-485 | 〃 | | 〃 | 〃 | $SCH_3$ | 〃 | 〃 | | 〃 | $n_D^{22.6}$ =1.5756 |
| I-486 | 〃 | | 〃 | 〃 | $OCH_3$ | 〃 | 〃 | | 〃 | $n_D^{22.7}$ =1.5445 |
| I-487 | 〃 | | $CHEt_2$ | 〃 | Cl | 〃 | 〃 | | 〃 | $n_D^{22.8}$ =1.5606 |
| I-488 | 〃 | | 〃 | 〃 | $SCH_3$ | 〃 | 〃 | | 〃 | $n_D^{22.8}$ =1.5803 |
| I-489 | 〃 | | 〃 | 〃 | $OCH_3$ | 〃 | 〃 | | 〃 | $n_D^{22.9}$ =1.5507 |
| I-490 | 〃 | | isoPr | 〃 | F | 〃 | 〃 | | 〃 | $n_D^{22.4}$ =1.5582 |
| I-491 | 〃 | | 〃 | 〃 | $SO_2CH_3$ | 〃 | 〃 | | 〃 | $n_D^{24.5}$ =1.5734 |
| I-492 | 〃 | | 〃 | $\overset{\underset{\|}{O}}{C}OEt$ | H | 〃 | 〃 | | 〃 | $n_D^{23.1}$ =1.5486 |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-493 | =O | | isoPr | O<br>‖<br>COH | H | Cl | H | | 0 | [102-103 ] |
| I-494 | 〃 | | 〃 | O<br>‖<br>$CNMe_2$ | 〃 | 〃 | 〃 | | 〃 | [96-98 ] |
| I-495 | 〃 | | 〃 | O<br>‖<br>$CNH_2$ | 〃 | 〃 | 〃 | | 〃 | [83-86 ] |
| I-496 | 〃 | | $C_2H_5$ | H | Cl | 〃 | 〃 | | 〃 | $n_D^{22.8}$ =1.5874 |
| I-497 | 〃 | | 〃 | 〃 | $SCH_3$ | 〃 | 〃 | | 〃 | [66-68 ] |
| I-498 | 〃 | | $CF_3$ | $CH=CH_2$ | $NH_2$ | 〃 | 〃 | | 〃 | [152-153 ] |
| I-499 | 〃 | | isoPr | $SO_2CH_3$ | H | 〃 | 〃 | | 〃 | [105-106 ] |
| I-500 | 〃 | | 〃 | $SCH_3$ | 〃 | 〃 | 〃 | | 〃 | $n_D^{22.1}$ =1.6034 |
| I-501 | 〃 | | 〃 | I | Cl | 〃 | 〃 | | 〃 | [127-128 ] |
| I-502 | 〃 | | $CF_3$ | $CH_2CH_2CH_2$<br>\|<br>$H_3CCOO$ | $OCH_3$ | 〃 | Cl | | 〃 | [125-127 ] |
| I-503 | 〃 | | OH | isoPr | H | 〃 | H | | 〃 | [124-126 ] |
| I-504 | 〃 | | Cl | 〃 | 〃 | 〃 | 〃 | | 〃 | [96-98 ] |
| I-505 | 〃 | | $NHCH_3$ | 〃 | 〃 | 〃 | 〃 | | 〃 | [125-126 ] |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-506 | =0 | | $OCH_3$ | isoPr | H | Cl | H | | 0 | $n_D^{20.3}$ =1.5670 |
| I-507 | 〃 | | $SCH_3$ | 〃 | 〃 | 〃 | 〃 | | 〃 | $n_D^{20.1}$ =1.6028 |
| I-508 | 〃 | | $CF_3$ | CH=$CH_2$ | $NHCH_3$ | F | F | | 〃 | [96-98 ] |
| I-509 | 〃 | | 〃 | 〃 | $OCH_3$ | 〃 | 〃 | | 〃 | [131-133 ] |
| I-510 | 〃 | | 〃 | 〃 | OH | $CH_3$ | $CH_3$ | 4-$CH_3$ | 1 | [238-240 ] |
| I-511 | 〃 | | Cl | isoPr | $OCH_3$ | Cl | H | | 0 | [120-121 ] |
| I-512 | 〃 | | $OCH_3$ | 〃 | 〃 | 〃 | 〃 | | 〃 | $n_D^{22.3}$ =1.5569 |
| I-513 | 〃 | | 〃 | 〃 | $SCH_3$ | 〃 | 〃 | | 〃 | [66-67 ] |
| I-514 | 〃 | | OH | 〃 | Cl | 〃 | 〃 | | 〃 | [184-185 ] |
| I-515 | 〃 | | $SCH_3$ | 〃 | 〃 | 〃 | 〃 | | 〃 | [75-77 ] |
| I-516 | 〃 | | 〃 | 〃 | OH | 〃 | 〃 | | 〃 | [189.5-190.5 |
| I-517 | 〃 | | 〃 | 〃 | $SCH_3$ | 〃 | 〃 | | 〃 | [89-90 ] |
| I-518 | 〃 | | $NHCH_3$ | 〃 | Cl | 〃 | 〃 | | 〃 | [148-149 ] |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-519 | =0 | | $NHCH_3$ | isoPr | $SCH_3$ | Cl | H | | 0 | [108-109] |
| I-520 | 〃 | | Cl | isoPr | Cl | 〃 | H | | 0 | [127-128] |
| I-521 | 〃 | | $CF_3$ | $CH_2CH_2CH_2$<br>\|<br>$H_3CCOO$ | SH | 〃 | F | | 〃 | [108-110] |
| I-522 | 〃 | | $CH_3$ | $CH_2CH_2Cl$ | 〃 | 〃 | H | | 〃 | [122-124] |
| I-523 | 〃 | | $CF_3$ | $CH_2CH_2OH$ | OH | 〃 | Cl | 4-Cl | 1 | [135-140] dec. |
| I-524 | 〃 | | 〃 | 〃 | 〃 | $CH_3$ | $CH_3$ | 4-$CH_3$ | 〃 | [220-221] |
| I-525 | 〃 | | 〃 | $CH_2CH_2Cl$ | 〃 | 〃 | 〃 | 〃 | 〃 | [198-199] |
| I-526 | 〃 | | H | $CH=CH_2$ | Cl | Cl | H | | 0 | 170° dec. |
| I-527 | 〃 | | 〃 | $CH_2CH_2Cl$ | 〃 | 〃 | 〃 | | 〃 | [71-3] |
| I-528 | 〃 | | 〃 | 〃 | $SCH_3$ | 〃 | 〃 | | 〃 | $n_D^{23.2}$=1.5678 |
| I-529 | 〃 | | 〃 | H | $OCF_2H$ | 〃 | 〃 | | 〃 | $n_D^{23.4}$=1.4985 |
| I-530 | 〃 | | isoPr | C≡CTMS *1 | Cl | 〃 | 〃 | | 〃 | [78-80] |
| I-531 | 〃 | | $PO(OEt)_2$ | H | 〃 | 〃 | 〃 | | 〃 | |

*1 TMS=Trimethylsilyl

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-532 | =O | | $PO(OEt)_2$ | H | $SCH_3$ | Cl | H | | 0 | |
| I-533 | 〃 | | 〃 | 〃 | $NHCH_3$ | 〃 | 〃 | | 〃 | |
| I-534 | 〃 | | COOEt | 〃 | Cl | 〃 | 〃 | | 〃 | |
| I-535 | 〃 | | 〃 | 〃 | $SCH_3$ | 〃 | 〃 | | 〃 | |
| I-536 | 〃 | | 〃 | 〃 | $NHCH_3$ | 〃 | 〃 | | 〃 | |
| I-537 | 〃 | | CN | 〃 | Cl | 〃 | 〃 | | 〃 | |
| I-538 | 〃 | | 〃 | 〃 | $SCH_3$ | 〃 | 〃 | | 〃 | |
| I-539 | 〃 | | 〃 | 〃 | $NHCH_3$ | 〃 | 〃 | | 〃 | |
| I-540 | 〃 | | $CF_3$ | 〃 | Cl | $-CON(CH_3)_2$ | 〃 | | 〃 | |
| I-541 | 〃 | | 〃 | 〃 | 〃 | $-CH=CH_2$ | 〃 | | 〃 | |
| I-542 | 〃 | | 〃 | 〃 | 〃 | $-C\equiv CH$ | 〃 | | 〃 | |
| I-543 | 〃 | | 〃 | 〃 | 〃 | 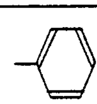 | 〃 | | 〃 | |
| I-544 | 〃 | | 〃 | 〃 | 〃 | 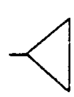 | 〃 | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-545 | =0 | | $CF_3$ | H | Cl | $-SCH_3$ | H | | 0 | |
| I-546 | 〃 | | 〃 | 〃 | 〃 | $-SO_2CH_3$ | 〃 | | 〃 | |
| I-547 | 〃 | | 〃 | 〃 | 〃 | 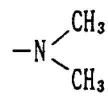 | 〃 | | 〃 | |
| I-548 | 〃 | | 〃 | 〃 | 〃 | Cl | 〃 | 4-Cl | 1 | |
| I-549 | 〃 | | 〃 | 〃 | 〃 | 〃 | 〃 | $4-CH_3$ | 〃 | |
| I-550 | 〃 | | 〃 | 〃 | 〃 | 〃 | 〃 | 4-F | 〃 | |
| I-551 | 〃 | | 〃 | -C≡C | 〃 | 〃 | 〃 | | 0 | |
| I-552 | 〃 | | 〃 | Cl | 〃 | 〃 | 〃 | | 〃 | |
| I-553 | 〃 | | 〃 | 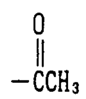 | 〃 | 〃 | 〃 | | 〃 | |
| I-554 | 〃 | | 〃 | 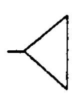 | 〃 | 〃 | 〃 | | 〃 | |
| I-555 | 〃 | | 〃 | 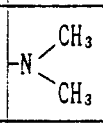 | 〃 | 〃 | 〃 | | 〃 | |
| I-556 | 〃 | | 〃 | CN | 〃 | 〃 | 〃 | | 〃 | |
| I-557 | 〃 | | 〃 | 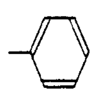 | 〃 | 〃 | 〃 | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-558 | =0 | | $CF_3$ | 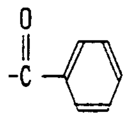 | Cl | Cl | H | | 0 | |
| I-559 | 〃 | | 〃 | $-OCH_3$ | 〃 | 〃 | 〃 | | 〃 | |
| I-560 | 〃 | | 〃 | H | 〃 | isoPr | 〃 | | 〃 | |
| I-561 | 〃 | | 〃 | 〃 | 〃 | -COOH | 〃 | | 〃 | |
| I-562 | 〃 | | 〃 | 〃 | 〃 | -COOEt | 〃 | | 〃 | |
| I-563 | 〃 | | 〃 | 〃 | 〃 | -CN | 〃 | | 〃 | |
| I-564 | 〃 | | 〃 | 〃 | O<br>‖<br>$P(OH)_2$ | Cl | 〃 | | 〃 | |
| I-565 | 〃 | | 〃 | 〃 | $CH=CH_2$ | 〃 | 〃 | | 〃 | |
| I-566 | 〃 | | $C_2F_5$ | 〃 | Cl | 〃 | 〃 | | 〃 | $n_D^{22.3}$ 1.5088 |
| I-567 | 〃 | | $C_3F_7$ | 〃 | 〃 | 〃 | 〃 | | 〃 | $n_D^{22.5}$ 1.4898 |
| I-568 | 〃 | | $C_2F_5$ | 〃 | $SCH_3$ | 〃 | 〃 | | 〃 | $n_D^{22.4}$ 1.5379 |
| I-569 | 〃 | | $C_3F_7$ | 〃 | 〃 | 〃 | 〃 | | 〃 | $n_D^{22.8}$ 1.5197 |
| I-570 | 〃 | | isoPr | 〃 | Cl | $CH_3$ | 〃 | 3-$CH_3$-<br>4-$OCH_3$ | 〃 | $n_D^{26.8}$ 1.5765 |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| I-571 | =O | | isoPr | H | $SCH_3$ | $CH_3$ | H | 3-$CH_3$-4-$OCH_3$ | 0 | $n_D^{26.5}$ 1.5922 |
| I-572 | 〃 | | 〃 | 〃 | Cl | 〃 | 〃 | 4-$OCH_3$-5-$CH_3$ | 〃 | $n_D^{26.5}$ 1.5739 |
| I-573 | 〃 | | 〃 | 〃 | $SCH_3$ | 〃 | 〃 | 〃 | 〃 | [126-7 ] |
| I-574 | 〃 | | 〃 | 〃 | Cl | $OCH_3$ | 〃 | 5-Br | 1 | $n_D^{26.2}$ 1.5883 |
| I-575 | 〃 | | 〃 | 〃 | $SCH_3$ | 〃 | 〃 | 〃 | 〃 | $n_D^{26.5}$ 1.5978 |
| I-576 | 〃 | | 〃 | 〃 | Cl | 〃 | 〃 | 5-F | 〃 | $n_D^{26.0}$ 1.5441 |
| I-577 | 〃 | | 〃 | 〃 | $SCH_3$ | 〃 | 〃 | 〃 | 〃 | $n_D^{26.5}$ 1.5692 |
| I-578 | 〃 | | $CF_3$ | 〃 | $PO(OEt)_2$ | Cl | 〃 | | 0 | $n_D^{24.5}$ 1.5092 |
| I-579 | 〃 | | 〃 | 〃 | 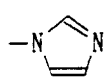 | 〃 | 〃 | | 〃 | [98−9 ] |
| I-580 | 〃 | | 〃 | 〃 | 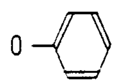 | 〃 | 〃 | | 〃 | $n_D^{24.5}$ 1.5612 |
| I-581 | 〃 | | 〃 | 〃 | 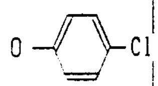 | 〃 | 〃 | | 〃 | $n_D^{24.5}$ 1.5650 |
| I-582 | 〃 | | 〃 | 〃 | $NHNHC(=O)OEt$ | 〃 | 〃 | | 〃 | [174-5 ] |
| I-583 | 〃 | | 〃 | 〃 | 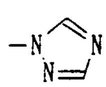 | 〃 | 〃 | | 〃 | [99-100] |
| I-584 | 〃 | | isoPr | 〃 | $OCF_2H$ | 〃 | 〃 | | 〃 | $n_D^{23.5}$ 1.5304 |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| II-1 | H | OH | $CH_3$ | H | OH | Cl | Cl | | 0 | |
| II-2 | 〃 | 〃 | 〃 | 〃 | 〃 | Br | Br | | 〃 | |
| II-3 | 〃 | 〃 | 〃 | 〃 | 〃 | $CH_3$ | Cl | | 〃 | |
| II-4 | 〃 | 〃 | 〃 | 〃 | 〃 | $CH_3$ | $CH_3$ | | 〃 | |
| II-5 | 〃 | 〃 | 〃 | 〃 | 〃 | $CH_3$ | H | 3-$CH_3$ | 1 | |
| II-6 | 〃 | 〃 | 〃 | 〃 | 〃 | $CH_3$ | H | 4-$CH_3$ | 〃 | |
| II-7 | 〃 | 〃 | 〃 | 〃 | 〃 | $CH_3$ | H | 5-$CH_3$ | 〃 | |
| II-8 | H | 〃 | 〃 | 〃 | 〃 | $C_2H_5$ | $C_2H_5$ | | 0 | |
| II-9 | 〃 | $OC_2H_5$ | 〃 | 〃 | Cl | Cl | Cl | | 〃 | |
| II-10 | 〃 | 〃 | 〃 | 〃 | 〃 | Br | Br | | 〃 | |
| II-11 | 〃 | 〃 | 〃 | 〃 | 〃 | $CH_3$ | Cl | | 〃 | |
| II-12 | 〃 | 〃 | 〃 | 〃 | 〃 | $CH_3$ | $CH_3$ | | 〃 | |
| II-13 | 〃 | 〃 | 〃 | 〃 | 〃 | $CH_3$ | H | 3-$CH_3$ | 1 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| II-14 | H | $OC_2H_5$ | $CH_3$ | H | Cl | $CH_3$ | H | 4-$CH_3$ | 1 | |
| II-15 | 〃 | 〃 | 〃 | 〃 | 〃 | $CH_3$ | H | 5-$CH_3$ | 〃 | |
| II-16 | 〃 | 〃 | 〃 | 〃 | 〃 | $C_2H_5$ | $C_2H_5$ | | 0 | |
| II-17 | 〃 | Cl | 〃 | 〃 | $OCH_3$ | Cl | Cl | | 〃 | |
| II-18 | 〃 | 〃 | 〃 | 〃 | 〃 | Br | Br | | 〃 | |
| II-19 | 〃 | 〃 | 〃 | 〃 | 〃 | $CH_3$ | Cl | | 〃 | |
| II-20 | 〃 | 〃 | 〃 | 〃 | 〃 | $CH_3$ | $CH_3$ | | 〃 | |
| II-21 | 〃 | 〃 | 〃 | 〃 | 〃 | $CH_3$ | H | 3-$CH_3$ | 1 | |
| II-22 | 〃 | 〃 | 〃 | 〃 | 〃 | $CH_3$ | H | 4-$CH_3$ | 〃 | |
| II-23 | 〃 | 〃 | 〃 | 〃 | 〃 | $CH_3$ | H | 5-$CH_3$ | 〃 | |
| II-24 | 〃 | 〃 | 〃 | 〃 | 〃 | $C_2H_5$ | $C_2H_5$ | | 0 | |
| II-25 | =S | | 〃 | 〃 | $SCH_3$ | Cl | Cl | | 〃 | |
| II-26 | 〃 | | 〃 | 〃 | 〃 | Br | Br | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| II-27 | =S | | $CH_3$ | H | $SCH_3$ | $CH_3$ | Cl | | 0 | |
| II-28 | 〃 | | 〃 | 〃 | 〃 | $CH_3$ | $CH_3$ | | 〃 | |
| II-29 | 〃 | | 〃 | 〃 | 〃 | $CH_3$ | H | 3-$CH_3$ | 1 | |
| II-30 | 〃 | | 〃 | 〃 | 〃 | $CH_3$ | H | 4-$CH_3$ | 〃 | |
| II-31 | 〃 | | 〃 | 〃 | 〃 | $CH_3$ | H | 5-$CH_3$ | 〃 | |
| II-32 | 〃 | | 〃 | 〃 | 〃 | $C_2H_5$ | $C_2H_5$ | | 0 | |
| II-33 | H | $SC_2H_5$ | 〃 | 〃 | $SO_2CH_3$ | Cl | Cl | | 〃 | |
| II-34 | 〃 | 〃 | 〃 | 〃 | 〃 | Br | Br | | 〃 | |
| II-35 | 〃 | 〃 | 〃 | 〃 | 〃 | $CH_3$ | Cl | | 〃 | |
| II-36 | 〃 | 〃 | 〃 | 〃 | 〃 | $CH_3$ | $CH_3$ | | 〃 | |
| II-37 | 〃 | 〃 | 〃 | 〃 | 〃 | $CH_3$ | H | 3-$CH_3$ | 1 | |
| II-38 | 〃 | 〃 | 〃 | 〃 | 〃 | $CH_3$ | H | 4-$CH_3$ | 〃 | |
| II-39 | 〃 | 〃 | 〃 | 〃 | 〃 | $CH_3$ | H | 5-$CH_3$ | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| II-40 | H | $SC_2H_5$ | $CH_3$ | H | $SO_2CH_3$ | $C_2H_5$ | $C_2H_5$ | | 0 | |
| II-41 | nBu | OH | 〃 | 〃 | $NHCH_3$ | Cl | Cl | | 〃 | |
| II-42 | 〃 | 〃 | 〃 | 〃 | 〃 | Br | Br | | 〃 | |
| II-43 | 〃 | 〃 | 〃 | 〃 | 〃 | $CH_3$ | Cl | | 〃 | |
| II-44 | 〃 | 〃 | 〃 | 〃 | 〃 | $CH_3$ | $CH_3$ | | 〃 | |
| II-45 | 〃 | 〃 | 〃 | 〃 | 〃 | $CH_3$ | H | 3-$CH_3$ | 1 | |
| II-46 | 〃 | 〃 | 〃 | 〃 | 〃 | $CH_3$ | H | 4-$CH_3$ | 〃 | |
| II-47 | 〃 | 〃 | 〃 | 〃 | 〃 | $CH_3$ | H | 5-$CH_3$ | 〃 | |
| II-48 | 〃 | 〃 | 〃 | 〃 | 〃 | $C_2H_5$ | $C_2H_5$ | | 0 | |
| II-49 | H | $CH_3$ | $CF_3$ | 〃 | $SCH_3$ | Cl | Cl | | 〃 | [64-66] |
| II-50 | 〃 | $C_2H_5$ | 〃 | 〃 | 〃 | 〃 | 〃 | | 〃 | $n_D^{23.8}$ =1.5500 |
| II-51 | 〃 | isoPr | 〃 | 〃 | 〃 | 〃 | H | | 〃 | $n_D^{23.3}$ =1.5343 |
| II-52 | $CH_3$ | OH | 〃 | 〃 | 〃 | 〃 | 〃 | | 〃 | $n_D^{23.2}$ =1.5516 |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| II-53 | O<br>‖<br>=NNHCOEt | | isoPr | H | $SCH_3$ | Cl | H | | 0 | 〔85-87〕 |
| II-54 | H | C≡N | 〃 | 〃 | 〃 | 〃 | 〃 | | 〃 | $n_D^{21.6}$ =1.5760 |
| II-55 | OEt<br>\|<br>$=NCH_2P=O$<br>\|<br>OEt | | 〃 | 〃 | 〃 | 〃 | 〃 | | 〃 | $n_D^{21.8}$ =1.5496 |
| II-56 | 〃<br>(異性体) | | 〃 | 〃 | 〃 | 〃 | 〃 | | 〃 | $n_D^{22.1}$ =1.5487 |
| II-57 | OH<br>\|<br>$=NCH_2P=O$<br>\|<br>OH | | 〃 | 〃 | 〃 | 〃 | 〃 | | 〃 | amorphoas |
| II-58 | $-OCH_2-$ | | 〃 | 〃 | 〃 | 〃 | 〃 | | 〃 | $n_D^{22.4}$ =1.5759 |
| II-59 | H | OH | $CF_3$ | 〃 | 〃 | 〃 | 〃 | | 〃 | 〔86-88〕 |
| II-60 | $OCH_3$ | $CH_3$ | 〃 | 〃 | 〃 | 〃 | 〃 | | 〃 | $n_D^{21.7}$ =1.5662 |
| II-61 | H | OH | $NHCH_3$ | isoPr | $OCH_3$ | Cl | 〃 | | 〃 | 〔107-108〕 |
| II-62 | $=NCH_3$ | | $CF_3$ | $C_2H_4Br$ | $NHCH_3$ | F | F | | 〃 | 〔128-130〕 |
| II-63 | 〃 | | $NHCH_3$ | isoPr | Cl | Cl | H | | 〃 | 〔206-208〕 |
| II-64 | $=CH_2$ | | isoPr | H | $SCH_3$ | 〃 | 〃 | | 〃 | $n_D^{23.3}$ =1.5889 |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| II-65 | =CHCN | | isoPr | H | $SCH_3$ | Cl | H | | 0 | $n_D^{23.5}$ =1.5911 |
| II-66 | =NOH | | 〃 | 〃 | 〃 | 〃 | 〃 | | 0 | [153-156 ] |
| II-67 | =NOEt | | $CH_3$ | $C_2H_4CHMe(OMe)$ | $OCH_3$ | I | 〃 | | 〃 | $n_D^{25}$ =1.5881 |
| II-68 | H | OH | $CF_3$ | 〃 | 〃 | Cl | 〃 | | 0 | [76-8] |
| II-69 | 〃 | Cl | 〃 | 〃 | 〃 | 〃 | 〃 | | 〃 | $n_D^{22.2}$ =1.5215 |
| II-70 | =NO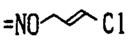 | | $CF_3$ | 〃 | 〃 | 〃 | Cl | | 0 | $n_D^{25.5}$ =1.5390 |
| II-71 | =NEt | | isoPr | H | $SCH_3$ | Cl | H | | 0 | |
| II-72 | =NMe | | 〃 | 〃 | 〃 | 〃 | 〃 | | 〃 | |
| II-73 | =NCHCOOEt (with $CH_3$ on CH) | | 〃 | 〃 | 〃 | 〃 | 〃 | | 〃 | |
| II-74 | $=NCH_2COOEt$ | | 〃 | 〃 | 〃 | 〃 | 〃 | | 〃 | |
| II-75 | =NOEt | | 〃 | 〃 | 〃 | 〃 | 〃 | | 〃 | $n_D^{22.6}$ 1.5848 |
| II-76 | =NO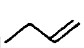 | | 〃 | 〃 | 〃 | 〃 | 〃 | | 〃 | |
| II-77 | $=NOCH_2C{\equiv}CH$ | | 〃 | 〃 | 〃 | 〃 | 〃 | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| Ⅱ-78 | $=NOCH_2Ph$ | | isoPr | H | $SCH_3$ | Cl | H | | 0 | |
| Ⅱ-79 | =NOMe | | 〃 | 〃 | 〃 | 〃 | 〃 | | 〃 | [73−74] |
| Ⅱ-80 | 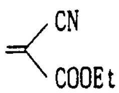 | | 〃 | 〃 | 〃 | 〃 | 〃 | | 〃 | |
| Ⅱ-81 | 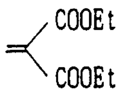 | | 〃 | 〃 | 〃 | 〃 | 〃 | | 〃 | |
| Ⅱ-82 | 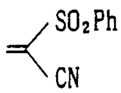 | | 〃 | 〃 | 〃 | 〃 | 〃 | | 〃 | |
| Ⅱ-83 | 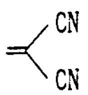 | | 〃 | 〃 | 〃 | 〃 | 〃 | | 〃 | |
| Ⅱ-84 | 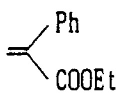 | | 〃 | 〃 | 〃 | 〃 | 〃 | | 〃 | |
| Ⅱ-85 | 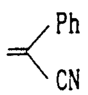 | | 〃 | 〃 | 〃 | 〃 | 〃 | | 〃 | |
| Ⅱ-86 | 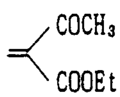 | | 〃 | 〃 | 〃 | 〃 | 〃 | | 〃 | |
| Ⅱ-87 | 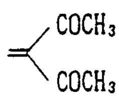 | | 〃 | 〃 | 〃 | 〃 | 〃 | | 〃 | |
| Ⅱ-88 | 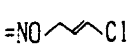 | | 〃 | 〃 | 〃 | 〃 | 〃 | | 〃 | |
| Ⅱ-89 | $=NOCH_2OCH_3$ | | 〃 | 〃 | 〃 | 〃 | 〃 | | 〃 | |
| Ⅱ-90 | $=NOCH_2SCH_3$ | | 〃 | 〃 | 〃 | 〃 | 〃 | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| II-91 | $=CHNO_2$ | | isoPr | H | $SCH_3$ | Cl | H | | 0 | |
| II-92 | 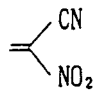 | | ″ | ″ | ″ | ″ | ″ | | ″ | |
| II-93 | 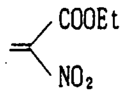 | | ″ | ″ | ″ | ″ | ″ | | ″ | |
| II-94 | 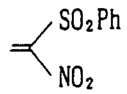 | | ″ | ″ | ″ | ″ | ″ | | ″ | |
| II-95 | $=CHCF_3$ | | ″ | ″ | ″ | ″ | ″ | | ″ | |
| II-96 | 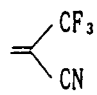 | | ″ | ″ | ″ | ″ | ″ | | ″ | |
| II-97 | 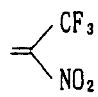 | | ″ | ″ | ″ | ″ | ″ | | ″ | |
| II-98 | 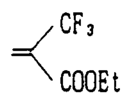 | | ″ | ″ | ″ | ″ | ″ | | ″ | |
| II-99 | =CHCOOEt | | ″ | ″ | ″ | ″ | ″ | | ″ | $n_D^{22.8}$ 1.5773 |
| II-100 | $=CHCH_2COOEt$ | | ″ | ″ | ″ | ″ | ″ | | ″ | |
| II-101 | SMe | H | ″ | ″ | ″ | ″ | ″ | | ″ | |
| II-102 | H<br>NMe | H | ″ | ″ | ″ | ″ | ″ | | ″ | |
| II-103 | 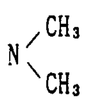 | H | ″ | ″ | ″ | ″ | ″ | | ″ | |

| No. | $R^1$ | $R^2$ | $R^3$ | $R^4$ | $R^5$ | $R^6$ | $R^7$ | $R^8$ | n | Physical Constants. |
|---|---|---|---|---|---|---|---|---|---|---|
| II-104 | NH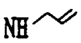 | H | isoPr | H | $SCH_3$ | Cl | H | | 0 | |
| II-105 | $NHCH_2C{\equiv}CH$ | H | 〃 | 〃 | 〃 | 〃 | 〃 | | 〃 | |
| II-106 | $NHCH_2OCH_3$ | H | 〃 | 〃 | 〃 | 〃 | 〃 | | 〃 | |
| II-107 | $-CH_2CH_2-$ | | 〃 | 〃 | 〃 | 〃 | 〃 | | 〃 | |
| II-108 | $CH_3$ | $CH_3$ | 〃 | 〃 | 〃 | 〃 | 〃 | | 〃 | |
| II-109 | NHCOOEt | H | 〃 | 〃 | 〃 | 〃 | 〃 | | 〃 | |
| II-110 | $NHCH_2COOEt$ | H | 〃 | 〃 | 〃 | 〃 | 〃 | | 〃 | |
| II-111 | NHCCOOEt<br>\|<br>$CH_3$ | H | 〃 | 〃 | 〃 | 〃 | 〃 | | 〃 | |
| II-112 | OH | CN | 〃 | 〃 | 〃 | 〃 | 〃 | | 〃 | |
| II-113 | SMe | SMe | | 〃 | 〃 | 〃 | 〃 | | 〃 | |
| II-114 | O<br>‖<br>$OCCH_3$ | H | 〃 | 〃 | 〃 | 〃 | 〃 | | 〃 | |
| II-115 | $-NHC_2H_4O-$ | | 〃 | 〃 | 〃 | 〃 | 〃 | | 〃 | |
| II-116 | $-NHC_2H_4S-$ | | 〃 | 〃 | 〃 | 〃 | 〃 | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| II-117 | $-NHC_2H_4NH-$ | | isoPr | H | $SCH_3$ | Cl | H | | 0 | |
| II-118 | $CH_3$ | OH | 〃 | 〃 | 〃 | 〃 | 〃 | | 〃 | |
| II-119 | H | $OCH_3$ | 〃 | 〃 | 〃 | 〃 | 〃 | | 〃 | |
| II-120 | 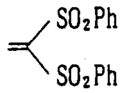 | | 〃 | 〃 | 〃 | 〃 | 〃 | | 〃 | |
| II-121 | 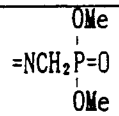 | | 〃 | 〃 | 〃 | 〃 | 〃 | | 〃 | |
| II-122 | 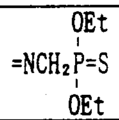 | | 〃 | 〃 | 〃 | 〃 | 〃 | | 〃 | |
| II-123 | $NO_2$ | H | $CF_3$ | 〃 | 〃 | 〃 | 〃 | | 〃 | |
| II-124 | $-CH=CH_2$ | 〃 | 〃 | 〃 | 〃 | 〃 | 〃 | | 〃 | |
| II-125 | $-C\equiv CH$ | 〃 | 〃 | 〃 | 〃 | 〃 | 〃 | | 〃 | |
| II-126 | 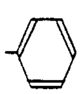 | 〃 | 〃 | 〃 | 〃 | 〃 | 〃 | | 〃 | |
| II-127 | | | | | | | | | | |
| II-128 | Br | H | 〃 | 〃 | 〃 | 〃 | 〃 | | 〃 | |
| II-129 | F | H | 〃 | 〃 | 〃 | 〃 | 〃 | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| II-130 | Cl | Cl | $CF_3$ | H | $SCH_3$ | Cl | H | | 0 | |
| II-131 | F | F | 〃 | 〃 | 〃 | 〃 | 〃 | | 〃 | |
| II-132 | Br | Br | 〃 | 〃 | 〃 | 〃 | 〃 | | 〃 | |
| II-133 | 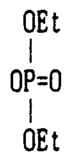 | H | 〃 | 〃 | Cl | $SCH_3$ | 〃 | | 〃 | |
| II-134 | =CHCOOH | | 〃 | 〃 | $SCH_3$ | Cl | 〃 | | 〃 | |
| II-135 | 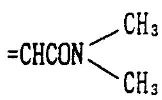 | | 〃 | 〃 | 〃 | 〃 | 〃 | | 〃 | |
| II-136 | =CHCN | | 〃 | 〃 | 〃 | 〃 | 〃 | | 〃 | |
| II-137 | $=CHCH_3$ | | 〃 | 〃 | 〃 | 〃 | 〃 | | 〃 | |
| II-138 | 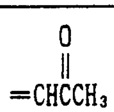 | | 〃 | 〃 | 〃 | 〃 | 〃 | | 〃 | |
| II-139 | $NH_2$ | H | 〃 | 〃 | 〃 | 〃 | 〃 | | 〃 | |
| II-140 | $COOCH_3$ | H | 〃 | 〃 | 〃 | 〃 | 〃 | | 〃 | |
| II-141 | COOH | H | 〃 | 〃 | 〃 | 〃 | 〃 | | 〃 | |

| No. | $R^1$ | $R^2$ | $R^3$ | $R^4$ | $R^5$ | $R^6$ | $R^7$ | $R^8$ | n | Physical Constants |
|---|---|---|---|---|---|---|---|---|---|---|
| II-142 | =N-isoPr | | iso-Pr | H | $SCH_3$ | Cl | H | | 0 | $n_D^{22.4}$ 1.5840 |
| II-143 | $=NCH_3$ | | 〃 | 〃 | 〃 | 〃 | 〃 | | 〃 | 〔47−51〕 |
| II-144 | =NEt | | 〃 | 〃 | 〃 | 〃 | 〃 | | 〃 | $n_D^{23.4}$ 1.5873 |
| II-145 | $=NOCH_2\overset{O}{\overset{\Vert}{C}}OEt$ | | | | | | | | | |
| II-146 | =CHCOOH | | 〃 | 〃 | 〃 | 〃 | 〃 | | 〃 | 〔148-150 〕 |
| II-147 | $NH_2$ | H | 〃 | 〃 | 〃 | 〃 | 〃 | | 〃 | $n_D^{22.5}$ 1.5707 |
| II-148 | H | H | $CF_3$ | 〃 | $PO(OEt)_2$ | 〃 | 〃 | | 〃 | $n_D^{24.5}$ 1.4990 |
| II-149 | $\begin{array}{c}OEt\\ \vert\\ OP{=}O\\ \vert\\ OEt\end{array}$ | H | iso-Pr | 〃 | Cl | 〃 | 〃 | | 〃 | $n_D^{25.5}$ 1.5260 |
| II-150 | $\begin{array}{c}OEt\\ \vert\\ P{=}O\\ \vert\\ OEt\end{array}$ | H | 〃 | 〃 | 〃 | 〃 | 〃 | | 〃 | $n_D^{25.5}$ 1.5352 |
| II-151 | $=NNH\overset{O}{\overset{\Vert}{C}}OEt$ | | $CF_3$ | 〃 | $NHNH\overset{O}{\overset{\Vert}{C}}OEt$ | 〃 | 〃 | | 〃 | $n_D^{24.0}$ 1.5405 |

Table. 2

Chemical Structure

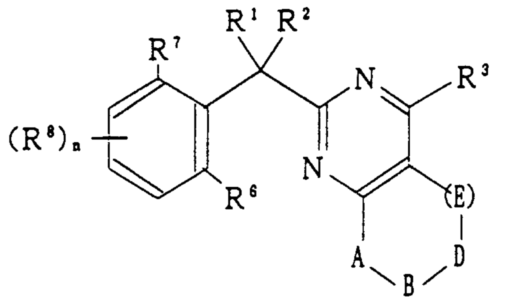

| No. | $R^1$ | $R^2$ | $R^3$ | A-B-D-(E)- | $R^6$, $R^7$, $R^8$ * | n | Physical Constants [ ] m.p.℃ |
|---|---|---|---|---|---|---|---|
| Ⅲ-1 | H, H | | OH | $CH_2$ $-CH_2CH_2CH_2-$ | 2-Cℓ | 0 | [240-242] |
| Ⅲ-2 | =0 | | OH | $CH_2$ $-CH_2CH_2CH_2-$ | 2-Cℓ | 0 | [210-213] |
| Ⅲ-3 | =0 | | Cℓ | $CH_2$ $-CH_2CH_2CH_2-$ | 2-Cℓ | 0 | [82-85] |
| Ⅲ-4 | =0 | | $OCH_3$ | $CH_2$ $-CH_2CH_2CH_2-$ | 2-Cℓ | 0 | [80-82] |
| Ⅲ-5 | =0 | | $SCH_3$ | $CH_2$ $-CH_2CH_2CH_2-$ | 2-Cℓ | 0 | [118-121] |
| Ⅲ-6 | H, H | | OH | CH =CH-CH=CH- | 2-Cℓ | 0 | [245-247] |
| Ⅲ-7 | =0 | | OH | CH =CH-CH=CH- | 2-Cℓ | 0 | [235-237] |
| Ⅲ-8 | =0 | | Cℓ | CH =CH-CH=CH- | 2-Cℓ | 0 | |
| Ⅲ-9 | =0 | | $SCH_3$ | CH =CH-CH=CH- | 2-Cℓ | 0 | [113-115] |
| Ⅲ-10 | H, H | | $CF_3$ | O $-CH_2CH_2-$ | 2-Cℓ | 0 | [91-94] |
| Ⅲ-11 | H, H | | $CF_3$ | O $-CH_2CH(OCH_3)-$ | 2-Cℓ | 0 | $n_D^{22.2}$ 1.5316 |
| Ⅲ-12 | OH, H | | $CF_3$ | O $-CH_2CH_2-$ | 2-Cℓ | 0 | [105-108] |
| Ⅲ-13 | =0 | | $CF_3$ | O $-CH_2CH_2-$ | 2-Cℓ | 0 | [101-104] |
| Ⅲ-14 | =0 | | $CF_3$ | O $-CH_2CH_2-$ | 2,6-$Cℓ_2$ | 0 | [138-140] |
| Ⅲ-15 | H, H | | $CF_3$ | O -CH=CH- | 2-Cℓ | 0 | [75-78] |
| Ⅲ-16 | =0 | | $CF_3$ | O -CH=CH- | 2-Cℓ | 0 | [112-114] |
| Ⅲ-17 | =0 | | $CF_3$ | NH $-CH_2CH_2-$ | 2-Cℓ | 0 | [220 up] |

| No. | $R^1$ | $R^2$ | $R^3$ | A-B-D-(E)- | $R^6, R^7, R^8$ | n | Physical Constants |
|---|---|---|---|---|---|---|---|
| III-18 | =O | | $CF_3$ | N $-CH_2CH_2-$<br>\|<br>$CH_3$ | 2-Cℓ | 0 | [ 85- 87 ] |
| III-19 | =O | | $CF_3$ | O $-CH_2CH_2CH_2-$ | 2-Cℓ | 0 | [ 75- 77 ] |
| III-20 | =O | | $CF_3$ | S $-CH_2CH_2-$ | 2-Cℓ | 0 | [ 99-102 ] |
| III-21 | =O | | $CF_3$ | O $-CH_2CH(Br)-$ | 2-Cℓ | 0 | powder**1 |
| III-22 | =O | | $CF_3$ | O $-CH_2CH_2CH-$<br>\|<br>Br | 2-Cℓ | 0 | $n_D^{22.2}$ 1.5361 |
| III-23 | =O | | $CF_3$ | S $-CH=CH-$ | 2-Cℓ | 0 | [ 78- 80 ] |
| III-24 | =O | | $CF_3$ | O $-CHCH_2-$<br>\|<br>$C_2H_5$ | 2-Cℓ | 0 | $n_D^{22.8}$ 1.5348 |
| III-25 | =O | | $CF_3$ | O $-C=CH-$<br>\|<br>$C_2H_5$ | 2-Cℓ | 0 | |
| III-26 | =O | | $CF_3$ | N $-CH=CH-$<br>\|<br>$CH_3$ | 2-Cℓ | 0 | [ 90- 91 ] |
| III-27 | =O | | $CF_3$ | O $-CH=CH-$ | 2,6-$Cℓ_2$ | 0 | [168-171 ] |
| III-28 | =O | | $CF_3$ | S $-CH_2CH_2-$ | 2,6-$Cℓ_2$ | 0 | [182-184 ] |
| III-29 | =O | | $CF_3$ | S $-CH=CH-$ | 2,6-$Cℓ_2$ | 0 | [136-137 ] |
| III-30 | =O | | $CF_3$ | O $-CH_2CH_2-$ | 2-Cℓ-6-F | 0 | [126-127 ] |
| III-31 | =O | | $CF_3$ | O $-CH=CH-$ | 2-Cℓ-6-F | 0 | [150-153 ] |
| III-32 | =O | | $CF_3$ | S $-CH_2CH_2-$ | 2-Cℓ-6-F | 0 | [133-134 ] |
| III-33 | =O | | $CF_3$ | S $-CH=CH-$ | 2-Cℓ-6-F | 0 | [125-128 ] |
| III-34 | =O | | $CF_3$ | O $-CH_2CH_2-$ | 2,6-$F_2$ | 0 | [130-132 ] |
| III-35 | =O | | $CF_3$ | O $-CH=CH-$ | 2,6-$F_2$ | 0 | |
| III-36 | =O | | i-Pr | O $-C=CH-$<br>\|<br>$C_6H_5$ | 2-Cℓ | 0 | [170-171 ] |

| No. | $R^1$ $R^2$ | $R^3$ | A-B-D-(E)- | $R^6$, $R^7$, $R^8$ | n | Physical Constants |
|---|---|---|---|---|---|---|
| Ⅲ-37 | =O | $CF_3$ | O -$CHCH_2CH_2$-<br>\|<br>$CH_3$ | 2-Cℓ | 0 | powder**2 |
| Ⅲ-38 | =O | $CF_3$ | O -$CHCH_2$-<br>\|<br>$CH_3$ | 2-Cℓ | 0 | 〔74- 76〕 |
| Ⅲ-39 | =O | iPr | O -CH=CH- | 2-Cℓ | 0 | $n_D^{22.3}$ 1.5941 |
| Ⅲ-40 | H, H | $CF_3$ | O -$CH_2CH_2$- | 2-Cℓ-6-F | 0 | 〔144-145〕 |
| Ⅲ-41 | =O | $CF_3$ | O -$CH_2CH_2CH_2$- | 2-Cℓ-6-F | 0 | 〔123-125〕 |
| Ⅲ-42 | H, H | $CF_3$ | O -$CH_2CH_2CH_2$- | 2, 6-$Cℓ_2$ | 0 | 〔166-168〕 |
| Ⅲ-43 | =O | $CF_3$ | O -$CH_2CH_2CH_2$- | 2, 6-$Cℓ_2$ | 0 | 〔173-175〕 |
| Ⅲ-44 | H, H | $CF_3$ | O -$CH_2CH_2$- | 2, 6-$F_2$ | 0 | 〔143-145〕 |
| Ⅲ-45 | H, H | $CF_3$ | S -$CH_2CH_2$- | 2, 6-$F_2$ | 0 | 〔120〕 |
| Ⅲ-46 | =O | $CF_3$ | S -$CH_2CH_2$- | 2, 6-$F_2$ | 0 | 〔129〕 |
| Ⅲ-47 | =O | $CF_3$ | S -CH=CH- | 2, 6-$F_2$ | 0 | 〔175〕 |
| Ⅲ-48 | =O | $CF_3$ | -N -$CH_2CH_2$-<br>\|<br>$CH_3$ | 2, 6-$F_2$ | 0 | 〔118-120〕 |
| Ⅲ-49 | =O | $CH_3$ | O -$CH_2CH_2$- | 2-Cℓ | 0 | 〔157-159〕 |
| Ⅲ-50 | =O | $CF_3$ | S -$CH_2CH_2CH_2$- | 2-Cℓ | 0 | $n_D^{23.7}$ 1.5961 |
| Ⅲ-51 | =O | $CF_3$ | O -$CH_2CH_2$- | 2, 4, 6-$(CH_3)_3$ | 1 | 〔113-114〕 |
| Ⅲ-52 | =O | $CF_3$ | O -$CH_2CH_2$- | 2, 4, 6-$Cℓ_3$ | 1 | 〔112-113〕 |
| Ⅲ-53 | H, H | $CF_3$ | O -$CH_2CH_2$- | 2-$OCH_3$ | 0 | 〔102-103〕 |
| Ⅲ-54 | H, H | $CF_3$ | O -$CH_2CH_2$- | 2-$NO_2$ | 0 | 〔138-140〕 |
| Ⅲ-55 | =O | H | O -$CH_2CH_2$- | 2-Cℓ | 0 | 〔125-127〕 |
| Ⅲ-56 | =O | $CF_3$ | NH -$CH_2CH_2CH_2$- | 2-Cℓ | 0 | 〔180-183〕 |
| Ⅲ-57 | O<br>‖<br>H, $OCCH_3$ | $CF_3$ | O -$CH_2CH_2$- | 2-$OCH_3$ | 0 | 〔94- 96〕 |

| No. | $R^1$ $R^2$ | $R^3$ | A-B-D-(E)- | $R^6$, $R^7$, $R^8$ | n | Physical Constants |
|---|---|---|---|---|---|---|
| III-58 | H, H | H | S $-CH_2CH_2-$ | 2-Cℓ | 0 | [ 76- 78 ] |
| III-59 | H, H | $CF_3$ | S $-CH_2CH_2-$ | 2-Cℓ-6-F | 0 | [125-128 ] |
| III-60 | H, H | $CF_3$ | -N $-CH_2CH_2-$<br>$CH_3$ | 2-Cℓ-6-F | 0 | [ 78- 81 ] |
| III-61 | H, H | $CF_3$ | S $-CHCH_2CH_2-$<br>$CH_3$ | 2-Cℓ | 0 | [ 92- 95 ] |
| III-62 | =O | H | S $-CH_2CH_2-$ | 2-Cℓ | 0 | [ 95- 97 ] |
| III-63 | H, H | $CF_3$ | S $-CH_2CH_2-$ | 2-Cℓ | 0 | [115-117 ] |
| III-64 | H, H | $CF_3$ | O $-CHCH_2CH_2-$<br>$CH_3$ | 2-Cℓ | 0 | $n_D^{22.7}$ 1.5399 |
| III-65 | =O | $CF_3$ | O $-CH_2CH_2-$ | 2-$OCH_3$ | 0 | [125-126 ] |
| III-66 | H, H | $CF_3$ | O $-CH_2CH_2-$ | 2, 3-$Cℓ_2$-4-$SCH_3$ | 2 | [ 97- 98 ] |
| III-67 | =O | $CF_3$ | O $-CH_2CH_2-$ | 2, 3-$Cℓ_2$-4-$SCH_3$ | 2 | |
| III-68 | =O | $CF_3$ | S $-CH_2CH_2-$ | 2, 4, 6-$Cℓ_3$ | 1 | [139-140 ] |
| III-69 | H, H | $CF_3$ | O $-CH_2CH_2-$ | 2-$CH_3$ | 0 | [ 85- 86 ] |
| III-70 | =O | $CF_3$ | O $-CH_2CH_2-$ | 2-$CH_3$ | 0 | |
| III-71 | H, H | $CF_3$ | O $-CH_2CH_2-$ | 2-F | 0 | [ 71- 72 ] |
| III-72 | =O | $CF_3$ | S $-CH_2CH_2-$ | 2-F | 0 | |
| III-73 | =O | $CF_3$ | -N -CH=CH-<br>$CH_3$ | 2-Cℓ-6-F | 0 | [105-107 ] |
| III-74 | =O | $CF_3$ | O $-CH_2CH(Br)-$ | 2-Cℓ-6-F | 0 | [157-161 ] |
| III-75 | H, H | $CF_3$ | -N $-CH_2CH_2-$<br>$CH_3$ | 2-Cℓ | 0 | [ 93- 95 ] |
| III-76 | =O | $CF_3$ | S $-CHCH_2CH_2-$<br>$CH_3$ | 2-Cℓ | 0 | powder**3 |

| No. | $R^1$ $R^2$ | $R^3$ | A-B-D-(E)- | $R^6$, $R^7$, $R^8$ | n | Physical Constants |
|---|---|---|---|---|---|---|
| Ⅲ-77 | H, H | $CF_3$ | N -$CHCH_2CH_2$-<br>\| \|<br>$CH_3$ $CH_3$ | 2-Cℓ | 0 | $n_D^{23.3}$ 1.5527 |
| Ⅲ-78 | =O | $CF_3$ | N -$CHCH_2CH_2$-<br>\| \|<br>$CH_3$ $CH_3$ | 2-Cℓ | 0 | |
| Ⅲ-79 | =O | $CF_3$ | N -CH-$CH_2CH_2$-<br>\| \|<br>Me Me | 2-Cℓ | 0 | 〔91- 93〕 |
| Ⅲ-80 | H, H | $CF_3$ | NH -CH-$CH_2CH_2$-<br>\|<br>Me | 2-Cℓ | 0 | $n_D^{22.5}$ 1.5597 |
| Ⅲ-81 | O<br>‖<br>=$NCH_2P(OEt)_2$ | $CF_3$ | O -$CH_2$-$CH_2$- | 2-Cℓ-6F | 0 | 〔139-140〕 |
| Ⅲ-82 | O<br>‖<br>=$NCH_2P(OEt)_2$ | $CF_3$ | O -$CH_2$-$CH_2$- | 2-Cℓ | 0 | $n_D^{24.5}$ 1.5335 |
| Ⅲ-83 | =O | $CF_3$ | NH -CH-$CH_2CH_2$-<br>\|<br>Me | 2-Cℓ | 0 | 〔140-143〕 |
| Ⅲ-84 | =O | $CF_3$ | O -$CH_2$-$CH_2$- | 2-F | 0 | 〔94- 95〕 |
| Ⅲ-85 | =O | $CF_3$ | NH -CH=CH- | 2-Cℓ-6F | 0 | 〔270 ℃ up〕 |
| Ⅲ-86 | H, H | $CF_3$ | N-$CH_2$Ph -$CH_2CH_2$- | 2-Cℓ | 0 | $n_D^{24.0}$ 1.5787 |
| Ⅲ-87 | =O | $CF_3$ | N-$CH_2$Ph -$CH_2CH_2$- | 2-Cℓ | 0 | 〔100-102〕 |
| Ⅲ-88 | =O | $CF_3$ | N-$CH_2$Ph -CH=CH- | 2-Cℓ | 0 | 〔119-120〕 |
| Ⅲ-89 | H, OAc | $CF_3$ | O -$CH_2$-$CH_2$- | 2-Me | 0 | $n_D^{22.6}$ 1.5237 |
| Ⅲ-90 | H, 2-Me-Ph | $CF_3$ | O -$CH_2$-$CH_2$- | 2-Me | 0 | 〔177-178〕 |
| Ⅲ-91 | =O | $CF_3$ | O -$CH_2$-$CH_2$- | 2-Me | 0 | 〔132-133〕 |
| Ⅲ-92 | H, H | $CF_3$ | O -$CH_2$-$CH_2$- | 2-$NH_2$ | 0 | 〔78- 79〕 |
| Ⅲ-93 | O<br>‖<br>H, $OP(OEt)_2$ | $CF_3$ | S -CH=CH- | 2-Cℓ | 0 | 〔105-106〕 |

| No. | $R^1$ $R^2$ | $R^3$ | A-B-D-(E)- | $R^6, R^7, R^8$ | n | Physical Constants |
|---|---|---|---|---|---|---|
| III-94 | $=NCH_2\overset{O}{\overset{\|}{P}}(OEt)_2$ | $CF_3$ | S -CH=CH- | 2-Cℓ | 0 | [ 96- 98 ] |
| III-95 | $=NCH_2\overset{O}{\overset{\|}{P}}(OEt)_2$ (isomer) | $CF_3$ | S -CH=CH- | 2-Cℓ | 0 | [104-105 ] |
| III-96 | H, H | $CF_3$ | O $-CH_2CH_2-$ | 2, 4-$Me_2$ | 1 | [ 87- 88 ] |
| III-97 | =O | $CF_3$ | O $-CH_2CH_2-$ | 2, 4-$Me_2$ | 1 | [112-113 ] |
| III-98 | $=CHCO_2Et$ | $CF_3$ | O $-CH_2CH_2-$ | 2-Cℓ | 0 | $n_D^{24.0}$ 1.5530 |
| III-99 | =NMe | $CF_3$ | O $-CH_2CH_2-$ | 2-Cℓ | 0 | $n_D^{24.3}$ 1.5579 |
| III-100 | $=CHCO_2H$ | $CF_3$ | O $-CH_2CH_2-$ | 2-Cℓ | 0 | [178-180 ] |
| III-101 | $=CHCONHBu^n$ | $CF_3$ | O $-CH_2CH_2-$ | 2-Cℓ | 0 | powder **4 |
| III-102 | H, H | $CF_3$ | O $-\underset{Me}{\underset{\vert}{CH}}CH_2CH_2-$ | 2Cℓ-6F | 0 | [ 97- 98 ] |
| III-103 | =O | $CF_3$ | O $-\underset{Me}{\underset{\vert}{CH}}CH_2CH_2-$ | 2Cℓ-6F | 0 | powder **5 |
| III-104 | $=NCH_2\overset{O}{\overset{\|}{P}}(OEt)_2$ | $CF_3$ | O -CH=CH- | 2-Cℓ | 0 | powder **6 |
| III-105 | =NOMe | $CF_3$ | O $-CH_2CH_2-$ | 2-Cℓ | 0 | $n_D^{25.7}$ 1.5543 |
| III-106 | $=NNH_2$ | $CF_3$ | O $-CH_2CH_2-$ | 2-Cℓ | 0 | 218 dec |
| III-107 | $=NNHCH_3$ (syn) | $CF_3$ | O $-CH_2CH_2-$ | 2-Cℓ | 0 | [164-165 ] |
| III-108 | $=NNHCH_3$ (anti) | $CF_3$ | O $-CH_2-CH_2-$ | 2-Cℓ | 0 | powder **7 |
| III-109 | =NNHEt (syn) | $CF_3$ | O $-CH_2CH_2-$ | 2-Cℓ | 0 | [133-135 ] |
| III-110 | =NNHEt (anti) | $CF_3$ | O $-CH_2CH_2-$ | 2-Cℓ | 0 | powder **8 |
| III-111 | =NNHPh | $CF_3$ | O $-CH_2CH_2-$ | 2-Cℓ | 0 | 210 dec |
| III-112 | H, OH | $CF_3$ | O -CH=CH- | 2-Cℓ | 0 | [ 82- 3 ] |

| No. | $R^1$ $R^2$ | $R^3$ | A-B-D-(E)- | $R^6, R^7, R^8$ | n | Physical Constants |
|---|---|---|---|---|---|---|
| Ⅲ-113 | $=NCH_2CH_2CO_2Et$ | $CF_3$ | O $-CH_2CH_2-$ | 2-Cℓ | 0 | $n_D^{23.5}$ 1.5326 |
| Ⅲ-114 | $=NN(CH_3)_2$ | $CF_3$ | O $-CH_2CH_2-$ | 2-Cℓ | 0 | powder **9 |
| Ⅲ-115 | H, H | $CF_3$ | O $-CH_2CH_2-$ | 2-NHAc | 0 | [112-113] |
| Ⅲ-116 | =O | $CF_3$ | O $-CH_2CH_2-$ | 2-NHAc | 0 | [187-188] |
| Ⅲ-117 | H, H | $CF_3$ | NH $-CH(Me)CH_2CH_2-$ | 2-Cℓ-6-F | 0 | [114-116] |
| Ⅲ-118 | =O | $CF_3$ | NH $-CH(Me)CH_2CH_2-$ | 2-Cℓ-6-F | 0 | [160-163] |
| Ⅲ-119 | H, H | $CF_3$ | S $-CH_2CH_2CH_2-$ | 2-Cℓ | 0 | [120-121] |
| Ⅲ-120 | H, H | $CF_3$ | S $-CH(Me)CH_2CH_2-$ | 2-Cℓ-6-F | 0 | |
| Ⅲ-121 | =O | $CF_3$ | S $-CH(Me)CH_2CH_2-$ | 2Cℓ-6F | 0 | [116-118] |
| Ⅲ-122 | H, Cℓ | $CF_3$ | O -CH=CH- | 2-Cℓ | 0 | [ 85- 87] |
| Ⅲ-123 | $=C(CN)_2$ | $CF_3$ | O $-CH_2-CH_2-$ | 2-Cℓ | 0 | [147-148] |
| Ⅲ-124 | H, H | $CF_3$ | O $-CH_2CH_2-$ | $2-CF_3$ | 0 | [ 80- 81] |
| Ⅲ-125 | H, H | $CF_3$ | O -CH=CH- | $2-CF_3$ | 0 | |
| Ⅲ-126 | H, H | $CF_3$ | O $-CH_2CH_2CH_2-$ | $2-CF_3$ | 0 | |
| Ⅲ-127 | H, H | i-Pr | O $-CH_2CH_2-$ | $2-CF_3$ | 0 | |
| Ⅲ-128 | H, H | i-Pr | O -CH=CH- | $2-CF_3$ | 0 | |
| Ⅲ-129 | H, H | Et | O $-CH_2CH_2-$ | $2-CF_3$ | 0 | |
| Ⅲ-130 | H, H | Et | O -CH=CH- | $2-CF_3$ | 0 | |
| Ⅲ-131 | H, H | $CF_3$ | S $-CH_2CH_2-$ | $2-CF_3$ | 0 | |
| Ⅲ-132 | H, H | $CF_3$ | S -CH=CH- | $2-CF_3$ | 0 | |
| Ⅲ-133 | H, H | i-Pr | S $-CH_2CH_2-$ | $2-CF_3$ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-134 | H, H | | i-Pr | S -CH=CH- | 2-$CF_3$ | 0 | |
| III-135 | H, H | | Et | S -$CH_2CH_2$- | 2-$CF_3$ | 0 | |
| III-136 | H, H | | Et | S -CH=CH- | 2-$CF_3$ | 0 | |
| III-137 | H, H | | $CF_3$ | O -CH=CH- | 2-$CH_3$ | 0 | |
| III-138 | H, H | | $CF_3$ | O -$CH_2CH_2CH_2$- | 2-$CH_3$ | 0 | |
| III-139 | H, H | | $CF_3$ | O -$CH(Me)CH_2CH_2$- | 2-$CH_3$ | 0 | |
| III-140 | H, H | | i-Pr | O -$CH_2CH_2$- | 2-$CH_3$ | 0 | |
| III-141 | H, H | | i-Pr | O -CH=CH- | 2-$CH_3$ | 0 | |
| III-142 | H, H | | Et | O -$CH_2CH_2$- | 2-$CH_3$ | 0 | |
| III-143 | H, H | | Et | O -CH=CH- | 2-$CH_3$ | 0 | |
| III-144 | H, H | | $CF_3$ | S -$CH_2CH_2$- | 2-$CH_3$ | 0 | [ 77- 78 ] |
| III-145 | H, H | | $CF_3$ | S -CH=CH- | 2-$CH_3$ | 0 | |
| III-146 | H, H | | Et | S -$CH_2CH_2$- | 2-$CH_3$ | 0 | |
| III-147 | H, H | | Et | S -CH=CH- | 2-$CH_3$ | 0 | |
| III-148 | H, H | | i-Pr | S -CH=CH- | 2-$CH_3$ | 0 | |
| III-149 | H, H | | i-Pr | S -$CH_2CH_2$- | 2-$CH_3$ | 0 | |
| III-150 | H, H | | $CF_3$ | NMe -$CH_2CH_2$- | 2-$CH_3$ | 0 | [ 61- 63 ] |
| III-151 | H, H | | $CF_3$ | NMe -CH=CH- | 2-$CH_3$ | 0 | |
| III-152 | H, H | | $CF_3$ | NH -$CH_2CH_2$- | 2-$CH_3$ | 0 | |
| III-153 | H, H | | $CF_3$ | NH -CH=CH- | 2-$CH_3$ | 0 | |
| III-154 | H, H | | i-Pr | NMe -$CH_2CH_2$- | 2-$CH_3$ | 0 | |
| III-155 | H, H | | i-Pr | NMe -CH=CH- | 2-$CH_3$ | 0 | |
| III-156 | H, H | | i-Pr | NH -$CH_2CH_2$- | 2-$CH_3$ | 0 | |
| III-157 | H, H | | i-Pr | NH -CH=CH- | 2-$CH_3$ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-158 | H, H | | $CF_3$ | O -CH=CMe- | 2-Cℓ | 0 | |
| III-159 | H, H | | $CF_3$ | S -CH=CMe- | 2-Cℓ | 0 | |
| III-160 | H, H | | $CF_3$ | NH -CH=CMe- | 2-Cℓ | 0 | |
| III-161 | H, H | | $CF_3$ | NMe -CH=CMe- | 2-Cℓ | 0 | |
| III-162 | H, H | | $CF_3$ | $CH_2$ -O-$CH_2$- | 2-Cℓ | 0 | |
| III-163 | H, H | | $CF_3$ | $CH_2$ -S-$CH_2$- | 2-Cℓ | 0 | |
| III-164 | H, H | | $CF_3$ | $CH_2$ -NH-$CH_2$- | 2-Cℓ | 0 | |
| III-165 | H, H | | $CF_3$ | $CH_2$ -N(Me)-$CH_2$- | 2-Cℓ | 0 | |
| III-166 | H, H | | $CF_3$ | $CH_2$ -N=CH- | 2-Cℓ | 0 | |
| III-167 | H, H | | $CF_3$ | $CH_2$ -O-$CH_2$-$CH_2$- | 2-Cℓ | 0 | |
| III-168 | H, H | | $CF_3$ | $CH_2$ -S-$CH_2$-$CH_2$- | 2-Cℓ | 0 | |
| III-169 | H, H | | $CF_3$ | $CH_2$ -NH-$CH_2CH_2$- | 2-Cℓ | 0 | |
| III-170 | H, H | | $CF_3$ | $CH_2$ -NMe-$CH_2CH_2$- | 2-Cℓ | 0 | |
| III-171 | H, H | | $CF_3$ | $CH_2$ -O-CH=CH- | 2-Cℓ | 0 | |
| III-172 | H, H | | $CF_3$ | $CH_2$ -S-CH=CH- | 2-Cℓ | 0 | |
| III-173 | H, H | | $CF_3$ | $CH_2$ -NH-CH=CH- | 2-Cℓ | 0 | |
| III-174 | H, H | | $CF_3$ | $CH_2$ -NMe-CH=CH- | 2-Cℓ | 0 | |
| III-175 | H, H | | $CF_3$ | CH=N-CH=CH- | 2-Cℓ | 0 | |
| III-176 | H, H | | $CF_3$ | $CH_2$ -$CH_2$-O- | 2-Cℓ | 0 | |
| III-177 | H, H | | $CF_3$ | $CH_2$ -$CH_2$-N(H)- | 2-Cℓ | 0 | |
| III-178 | H, H | | $CF_3$ | $CH_2$ -$CH_2$-S- | 2-Cℓ | 0 | |
| III-179 | H, H | | $CF_3$ | $CH_2$ -$CH_2$-NMe- | 2-Cℓ | 0 | |
| III-180 | H, H | | $CF_3$ | CH=CH-O- | 2-Cℓ | 0 | |
| III-181 | H, H | | $CF_3$ | CH=CH-S- | 2-Cℓ | 0 | |

| No. | $R^1$ $R^2$ | $R^3$ | A-B-D-(E)- | $R^6, R^7, R^8$ | n | Physical Constants |
|---|---|---|---|---|---|---|
| Ⅲ-182 | H, H | $CF_3$ | CH=CH-NH- | 2-Cℓ | 0 | |
| Ⅲ-183 | H, H | $CF_3$ | CH=CH-NMe- | 2-Cℓ | 0 | |
| Ⅲ-184 | H, H | $CF_3$ | $CH_2$ $-CH_2CH_2$-O- | 2-Cℓ | 0 | |
| Ⅲ-185 | H, H | $CF_3$ | $CH_2$ $-CH_2CH_2$-NH- | 2-Cℓ | 0 | |
| Ⅲ-186 | H, H | $CF_3$ | $CH_2$ $-CH_2CH_2$-S- | 2-Cℓ | 0 | |
| Ⅲ-187 | H, H | $CF_3$ | $CH_2$ $-CH_2CH_2$-NMe- | 2-Cℓ | 0 | |
| Ⅲ-188 | H, H | Et | NMe $-CH_2CH_2-$ | 2-Me | 0 | |
| Ⅲ-189 | H, H | Et | NMe -CH=CH- | 2-Me | 0 | |
| Ⅲ-190 | H, H | Et | NH $-CH_2CH_2-$ | 2-Me | 0 | |
| Ⅲ-191 | H, H | Et | NH -CH=CH- | 2-Me | 0 | |
| Ⅲ-192 | H, H | i-Pr | O $-CH_2CH_2-$ | 2-Cℓ | 0 | |
| Ⅲ-193 | H, H | i-Pr | O $-CH_2CH_2CH_2-$ | 2-Cℓ | 0 | |
| Ⅲ-194 | H, H | i-Pr | O $-CH(Me)CH_2CH_2-$ | 2-Cℓ | 0 | |
| Ⅲ-195 | H, H | i-Pr | S $-CH_2CH_2-$ | 2-Cℓ | 0 | |
| Ⅲ-196 | H, H | i-Pr | S -CH=CH- | 2-Cℓ | 0 | |
| Ⅲ-197 | H, H | i-Pr | NMe $-CH_2CH_2-$ | 2-Cℓ | 0 | |
| Ⅲ-198 | H, H | i-Pr | NMe -CH=CH- | 2-Cℓ | 0 | |
| Ⅲ-199 | H, H | i-Pr | NH $-CH_2CH_2-$ | 2-Cℓ | 0 | |
| Ⅲ-200 | H, H | i-Pr | NH -CH=CH- | 2-Cℓ | 0 | |
| Ⅲ-201 | H, H | Et | O $-CH_2CH_2-$ | 2-Cℓ | 0 | |
| Ⅲ-202 | H, H | Et | O -CH=CH- | 2-Cℓ | 0 | |
| Ⅲ-203 | H, H | Et | O $-CH_2CH_2CH_2-$ | 2-Cℓ | 0 | |
| Ⅲ-204 | H, H | Et | S $-CH_2CH_2-$ | 2-Cℓ | 0 | |
| Ⅲ-205 | H, H | Et | S -CH=CH- | 2-Cℓ | 0 | |

| No. | $R^1$ $R^2$ | $R^3$ | A-B-D-(E)- | $R^6$, $R^7$, $R^8$ | n | Physical Constants |
|---|---|---|---|---|---|---|
| Ⅲ-206 | H, H | Et | NMe $-CH_2CH_2-$ | 2-Cℓ | 0 | |
| Ⅲ-207 | H, H | Et | NMe -CH=CH- | 2-Cℓ | 0 | |
| Ⅲ-208 | H, H | Et | NH $-CH_2CH_2-$ | 2-Cℓ | 0 | |
| Ⅲ-209 | H, H | Et | NH -CH=CH- | 2-Cℓ | 0 | |
| Ⅲ-210 | H, H | $CF_2CF_3$ | O $-CH_2CH_2-$ | 2-Cℓ | 0 | |
| Ⅲ-211 | H, H | $CF_2CF_3$ | O -CH=CH- | 2-Cℓ | 0 | |
| Ⅲ-212 | H, H | $CF_2CF_3$ | S $-CH_2CH_2-$ | 2-Cℓ | 0 | |
| Ⅲ-213 | H, H | $CF_2CF_3$ | S -CH=CH- | 2-Cℓ | 0 | |
| Ⅲ-214 | H, H | OMe | O $-CH_2CH_2-$ | 2-Cℓ | 0 | |
| Ⅲ-215 | H, H | OMe | O -CH=CH- | 2-Cℓ | 0 | |
| Ⅲ-216 | H, H | $OCF_3$ | O $-CH_2CH_2-$ | 2-Cℓ | 0 | |
| Ⅲ-217 | H, H | $OCF_3$ | O -CH=CH- | 2-Cℓ | 0 | |
| Ⅲ-218 | H, H | SMe | O $-CH_2CH_2-$ | 2-Cℓ | 0 | |
| Ⅲ-219 | H, H | NHMe | O $-CH_2CH_2-$ | 2-Cℓ | 0 | |
| Ⅲ-220 | H, H | $NMe_2$ | O $-CH_2CH_2-$ | 2-Cℓ | 0 | |
| Ⅲ-221 | H, H | CN | O $-CH_2CH_2-$ | 2-Cℓ | 0 | |
| Ⅲ-222 | H, H | C≡CH | O $-CH_2CH_2-$ | 2-Cℓ | 0 | |
| Ⅲ-223 | H, H | $CH=CH_2$ | O $-CH_2CH_2-$ | 2-Cℓ | 0 | |
| Ⅲ-224 | =O | $CF_3$ | O $-CH_2CH_2-$ | $2-CF_3$ | 0 | [126-127 ] |
| Ⅲ-225 | =O | $CF_3$ | O -CH=CH- | $2-CF_3$ | 0 | |
| Ⅲ-226 | =O | $CF_3$ | O $-CH_2CH_2CH_2-$ | $2-CF_3$ | 0 | |
| Ⅲ-227 | =O | i-Pr | O $-CH_2CH_2-$ | $2-CF_3$ | 0 | |
| Ⅲ-228 | =O | i-Pr | O -CH=CH- | $2-CF_3$ | 0 | |
| Ⅲ-229 | =O | Et | O $-CH_2CH_2-$ | $2-CF_3$ | 0 | |
| Ⅲ-230 | =O | Et | O -CH=CH- | $2-CF_3$ | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| Ⅲ-231 | =O | | $CF_3$ | S $-CH_2CH_2-$ | 2-$CF_3$ | 0 | |
| Ⅲ-232 | =O | | $CF_3$ | S $-CH=CH-$ | 2-$CF_3$ | 0 | |
| Ⅲ-233 | =O | | i-Pr | S $-CH_2CH_2-$ | 2-$CF_3$ | 0 | |
| Ⅲ-234 | =O | | i-Pr | S $-CH=CH-$ | 2-$CF_3$ | 0 | |
| Ⅲ-235 | =O | | Et | S $-CH_2CH_2-$ | 2-$CF_3$ | 0 | |
| Ⅲ-236 | =O | | Et | S $-CH=CH-$ | 2-$CF_3$ | 0 | |
| Ⅲ-237 | =O | | $CF_3$ | O $-CH=CH-$ | 2-$CH_3$ | 0 | |
| Ⅲ-238 | =O | | $CF_3$ | O $-CH_2CH_2CH_2-$ | 2-$CH_3$ | 0 | |
| Ⅲ-239 | =O | | $CF_3$ | O $-CH(Me)CH_2CH_2-$ | 2-$CH_3$ | 0 | |
| Ⅲ-240 | =O | | i-Pr | O $-CH_2CH_2-$ | 2-$CH_3$ | 0 | |
| Ⅲ-241 | =O | | i-Pr | O $-CH=CH-$ | 2-$CH_3$ | 0 | |
| Ⅲ-242 | =O | | Et | O $-CH_2CH_2-$ | 2-$CH_3$ | 0 | |
| Ⅲ-243 | =O | | Et | O $-CH=CH-$ | 2-$CH_3$ | 0 | |
| Ⅲ-244 | =O | | $CF_3$ | S $-CH_2CH_2-$ | 2-$CH_3$ | 0 | 〔130-131〕 |
| Ⅲ-245 | =O | | $CF_3$ | S $-CH=CH-$ | 2-$CH_3$ | 0 | |
| Ⅲ-246 | =O | | Et | S $-CH_2CH_2-$ | 2-$CH_3$ | 0 | |
| Ⅲ-247 | =O | | Et | S $-CH=CH-$ | 2-$CH_3$ | 0 | |
| Ⅲ-248 | =O | | i-Pr | S $-CH=CH-$ | 2-$CH_3$ | 0 | |
| Ⅲ-249 | =O | | i-Pr | S $-CH_2CH_2-$ | 2-$CH_3$ | 0 | |
| Ⅲ-250 | =O | | $CF_3$ | NMe $-CH_2CH_2-$ | 2-$CH_3$ | 0 | 〔98-100〕 |
| Ⅲ-251 | =O | | $CF_3$ | NMe $-CH=CH-$ | 2-$CH_3$ | 0 | 〔113-114〕 |
| Ⅲ-252 | =O | | $CF_3$ | NH $-CH_2CH_2-$ | 2-$CH_3$ | 0 | |
| Ⅲ-253 | =O | | $CF_3$ | NH $-CH=CH-$ | 2-$CH_3$ | 0 | |
| Ⅲ-254 | =O | | i-Pr | NMe $-CH_2CH_2-$ | 2-$CH_3$ | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| III-255 | =O | | i-Pr | NMe -CH=CH- | 2-$CH_3$ | 0 | |
| III-256 | =O | | i-Pr | NH -$CH_2CH_2$- | 2-$CH_3$ | 0 | |
| III-257 | =O | | i-Pr | NH -CH=CH- | 2-$CH_3$ | 0 | |
| III-258 | =O | | $CF_3$ | O -CH=CMe- | 2-Cl | 0 | |
| III-259 | =O | | $CF_3$ | S -CH=CMe- | 2-Cl | 0 | |
| III-260 | =O | | $CF_3$ | NH -CH=CMe- | 2-Cl | 0 | |
| III-261 | =O | | $CF_3$ | NMe -CH=CMe- | 2-Cl | 0 | |
| III-262 | =O | | $CF_3$ | $CH_2$ -O-$CH_2$- | 2-Cl | 0 | |
| III-263 | =O | | $CF_3$ | $CH_2$ -S-$CH_2$- | 2-Cl | 0 | |
| III-264 | =O | | $CF_3$ | $CH_2$ -NH-$CH_2$- | 2-Cl | 0 | |
| III-265 | =O | | $CF_3$ | $CH_2$ -N-$CH_2$-<br>Me | 2-Cl | 0 | |
| III-266 | =O | | $CF_3$ | $CH_2$ -N-CH- | 2-Cl | 0 | |
| III-267 | =O | | $CF_3$ | $CH_2$ -O-$CH_2$-$CH_2$- | 2-Cl | 0 | |
| III-268 | =O | | $CF_3$ | $CH_2$ -S-$CH_2$-$CH_2$- | 2-Cl | 0 | |
| III-269 | =O | | $CF_3$ | $CH_2$ -NH-$CH_2CH_2$- | 2-Cl | 0 | |
| III-270 | =O | | $CF_3$ | $CH_2$ -NMe-$CH_2CH_2$- | 2-Cl | 0 | |
| III-271 | =O | | $CF_3$ | $CH_2$ -O-CH=CH- | 2-Cl | 0 | |
| III-272 | =O | | $CF_3$ | $CH_2$ -S-CH=CH- | 2-Cl | 0 | |
| III-273 | =O | | $CF_3$ | $CH_2$ -NH-CH=CH- | 2-Cl | 0 | |
| III-274 | =O | | $CF_3$ | $CH_2$ -NMe-CH=CH- | 2-Cl | 0 | |
| III-275 | =O | | $CF_3$ | CH=N-CH=CH- | 2-Cl | 0 | |
| III-276 | =O | | $CF_3$ | $CH_2$ -$CH_2$-O- | 2-Cl | 0 | |
| III-277 | =O | | $CF_3$ | $CH_2$ -$CH_2$-N-<br>H | 2-Cl | 0 | |
| III-278 | =O | | $CF_3$ | $CH_2$ -$CH_2$-S- | 2-Cl | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| III-279 | =O | | $CF_3$ | $CH_2$ $-CH_2-$NMe- | 2-Cℓ | 0 | |
| III-280 | =O | | $CF_3$ | CH=CH-O- | 2-Cℓ | 0 | |
| III-281 | =O | | $CF_3$ | CH=CH-S- | 2-Cℓ | 0 | |
| III-282 | =O | | $CF_3$ | CH=CH-NH- | 2-Cℓ | 0 | |
| III-283 | =O | | $CF_3$ | CH=CH-NMe- | 2-Cℓ | 0 | |
| III-284 | =O | | $CF_3$ | $CH_2CH_2CH_2$-O- | 2-Cℓ | 0 | |
| III-285 | =O | | $CF_3$ | $CH_2CH_2CH_2$-NH- | 2-Cℓ | 0 | |
| III-286 | =O | | $CF_3$ | $CH_2CH_2CH_2$-S- | 2-Cℓ | 0 | |
| III-287 | =O | | $CF_3$ | $CH_2CH_2CH_2$-NMe- | 2-Cℓ | 0 | |
| III-288 | =O | | Et | NMe $-CH_2CH_2-$ | 2-Me | 0 | |
| III-289 | =O | | Et | NMe -CH=CH- | 2-Me | 0 | |
| III-290 | =O | | Et | NH $-CH_2CH_2-$ | 2-Me | 0 | |
| III-291 | =O | | Et | NH -CH=CH- | 2-Me | 0 | |
| III-292 | =O | | i-Pr | O $-CH_2CH_2-$ | 2-Cℓ | 0 | |
| III-293 | =O | | i-Pr | O $-CH_2CH_2CH_2-$ | 2-Cℓ | 0 | |
| III-294 | =O | | i-Pr | O $-CH(Me)CH_2CH_2-$ | 2-Cℓ | 0 | |
| III-295 | =O | | i-Pr | S $-CH_2CH_2-$ | 2-Cℓ | 0 | |
| III-296 | =O | | i-Pr | S -CH=CH- | 2-Cℓ | 0 | |
| III-297 | =O | | i-Pr | NMe $-CH_2CH_2-$ | 2-Cℓ | 0 | |
| III-298 | =O | | i-Pr | NMe -CH=CH- | 2-Cℓ | 0 | |
| III-299 | =O | | i-Pr | NH $-CH_2CH_2-$ | 2-Cℓ | 0 | |
| III-300 | =O | | i-Pr | NH -CH=CH- | 2-Cℓ | 0 | |
| III-301 | =O | | Et | O $-CH_2CH_2-$ | 2-Cℓ | 0 | |
| III-302 | =O | | Et | O -CH=CH- | 2-Cℓ | 0 | |

| no. | $R^1$ | $R^2$ | $R^3$ | A-B-D-(E)- | $R^6$, $R^7$, $R^8$ | n | Physical Constants |
|---|---|---|---|---|---|---|---|
| III-303 | =O | | Et | O $-CH_2CH_2CH_2-$ | 2-Cℓ | 0 | |
| III-304 | =O | | Et | S $-CH_2CH_2-$ | 2-Cℓ | 0 | |
| III-305 | =O | | Et | S -CH=CH- | 2-Cℓ | 0 | |
| III-306 | =O | | Et | NMe $-CH_2CH_2-$ | 2-Cℓ | 0 | |
| III-307 | =O | | Et | NMe -CH=CH- | 2-Cℓ | 0 | |
| III-308 | =O | | Et | NH $-CH_2CH_2-$ | 2-Cℓ | 0 | |
| III-309 | =O | | Et | NH -CH=CH- | 2-Cℓ | 0 | |
| III-310 | =O | | $CF_2CF_3$ | O $-CH_2CH_2-$ | 2-Cℓ | 0 | |
| III-311 | =O | | $CF_2CF_3$ | O -CH=CH- | 2-Cℓ | 0 | |
| III-312 | =O | | $CF_2CF_3$ | S $-CH_2CH_2-$ | 2-Cℓ | 0 | |
| III-313 | =O | | $CF_2CF_3$ | S -CH=CH- | 2-Cℓ | 0 | |
| III-314 | =O | | OMe | O $-CH_2CH_2-$ | 2-Cℓ | 0 | |
| III-315 | =O | | OMe | O -CH=CH- | 2-Cℓ | 0 | |
| III-316 | =O | | $OCF_3$ | O $-CH_2CH_2-$ | 2-Cℓ | 0 | |
| III-317 | =O | | $OCF_3$ | O -CH=CH- | 2-Cℓ | 0 | |
| III-318 | =O | | SMe | O $-CH_2CH_2-$ | 2-Cℓ | 0 | |
| III-319 | =O | | NHMe | O $-CH_2CH_2-$ | 2-Cℓ | 0 | |
| III-320 | =O | | $NMe_2$ | O $-CH_2CH_2-$ | 2-Cℓ | 0 | |
| III-321 | =O | | CN | O $-CH_2CH_2-$ | 2-Cℓ | 0 | |
| III-322 | =O | | C≡CH | O $-CH_2CH_2-$ | 2-Cℓ | 0 | |
| III-323 | =O | | $CH=CH_2$ | O $-CH_2CH_2-$ | 2-Cℓ | 0 | |
| III-324 | =O | | Et | $CH_2-OCH_2-$ | 2-Cℓ | 0 | |
| III-325 | =O | | Et | $CH_2-OCH_2-$ | 2-Cℓ | 0 | |
| III-326 | =O | | i-Pr | $CH_2-OCH_2-$ | 2-Cℓ | 0 | |
| III-327 | =O | | Cℓ | $CH_2-OCH_2-$ | 2-Cℓ | 0 | |

| no. | $R^1$ | $R^2$ | $R^3$ | A-B-D-(E)- | $R^6, R^7, R^8$ | n | Physical Constants |
|---|---|---|---|---|---|---|---|
| III-328 | =O | | $OCH_3$ | $CH_2-OCH_2-$ | 2-Cℓ | 0 | |
| III-329 | =O | | $SCH_3$ | $CH_2-OCH_2-$ | 2-Cℓ | 0 | |
| III-330 | =O | | $NHCH_3$ | $CH_2-OCH_2-$ | 2-Cℓ | 0 | |
| III-331 | =O | | $CF_3$ | $CH_2-OCH_2-$ | 2-Cℓ | 0 | |
| III-332 | =O | | Et | $CH_2-OCH_2-$ | 2-Cℓ | 0 | |
| III-333 | =O | | i-Pr | $CH_2-OCH_2-$ | 2-Cℓ | 0 | |
| III-334 | =O | | Cℓ | $CH_2-OCH_2-$ | 2-Cℓ | 0 | |
| III-335 | =O | | $OCH_3$ | $CH_2-OCH_2-$ | 2-Cℓ | 0 | |
| III-336 | =O | | $SCH_3$ | $CH_2-OCH_2-$ | 2-Cℓ | 0 | |
| III-337 | =O | | $NHCH_3$ | $CH_2-OCH_2-$ | 2-Cℓ | 0 | |
| III-338 | =O | | $CF_3$ | $CH_2-OCH_2-$ | 2-Cℓ | 0 | |
| III-339 | =O | | Et | $CH_2-OCH_2-$ | 2-Cℓ | 0 | |
| III-340 | =O | | i-Pr | $CH_2-OCH_2-$ | 2-Cℓ | 0 | |
| III-341 | =O | | Cℓ | $CH_2-OCH_2-$ | 2-Cℓ | 0 | |
| III-342 | =O | | $OCH_3$ | $CH_2-OCH_2-$ | 2-Cℓ | 0 | |
| III-343 | =O | | $SCH_3$ | $CH_2-OCH_2-$ | 2-Cℓ | 0 | |
| III-344 | =O | | $NHCH_3$ | $CH_2-OCH_2-$ | 2-Cℓ | 0 | |
| III-345 | =O | | $CF_2CF_3$ | $CH_2-CH_2O-$ | 2-Cℓ | 0 | |
| III-346 | =O | | Et | $CH_2-CH_2O-$ | 2-Cℓ | 0 | |
| III-347 | =O | | i-Pr | $CH_2-CH_2O-$ | 2-Cℓ | 0 | |
| III-348 | =O | | Cℓ | $CH_2-CH_2O-$ | 2-Cℓ | 0 | |
| III-349 | =O | | $OCH_3$ | $CH_2-CH_2O-$ | 2-Cℓ | 0 | |
| III-350 | =O | | $SCH_3$ | $CH_2-CH_2O-$ | 2-Cℓ | 0 | |
| III-351 | =O | | $NHCH_3$ | $CH_2-CH_2O-$ | 2-Cℓ | 0 | |
| III-352 | =O | | $CF_3$ | $CH_2-CH_2O-$ | 2-Cℓ, 6-F | 0 | |

| no. | $R^1$ | $R^2$ | $R^3$ | A-B-D-(E)- | $R^6, R^7, R^8$ | n | Physical Constants |
|---|---|---|---|---|---|---|---|
| III-353 | =O | | Et | $CH_2$-$CH_2$O- | 2-Cℓ, 6-F | 0 | |
| III-354 | =O | | i-Pr | $CH_2$-$CH_2$O- | 2-Cℓ, 6-F | 0 | |
| III-355 | =O | | Cℓ | $CH_2$-$CH_2$O- | 2-Cℓ, 6-F | 0 | |
| III-356 | =O | | $OCH_3$ | $CH_2$-$CH_2$O- | 2-Cℓ, 6-F | 0 | |
| III-357 | =O | | $SCH_3$ | $CH_2$-$CH_2$O- | 2-Cℓ, 6-F | 0 | |
| III-358 | =O | | $NHCH_3$ | $CH_2$-$CH_2$O- | 2-Cℓ, 6-F | 0 | |
| III-359 | =O | | $CF_3$ | $CH_2$-$CH_2$O- | 2, 6-$Cℓ_2$ | 0 | |
| III-360 | =O | | Et | $CH_2$-$CH_2$O- | 2, 6-$Cℓ_2$ | 0 | |
| III-361 | =O | | i-Pr | $CH_2$-$CH_2$O- | 2, 6-$Cℓ_2$ | 0 | |
| III-362 | =O | | Cℓ | $CH_2$-$CH_2$O- | 2, 6-$Cℓ_2$ | 0 | |
| III-363 | =O | | $OCH_3$ | $CH_2$-$CH_2$O- | 2, 6-$Cℓ_2$ | 0 | |
| III-364 | =O | | $SCH_3$ | $CH_2$-$CH_2$O- | 2, 6-$Cℓ_2$ | 0 | |
| III-365 | =O | | $NHCH_3$ | $CH_2$-$CH_2$O- | 2, 6-$Cℓ_2$ | 0 | |
| III-366 | =O | | $CF_2CF_3$ | -CH=CH-O- | 2-Cℓ | 0 | |
| III-367 | =O | | Et | -CH=CH-O- | 2-Cℓ | 0 | |
| III-368 | =O | | i-Pr | -CH=CH-O- | 2-Cℓ | 0 | |
| III-369 | =O | | Cℓ | -CH=CH-O- | 2-Cℓ | 0 | |
| III-370 | =O | | $OCH_3$ | -CH=CH-O- | 2-Cℓ | 0 | |
| III-371 | =O | | $SCH_3$ | -CH=CH-O- | 2-Cℓ | 0 | |
| III-372 | =O | | $NHCH_3$ | -CH=CH-O- | 2-Cℓ | 0 | |
| III-373 | =O | | $CF_3$ | -CH=CH-O- | 2-Cℓ, 6-F | 0 | |
| III-374 | =O | | Et | -CH=CH-O- | 2-Cℓ, 6-F | 0 | |
| III-375 | =O | | i-Pr | -CH=CH-O- | 2-Cℓ, 6-F | 0 | |
| III-376 | =O | | Cℓ | -CH=CH-O- | 2-Cℓ, 6-F | 0 | |
| III-377 | =O | | $OCH_3$ | -CH=CH-O- | 2-Cℓ, 6-F | 0 | |

| no. | $R^1$ | $R^2$ | $R^3$ | A-B-D-(E)- | $R^6$, $R^7$, $R^8$ | n | Physical Constants |
|---|---|---|---|---|---|---|---|
| III-378 | =O | | $SCH_3$ | -CH=CH-O- | 2-Cℓ, 6-F | 0 | |
| III-379 | =O | | $NHCH_3$ | -CH=CH-O- | 2-Cℓ, 6-F | 0 | |
| III-380 | =O | | $CF_3$ | -CH=CH-O- | 2, 6-$Cℓ_2$ | 0 | |
| III-381 | =O | | Et | -CH=CH-O- | 2, 6-$Cℓ_2$ | 0 | |
| III-382 | =O | | i-Pr | -CH=CH-O- | 2, 6-$Cℓ_2$ | 0 | |
| III-383 | =O | | Cℓ | -CH=CH-O- | 2, 6-$Cℓ_2$ | 0 | |
| III-384 | =O | | $OCH_3$ | -CH=CH-O- | 2, 6-$Cℓ_2$ | 0 | |
| III-385 | =O | | $SCH_3$ | -CH=CH-O- | 2, 6-$Cℓ_2$ | 0 | |
| III-386 | =O | | $NHCH_3$ | -CH=CH-O- | 2, 6-$Cℓ_2$ | 0 | |
| III-387 | =O | | $CF_2CF_3$ | $-CH_2$ S $CH_2-$ | 2-Cℓ | 0 | |
| III-388 | =O | | Et | $-CH_2$ S $CH_2-$ | 2-Cℓ | 0 | |
| III-389 | =O | | i-Pr | $-CH_2$ S $CH_2-$ | 2-Cℓ | 0 | |
| III-390 | =O | | Cℓ | $-CH_2$ S $CH_2-$ | 2-Cℓ | 0 | |
| III-391 | =O | | $OCH_3$ | $-CH_2$ S $CH_2-$ | 2-Cℓ | 0 | |
| III-392 | =O | | $SCH_3$ | $-CH_2$ S $CH_2-$ | 2-Cℓ | 0 | |
| III-393 | =O | | $NHCH_3$ | $-CH_2$ S $CH_2-$ | 2-Cℓ | 0 | |
| III-394 | =O | | $CF_3$ | $-CH_2$ S $CH_2-$ | 2-Cℓ, 6-F | 0 | |
| III-395 | =O | | Et | $-CH_2$ S $CH_2-$ | 2-Cℓ, 6-F | 0 | |
| III-396 | =O | | i-Pr | $-CH_2$ S $CH_2-$ | 2-Cℓ, 6-F | 0 | |
| III-397 | =O | | Cℓ | $-CH_2$ S $CH_2-$ | 2-Cℓ, 6-F | 0 | |
| III-398 | =O | | $OCH_3$ | $-CH_2$ S $CH_2-$ | 2-Cℓ, 6-F | 0 | |
| III-399 | =O | | $SCH_3$ | $-CH_2$ S $CH_2-$ | 2-Cℓ, 6-F | 0 | |
| III-400 | =O | | $NHCH_3$ | $-CH_2$ S $CH_2-$ | 2-Cℓ, 6-F | 0 | |
| III-401 | =O | | $CF_3$ | $-CH_2$ S $CH_2-$ | 2, 6-$Cℓ_2$ | 0 | |
| III-402 | =O | | Et | $-CH_2$ S $CH_2-$ | 2, 6-$Cℓ_2$ | 0 | |

| no. | $R^1$ | $R^2$ | $R^3$ | A-B-D-(E)- | $R^6, R^7, R^8$ | n | Physical Constants |
|---|---|---|---|---|---|---|---|
| III-403 | =O | | i-Pr | $-CH_2\ S\ CH_2-$ | 2,6-$Cl_2$ | 0 | |
| III-404 | =O | | Cl | $-CH_2\ S\ CH_2-$ | 2,6-$Cl_2$ | 0 | |
| III-405 | =O | | $OCH_3$ | $-CH_2\ S\ CH_2-$ | 2,6-$Cl_2$ | 0 | |
| III-406 | =O | | $SCH_3$ | $-CH_2\ S\ CH_2-$ | 2,6-$Cl_2$ | 0 | |
| III-407 | =O | | $NHCH_3$ | $-CH_2\ S\ CH_2-$ | 2,6-$Cl_2$ | 0 | |
| III-408 | =O | | $CF_2CF_3$ | $-CH_2CH_2S-$ | 2-Cl | 0 | |
| III-409 | =O | | Et | $-CH_2CH_2S-$ | 2-Cl | 0 | |
| III-410 | =O | | i-Pr | $-CH_2CH_2S-$ | 2-Cl | 0 | |
| III-411 | =O | | Cl | $-CH_2CH_2S-$ | 2-Cl | 0 | |
| III-412 | =O | | $OCH_3$ | $-CH_2CH_2S-$ | 2-Cl | 0 | |
| III-413 | =O | | $SCH_3$ | $-CH_2CH_2S-$ | 2-Cl | 0 | |
| III-414 | =O | | $NHCH_3$ | $-CH_2CH_2S-$ | 2-Cl | 0 | |
| III-415 | =O | | $CF_3$ | $-CH_2CH_2S-$ | 2-Cl, 6-F | 0 | |
| III-416 | =O | | Et | $-CH_2CH_2S-$ | 2-Cl, 6-F | 0 | |
| III-417 | =O | | i-Pr | $-CH_2CH_2S-$ | 2-Cl, 6-F | 0 | |
| III-418 | =O | | Cl | $-CH_2CH_2S-$ | 2-Cl, 6-F | 0 | |
| III-419 | =O | | $OCH_3$ | $-CH_2CH_2S-$ | 2-Cl, 6-F | 0 | |
| III-420 | =O | | $SCH_3$ | $-CH_2CH_2S-$ | 2-Cl, 6-F | 0 | |
| III-421 | =O | | $NHCH_3$ | $-CH_2CH_2S-$ | 2-Cl, 6-F | 0 | |
| III-422 | =O | | $CF_3$ | $-CH_2CH_2S-$ | 2,6-$Cl_2$ | 0 | |
| III-423 | =O | | Et | $-CH_2CH_2S-$ | 2,6-$Cl_2$ | 0 | |
| III-424 | =O | | i-Pr | $-CH_2CH_2S-$ | 2,6-$Cl_2$ | 0 | |
| III-425 | =O | | Cl | $-CH_2CH_2S-$ | 2,6-$Cl_2$ | 0 | |
| III-426 | =O | | $OCH_3$ | $-CH_2CH_2S-$ | 2,6-$Cl_2$ | 0 | |
| III-427 | =O | | $SCH_3$ | $-CH_2CH_2S-$ | 2,6-$Cl_2$ | 0 | |

| no. | $R^1$ | $R^2$ | $R^3$ | A-B-D-(E)- | $R^6, R^7, R^8$ | n | Physical Constants |
|---|---|---|---|---|---|---|---|
| III-428 | =O | | $NHCH_3$ | $-CH_2$ S $CH_2-$ | $2,6-C\ell_2$ | 0 | |
| III-429 | =O | | $CF_2CF_3$ | -CH=CH-S- | 2-Cℓ | 0 | |
| III-430 | =O | | Et | -CH=CH-S- | 2-Cℓ | 0 | |
| III-431 | =O | | i-Pr | -CH=CH-S- | 2-Cℓ | 0 | |
| III-432 | =O | | Cℓ | -CH=CH-S- | 2-Cℓ | 0 | [141-144] |
| III-433 | =O | | $OCH_3$ | -CH=CH-S- | 2-Cℓ | 0 | [137-138] |
| III-434 | =O | | $SCH_3$ | -CH=CH-S- | 2-Cℓ | 0 | [146-148] |
| III-435 | =O | | $NHCH_3$ | -CH=CH-S- | 2-Cℓ | 0 | |
| III-436 | =O | | $CF_3$ | -CH=CH-S- | 2-Cℓ, 6-F | 0 | |
| III-437 | =O | | Et | -CH=CH-S- | 2-Cℓ, 6-F | 0 | |
| III-438 | =O | | i-Pr | -CH=CH-S- | 2-Cℓ, 6-F | 0 | |
| III-439 | =O | | Cℓ | -CH=CH-S- | 2-Cℓ, 6-F | 0 | |
| III-440 | =O | | $OCH_3$ | -CH=CH-S- | 2-Cℓ, 6-F | 0 | |
| III-441 | =O | | $SCH_3$ | -CH=CH-S- | 2-Cℓ, 6-F | 0 | |
| III-442 | =O | | $NHCH_3$ | -CH=CH-S- | 2-Cℓ, 6-F | 0 | |
| III-443 | =O | | $CF_3$ | -CH=CH-S- | $2,6-C\ell_2$ | 0 | |
| III-444 | =O | | Et | -CH=CH-S- | $2,6-C\ell_2$ | 0 | |
| III-445 | =O | | i-Pr | -CH=CH-S- | $2,6-C\ell_2$ | 0 | |
| III-446 | =O | | Cℓ | -CH=CH-S- | $2,6-C\ell_2$ | 0 | |
| III-447 | =O | | $OCH_3$ | -CH=CH-S- | $2,6-C\ell_2$ | 0 | |
| III-448 | =O | | $SCH_3$ | -CH=CH-S- | $2,6-C\ell_2$ | 0 | |
| III-449 | =O | | $NHCH_3$ | -CH=CH-S- | $2,6-C\ell_2$ | 0 | |
| III-450 | =O | | $CF_2CF_3$ | $-CH_2N(CH_3)CH_2-$ | 2-Cℓ | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| III-451 | =O | | Et | $-CH_2\ N\ CH_2-$<br>\|<br>$CH_3$ | 2-Cℓ | 0 | |
| III-452 | =O | | i-Pr | $-CH_2\ N\ CH_2-$<br>\|<br>$CH_3$ | 2-Cℓ | 0 | |
| III-453 | =O | | Cℓ | $-CH_2\ N\ CH_2-$<br>\|<br>$CH_3$ | 2-Cℓ | 0 | |
| III-454 | =O | | $OCH_3$ | $-CH_2\ N\ CH_2-$<br>\|<br>$CH_3$ | 2-Cℓ | 0 | |
| III-455 | =O | | $SCH_3$ | $-CH_2\ N\ CH_2-$<br>\|<br>$CH_3$ | 2-Cℓ | 0 | |
| III-456 | =O | | $NHCH_3$ | $-CH_2\ N\ CH_2-$<br>\|<br>$CH_3$ | 2-Cℓ | 0 | |
| III-457 | =O | | $CF_3$ | $-CH_2\ N\ CH_2-$<br>\|<br>$CH_3$ | 2-Cℓ, 6-F | 0 | |
| III-458 | =O | | Et | $-CH_2\ N\ CH_2-$<br>\|<br>$CH_3$ | 2-Cℓ, 6-F | 0 | |
| III-459 | =O | | i-Pr | $-CH_2\ N\ CH_2-$<br>\|<br>$CH_3$ | 2-Cℓ, 6-F | 0 | |
| III-460 | =O | | Cℓ | $-CH_2\ N\ CH_2-$<br>\|<br>$CH_3$ | 2-Cℓ, 6-F | 0 | |
| III-461 | =O | | $OCH_3$ | $-CH_2\ N\ CH_2-$<br>\|<br>$CH_3$ | 2-Cℓ, 6-F | 0 | |
| III-462 | =O | | $SCH_3$ | $-CH_2\ N\ CH_2-$<br>\|<br>$CH_3$ | 2-Cℓ, 6-F | 0 | |

| No. | $R^1$ | $R^2$ | $R^3$ | A-B-D-(E)- | $R^6, R^7, R^8$ | n | Physical Constants |
|---|---|---|---|---|---|---|---|
| III-463 | =O | | $NHCH_3$ | $-CH_2N(CH_3)CH_2-$ | 2-Cℓ, 6-F | 0 | |
| III-464 | =O | | $CF_3$ | $-CH_2N(CH_3)CH_2-$ | 2, 6-Cℓ$_2$ | 0 | |
| III-465 | =O | | Et | $-CH_2N(CH_3)CH_2-$ | 2, 6-Cℓ$_2$ | 0 | |
| III-466 | =O | | i-Pr | $-CH_2N(CH_3)CH_2-$ | 2, 6-Cℓ$_2$ | 0 | |
| III-467 | =O | | Cℓ | $-CH_2N(CH_3)CH_2-$ | 2, 6-Cℓ$_2$ | 0 | |
| III-468 | =O | | $OCH_3$ | $-CH_2N(CH_3)CH_2-$ | 2, 6-Cℓ$_2$ | 0 | |
| III-469 | =O | | $SCH_3$ | $-CH_2N(CH_3)CH_2-$ | 2, 6-Cℓ$_2$ | 0 | |
| III-470 | =O | | $NHCH_3$ | $-CH_2N(CH_3)CH_2-$ | 2, 6-Cℓ$_2$ | 0 | |
| III-471 | =O | | $CF_2CF_3$ | $-CH_2CH_2N(CH_3)-$ | 2-Cℓ | 0 | |
| III-472 | =O | | Et | $-CH_2CH_2N(CH_3)-$ | 2-Cℓ | 0 | |
| III-473 | =O | | i-Pr | $-CH_2CH_2N(CH_3)-$ | 2-Cℓ | 0 | |
| III-474 | =O | | Cℓ | $-CH_2CH_2N(CH_3)-$ | 2-Cℓ | 0 | |
| III-475 | =O | | $OCH_3$ | $-CH_2CH_2N(CH_3)-$ | 2-Cℓ | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| III-476 | =O | | $SCH_3$ | $-CH_2CH_2N-$<br>\|<br>$CH_3$ | 2-Cl | 0 | |
| III-477 | =O | | $NHCH_3$ | $-CH_2CH_2N-$<br>\|<br>$CH_3$ | 2-Cl | 0 | |
| III-478 | =O | | $CF_3$ | $-CH_2CH_2N-$<br>\|<br>$CH_3$ | 2-Cl, 6-F | 0 | |
| III-479 | =O | | Et | $-CH_2CH_2N-$<br>\|<br>$CH_3$ | 2-Cl, 6-F | 0 | |
| III-480 | =O | | i-Pr | $-CH_2CH_2N-$<br>\|<br>$CH_3$ | 2-Cl, 6-F | 0 | |
| III-481 | =O | | Cl | $-CH_2CH_2N-$<br>\|<br>$CH_3$ | 2-Cl, 6-F | 0 | |
| III-482 | =O | | $OCH_3$ | $-CH_2CH_2N-$<br>\|<br>$CH_3$ | 2-Cl, 6-F | 0 | |
| III-483 | =O | | $SCH_3$ | $-CH_2CH_2N-$<br>\|<br>$CH_3$ | 2-Cl, 6-F | 0 | |
| III-484 | =O | | $NHCH_3$ | $-CH_2CH_2N-$<br>\|<br>$CH_3$ | 2-Cl, 6-F | 0 | |
| III-485 | =O | | $CF_3$ | $-CH_2CH_2N-$<br>\|<br>$CH_3$ | 2,6-$Cl_2$ | 0 | |
| III-486 | =O | | Et | $-CH_2CH_2N-$<br>\|<br>$CH_3$ | 2,6-$Cl_2$ | 0 | |
| III-487 | =O | | i-Pr | $-CH_2CH_2N-$<br>\|<br>$CH_3$ | 2,6-$Cl_2$ | 0 | |
| III-488 | =O | | Cl | $-CH_2CH_2N-$<br>\|<br>$CH_3$ | 2,6-$Cl_2$ | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| III-489 | =O | | $OCH_3$ | $-CH_2CH_2N-$<br>\|<br>$CH_3$ | 2,6-Cℓ$_2$ | 0 | |
| III-490 | =O | | $SCH_3$ | $-CH_2CH_2N-$<br>\|<br>$CH_3$ | 2,6-Cℓ$_2$ | 0 | |
| III-491 | =O | | $NHCH_3$ | $-CH_2CH_2N-$<br>\|<br>$CH_3$ | 2,6-Cℓ$_2$ | 0 | |
| III-492 | =O | | $CF_2CF_3$ | -CH=CH-N-<br>\|<br>$CH_3$ | 2-Cℓ | 0 | |
| III-493 | =O | | Et | -CH=CH-N-<br>\|<br>$CH_3$ | 2-Cℓ | 0 | |
| III-494 | =O | | i-Pr | -CH=CH-N-<br>\|<br>$CH_3$ | 2-Cℓ | 0 | |
| III-495 | =O | | Cℓ | -CH=CH-N-<br>\|<br>$CH_3$ | 2-Cℓ | 0 | |
| III-496 | =O | | $OCH_3$ | -CH=CH-N-<br>\|<br>$CH_3$ | 2-Cℓ | 0 | |
| III-497 | =O | | $SCH_3$ | -CH=CH-N-<br>\|<br>$CH_3$ | 2-Cℓ | 0 | |
| III-498 | =O | | $NHCH_3$ | -CH=CH-N-<br>\|<br>$CH_3$ | 2-Cℓ | 0 | |
| III-499 | =O | | $CF_3$ | -CH=CH-N-<br>\|<br>$CH_3$ | 2-Cℓ, 6-F | 0 | |
| III-500 | =O | | Et | -CH=CH-N-<br>\|<br>$CH_3$ | 2-Cℓ, 6-F | 0 | |
| III-501 | =O | | i-Pr | -CH=CH-N-<br>\|<br>$CH_3$ | 2-Cℓ, 6-F | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| III-502 | =O | | Cℓ | -CH=CH-N($CH_3$)- | 2-Cℓ, 6-F | 0 | |
| III-503 | =O | | $OCH_3$ | -CH=CH-N($CH_3$)- | 2-Cℓ, 6-F | 0 | |
| III-504 | =O | | $SCH_3$ | -CH=CH-N($CH_3$)- | 2-Cℓ, 6-F | 0 | |
| III-505 | =O | | $NHCH_3$ | -CH=CH-N($CH_3$)- | 2-Cℓ, 6-F | 0 | |
| III-506 | =O | | $CF_3$ | -CH=CH-N($CH_3$)- | 2, 6-$Cℓ_2$ | 0 | |
| III-507 | =O | | Et | -CH=CH-N($CH_3$)- | 2, 6-$Cℓ_2$ | 0 | |
| III-508 | =O | | i-Pr | -CH=CH-N($CH_3$)- | 2, 6-$Cℓ_2$ | 0 | |
| III-509 | =O | | Cℓ | -CH=CH-N($CH_3$)- | 2, 6-$Cℓ_2$ | 0 | |
| III-510 | =O | | $OCH_3$ | -CH=CH-N($CH_3$)- | 2, 6-$Cℓ_2$ | 0 | |
| III-511 | =O | | $SCH_3$ | -CH=CH-N($CH_3$)- | 2, 6-$Cℓ_2$ | 0 | |
| III-512 | =O | | $NHCH_3$ | -CH=CH-N($CH_3$)- | 2, 6-$Cℓ_2$ | 0 | |
| III-513 | =O | | $CF_2CF_3$ | $-CH_2CH_2CH_2S-$ | 2-Cℓ | 0 | |
| III-514 | =O | | Et | $-CH_2CH_2CH_2S-$ | 2-Cℓ | 0 | |
| III-515 | =O | | i-Pr | $-CH_2CH_2CH_2S-$ | 2-Cℓ | 0 | |
| III-516 | =O | | Cℓ | $-CH_2CH_2CH_2S-$ | 2-Cℓ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-517 | =O | | $OCH_3$ | $-CH_2CH_2CH_2S-$ | 2-Cℓ | 0 | |
| III-518 | =O | | $SCH_3$ | $-CH_2CH_2CH_2S-$ | 2-Cℓ | 0 | |
| III-519 | =O | | $NHCH_3$ | $-CH_2CH_2CH_2S-$ | 2-Cℓ | 0 | |
| III-520 | =O | | $CF_3$ | $-CH_2CH_2CH_2S-$ | 2-Cℓ, 6-F | 0 | |
| III-521 | =O | | Et | $-CH_2CH_2CH_2S-$ | 2-Cℓ, 6-F | 0 | |
| III-522 | =O | | i-Pr | $-CH_2CH_2CH_2S-$ | 2-Cℓ, 6-F | 0 | |
| III-523 | =O | | Cℓ | $-CH_2CH_2CH_2S-$ | 2-Cℓ, 6-F | 0 | |
| III-524 | =O | | $OCH_3$ | $-CH_2CH_2CH_2S-$ | 2-Cℓ, 6-F | 0 | |
| III-525 | =O | | $SCH_3$ | $-CH_2CH_2CH_2S-$ | 2-Cℓ, 6-F | 0 | |
| III-526 | =O | | $NHCH_3$ | $-CH_2CH_2CH_2S-$ | 2-Cℓ, 6-F | 0 | |
| III-527 | =O | | $CF_3$ | $-CH_2CH_2CH_2S-$ | 2, 6-Cℓ$_2$ | 0 | |
| III-528 | =O | | Et | $-CH_2CH_2CH_2S-$ | 2, 6-Cℓ$_2$ | 0 | |
| III-529 | =O | | i-Pr | $-CH_2CH_2CH_2S-$ | 2, 6-Cℓ$_2$ | 0 | |
| III-530 | =O | | Cℓ | $-CH_2CH_2CH_2S-$ | 2, 6-Cℓ$_2$ | 0 | |
| III-531 | =O | | $OCH_3$ | $-CH_2CH_2CH_2S-$ | 2, 6-Cℓ$_2$ | 0 | |
| III-532 | =O | | $SCH_3$ | $-CH_2CH_2CH_2S-$ | 2, 6-Cℓ$_2$ | 0 | |
| III-533 | =O | | $NHCH_3$ | $-CH_2CH_2CH_2S-$ | 2, 6-Cℓ$_2$ | 0 | |
| III-534 | =O | | $CF_3$ | $-CH_2CH_2CH_2S-$ | 2-Cℓ | 0 | |
| III-535 | =O | | Et | $-CH_2CH_2CH_2S-$ | 2-Cℓ | 0 | |
| III-536 | =O | | i-Pr | $-CH_2CH_2CH_2S-$ | 2-Cℓ | 0 | |
| III-537 | =O | | Cℓ | $-CH_2CH_2CH_2S-$ | 2-Cℓ | 0 | |
| III-538 | =O | | $OCH_3$ | $-CH_2CH_2CH_2S-$ | 2-Cℓ | 0 | |
| III-539 | =O | | $SCH_3$ | $-CH_2CH_2CH_2S-$ | 2-Cℓ | 0 | |
| III-540 | =O | | $NHCH_3$ | $-CH_2CH_2CH_2S-$ | 2-Cℓ | 0 | |
| III-541 | =O | | $CF_3$ | $-CH_2CH_2CH_2S-$ | 2-Cℓ, 6-F | 0 | |
| III-542 | =O | | Et | $-CH_2CH_2CH_2S-$ | 2-Cℓ, 6-F | 0 | |

| No. | $R^1$ | $R^2$ | $R^3$ | A-B-D-(E)- | $R^6, R^7, R^8$ | n | Physical Constants |
|---|---|---|---|---|---|---|---|
| III-543 | =O | | i-Pr | $-CH_2CH_2CH_2S-$ | 2-Cℓ, 6-F | 0 | |
| III-544 | =O | | Cℓ | $-CH_2CH_2CH_2S-$ | 2-Cℓ, 6-F | 0 | |
| III-545 | =O | | $OCH_3$ | $-CH_2CH_2CH_2S-$ | 2-Cℓ, 6-F | 0 | |
| III-546 | =O | | $SCH_3$ | $-CH_2CH_2CH_2S-$ | 2-Cℓ, 6-F | 0 | |
| III-547 | =O | | $NHCH_3$ | $-CH_2CH_2CH_2S-$ | 2-Cℓ, 6-F | 0 | |
| III-548 | =O | | $CF_3$ | $-CH_2CH_2CH_2S-$ | 2, 6-$Cℓ_2$ | 0 | |
| III-549 | =O | | Et | $-CH_2CH_2CH_2S-$ | 2, 6-$Cℓ_2$ | 0 | |
| III-550 | =O | | i-Pr | $-CH_2CH_2CH_2S-$ | 2, 6-$Cℓ_2$ | 0 | |
| III-551 | =O | | Cℓ | $-CH_2CH_2CH_2S-$ | 2, 6-$Cℓ_2$ | 0 | |
| III-552 | =O | | $OCH_3$ | $-CH_2CH_2CH_2S-$ | 2, 6-$Cℓ_2$ | 0 | |
| III-553 | =O | | $SCH_3$ | $-CH_2CH_2CH_2S-$ | 2, 6-$Cℓ_2$ | 0 | |
| III-554 | =O | | $NHCH_3$ | $-CH_2CH_2CH_2S-$ | 2, 6-$Cℓ_2$ | 0 | |
| III-555 | =O | | $CF_2CF_3$ | $-CH_2SCH_2CH_2-$ | 2-Cℓ | 0 | |
| III-556 | =O | | Et | $-CH_2SCH_2CH_2-$ | 2-Cℓ | 0 | |
| III-557 | =O | | i-Pr | $-CH_2SCH_2CH_2-$ | 2-Cℓ | 0 | |
| III-558 | =O | | Cℓ | $-CH_2SCH_2CH_2-$ | 2-Cℓ | 0 | |
| III-559 | =O | | $OCH_3$ | $-CH_2SCH_2CH_2-$ | 2-Cℓ | 0 | |
| III-560 | =O | | $SCH_3$ | $-CH_2SCH_2CH_2-$ | 2-Cℓ | 0 | |
| III-561 | =O | | $NHCH_3$ | $-CH_2SCH_2CH_2-$ | 2-Cℓ | 0 | |
| III-562 | =O | | $CF_3$ | $-CH_2SCH_2CH_2-$ | 2-Cℓ, 6-F | 0 | |
| III-563 | =O | | Et | $-CH_2SCH_2CH_2-$ | 2-Cℓ, 6-F | 0 | |
| III-564 | =O | | i-Pr | $-CH_2SCH_2CH_2-$ | 2-Cℓ, 6-F | 0 | |
| III-565 | =O | | Cℓ | $-CH_2SCH_2CH_2-$ | 2-Cℓ, 6-F | 0 | |
| III-566 | =O | | $OCH_3$ | $-CH_2SCH_2CH_2-$ | 2-Cℓ, 6-F | 0 | |
| III-567 | =O | | $SCH_3$ | $-CH_2SCH_2CH_2-$ | 2-Cℓ, 6-F | 0 | |
| III-568 | =O | | $NHCH_3$ | $-CH_2SCH_2CH_2-$ | 2-Cℓ, 6-F | 0 | |

| No. | $R^1$ | $R^2$ | $R^3$ | A-B-D-(E)- | $R^6, R^7, R^8$ | n | Physical Constants |
|---|---|---|---|---|---|---|---|
| Ⅲ-569 | =0 | | $CF_3$ | $-CH_2SCH_2CH_2-$ | 2, 6-$C\ell_2$ | 0 | |
| Ⅲ-570 | =0 | | Et | $-CH_2SCH_2CH_2-$ | 2, 6-$C\ell_2$ | 0 | |
| Ⅲ-571 | =0 | | i-Pr | $-CH_2SCH_2CH_2-$ | 2, 6-$C\ell_2$ | 0 | |
| Ⅲ-572 | =0 | | $C\ell$ | $-CH_2SCH_2CH_2-$ | 2, 6-$C\ell_2$ | 0 | |
| Ⅲ-573 | =0 | | $OCH_3$ | $-CH_2SCH_2CH_2-$ | 2, 6-$C\ell_2$ | 0 | |
| Ⅲ-574 | =0 | | $SCH_3$ | $-CH_2SCH_2CH_2-$ | 2, 6-$C\ell_2$ | 0 | |
| Ⅲ-575 | =0 | | $NHCH_3$ | $-CH_2SCH_2CH_2-$ | 2, 6-$C\ell_2$ | 0 | |
| Ⅲ-576 | =0 | | $CF_2CF_3$ | $-CH_2CH_2CH_2O-$ | 2-$C\ell$ | 0 | |
| Ⅲ-577 | =0 | | Et | $-CH_2CH_2CH_2O-$ | 2-$C\ell$ | 0 | |
| Ⅲ-578 | =0 | | i-Pr | $-CH_2CH_2CH_2O-$ | 2-$C\ell$ | 0 | |
| Ⅲ-579 | =0 | | $C\ell$ | $-CH_2CH_2CH_2O-$ | 2-$C\ell$ | 0 | |
| Ⅲ-580 | =0 | | $OCH_3$ | $-CH_2CH_2CH_2O-$ | 2-$C\ell$ | 0 | |
| Ⅲ-581 | =0 | | $SCH_3$ | $-CH_2CH_2CH_2O-$ | 2-$C\ell$ | 0 | |
| Ⅲ-582 | =0 | | $NHCH_3$ | $-CH_2CH_2CH_2O-$ | 2-$C\ell$ | 0 | |
| Ⅲ-583 | =0 | | $CF_3$ | $-CH_2CH_2CH_2O-$ | 2-$C\ell$, 6-F | 0 | |
| Ⅲ-584 | =0 | | Et | $-CH_2CH_2CH_2O-$ | 2-$C\ell$, 6-F | 0 | |
| Ⅲ-585 | =0 | | i-Pr | $-CH_2CH_2CH_2O-$ | 2-$C\ell$, 6-F | 0 | |
| Ⅲ-586 | =0 | | $C\ell$ | $-CH_2CH_2CH_2O-$ | 2-$C\ell$, 6-F | 0 | |
| Ⅲ-587 | =0 | | $OCH_3$ | $-CH_2CH_2CH_2O-$ | 2-$C\ell$, 6-F | 0 | |
| Ⅲ-588 | =0 | | $SCH_3$ | $-CH_2CH_2CH_2O-$ | 2-$C\ell$, 6-F | 0 | |
| Ⅲ-589 | =0 | | $NHCH_3$ | $-CH_2CH_2CH_2O-$ | 2-$C\ell$, 6-F | 0 | |
| Ⅲ-590 | =0 | | $CF_3$ | $-CH_2CH_2CH_2O-$ | 2, 6-$C\ell_2$ | 0 | |
| Ⅲ-591 | =0 | | Et | $-CH_2CH_2CH_2O-$ | 2, 6-$C\ell_2$ | 0 | |
| Ⅲ-592 | =0 | | i-Pr | $-CH_2CH_2CH_2O-$ | 2, 6-$C\ell_2$ | 0 | |
| Ⅲ-593 | =0 | | $C\ell$ | $-CH_2CH_2CH_2O-$ | 2, 6-$C\ell_2$ | 0 | |
| Ⅲ-594 | =0 | | $OCH_3$ | $-CH_2CH_2CH_2O-$ | 2, 6-$C\ell_2$ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-595 | =0 | | $SCH_3$ | $-CH_2CH_2CH_2O-$ | 2, 6-$Cl_2$ | 0 | |
| III-596 | =0 | | $NHCH_3$ | $-CH_2CH_2CH_2O-$ | 2, 6-$Cl_2$ | 0 | |
| III-597 | =0 | | $CF_3$ | $-CH_2CH_2OCH_2-$ | 2-Cl | 0 | |
| III-598 | =0 | | Et | $-CH_2CH_2OCH_2-$ | 2-Cl | 0 | |
| III-599 | =0 | | i-Pr | $-CH_2CH_2OCH_2-$ | 2-Cl | 0 | |
| III-600 | =0 | | Cl | $-CH_2CH_2OCH_2-$ | 2-Cl | 0 | |
| III-601 | =0 | | $OCH_3$ | $-CH_2CH_2OCH_2-$ | 2-Cl | 0 | |
| III-602 | =0 | | $SCH_3$ | $-CH_2CH_2OCH_2-$ | 2-Cl | 0 | |
| III-603 | =0 | | $NHCH_3$ | $-CH_2CH_2OCH_2-$ | 2-Cl | 0 | |
| III-604 | =0 | | $CF_3$ | $-CH_2CH_2OCH_2-$ | 2-Cl, 6-F | 0 | |
| III-605 | =0 | | Et | $-CH_2CH_2OCH_2-$ | 2-Cl, 6-F | 0 | |
| III-606 | =0 | | i-Pr | $-CH_2CH_2OCH_2-$ | 2-Cl, 6-F | 0 | |
| III-607 | =0 | | Cl | $-CH_2CH_2OCH_2-$ | 2-Cl, 6-F | 0 | |
| III-608 | =0 | | $OCH_3$ | $-CH_2CH_2OCH_2-$ | 2-Cl, 6-F | 0 | |
| III-609 | =0 | | $SCH_3$ | $-CH_2CH_2OCH_2-$ | 2-Cl, 6-F | 0 | |
| III-610 | =0 | | $NHCH_3$ | $-CH_2CH_2OCH_2-$ | 2-Cl, 6-F | 0 | |
| III-611 | =0 | | $CF_3$ | $-CH_2CH_2OCH_2-$ | 2, 6-$Cl_2$ | 0 | |
| III-612 | =0 | | Et | $-CH_2CH_2OCH_2-$ | 2, 6-$Cl_2$ | 0 | |
| III-613 | =0 | | i-Pr | $-CH_2CH_2OCH_2-$ | 2, 6-$Cl_2$ | 0 | |
| III-614 | =0 | | Cl | $-CH_2CH_2OCH_2-$ | 2, 6-$Cl_2$ | 0 | |
| III-615 | =0 | | $OCH_3$ | $-CH_2CH_2OCH_2-$ | 2, 6-$Cl_2$ | 0 | |
| III-616 | =0 | | $SCH_3$ | $-CH_2CH_2OCH_2-$ | 2, 6-$Cl_2$ | 0 | |
| III-617 | =0 | | $NHCH_3$ | $-CH_2CH_2OCH_2-$ | 2, 6-$Cl_2$ | 0 | |
| III-618 | =0 | | $CF_2CF_3$ | $-CH_2OCH_2CH_2-$ | 2-Cl | 0 | |
| III-619 | =0 | | Et | $-CH_2OCH_2CH_2-$ | 2-Cl | 0 | |
| III-620 | =0 | | i-Pr | $-CH_2OCH_2CH_2-$ | 2-Cl | 0 | |

| No. | $R^1$ | $R^2$ | $R^3$ | A-B-D-(E)- | $R^6, R^7, R^8$ | n | Physical Constants |
|---|---|---|---|---|---|---|---|
| III-621 | =O | | Cℓ | $-CH_2OCH_2CH_2-$ | 2-Cℓ | 0 | |
| III-622 | =O | | $OCH_3$ | $-CH_2OCH_2CH_2-$ | 2-Cℓ | 0 | |
| III-623 | =O | | $SCH_3$ | $-CH_2OCH_2CH_2-$ | 2-Cℓ | 0 | |
| III-624 | =O | | $NHCH_3$ | $-CH_2OCH_2CH_2-$ | 2-Cℓ | 0 | |
| III-625 | =O | | $CF_3$ | $-CH_2OCH_2CH_2-$ | 2-Cℓ, 6-F | 0 | |
| III-626 | =O | | Et | $-CH_2OCH_2CH_2-$ | 2-Cℓ, 6-F | 0 | |
| III-627 | =O | | i-Pr | $-CH_2OCH_2CH_2-$ | 2-Cℓ, 6-F | 0 | |
| III-628 | =O | | Cℓ | $-CH_2OCH_2CH_2-$ | 2-Cℓ, 6-F | 0 | |
| III-629 | =O | | $OCH_3$ | $-CH_2OCH_2CH_2-$ | 2-Cℓ, 6-F | 0 | |
| III-630 | =O | | $SCH_3$ | $-CH_2OCH_2CH_2-$ | 2-Cℓ, 6-F | 0 | |
| III-631 | =O | | $NHCH_3$ | $-CH_2OCH_2CH_2-$ | 2-Cℓ, 6-F | 0 | |
| III-632 | =O | | $CF_3$ | $-CH_2OCH_2CH_2-$ | 2, 6-$Cℓ_2$ | 0 | |
| III-633 | =O | | Et | $-CH_2OCH_2CH_2-$ | 2, 6-$Cℓ_2$ | 0 | |
| III-634 | =O | | i-Pr | $-CH_2OCH_2CH_2-$ | 2, 6-$Cℓ_2$ | 0 | |
| III-635 | =O | | Cℓ | $-CH_2OCH_2CH_2-$ | 2, 6-$Cℓ_2$ | 0 | |
| III-636 | =O | | $OCH_3$ | $-CH_2OCH_2CH_2-$ | 2, 6-$Cℓ_2$ | 0 | |
| III-637 | =O | | $SCH_3$ | $-CH_2OCH_2CH_2-$ | 2, 6-$Cℓ_2$ | 0 | |
| III-638 | =O | | $NHCH_3$ | $-CH_2OCH_2CH_2-$ | 2, 6-$Cℓ_2$ | 0 | |
| III-639 | =O | | $CF_2CF_3$ | $-CH_2N(CH_3)CH_2CH_2-$ | 2, 6-$Cℓ_2$ | 0 | |
| III-640 | =O | | Et | $-CH_2N(CH_3)CH_2CH_2-$ | 2-Cℓ | 0 | |
| III-641 | =O | | i-Pr | $-CH_2N(CH_3)CH_2CH_2-$ | 2-Cℓ | 0 | |
| III-642 | =O | | Cℓ | $-CH_2N(CH_3)CH_2CH_2-$ | 2-Cℓ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-643 | =O | | $OCH_3$ | $-CH_2\underset{CH_3}{\underset{\mid}{N}}CH_2CH_2$ | 2-Cℓ | 0 | |
| III-644 | =O | | $SCH_3$ | $-CH_2\underset{CH_3}{\underset{\mid}{N}}CH_2CH_2$ | 2-Cℓ | 0 | |
| III-645 | =O | | $NHCH_3$ | $-CH_2\underset{CH_3}{\underset{\mid}{N}}CH_2CH_2$ | 2-Cℓ | 0 | |
| III-646 | =O | | $CF_3$ | $-CH_2\underset{CH_3}{\underset{\mid}{N}}CH_2CH_2$ | 2-Cℓ, 6-F | 0 | |
| III-647 | =O | | Et | $-CH_2\underset{CH_3}{\underset{\mid}{N}}CH_2CH_2$ | 2-Cℓ, 6-F | 0 | |
| III-648 | =O | | i-Pr | $-CH_2\underset{CH_3}{\underset{\mid}{N}}CH_2CH_2$ | 2-Cℓ, 6-F | 0 | |
| III-649 | =O | | Cℓ | $-CH_2\underset{CH_3}{\underset{\mid}{N}}CH_2CH_2$ | 2-Cℓ, 6-F | 0 | |
| III-650 | =O | | $OCH_3$ | $-CH_2\underset{CH_3}{\underset{\mid}{N}}CH_2CH_2$ | 2-Cℓ, 6-F | 0 | |
| III-651 | =O | | $SCH_3$ | $-CH_2\underset{CH_3}{\underset{\mid}{N}}CH_2CH_2$ | 2-Cℓ, 6-F | 0 | |
| III-652 | =O | | $NHCH_3$ | $-CH_2\underset{CH_3}{\underset{\mid}{N}}CH_2CH_2$ | 2-Cℓ, 6-F | 0 | |
| III-653 | =O | | $CF_3$ | $-CH_2\underset{CH_3}{\underset{\mid}{N}}CH_2CH_2$ | 2, 6-Cℓ$_2$ | 0 | |
| III-654 | =O | | Et | $-CH_2\underset{CH_3}{\underset{\mid}{N}}CH_2CH_2$ | 2, 6-Cℓ$_2$ | 0 | |
| III-655 | =O | | i-Pr | $-CH_2\underset{CH_3}{\underset{\mid}{N}}CH_2CH_2$ | 2, 6-Cℓ$_2$ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-656 | =0 | | Cℓ | $-CH_2\underset{CH_3}{N}CH_2CH_2$ | 2,6-$Cℓ_2$ | 0 | |
| III-657 | =0 | | $OCH_3$ | $-CH_2\underset{CH_3}{N}CH_2CH_2$ | 2,6-$Cℓ_2$ | 0 | |
| III-658 | =0 | | $SCH_3$ | $-CH_2\underset{CH_3}{N}CH_2CH_2$. | 2,6-$Cℓ_2$ | 0 | |
| III-659 | =0 | | $NHCH_3$ | $-CH_2\underset{CH_3}{N}CH_2CH_2$ | 2,6-$Cℓ_2$ | 0 | |
| III-660 | =0 | | $CF_2CF_3$ | $-CH_2\underset{H}{N}CH_2CH_2-$ | 2-Cℓ | 0 | |
| III-661 | =0 | | Et | $-CH_2\underset{H}{N}CH_2CH_2-$ | 2-Cℓ | 0 | |
| III-662 | =0 | | i-Pr | $-CH_2\underset{H}{N}CH_2CH_2-$ | 2-Cℓ | 0 | |
| III-663 | =0 | | Cℓ | $-CH_2\underset{H}{N}CH_2CH_2-$ | 2-Cℓ | 0 | |
| III-664 | =0 | | $OCH_3$ | $-CH_2\underset{H}{N}CH_2CH_2-$ | 2-Cℓ | 0 | |
| III-665 | =0 | | $SCH_3$ | $-CH_2\underset{H}{N}CH_2CH_2-$ | 2-Cℓ | 0 | |
| III-666 | =0 | | $NHCH_3$ | $-CH_2\underset{H}{N}CH_2CH_2-$ | 2-Cℓ | 0 | |
| III-667 | =0 | | $CF_3$ | $-CH_2\underset{H}{N}CH_2CH_2-$ | 2-Cℓ,6-F | 0 | |
| III-668 | =0 | | Et | $-CH_2\underset{H}{N}CH_2CH_2-$ | 2-Cℓ,6-F | 0 | |
| III-669 | =0 | | i-Pr | $-CH_2\underset{H}{N}CH_2CH_2-$ | 2-Cℓ,6-F | 0 | |
| III-670 | =0 | | Cℓ | $-CH_2\underset{H}{N}CH_2CH_2-$ | 2-Cℓ,6-F | 0 | |
| III-671 | =0 | | $OCH_3$ | $-CH_2\underset{H}{N}CH_2CH_2-$ | 2-Cℓ,6-F | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| III-672 | =O | | $SCH_3$ | $-CH_2\underset{H}{N}CH_2CH_2-$ | 2-Cl, 6-F | 0 | |
| III-673 | =O | | $NHCH_3$ | $-CH_2\underset{H}{N}CH_2CH_2-$ | 2-Cl, 6-F | 0 | |
| III-674 | =O | | $CF_3$ | $-CH_2\underset{H}{N}CH_2CH_2-$ | 2-Cl, 6-F | 0 | |
| III-675 | =O | | Et | $-CH_2\underset{H}{N}CH_2CH_2-$ | 2,6-$Cl_2$ | 0 | |
| III-676 | =O | | i-Pr | $-CH_2\underset{H}{N}CH_2CH_2-$ | 2,6-$Cl_2$ | 0 | |
| III-677 | =O | | Cl | $-CH_2\underset{H}{N}CH_2CH_2-$ | 2,6-$Cl_2$ | 0 | |
| III-678 | =O | | $OCH_3$ | $-CH_2\underset{H}{N}CH_2CH_2-$ | 2,6-$Cl_2$ | 0 | |
| III-679 | =O | | $SCH_3$ | $-CH_2\underset{H}{N}CH_2CH_2-$ | 2,6-$Cl_2$ | 0 | |
| III-680 | =O | | $NHCH_3$ | $-CH_2\underset{H}{N}CH_2CH_2-$ | 2,6-$Cl_2$ | 0 | |
| III-681 | =O | | $CF_3$ | $-CH_2CH_2\underset{H}{N}CH_2-$ | 2-Cl | 0 | |
| III-682 | =O | | Et | $-CH_2CH_2\underset{H}{N}CH_2-$ | 2-Cl | 0 | |
| III-683 | =O | | i-Pr | $-CH_2CH_2\underset{H}{N}CH_2-$ | 2-Cl | 0 | |
| III-684 | =O | | Cl | $-CH_2CH_2\underset{H}{N}CH_2-$ | 2-Cl | 0 | |
| III-685 | =O | | $OCH_3$ | $-CH_2CH_2\underset{H}{N}CH_2-$ | 2-Cl | 0 | |
| III-686 | =O | | $SCH_3$ | $-CH_2CH_2\underset{H}{N}CH_2-$ | 2-Cl | 0 | |
| III-687 | =O | | $NHCH_3$ | $-CH_2CH_2\underset{H}{N}CH_2-$ | 2-Cl | 0 | |
| III-688 | =O | | $CF_3$ | $-CH_2CH_2\underset{H}{N}CH_2-$ | 2-Cl, 6-F | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| III-689 | =O | | Et | $-CH_2CH_2NCH_2-$<br>H | 2-Cl, 6-F | 0 | |
| III-690 | =O | | i-Pr | $-CH_2CH_2NCH_2-$<br>H | 2-Cl, 6-F | 0 | |
| III-691 | =O | | Cl | $-CH_2CH_2NCH_2-$<br>H | 2-Cl, 6-F | 0 | |
| III-692 | =O | | $OCH_3$ | $-CH_2CH_2NCH_2-$<br>H | 2-Cl, 6-F | 0 | |
| III-693 | =O | | $SCH_3$ | $-CH_2CH_2NCH_2-$<br>H | 2-Cl, 6-F | 0 | |
| III-694 | =O | | $NHCH_3$ | $-CH_2CH_2NCH_2-$<br>H | 2-Cl, 6-F | 0 | |
| III-695 | =O | | $CF_3$ | $-CH_2CH_2NCH_2-$<br>H | 2, 6-$Cl_2$ | 0 | |
| III-696 | =O | | Et | $-CH_2CH_2NCH_2-$<br>H | 2, 6-$Cl_2$ | 0 | |
| III-697 | =O | | i-Pr | $-CH_2CH_2NCH_2-$<br>H | 2, 6-$Cl_2$ | 0 | |
| III-698 | =O | | Cl | $-CH_2CH_2NCH_2-$<br>H | 2, 6-$Cl_2$ | 0 | |
| III-699 | =O | | $OCH_3$ | $-CH_2CH_2NCH_2-$<br>H | 2, 6-$Cl_2$ | 0 | |
| III-700 | =O | | $SCH_3$ | $-CH_2CH_2NCH_2-$<br>H | 2, 6-$Cl_2$ | 0 | |
| III-701 | =O | | $NHCH_3$ | $-CH_2CH_2NCH_2-$<br>H | 2, 6-$Cl_2$ | 0 | |
| III-702 | =O | | $CF_3$ | $-CH_2CH_2NCH_2-$<br>$CH_3$ | 2-Cl | 0 | |
| III-703 | =O | | Et | $-CH_2CH_2NCH_2-$<br>$CH_3$ | 2-Cl | 0 | |
| III-704 | =O | | i-Pr | $-CH_2CH_2NCH_2-$<br>$CH_3$ | 2-Cl | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| Ⅲ-705 | =O | | Cℓ | $-CH_2CH_2NCH_2-$<br>\|<br>$CH_3$ | 2-Cℓ | 0 | |
| Ⅲ-706 | =O | | $OCH_3$ | $-CH_2CH_2NCH_2-$<br>\|<br>$CH_3$ | 2-Cℓ | 0 | |
| Ⅲ-707 | =O | | $SCH_3$ | $-CH_2CH_2NCH_2-$<br>\|<br>$CH_3$ | 2-Cℓ | 0 | |
| Ⅲ-708 | =O | | $NHCH_3$ | $-CH_2CH_2NCH_2-$<br>\|<br>$CH_3$ | 2-Cℓ | 0 | |
| Ⅲ-709 | =O | | $CF_3$ | $-CH_2CH_2NCH_2-$<br>\|<br>$CH_3$ | 2-Cℓ, 6-F | 0 | |
| Ⅲ-710 | =O | | Et | $-CH_2CH_2NCH_2-$<br>\|<br>$CH_3$ | 2-Cℓ, 6-F | 0 | |
| Ⅲ-711 | =O | | i-Pr | $-CH_2CH_2NCH_2-$<br>\|<br>$CH_3$ | 2-Cℓ, 6-F | 0 | |
| Ⅲ-712 | =O | | Cℓ | $-CH_2CH_2NCH_2-$<br>\|<br>$CH_3$ | 2-Cℓ, 6-F | 0 | |
| Ⅲ-713 | =O | | $OCH_3$ | $-CH_2CH_2NCH_2-$<br>\|<br>$CH_3$ | 2-Cℓ, 6-F | 0 | |
| Ⅲ-714 | =O | | $SCH_3$ | $-CH_2CH_2NCH_2-$<br>\|<br>$CH_3$ | 2-Cℓ, 6-F | 0 | |
| Ⅲ-715 | =O | | $NHCH_3$ | $-CH_2CH_2NCH_2-$<br>\|<br>$CH_3$ | 2-Cℓ, 6-F | 0 | |
| Ⅲ-716 | =O | | $CF_3$ | $-CH_2CH_2NCH_2-$<br>\|<br>$CH_3$ | 2, 6-$Cℓ_2$ | 0 | |
| Ⅲ-717 | =O | | Et | $-CH_2CH_2NCH_2-$<br>\|<br>$CH_3$ | 2, 6-$Cℓ_2$ | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| Ⅲ-718 | =O | | i-Pr | $-CH_2CH_2NCH_2-$<br>\|<br>$CH_3$ | 2, 6-$C\ell_2$ | 0 | |
| Ⅲ-719 | =O | | $C\ell$ | $-CH_2CH_2NCH_2-$<br>\|<br>$CH_3$ | 2, 6-$C\ell_2$ | 0 | |
| Ⅲ-720 | =O | | $OCH_3$ | $-CH_2CH_2NCH_2-$<br>\|<br>$CH_3$ | 2, 6-$C\ell_2$ | 0 | |
| Ⅲ-721 | =O | | $SCH_3$ | $-CH_2CH_2NCH_2-$<br>\|<br>$CH_3$ | 2, 6-$C\ell_2$ | 0 | |
| Ⅲ-722 | =O | | $NHCH_3$ | $-CH_2CH_2NCH_2-$<br>\|<br>$CH_3$ | 2, 6-$C\ell_2$ | 0 | |
| Ⅲ-723 | =O | | Et | $-CH_2CH_2CH_2N-$<br>H | 2-$C\ell$ | 0 | |
| Ⅲ-724 | =O | | i-Pr | $-CH_2CH_2CH_2N-$<br>H | 2-$C\ell$ | 0 | |
| Ⅲ-725 | =O | | $C\ell$ | $-CH_2CH_2CH_2N-$<br>H | 2-$C\ell$ | 0 | |
| Ⅲ-726 | =O | | $OCH_3$ | $-CH_2CH_2CH_2N-$<br>H | 2-$C\ell$ | 0 | |
| Ⅲ-727 | =O | | $SCH_3$ | $-CH_2CH_2CH_2N-$<br>H | 2-$C\ell$ | 0 | |
| Ⅲ-728 | =O | | $NHCH_3$ | $-CH_2CH_2CH_2N-$<br>H | 2-$C\ell$ | 0 | |
| Ⅲ-729 | =O | | $CF_3$ | $-CH_2CH_2CH_2N-$<br>H | 2-$C\ell$, 6-F | 0 | |
| Ⅲ-730 | =O | | Et | $-CH_2CH_2CH_2N-$<br>H | 2-$C\ell$, 6-F | 0 | |
| Ⅲ-731 | =O | | i-Pr | $-CH_2CH_2CH_2N-$<br>H | 2-$C\ell$, 6-F | 0 | |
| Ⅲ-732 | =O | | $C\ell$ | $-CH_2CH_2CH_2N-$<br>H | 2-$C\ell$, 6-F | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| III-733 | =O | | $OCH_3$ | $-CH_2CH_2CH_2N-$<br>H | 2-$C\ell$, 6-F | 0 | |
| III-734 | =O | | $SCH_3$ | $-CH_2CH_2CH_2N-$<br>H | 2-$C\ell$, 6-F | 0 | |
| III-735 | =O | | $NHCH_3$ | $-CH_2CH_2CH_2N-$<br>H | 2-$C\ell$, 6-F | 0 | |
| III-736 | =O | | $CF_3$ | $-CH_2CH_2CH_2N-$<br>H | 2, 6-$C\ell_2$ | 0 | |
| III-737 | =O | | Et | $-CH_2CH_2CH_2N-$<br>H | 2, 6-$C\ell_2$ | 0 | |
| III-738 | =O | | i-Pr | $-CH_2CH_2CH_2N-$<br>H | 2, 6-$C\ell_2$ | 0 | |
| III-739 | =O | | $C\ell$ | $-CH_2CH_2CH_2N-$<br>H | 2, 6-$C\ell_2$ | 0 | |
| III-740 | =O | | $OCH_3$ | $-CH_2CH_2CH_2N-$<br>H | 2, 6-$C\ell_2$ | 0 | |
| III-741 | =O | | $SCH_3$ | $-CH_2CH_2CH_2N-$<br>H | 2, 6-$C\ell_2$ | 0 | |
| III-742 | =O | | $NHCH_3$ | $-CH_2CH_2CH_2N-$<br>H | 2, 6-$C\ell_2$ | 0 | |
| III-743 | =O | | Et | $-CH_2CH_2CH_2N-$<br>\|<br>$CH_3$ | 2-$C\ell$ | 0 | |
| III-744 | =O | | i-Pr | $-CH_2CH_2CH_2N-$<br>\|<br>$CH_3$ | 2-$C\ell$ | 0 | |
| III-745 | =O | | $C\ell$ | $-CH_2CH_2CH_2N-$<br>\|<br>$CH_3$ | 2-$C\ell$ | 0 | |
| III-746 | =O | | $OCH_3$ | $-CH_2CH_2CH_2N-$<br>\|<br>$CH_3$ | 2-$C\ell$ | 0 | |
| III-747 | =O | | $SCH_3$ | $-CH_2CH_2CH_2N-$<br>\|<br>$CH_3$ | 2-$C\ell$ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-748 | =O | | $NHCH_3$ | $-CH_2CH_2CH_2N-$<br>\|<br>$CH_3$ | 2-Cℓ | 0 | |
| III-749 | =O | | $CF_3$ | $-CH_2CH_2CH_2N-$<br>\|<br>$CH_3$ | 2-Cℓ, 6-F | 0 | |
| III-750 | =O | | Et | $-CH_2CH_2CH_2N-$<br>\|<br>$CH_3$ | 2-Cℓ, 6-F | 0 | |
| III-751 | =O | | i-Pr | $-CH_2CH_2CH_2N-$<br>\|<br>$CH_3$ | 2-Cℓ, 6-F | 0 | |
| III-752 | =O | | Cℓ | $-CH_2CH_2CH_2N-$<br>\|<br>$CH_3$ | 2-Cℓ, 6-F | 0 | |
| III-753 | =O | | $OCH_3$ | $-CH_2CH_2CH_2N-$<br>\|<br>$CH_3$ | 2-Cℓ, 6-F | 0 | |
| III-754 | =O | | $SCH_3$ | $-CH_2CH_2CH_2N-$<br>\|<br>$CH_3$ | 2-Cℓ, 6-F | 0 | |
| III-755 | =O | | $NHCH_3$ | $-CH_2CH_2CH_2N-$<br>\|<br>$CH_3$ | 2-Cℓ, 6-F | 0 | |
| III-756 | =O | | $CF_3$ | $-CH_2CH_2CH_2N-$<br>\|<br>$CH_3$ | 2, 6-$Cℓ_2$ | 0 | |
| III-757 | =O | | Et | $-CH_2CH_2CH_2N-$<br>\|<br>$CH_3$ | 2, 6-$Cℓ_2$ | 0 | |
| III-758 | =O | | i-Pr | $-CH_2CH_2CH_2N-$<br>\|<br>$CH_3$ | 2, 6-$Cℓ_2$ | 0 | |
| III-759 | =O | | Cℓ | $-CH_2CH_2CH_2N-$<br>\|<br>$CH_3$ | 2, 6-$Cℓ_2$ | 0 | |
| III-760 | =O | | $OCH_3$ | $-CH_2CH_2CH_2N-$<br>\|<br>$CH_3$ | 2, 6-$Cℓ_2$ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-761 | =O | | $SCH_3$ | $-CH_2CH_2CH_2N-$<br>$CH_3$ | 2,6-$Cl_2$ | 0 | |
| III-762 | =O | | $NHCH_3$ | $-CH_2CH_2CH_2N-$<br>$CH_3$ | 2,6-$Cl_2$ | 0 | |
| III-763 | =O | | Cl | -CH=CH-S-CH=CH- | 2-Cl | 0 | |
| III-764 | =O | | $OCH_3$ | -CH=CH-S-CH=CH- | 2-Cl | 0 | |
| III-765 | =O | | $SCH_3$ | -CH=CH-S-CH=CH- | 2-Cl | 0 | |
| III-766 | =O | | $NHCH_3$ | -CH=CH-S-CH=CH- | 2-Cl | 0 | |
| III-767 | =O | | $CF_3$ | -CH=CH-S-CH=CH- | 2-Cl | 0 | |
| III-768 | =O | | Et | -CH=CH-S-CH=CH- | 2-Cl | 0 | |
| III-769 | =O | | i-Pr | -CH=CH-S-CH=CH- | 2-Cl | 0 | |
| III-770 | =O | | Cl | -CH=CH-S-CH=CH- | 2-Cl, 6-F | | |
| III-771 | =O | | $OCH_3$ | -CH=CH-S-CH=CH- | 2-Cl, 6-F | 0 | |
| III-772 | =O | | $SCH_3$ | -CH=CH-S-CH=CH- | 2-Cl, 6-F | | |
| III-773 | =O | | $NHCH_3$ | -CH=CH-S-CH=CH- | 2-Cl, 6-F | 0 | |
| III-774 | =O | | $CF_3$ | -CH=CH-S-CH=CH- | 2-Cl, 6-F | | |
| III-775 | =O | | Et | -CH=CH-S-CH=CH- | 2-Cl, 6-F | 0 | |
| III-776 | =O | | i-Pr | -CH=CH-S-CH=CH- | 2-Cl, 6-F | | |
| III-777 | =O | | Cl | -CH=CH-S-CH=CH- | 2,6-$Cl_2$ | 0 | |
| III-778 | =O | | $OCH_3$ | -CH=CH-S-CH=CH- | 2,6-$Cl_2$ | | |
| III-779 | =O | | $SCH_3$ | -CH=CH-S-CH=CH- | 2,6-$Cl_2$ | 0 | |
| III-780 | =O | | $NHCH_3$ | -CH=CH-S-CH=CH- | 2,6-$Cl_2$ | | |
| III-781 | =O | | $CF_3$ | -CH=CH-S-CH=CH- | 2,6-$Cl_2$ | 0 | |
| III-782 | =O | | Et | -CH=CH-S-CH=CH- | 2,6-$Cl_2$ | | |
| III-783 | =O | | i-Pr | -CH=CH-S-CH=CH- | 2,6-$Cl_2$ | 0 | |
| III-784 | $=NNH_2$ | | $CF_3$ | O $-CH_2CH_2-$ | 2-Cl | 0 | |

| No. | $R^1$ | $R^2$ | $R^3$ | A-B-D-(E)- | $R^6, R^7, R^8$ | n | Physical Constants |
|---|---|---|---|---|---|---|---|
| III-785 | $=NNHCH_3$ | | $CF_3$ | O $-CH_2CH_2-$ | 2-Cℓ | 0 | |
| III-786 | 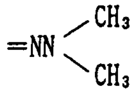 | | $CF_3$ | O $-CH_2CH_2-$ | 2-Cℓ | 0 | |
| III-787 | =NNHCOOEt | | $CF_3$ | O $-CH_2CH_2-$ | 2-Cℓ | 0 | |
| III-788 | $=NH_2$ | H | $CF_3$ | O $-CH_2CH_2-$ | 2-Cℓ | 0 | |
| III-789 | =NHMe | H | $CF_3$ | O $-CH_2CH_2-$ | 2-Cℓ | 0 | |
| III-790 | 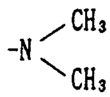 | H | $CF_3$ | O $-CH_2CH_2-$ | 2-Cℓ | 0 | |
| III-791 | 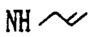 | H | $CF_3$ | O $-CH_2CH_2-$ | 2-Cℓ | 0 | |
| III-792 | $NHCH_2C{\equiv}CH$ | H | $CF_3$ | O $-CH_2CH_2-$ | 2-Cℓ | 0 | |
| III-793 | $NHCH_2OCH_3$ | H | $CF_3$ | O $-CH_2CH_2-$ | 2-Cℓ | 0 | |
| III-794 | NHCOOEt | H | $CF_3$ | O $-CH_2CH_2-$ | 2-Cℓ | 0 | |
| III-795 | $NHCH_2COOEt$ | H | $CF_3$ | O $-CH_2CH_2-$ | 2-Cℓ | 0 | |
| III-796 | 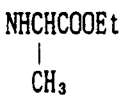 | H | $CF_3$ | O $-CH_2CH_2-$ | 2-Cℓ | 0 | |
| III-797 | $-NHNH_2$ | | $CF_3$ | O $-CH_2CH_2-$ | 2-Cℓ | 0 | |
| III-798 | $-NHNHCH_3$ | | $CF_3$ | O $-CH_2CH_2-$ | 2-Cℓ | 0 | |
| III-799 | 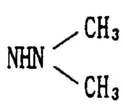 | | $CF_3$ | O $-CH_2CH_2-$ | 2-Cℓ | 0 | |
| III-800 | =NHNHCOOEt | | $CF_3$ | O $-CH_2CH_2-$ | 2-Cℓ | 0 | |
| III-801 | =NH | | $CF_3$ | O $-CH_2CH_2-$ | 2-Cℓ | 0 | |
| III-802 | $=NCH_3$ | | $CF_3$ | O $-CH_2CH_2-$ | 2-Cℓ | 0 | |
| III-803 | =NEt | | $CF_3$ | O $-CH_2CH_2-$ | 2-Cℓ | 0 | |
| III-804 | =NOH | | $CF_3$ | O $-CH_2CH_2-$ | 2-Cℓ | 0 | |
| III-805 | $=NOCH_3$ | | $CF_3$ | O $-CH_2CH_2-$ | 2-Cℓ | 0 | |
| III-806 | =NOEt | | $CF_3$ | O $-CH_2CH_2-$ | 2-Cℓ | 0 | |

| No. | $R^1$ $R^2$ | $R^3$ | A-B-D-(E)- | $R^6$, $R^7$, $R^8$ | n | Physical Constants |
|---|---|---|---|---|---|---|
| III-807 | =NO 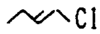 | $CF_3$ | 0 $-CH_2CH_2-$ | 2-Cℓ | 0 | |
| III-808 | $=NCHCOOEt$<br>$CH_3$ | $CF_3$ | 0 $-CH_2CH_2-$ | 2-Cℓ | 0 | |
| III-809 | $=NCH_2COOEt$ | $CF_3$ | 0 $-CH_2CH_2-$ | 2-Cℓ | 0 | |
| III-810 | =NO 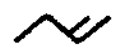 | $CF_3$ | 0 $-CH_2CH_2-$ | 2-Cℓ | 0 | |
| III-811 | =NO 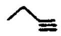 | $CF_3$ | 0 $-CH_2CH_2-$ | 2-Cℓ | 0 | |
| III-812 | $=NOCH_2Ph$ | $CF_3$ | 0 $-CH_2CH_2-$ | 2-Cℓ | 0 | |
| III-813 | $=NOCH_2OCH_3$ | $CF_3$ | 0 $-CH_2CH_2-$ | 2-Cℓ | 0 | |
| III-814 | $=NOCH_2SCH_3$ | $CF_3$ | 0 $-CH_2CH_2-$ | 2-Cℓ | 0 | |
| III-815 | $=CH_2$ | $CF_3$ | 0 $-CH_2CH_2-$ | 2-Cℓ | 0 | |
| III-816 | $=CHCH_3$ | $CF_3$ | 0 $-CH_2CH_2-$ | 2-Cℓ | 0 | |
| III-817 | $=CHCF_3$ | $CF_3$ | 0 $-CH_2CH_2-$ | 2-Cℓ | 0 | |
| III-818 | $-OCH_2-$ | $CF_3$ | 0 $-CH_2CH_2-$ | 2-Cℓ | 0 | |
| III-819 | $-CH_2CH_2-$ | $CF_3$ | 0 $-CH_2CH_2-$ | 2-Cℓ | 0 | |
| III-820 | $-NHCH_2H_4O-$ | $CF_3$ | 0 $-CH_2CH_2-$ | 2-Cℓ | 0 | |
| III-821 | $-NHC_2H_4S-$ | $CF_3$ | 0 $-CH_2CH_2-$ | 2-Cℓ | 0 | |
| III-822 | $-NHC_2H_4NH-$ | $CF_3$ | 0 $-CH_2CH_2-$ | 2-Cℓ | 0 | |
| III-823 | 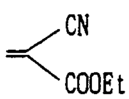 | $CF_3$ | 0 $-CH_2CH_2-$ | 2-Cℓ | 0 | |
| III-824 | 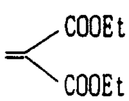 | $CF_3$ | 0 $-CH_2CH_2-$ | 2-Cℓ | 0 | **10 |
| III-825 | 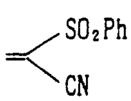 | $CF_3$ | 0 $-CH_2CH_2-$ | 2-Cℓ | 0 | |
| III-826 | 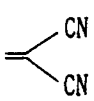 | $CF_3$ | 0 $-CH_2CH_2-$ | 2-Cℓ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-827 | | 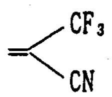 | $CF_3$ | O $-CH_2CH_2-$ | 2-Cℓ | 0 | |
| III-828 | | 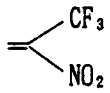 | $CF_3$ | O $-CH_2CH_2-$ | 2-Cℓ | 0 | |
| III-829 | | 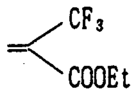 | $CF_3$ | O $-CH_2CH_2-$ | 2-Cℓ | 0 | |
| III-830 | $=CHCH_2CO_2Et$ | | $CF_3$ | O $-CH_2CH_2-$ | 2-Cℓ | 0 | |
| III-831 | 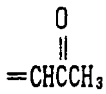 | | $CF_3$ | O $-CH_2CH_2-$ | 2-Cℓ | 0 | |
| III-832 | 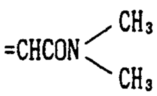 | | $CF_3$ | O $-CH_2CH_2-$ | 2-Cℓ | 0 | |
| III-833 | $=CHNO_2$ | | $CF_3$ | O $-CH_2CH_2-$ | 2-Cℓ | 0 | |
| III-834 | =O | | $CF_3$ | O $-CH_2CH_2-$ | 2-Et | 0 | [82−84] |
| III-835 | =O | | $CF_3$ | O -CH=CH- | 2-Et | 0 | |
| III-836 | =O | | $CF_3$ | O $-CH_2CH_2CH_2-$ | 2-Et | 0 | |
| III-837 | =O | | $CF_3$ | S $-CH_2CH_2-$ | 2-Et | 0 | |
| III-838 | =O | | $CF_3$ | S -CH=CH- | 2-Et | 0 | |
| III-839 | =O | | $CF_3$ | S $-CH_2CH_2CH_2-$ | 2-Et | 0 | |
| III-840 | =O | | $CF_3$ | NH $-CH_2CH_2-$ | 2-Et | | |
| III-841 | =O | | $CF_3$ | NH -CH=CH- | 2-Et | 0 | |
| III-842 | =O | | $CF_3$ | NH $-CH_2CH_2CH_2-$ | 2-Et | 0 | |
| III-843 | =O | | $CF_3$ | 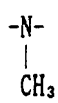 $-CH_2CH_2-$ | 2-Et | 0 | |
| III-844 | =O | | $CF_3$ | 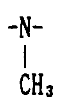 -CH=CH- | 2-Et | 0 | |

| No. | $R^1$ | $R^2$ | $R^3$ | A-B-D-(E)- | $R^6, R^7, R^8$ | n | Physical Constants |
|---|---|---|---|---|---|---|---|
| Ⅲ-845 | =O | | $CF_3$ | $-N(CH_3)-$ $-CH_2CH_2CH_2-$ | 2-Et | 0 | |
| Ⅲ-846 | =O | | $CF_3$ | $-N(CH_2OEt)-$ $-CH_2CH_2-$ | 2-Et | 0 | |
| Ⅲ-847 | =O | | $CF_3$ | $-N(CH_2OEt)-$ $-CH=CH-$ | 2-Et | 0 | |
| Ⅲ-848 | =O | | $CF_3$ | $-N(CH_2OEt)-$ $-CH_2CH_2CH_2-$ | 2-Et | 0 | |
| Ⅲ-849 | =O | | $CF_3$ | O $-CH_2CH_2-$ | 2-COOEt | 0 | |
| Ⅲ-850 | =O | | $CF_3$ | O $-CH=CH-$ | 2-COOEt | 0 | |
| Ⅲ-851 | =O | | $CF_3$ | O $-CH_2CH_2CH_2-$ | 2-COOEt | 0 | |
| Ⅲ-852 | =O | | $CF_3$ | S $-CH_2CH_2-$ | 2-COOEt | 0 | |
| Ⅲ-853 | =O | | $CF_3$ | S $-CH=CH-$ | 2-COOEt | 0 | |
| Ⅲ-854 | =O | | $CF_3$ | S $-CH_2CH_2CH_2-$ | 2-COOEt | 0 | |
| Ⅲ-855 | =O | | $CF_3$ | -NH- $-CH_2CH_2-$ | 2-COOEt | 0 | |
| Ⅲ-856 | =O | | $CF_3$ | -NH- $-CH=CH-$ | 2-COOEt | 0 | |
| Ⅲ-857 | =O | | $CF_3$ | -NH- $-CH_2CH_2CH_2-$ | 2-COOEt | 0 | |
| Ⅲ-858 | =O | | $CF_3$ | $-N(CH_3)-$ $-CH_2CH_2-$ | 2-COOEt | 0 | |
| Ⅲ-859 | =O | | $CF_3$ | $-N(CH_3)-$ $-CH=CH-$ | 2-COOEt | 0 | |
| Ⅲ-860 | =O | | $CF_3$ | $-N(CH_3)-$ $-CH_2CH_2CH_2-$ | 2-COOEt | 0 | |
| Ⅲ-861 | =O | | $CF_3$ | $-N(CH_2OCH_2CH_3)-$ $-CH_2CH_2-$ | 2-Et | 0 | |

| No. | $R^1$ | $R^2$ | $R^3$ | A-B-D-(E)- | $R^6, R^7, R^8$ | n | Physical Constants |
|---|---|---|---|---|---|---|---|
| III-862 | =O | | $CF_3$ | -N- -CH=CH-<br>$CH_2OCH_2CH_3$ | 2-COOEt | 0 | |
| III-863 | =O | | $CF_3$ | -N- $-CH_2CH_2CH_2-$<br>$CH_2OCH_2CH_3$ | 2-COOEt | 0 | |
| III-864 | =CHCOOH | | $CF_3$ | O | 2-Cℓ | 0 | |
| III-865 | =CHCOOH | | $CF_3$ | O -CH=CH- | 2-Cℓ | 0 | |
| III-866 | =CHCOOH | | $CF_3$ | O $-CH_2CH_2CH_2-$ | 2-Cℓ | 0 | |
| III-867 | =CHCOOH | | $CF_3$ | S $-CH_2CH_2-$ | 2-Cℓ | 0 | |
| III-868 | =CHCOOH | | $CF_3$ | S -CH=CH- | 2-Cℓ | 0 | |
| III-869 | =CHCOOH | | $CF_3$ | S $-CH_2CH_2CH_2-$ | 2-Cℓ | 0 | |
| III-870 | =CHCOOH | | $CF_3$ | -NH- $-CH_2CH_2-$ | 2-Cℓ | 0 | |
| III-871 | =CHCOOH | | $CF_3$ | -NH- -CH=CH- | 2-Cℓ | 0 | |
| III-872 | =CHCOOH | | $CF_3$ | -NH- $-CH_2CH_2CH_2-$ | 2-Cℓ | 0 | |
| III-873 | =CHCOOH | | $CF_3$ | -N- $-CH_2CH_2-$<br>$CH_3$ | 2-Cℓ | 0 | |
| III-874 | =CHCOOH | | $CF_3$ | -N- -CH=CH-<br>$CH_3$ | 2-Cℓ | 0 | |
| III-875 | =CHCOOH | | $CF_3$ | -N- $-CH_2CH_2CH_3-$<br>$CH_3$ | 2-Cℓ | 0 | |
| III-876 | =CHCOOH | | $CF_3$ | -N- $-CH_2CH_2-$<br>$CH_2OEt$ | 2-Cℓ | 0 | |
| III-877 | =CHCOOH | | $CF_3$ | -N- -CH=CH-<br>$CH_2OEt$ | 2-Cℓ | 0 | |
| III-878 | =CHCOOH | | $CF_3$ | -N- $-CH_2CH_2CH_2-$<br>$CH_2OEt$ | 2-Cℓ | 0 | |

| No. | $R^1$ $R^2$ | $R^3$ | A-B-D-(E)- | $R^6, R^7, R^8$ | n | Physical Constants |
|---|---|---|---|---|---|---|
| Ⅲ-879 | =CHCOOH | i-Pr | O $-CH_2CH_2-$ | 2-Cℓ | 0 | |
| Ⅲ-880 | =CHCOOH | i-Pr | O -CH=CH- | 2-Cℓ | 0 | |
| Ⅲ-881 | =CHCOOH | i-Pr | O $-CH_2CH_2CH_2-$ | 2-Cℓ | 0 | |
| Ⅲ-882 | =CHCOOH | i-Pr | S $-CH_2CH_2-$ | 2-Cℓ | 0 | |
| Ⅲ-883 | =CHCOOH | i-Pr | S -CH=CH- | 2-Cℓ | 0 | |
| Ⅲ-884 | =CHCOOH | i-Pr | S $-CH_2CH_2CH_2-$ | 2-Cℓ | 0 | |
| Ⅲ-885 | =CHCOOH | i-Pr | -NH- $-CH_2CH_2-$ | 2-Cℓ | 0 | |
| Ⅲ-886 | =CHCOOH | i-Pr | -NH- -CH=CH- | 2-Cℓ | 0 | |
| Ⅲ-887 | =CHCOOH | i-Pr | -NH- $-CH_2CH_2CH_2-$ | 2-Cℓ | 0 | |
| Ⅲ-888 | =CHCOOH | i-Pr | -N($CH_3$)- $-CH_2CH_2-$ | 2-Cℓ | 0 | |
| Ⅲ-889 | =CHCOOH | i-Pr | -N($CH_3$)- -CH=CH- | 2-Cℓ | 0 | |
| Ⅲ-890 | =CHCOOH | i-Pr | -N($CH_3$)- $-CH_2CH_2CH_2-$ | 2-Cℓ | 0 | |
| Ⅲ-891 | =CHCOOH | i-Pr | -N($CH_2OEt$)- $-CH_2CH_2-$ | 2-Cℓ | 0 | |
| Ⅲ-892 | =CHCOOH | i-Pr | -N($CH_2OEt$)- -CH=CH- | 2-Cℓ | 0 | |
| Ⅲ-893 | =CHCOOH | i-Pr | -N($CH_2OEt$)- $-CH_2CH_2CH_2-$ | 2-Cℓ | 0 | |
| Ⅲ-894 | =CHCOOH | Et | O $-CH_2CH_2-$ | 2-Cℓ | 0 | |
| Ⅲ-895 | =CHCOOH | Et | O -CH=CH- | 2-Cℓ | 0 | |
| Ⅲ-896 | =CHCOOH | Et | O $-CH_2CH_2CH_2-$ | 2-Cℓ | 0 | |
| Ⅲ-897 | =CHCOOH | Et | S $-CH_2CH_2-$ | 2-Cℓ | 0 | |
| Ⅲ-898 | =CHCOOH | Et | S -CH=CH- | 2-Cℓ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-899 | =CHCOOH | | Et | S $-CH_2CH_2CH_2-$ | 2-Cℓ | 0 | |
| III-900 | =CHCOOH | | Et | -NH- $-CH_2CH_2-$ | 2-Cℓ | 0 | |
| III-901 | =CHCOOH | | Et | -NH- -CH=CH- | 2-Cℓ | 0 | |
| III-902 | =CHCOOH | | Et | -NH- $-CH_2CH_2CH_2-$ | 2-Cℓ | 0 | |
| III-903 | =CHCOOH | | Et | $-N(CH_3)-$ $-CH_2CH_2-$ | 2-Cℓ | 0 | |
| III-904 | =CHCOOH | | Et | $-N(CH_3)-$ -CH=CH- | 2-Cℓ | 0 | |
| III-905 | =CHCOOH | | Et | $-N(CH_3)-$ $-CH_2CH_2CH_2-$ | 2-Cℓ | 0 | |
| III-906 | =CHCOOH | | Et | $-N(CH_2OEt)-$ $-CH_2CH_2-$ | 2-Cℓ | 0 | |
| III-907 | =CHCOOH | | Et | $-N(CH_2OEt)-$ -CH=CH- | 2-Cℓ | 0 | |
| III-908 | =CHCOOH | | Et | $-N(CH_2OEt)-$ $-CH_2CH_2CH_2-$ | 2-Cℓ | 0 | |
| III-909 | =CHCOOH | | $CF_3$ | O $-CH_2CH_2-$ | 2-Cℓ, 6-F | 0 | |
| III-910 | =CHCOOH | | $CF_3$ | O -CH=CH- | 2-Cℓ, 6-F | 0 | |
| III-911 | =CHCOOH | | $CF_3$ | O $-CH_2CH_2CH_2-$ | 2-Cℓ, 6-F | 0 | |
| III-912 | =CHCOOH | | Et | O $-CH_2CH_2-$ | 2-Cℓ, 6-F | 0 | |
| III-913 | =CHCOOH | | Et | O -CH=CH- | 2-Cℓ, 6-F | 0 | |
| III-914 | =CHCOOH | | Et | O $-CH_2CH_2CH_2-$ | 2-Cℓ, 6-F | 0 | |
| III-915 | =CHCOOH | | i-Pr | O $-CH_2CH_2-$ | 2-Cℓ, 6-F | 0 | |
| III-916 | =CHCOOH | | i-Pr | O -CH=CH- | 2-Cℓ, 6-F | 0 | |
| III-917 | =CHCOOH | | i-Pr | O $-CH_2CH_2CH_2-$ | 2-Cℓ, 6-F | 0 | |
| III-918 | =CHCOOH | | $CF_3$ | S $-CH_2CH_2-$ | 2-Cℓ, 6-F | 0 | |

| No. | $R^1$ $R^2$ | $R^3$ | A-B-D-(E)- | $R^6, R^7, R^8$ | n | Physical Constants |
|---|---|---|---|---|---|---|
| III-919 | =CHCOOH | $CF_3$ | S -CH=CH- | 2-Cℓ, 6-F | 0 | [218] |
| III-920 | =CHCOOH | $CF_3$ | S $-CH_2CH_2CH_2-$ | 2-Cℓ, 6-F | 0 | |
| III-921 | =CHCOOH | Et | S $-CH_2CH_2-$ | 2-Cℓ, 6-F | 0 | |
| III-922 | =CHCOOH | Et | S -CH=CH- | 2-Cℓ, 6-F | 0 | |
| III-923 | =CHCOOH | Et | S $-CH_2CH_2CH_2-$ | 2-Cℓ, 6-F | 0 | |
| III-924 | =CHCOOH | i-Pr | S $-CH_2CH_2-$ | 2-Cℓ, 6-F | 0 | |
| III-925 | =CHCOOH | i-Pr | S -CH=CH- | 2-Cℓ, 6-F | 0 | |
| III-926 | =CHCOOH | i-Pr | S $-CH_2CH_2CH_2-$ | 2-Cℓ, 6-F | 0 | |
| III-927 | =CHCOOH | $CF_3$ | -NH- $-CH_2CH_2-$ | 2-Cℓ, 6-F | 0 | |
| III-928 | =CHCOOH | $CF_3$ | -NH- -CH=CH- | 2-Cℓ, 6-F | 0 | |
| III-929 | =CHCOOH | $CF_3$ | -NH- $-CH_2CH_2CH_2-$ | 2-Cℓ, 6-F | 0 | |
| III-930 | =CHCOOH | Et | -NH- $-CH_2CH_2-$ | 2-Cℓ, 6-F | 0 | |
| III-931 | =CHCOOH | Et | -NH- -CH=CH- | 2-Cℓ, 6-F | 0 | |
| III-932 | =CHCOOH | Et | -NH- $-CH_2CH_2CH_2-$ | 2-Cℓ, 6-F | 0 | |
| III-933 | =CHCOOH | i-Pr | -NH- $-CH_2CH_2-$ | 2-Cℓ, 6-F | 0 | |
| III-934 | =CHCOOH | i-Pr | -NH- -CH=CH- | 2-Cℓ, 6-F | 0 | |
| III-935 | =CHCOOH | i-Pr | -NH- $-CH_2CH_2CH_2-$ | 2-Cℓ, 6-F | 0 | |
| III-936 | =CHCOOH | $CF_3$ | $-N(CH_3)-$ $-CH_2CH_2-$ | 2-Cℓ, 6-F | 0 | |
| III-937 | =CHCOOH | $CF_3$ | $-N(CH_3)-$ -CH=CH- | 2-Cℓ, 6-F | 0 | |
| III-938 | =CHCOOH | $CF_3$ | $-N(CH_3)-$ $-CH_2CH_2CH_2-$ | 2-Cℓ, 6-F | 0 | |
| III-939 | =CHCOOH | Et | $-N(CH_3)-$ $-CH_2CH_2-$ | 2-Cℓ, 6-F | 0 | |

| No. | $R^1$ | $R^2$ | $R^3$ | A-B-D-(E)- | $R^6, R^7, R^8$ | n | Physical Constants |
|---|---|---|---|---|---|---|---|
| III-940 | =CHCOOH | | Et | -N(CH₃)- -CH=CH- | 2-Cℓ, 6-F | 0 | |
| III-941 | =CHCOOH | | Et | -N(CH₃)- $-CH_2CH_2CH_2-$ | 2-Cℓ, 6-F | 0 | |
| III-942 | =CHCOOH | | i-Pr | -N(CH₃)- $-CH_2CH_2-$ | 2-Cℓ, 6-F | 0 | |
| III-943 | =CHCOOH | | i-Pr | -N(CH₃)- -CH=CH- | 2-Cℓ, 6-F | 0 | |
| III-944 | =CHCOOH | | i-Pr | -N(CH₃)- $-CH_2CH_2CH_2-$ | 2-Cℓ, 6-F | 0 | |
| III-945 | =CHCOOH | | $CF_3$ | -N($CH_2OEt$)- $-CH_2CH_2-$ | 2-Cℓ, 6-F | 0 | |
| III-946 | =CHCOOH | | $CF_3$ | -N($CH_2OEt$)- -CH=CH- | 2-Cℓ, 6-F | 0 | |
| III-947 | =CHCOOH | | $CF_3$ | -N($CH_2OEt$)- $-CH_2CH_2CH_2-$ | 2-Cℓ, 6-F | 0 | |
| III-948 | =CHCOOH | | Et | -N($CH_2OEt$)- $-CH_2CH_2-$ | 2-Cℓ, 6-F | 0 | |
| III-949 | =CHCOOH | | Et | -N($CH_2OEt$)- -CH=CH- | 2-Cℓ, 6-F | 0 | |
| III-950 | =CHCOOH | | Et | -N($CH_2OEt$)- $-CH_2CH_2CH_2-$ | 2-Cℓ, 6-F | 0 | |
| III-951 | =CHCOOH | | i-Pr | -N($CH_2OEt$)- $-CH_2CH_2-$ | 2-Cℓ, 6-F | 0 | |
| III-952 | =CHCOOH | | i-Pr | -N($CH_2OEt$)- -CH=CH- | 2-Cℓ, 6-F | 0 | |

| No. | $R^1$ | $R^2$ | $R^3$ | A-B-D-(E)- | $R^6, R^7, R^8$ | n | Physical Constants |
|---|---|---|---|---|---|---|---|
| III-953 | =CHCOOH | | i-Pr | -N(CH₂OEt)- $-CH_2CH_2CH_2-$ | 2-Cℓ, 6-F | 0 | |
| III-954 | =CHCOOH | | $CF_3$ | O $-CH_2CH_2-$ | 2,6-$Cℓ_2$ | 0 | |
| III-955 | =CHCOOH | | $CF_3$ | O -CH=CH- | 2,6-$Cℓ_2$ | 0 | |
| III-956 | =CHCOOH | | $CF_3$ | O $-CH_2CH_2CH_2-$ | 2,6-$Cℓ_2$ | 0 | |
| III-957 | =CHCOOH | | Et | O $-CH_2CH_2-$ | 2,6-$Cℓ_2$ | 0 | |
| III-958 | =CHCOOH | | Et | O -CH=CH- | 2,6-$Cℓ_2$ | 0 | |
| III-959 | =CHCOOH | | Et | O $-CH_2CH_2CH_2-$ | 2,6-$Cℓ_2$ | 0 | |
| III-960 | =CHCOOH | | i-Pr | O $-CH_2CH_2-$ | 2,6-$Cℓ_2$ | 0 | |
| III-961 | =CHCOOH | | i-Pr | O -CH=CH- | 2,6-$Cℓ_2$ | 0 | |
| III-962 | =CHCOOH | | i-Pr | O $-CH_2CH_2CH_2-$ | 2,6-$Cℓ_2$ | 0 | |
| III-963 | =CHCOOH | | $CF_3$ | S $-CH_2CH_2-$ | 2,6-$Cℓ_2$ | 0 | |
| III-964 | =CHCOOH | | $CF_3$ | S -CH=CH- | 2,6-$Cℓ_2$ | 0 | |
| III-965 | =CHCOOH | | $CF_3$ | S $-CH_2CH_2CH_2-$ | 2,6-$Cℓ_2$ | 0 | |
| III-966 | =CHCOOH | | Et | S $-CH_2CH_2-$ | 2,6-$Cℓ_2$ | 0 | |
| III-967 | =CHCOOH | | Et | S -CH=CH- | 2,6-$Cℓ_2$ | 0 | |
| III-968 | =CHCOOH | | Et | S $-CH_2CH_2CH_2-$ | 2,6-$Cℓ_2$ | 0 | |
| III-969 | =CHCOOH | | i-Pr | S $-CH_2CH_2-$ | 2,6-$Cℓ_2$ | 0 | |
| III-970 | =CHCOOH | | i-Pr | S -CH=CH- | 2,6-$Cℓ_2$ | 0 | |
| III-971 | =CHCOOH | | i-Pr | S $-CH_2CH_2CH_2-$ | 2,6-$Cℓ_2$ | 0 | |
| III-972 | =CHCOOH | | $CF_3$ | -NH- $-CH_2CH_2-$ | 2,6-$Cℓ_2$ | 0 | |
| III-973 | =CHCOOH | | $CF_3$ | -NH- -CH=CH- | 2,6-$Cℓ_2$ | 0 | |
| III-974 | =CHCOOH | | $CF_3$ | -NH- $-CH_2CH_2CH_2-$ | 2,6-$Cℓ_2$ | 0 | |
| III-975 | =CHCOOH | | Et | -NH- $-CH_2CH_2-$ | 2,6-$Cℓ_2$ | 0 | |
| III-976 | =CHCOOH | | Et | -NH- -CH=CH- | 2,6-$Cℓ_2$ | 0 | |
| III-977 | =CHCOOH | | Et | -NH- $-CH_2CH_2CH_2-$ | 2,6-$Cℓ_2$ | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| III-978 | =CHCOOH | | i-Pr | -NH- $-CH_2CH_2-$ | 2,6-$Cl_2$ | 0 | |
| III-979 | =CHCOOH | | i-Pr | -NH- -CH=CH- | 2,6-$Cl_2$ | 0 | |
| III-980 | =CHCOOH | | i-Pr | -NH- $-CH_2CH_2CH_2-$ | 2,6-$Cl_2$ | 0 | |
| III-981 | =CHCOOH | | $CF_3$ | -N($CH_3$)- $-CH_2CH_2-$ | 2,6-$Cl_2$ | 0 | |
| III-982 | =CHCOOH | | $CF_3$ | -N($CH_3$)- -CH=CH- | 2,6-$Cl_2$ | 0 | |
| III-983 | =CHCOOH | | $CF_3$ | -N($CH_3$)- $-CH_2CH_2CH_2-$ | 2,6-$Cl_2$ | 0 | |
| III-984 | =CHCOOH | | Et | -N($CH_3$)- $-CH_2CH_2-$ | 2,6-$Cl_2$ | 0 | |
| III-985 | =CHCOOH | | Et | -N($CH_3$)- -CH=CH- | 2,6-$Cl_2$ | 0 | |
| III-986 | =CHCOOH | | Et | -N($CH_3$)- $-CH_2CH_2CH_2-$ | 2,6-$Cl_2$ | 0 | |
| III-987 | =CHCOOH | | i-Pr | -N($CH_3$)- $-CH_2CH_2-$ | 2,6-$Cl_2$ | 0 | |
| III-988 | =CHCOOH | | i-Pr | -N($CH_3$)- -CH=CH- | 2,6-$Cl_2$ | 0 | |
| III-989 | =CHCOOH | | i-Pr | -N($CH_3$)- $-CH_2CH_2CH_2-$ | 2,6-$Cl_2$ | 0 | |
| III-990 | =CHCOOH | | $CF_3$ | -N($CH_2OEt$)- $-CH_2CH_2-$ | 2,6-$Cl_2$ | 0 | |
| III-991 | =CHCOOH | | $CF_3$ | -N($CH_2OEt$)- -CH=CH- | 2,6-$Cl_2$ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-992 | =CHCOOH | | $CF_3$ | -N(CH$_2$OEt)- $-CH_2CH_2CH_2-$ | 2,6-$Cl_2$ | 0 | |
| III-993 | =CHCOOH | | Et | -N(CH$_2$OEt)- $-CH_2CH_2-$ | 2,6-$Cl_2$ | 0 | |
| III-994 | =CHCOOH | | Et | -N(CH$_2$OEt)- -CH=CH- | 2,6-$Cl_2$ | 0 | |
| III-995 | =CHCOOH | | Et | -N(CH$_2$OEt)- $-CH_2CH_2CH_2-$ | 2,6-$Cl_2$ | 0 | |
| III-996 | =CHCOOH | | i-Pr | -N(CH$_2$OEt)- $-CH_2CH_2-$ | 2,6-$Cl_2$ | 0 | |
| III-997 | =CHCOOH | | i-Pr | -N(CH$_2$OEt)- -CH=CH- | 2,6-$Cl_2$ | 0 | |
| III-998 | =CHCOOH | | i-Pr | -N(CH$_2$OEt)- $-CH_2CH_2CH_2-$ | 2,6-$Cl_2$ | 0 | |
| III-999 | =O | | $CF_3$ | O $-CH_2CH_2-$ | 2-Cl, 4-Cl | 1 | |
| III-1000 | =O | | $CF_3$ | O $-CH_2CH_2-$ | 2-Cl, 4-F | 1 | |
| III-1001 | =O | | $CF_3$ | O $-CH_2CH_2-$ | 2-Cl, 4-$CH_3$ | 1 | |
| III-1002 | =O | | $CF_3$ | O $-CH_2CH_2-$ | 2-F, 4-F | 1 | [80−81] |
| III-1003 | =O | | $CF_3$ | O $-CH_2CH_2-$ | 2-F, 4-Cl | 1 | |
| III-1004 | =O | | $CF_3$ | O $-CH_2CH_2-$ | 2-F, 4-$CH_3$ | 1 | |
| III-1005 | =O | | $CF_3$ | O $-CH_2CH_2-$ | 2-$CH_3$, 4-Cl | 1 | |
| III-1006 | =O | | $CF_3$ | O $-CH_2CH_2-$ | 2-$CH_3$, 4-F | 1 | |
| III-1007 | =CHCOOEt | | $CF_3$ | O $-CH(CH_3)CH_2-$ | 2-Cl | 0 | |
| III-1008 | =CHCOOEt | | $CF_3$ | O -CH=CH- | 2-Cl | 0 | |
| III-1009 | =CHCOOEt | | $CF_3$ | O $-CH_2CH_2CH_2-$ | 2-Cl | 0 | |
| III-1010 | =CHCOOEt | | $CF_3$ | S $-CH_2CH_2-$ | 2-Cl | 0 | |

| No. | $R^1$ | $R^2$ | $R^3$ | A-B-D-(E)- | $R^6, R^7, R^8$ | n | Physical Constants |
|---|---|---|---|---|---|---|---|
| III-1011 | =CHCOOEt | | $CF_3$ | S -CH=CH- | 2-Cℓ | 0 | |
| III-1012 | =CHCOOEt | | $CF_3$ | S $-CH_2CH_2CH_2-$ | 2-Cℓ | 0 | |
| III-1013 | =CHCOOEt | | $CF_3$ | -NH- $-CH_2CH_2-$ | 2-Cℓ | 0 | |
| III-1014 | =CHCOOEt | | $CF_3$ | -NH- -CH=CH- | 2-Cℓ | 0 | |
| III-1015 | =CHCOOEt | | $CF_3$ | -NH- $-CH_2CH_2CH_2-$ | 2-Cℓ | 0 | |
| III-1016 | =CHCOOEt | | $CF_3$ | -N($CH_3$)- $-CH_2CH_2-$ | 2-Cℓ | 0 | |
| III-1017 | =CHCOOEt | | $CF_3$ | -N($CH_3$)- -CH=CH- | 2-Cℓ | 0 | |
| III-1018 | =CHCOOEt | | $CF_3$ | -N($CH_3$)- $-CH_2CH_2CH_2-$ | 2-Cℓ | 0 | |
| III-1019 | =CHCOOEt | | $CF_3$ | -N($CH_2OEt$)- $-CH_2CH_2-$ | 2-Cℓ | 0 | |
| III-1020 | =CHCOOEt | | $CF_3$ | -N($CH_2OEt$)- -CH=CH- | 2-Cℓ | 0 | |
| III-1021 | =CHCOOEt | | $CF_3$ | -N($CH_2OEt$)- $-CH_2CH_2CH_2-$ | 2-Cℓ | 0 | |
| III-1022 | =CHCOOEt | | i-Pr | O $-CH_2CH_2-$ | 2-Cℓ | 0 | |
| III-1023 | =CHCOOEt | | i-Pr | O -CH=CH- | 2-Cℓ | 0 | |
| III-1024 | =CHCOOEt | | i-Pr | O $-CH_2CH_2CH_2-$ | 2-Cℓ | 0 | |
| III-1025 | =CHCOOEt | | i-Pr | S $-CH_2CH_2-$ | 2-Cℓ | 0 | |
| III-1026 | =CHCOOEt | | i-Pr | S -CH=CH- | 2-Cℓ | 0 | |
| III-1027 | =CHCOOEt | | i-Pr | S $-CH_2CH_2CH_2-$ | 2-Cℓ | 0 | |
| III-1028 | =CHCOOEt | | i-Pr | -NH- $-CH_2CH_2-$ | 2-Cℓ | 0 | |
| III-1029 | =CHCOOEt | | i-Pr | -NH- -CH=CH- | 2-Cℓ | 0 | |
| III-1030 | =CHCOOEt | | i-Pr | -NH- $-CH_2CH_2CH_2-$ | 2-Cℓ | 0 | |

| No. | $R^1$ | $R^2$ | $R^3$ | A-B-D-(E)- | $R^6, R^7, R^8$ | n | Physical Constants |
|---|---|---|---|---|---|---|---|
| Ⅲ-1031 | =CHCOOEt | | i-Pr | -N(CH₃)- -$CH_2CH_2$- | 2-Cℓ | 0 | |
| Ⅲ-1032 | =CHCOOEt | | i-Pr | -N(CH₃)- -CH=CH- | 2-Cℓ | 0 | |
| Ⅲ-1033 | =CHCOOEt | | i-Pr | -N(CH₃)- -$CH_2CH_2CH_3$- | 2-Cℓ | 0 | |
| Ⅲ-1034 | =CHCOOEt | | i-Pr | -N($CH_2$OEt)- -$CH_2CH_2$- | 2-Cℓ | 0 | |
| Ⅲ-1035 | =CHCOOEt | | i-Pr | -N($CH_2$OEt)- -CH=CH- | 2-Cℓ | 0 | |
| Ⅲ-1036 | =CHCOOEt | | i-Pr | -N($CH_2$OEt)- -$CH_2CH_2CH_3$- | 2-Cℓ | 0 | |
| Ⅲ-1037 | =CHCOOEt | | Et | O -$CH_2CH_2$- | 2-Cℓ | 0 | |
| Ⅲ-1038 | =CHCOOEt | | Et | O -CH=CH- | 2-Cℓ | 0 | |
| Ⅲ-1039 | =CHCOOEt | | Et | O -$CH_2CH_2CH_2$- | 2-Cℓ | 0 | |
| Ⅲ-1040 | =CHCOOEt | | Et | S -$CH_2CH_2$- | 2-Cℓ | 0 | |
| Ⅲ-1041 | =CHCOOEt | | Et | S -CH=CH- | 2-Cℓ | 0 | |
| Ⅲ-1042 | =CHCOOEt | | Et | S -$CH_2CH_2CH_2$- | 2-Cℓ | 0 | |
| Ⅲ-1043 | =CHCOOEt | | Et | -NH- -$CH_2CH_2$- | 2-Cℓ | 0 | |
| Ⅲ-1044 | =CHCOOEt | | Et | -NH- -CH=CH- | 2-Cℓ | 0 | |
| Ⅲ-1045 | =CHCOOEt | | Et | -NH- -$CH_2CH_2CH_2$- | 2-Cℓ | 0 | |
| Ⅲ-1046 | =CHCOOEt | | Et | -N(CH₃)- -$CH_2CH_2$- | 2-Cℓ | 0 | |
| Ⅲ-1047 | =CHCOOEt | | Et | -N(CH₃)- -CH=CH- | 2-Cℓ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-1048 | =CHCOOEt | | Et | -N($CH_3$)- $-CH_2CH_2CH_2-$ | 2-Cℓ | 0 | |
| III-1049 | =CHCOOEt | | Et | -N($CH_2OEt$)- $-CH_2CH_2-$ | 2-Cℓ | 0 | |
| III-1050 | =CHCOOEt | | Et | -N($CH_2OEt$)- -CH=CH- | 2-Cℓ | 0 | |
| III-1051 | =CHCOOEt | | Et | -N($CH_2OEt$)- $-CH_2CH_2CH_2-$ | 2-Cℓ | 0 | |
| III-1052 | =CHCOOEt | | $CF_3$ | O $-CH_2CH_2-$ | 2-Cℓ, 6-F | 0 | |
| III-1053 | =CHCOOEt | | $CF_3$ | O -CH=CH- | 2-Cℓ, 6-F | 0 | |
| III-1054 | =CHCOOEt | | $CF_3$ | O $-CH_2CH_2CH_2-$ | 2-Cℓ, 6-F | 0 | |
| III-1055 | =CHCOOEt | | Et | O $-CH_2CH_2-$ | 2-Cℓ, 6-F | 0 | |
| III-1056 | =CHCOOEt | | Et | O -CH=CH- | 2-Cℓ, 6-F | 0 | |
| III-1057 | =CHCOOEt | | Et | O $-CH_2CH_2CH_2-$ | 2-Cℓ, 6-F | 0 | |
| III-1058 | =CHCOOEt | | i-Pr | O $-CH_2CH_2-$ | 2-Cℓ, 6-F | 0 | |
| III-1059 | =CHCOOEt | | i-Pr | O -CH=CH- | 2-Cℓ, 6-F | 0 | |
| III-1060 | =CHCOOEt | | i-Pr | O $-CH_2CH_2CH_2-$ | 2-Cℓ, 6-F | 0 | |
| III-1061 | =CHCOOEt | | $CF_3$ | S $-CH_2CH_2-$ | 2-Cℓ, 6-F | 0 | |
| III-1062 | =CHCOOEt | | $CF_3$ | S -CH=CH- | 2-Cℓ, 6-F | 0 | [98-100] |
| III-1063 | =CHCOOEt | | $CF_3$ | S $-CH_2CH_2CH_2-$ | 2-Cℓ, 6-F | 0 | |
| III-1064 | =CHCOOEt | | Et | S $-CH_2CH_2-$ | 2-Cℓ, 6-F | 0 | |
| III-1065 | =CHCOOEt | | Et | S -CH=CH- | 2-Cℓ, 6-F | 0 | |
| III-1066 | =CHCOOEt | | Et | S $-CH_2CH_2CH_2-$ | 2-Cℓ, 6-F | 0 | |
| III-1067 | =CHCOOEt | | i-Pr | S $-CH_2CH_2-$ | 2-Cℓ, 6-F | 0 | |
| III-1068 | =CHCOOEt | | i-Pr | S -CH=CH- | 2-Cℓ, 6-F | 0 | |
| III-1069 | =CHCOOEt | | i-Pr | S $-CH_2CH_2CH_2-$ | 2-Cℓ, 6-F | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| Ⅲ-1070 | =CHCOOEt | | $CF_3$ | $-NH-$ $-CH_2CH_2-$ | 2-Cℓ, 6-F | 0 | |
| Ⅲ-1071 | =CHCOOEt | | $CF_3$ | $-NH-$ $-CH=CH-$ | 2-Cℓ, 6-F | 0 | |
| Ⅲ-1072 | =CHCOOEt | | $CF_3$ | $-NH-$ $-CH_2CH_2CH_2-$ | 2-Cℓ, 6-F | 0 | |
| Ⅲ-1073 | =CHCOOEt | | Et | $-NH-$ $-CH_2CH_2-$ | 2-Cℓ, 6-F | 0 | |
| Ⅲ-1074 | =CHCOOEt | | Et | $-NH-$ $-CH=CH-$ | 2-Cℓ, 6-F | 0 | |
| Ⅲ-1075 | =CHCOOEt | | Et | $-NH-$ $-CH_2CH_2CH_2-$ | 2-Cℓ, 6-F | 0 | |
| Ⅲ-1076 | =CHCOOEt | | i-Pr | $-NH-$ $-CH_2CH_2-$ | 2-Cℓ, 6-F | 0 | |
| Ⅲ-1077 | =CHCOOEt | | i-Pr | $-NH-$ $-CH=CH-$ | 2-Cℓ, 6-F | 0 | |
| Ⅲ-1078 | =CHCOOEt | | i-Pr | $-NH-$ $-CH_2CH_2CH_2-$ | 2-Cℓ, 6-F | 0 | |
| Ⅲ-1079 | =CHCOOEt | | $CF_3$ | $-N(CH_3)-$ $-CH_2CH_2-$ | 2-Cℓ, 6-F | 0 | |
| Ⅲ-1080 | =CHCOOEt | | $CF_3$ | $-N(CH_3)-$ $-CH=CH-$ | 2-Cℓ, 6-F | 0 | |
| Ⅲ-1081 | =CHCOOEt | | $CF_3$ | $-N(CH_3)-$ $-CH_2CH_2CH_2-$ | 2-Cℓ, 6-F | 0 | |
| Ⅲ-1082 | =CHCOOEt | | Et | $-N(CH_3)-$ $-CH_2CH_2-$ | 2-Cℓ, 6-F | 0 | |
| Ⅲ-1083 | =CHCOOEt | | Et | $-N(CH_3)-$ $-CH=CH-$ | 2-Cℓ, 6-F | 0 | |
| Ⅲ-1084 | =CHCOOEt | | Et | $-N(CH_3)-$ $-CH_2CH_2CH_2-$ | 2-Cℓ, 6-F | 0 | |
| Ⅲ-1085 | =CHCOOEt | | i-Pr | $-N(CH_3)-$ $-CH_2CH_2-$ | 2-Cℓ, 6-F | 0 | |
| Ⅲ-1086 | =CHCOOEt | | i-Pr | $-N(CH_3)-$ $-CH=CH-$ | 2-Cℓ, 6-F | 0 | |

| No. | R¹ R² | R³ | A-B-D-(E)- | $R^6$, $R^7$, $R^8$ | n | Physical Constants |
|---|---|---|---|---|---|---|
| Ⅲ-1087 | =CHCOOEt | i-Pr | -N(CH₃)- -CH₂CH₂CH₂- | 2-Cℓ, 6-F | 0 | |
| Ⅲ-1088 | =CHCOOEt | CF₃ | -N(CH₂OEt)- -CH₂CH₂- | 2-Cℓ, 6-F | 0 | |
| Ⅲ-1089 | =CHCOOEt | CF₃ | -N(CH₂OEt)- -CH=CH- | 2-Cℓ, 6-F | 0 | |
| Ⅲ-1090 | =CHCOOEt | CF₃ | -N(CH₂OEt)- -CH₂CH₂CH₂- | 2-Cℓ, 6-F | 0 | |
| Ⅲ-1091 | =CHCOOEt | Et | -N(CH₂OEt)- -CH₂CH₂- | 2-Cℓ, 6-F | 0 | |
| Ⅲ-1092 | =CHCOOEt | Et | -N(CH₂OEt)- -CH=CH- | 2-Cℓ, 6-F | 0 | |
| Ⅲ-1093 | =CHCOOEt | Et | -N(CH₂OEt)- -CH₂CH₂CH₂- | 2-Cℓ, 6-F | 0 | |
| Ⅲ-1094 | =CHCOOEt | i-Pr | -N(CH₂OEt)- -CH₂CH₂- | 2-Cℓ, 6-F | 0 | |
| Ⅲ-1095 | =CHCOOEt | i-Pr | -N(CH₂OEt)- -CH=CH- | 2-Cℓ, 6-F | 0 | |
| Ⅲ-1096 | =CHCOOEt | i-Pr | -N(CH₂OEt)- -CH₂CH₂CH₂- | 2-Cℓ, 6-F | 0 | |
| Ⅲ-1097 | =CHCOOEt | CF₃ | O -CH₂CH₂- | 2, 6-Cℓ₂ | 0 | |
| Ⅲ-1098 | =CHCOOEt | CF₃ | O -CH=CH- | 2, 6-Cℓ₂ | 0 | |
| Ⅲ-1099 | =CHCOOEt | CF₃ | O -CH₂CH₂CH₂- | 2, 6-Cℓ₂ | 0 | ＊＊11 |
| Ⅲ-1100 | =CHCOOEt | Et | O -CH₂CH₂- | 2, 6-Cℓ₂ | 0 | |
| Ⅲ-1101 | =CHCOOEt | Et | O -CH=CH- | 2, 6-Cℓ₂ | 0 | |
| Ⅲ-1102 | =CHCOOEt | Et | O -CH₂CH₂CH₂- | 2, 6-Cℓ₂ | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| III-1103 | =CHCOOEt | | i-Pr | O $-CH_2CH_2-$ | 2,6-$Cl_2$ | 0 | |
| III-1104 | =CHCOOEt | | i-Pr | O -CH=CH- | 2,6-$Cl_2$ | 0 | |
| III-1105 | =CHCOOEt | | i-Pr | O $-CH_2CH_2CH_2-$ | 2,6-$Cl_2$ | 0 | |
| III-1106 | =CHCOOEt | | $CF_3$ | S $-CH_2CH_2-$ | 2,6-$Cl_2$ | 0 | |
| III-1107 | =CHCOOEt | | $CF_3$ | S -CH=CH- | 2,6-$Cl_2$ | 0 | |
| III-1108 | =CHCOOEt | | $CF_3$ | S $-CH_2CH_2CH_2-$ | 2,6-$Cl_2$ | 0 | |
| III-1109 | =CHCOOEt | | Et | S $-CH_2CH_2-$ | 2,6-$Cl_2$ | 0 | |
| III-1110 | =CHCOOEt | | Et | S -CH=CH- | 2,6-$Cl_2$ | 0 | |
| III-1111 | =CHCOOEt | | Et | S $-CH_2CH_2CH_2-$ | 2,6-$Cl_2$ | 0 | |
| III-1112 | =CHCOOEt | | i-Pr | S $-CH_2CH_2-$ | 2,6-$Cl_2$ | 0 | |
| III-1113 | =CHCOOEt | | i-Pr | S -CH=CH- | 2,6-$Cl_2$ | 0 | |
| III-1114 | =CHCOOEt | | i-Pr | S $-CH_2CH_2CH_2-$ | 2,6-$Cl_2$ | 0 | |
| III-1115 | =CHCOOEt | | $CF_3$ | -NH- $-CH_2CH_2-$ | 2,6-$Cl_2$ | 0 | |
| III-1116 | =CHCOOEt | | $CF_3$ | -NH- -CH=CH- | 2,6-$Cl_2$ | 0 | |
| III-1117 | =CHCOOEt | | $CF_3$ | -NH- $-CH_2CH_2CH_2-$ | 2,6-$Cl_2$ | 0 | |
| III-1118 | =CHCOOEt | | Et | -NH- $-CH_2CH_2-$ | 2,6-$Cl_2$ | 0 | |
| III-1119 | =CHCOOEt | | Et | -NH- -CH=CH- | 2,6-$Cl_2$ | 0 | |
| III-1120 | =CHCOOEt | | Et | -NH- $-CH_2CH_2CH_2-$ | 2,6-$Cl_2$ | 0 | |
| III-1121 | =CHCOOEt | | i-Pr | -NH- $-CH_2CH_2-$ | 2,6-$Cl_2$ | 0 | |
| III-1122 | =CHCOOEt | | i-Pr | -NH- -CH=CH- | 2,6-$Cl_2$ | 0 | |
| III-1123 | =CHCOOEt | | i-Pr | -NH- $-CH_2CH_2CH_2-$ | 2,6-$Cl_2$ | 0 | |
| III-1124 | =CHCOOEt | | $CF_3$ | $-N(CH_3)-$ $-CH_2CH_2-$ | 2,6-$Cl_2$ | 0 | |
| III-1125 | =CHCOOEt | | $CF_3$ | $-N(CH_3)-$ -CH=CH- | 2,6-$Cl_2$ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-1126 | =CHCOOEt | | $CF_3$ | $-N(CH_3)-$ $-CH_2CH_2CH_2-$ | 2,6-$Cl_2$ | 0 | |
| III-1127 | =CHCOOEt | | Et | $-N(CH_3)-$ $-CH_2CH_2-$ | 2,6-$Cl_2$ | 0 | |
| III-1128 | =CHCOOEt | | Et | $-N(CH_3)-$ $-CH=CH-$ | 2,6-$Cl_2$ | 0 | |
| III-1129 | =CHCOOEt | | Et | $-N(CH_3)-$ $-CH_2CH_2CH_2-$ | 2,6-$Cl_2$ | 0 | |
| III-1130 | =CHCOOEt | | i-Pr | $-N(CH_3)-$ $-CH_2CH_2-$ | 2,6-$Cl_2$ | 0 | |
| III-1131 | =CHCOOEt | | i-Pr | $-N(CH_3)-$ $-CH=CH-$ | 2,6-$Cl_2$ | 0 | |
| III-1132 | =CHCOOEt | | i-Pr | $-N(CH_3)-$ $-CH_2CH_2CH_2-$ | 2,6-$Cl_2$ | 0 | |
| III-1133 | =CHCOOEt | | $CF_3$ | $-N(CH_2OEt)-$ $-CH_2CH_2-$ | 2,6-$Cl_2$ | 0 | |
| III-1134 | =CHCOOEt | | $CF_3$ | $-N(CH_2OEt)-$ $-CH=CH-$ | 2,6-$Cl_2$ | 0 | |
| III-1135 | =CHCOOEt | | $CF_3$ | $-N(CH_2OEt)-$ $-CH_2CH_2CH_2-$ | 2,6-$Cl_2$ | 0 | |
| III-1136 | =CHCOOEt | | Et | $-N(CH_2OEt)-$ $-CH_2CH_2-$ | 2,6-$Cl_2$ | 0 | |
| III-1137 | =CHCOOEt | | Et | $-N(CH_2OEt)-$ $-CH=CH-$ | 2,6-$Cl_2$ | 0 | |
| III-1138 | =CHCOOEt | | Et | $-N(CH_2OEt)-$ $-CH_2CH_2CH_2-$ | 2,6-$Cl_2$ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-1139 | =CHCOOEt | | i-Pr | $-N(CH_2OEt)-$ $-CH_2CH_2-$ | 2,6-$Cl_2$ | 0 | |
| III-1140 | =CHCOOEt | | i-Pr | $-N(CH_2OEt)-$ $-CH=CH-$ | 2,6-$Cl_2$ | 0 | |
| III-1141 | =CHCOOEt | | i-Pr | $-N(CH_2OEt)-$ $-CH_2CH_2CH_2-$ | 2,6-$Cl_2$ | 0 | |
| III-1142 | $=NCH_2P(O)(OEt)_2$ | | $CF_3$ | O $-CH(CH_3)CH_2-$ | 2-Cl | 0 | |
| III-1143 | $=NCH_2P(O)(OEt)_2$ | | $CF_3$ | O $-CH(CH_3)CH_2-$ | 2-Cl | 0 | |
| III-1144 | $=NCH_2P(O)(OEt)_2$ | | $CF_3$ | O $-CH_2CH_2CH_2-$ | 2-Cl | 0 | |
| III-1145 | $=NCH_2P(O)(OEt)_2$ | | $CF_3$ | S $-CH_2CH_2-$ | 2-Cl | 0 | |
| III-1146 | $=NCH_2P(O)(OEt)_2$ | | $CF_3$ | S $-CH(CH_3)CH_2-$ | 2-Cl | 0 | |
| III-1147 | $=NCH_2P(O)(OEt)_2$ | | $CF_3$ | S $-CH_2CH_2CH_2-$ | 2-Cl | 0 | |
| III-1148 | $=NCH_2P(O)(OEt)_2$ | | $CF_3$ | -NH- $-CH_2CH_2-$ | 2-Cl | 0 | |
| III-1149 | $=NCH_2P(O)(OEt)_2$ | | $CF_3$ | -NH- $-CH=CH-$ | 2-Cl | 0 | |
| III-1150 | $=NCH_2P(O)(OEt)_2$ | | $CF_3$ | -NH- $-CH_2CH_2CH_2-$ | 2-Cl | 0 | |

| No. | $R^1$ | $R^2$ | $R^3$ | A-B-D-(E)- | $R^6, R^7, R^8$ | n | Physical Constants |
|---|---|---|---|---|---|---|---|
| Ⅲ-1151 | $=NCH_2\overset{\overset{O}{\|}}{P}(OEt)_2$ | | $CF_3$ | $-\underset{CH_3}{\underset{\|}{N}}-$ $-CH_2CH_2-$ | 2-Cℓ | 0 | |
| Ⅲ-1152 | $=NCH_2\overset{\overset{O}{\|}}{P}(OEt)_2$ | | $CF_3$ | $-\underset{CH_3}{\underset{\|}{N}}-$ $-CH=CH-$ | 2-Cℓ | 0 | |
| Ⅲ-1153 | $=NCH_2\overset{\overset{O}{\|}}{P}(OEt)_2$ | | $CF_3$ | $-\underset{CH_3}{\underset{\|}{N}}-$ $-CH_2CH_2CH_2-$ | 2-Cℓ | 0 | |
| Ⅲ-1154 | $=NCH_2\overset{\overset{O}{\|}}{P}(OEt)_2$ | | $CF_3$ | $-\underset{CH_2OEt}{\underset{\|}{N}}-$ $-CH_2CH_2-$ | 2-Cℓ | 0 | |
| Ⅲ-1155 | $=NCH_2\overset{\overset{O}{\|}}{P}(OEt)_2$ | | $CF_3$ | $-\underset{CH_2OEt}{\underset{\|}{N}}-$ $-CH=CH-$ | 2-Cℓ | 0 | |
| Ⅲ-1156 | $=NCH_2\overset{\overset{O}{\|}}{P}(OEt)_2$ | | $CF_3$ | $-\underset{CH_2OEt}{\underset{\|}{N}}-$ $CH_2CH_2CH_2-$ | 2-Cℓ | 0 | |
| Ⅲ-1157 | $=NCH_2\overset{\overset{O}{\|}}{P}(OEt)_2$ | | i-Pr | O $-CH_2CH_2-$ | 2-Cℓ | 0 | |
| Ⅲ-1158 | $=NCH_2\overset{\overset{O}{\|}}{P}(OEt)_2$ | | i-Pr | O $-CH=CH-$ | 2-Cℓ | 0 | |
| Ⅲ-1159 | $=NCH_2\overset{\overset{O}{\|}}{P}(OEt)_2$ | | i-Pr | O $-CH_2CH_2CH_2-$ | 2-Cℓ | 0 | |
| Ⅲ-1160 | $=NCH_2\overset{\overset{O}{\|}}{P}(OEt)_2$ | | i-Pr | S $-CH_2CH_2-$ | 2-Cℓ | 0 | |
| Ⅲ-1161 | $=NCH_2\overset{\overset{O}{\|}}{P}(OEt)_2$ | | i-Pr | S $-CH=CH-$ | 2-Cℓ | 0 | |
| Ⅲ-1162 | $=NCH_2\overset{\overset{O}{\|}}{P}(OEt)_2$ | | i-Pr | S $-CH_2CH_2CH_2-$ | 2-Cℓ | 0 | |
| Ⅲ-1163 | $=NCH_2\overset{\overset{O}{\|}}{P}(OEt)_2$ | | i-Pr | -NH- $-CH_2CH_2-$ | 2-Cℓ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-1164 | $=NCH_2\overset{O}{\overset{\|}{P}}(OEt)_2$ | | i-Pr | -NH- -CH=CH- | 2-Cℓ | 0 | |
| III-1165 | $=NCH_2\overset{O}{\overset{\|}{P}}(OEt)_2$ | | i-Pr | -NH- $-CH_2CH_2CH_2-$ | 2-Cℓ | 0 | |
| III-1166 | $=NCH_2\overset{O}{\overset{\|}{P}}(OEt)_2$ | | i-Pr | $-\underset{CH_3}{N}-$ $-CH_2CH_2-$ | 2-Cℓ | 0 | |
| III-1167 | $=NCH_2\overset{O}{\overset{\|}{P}}(OEt)_2$ | | i-Pr | $-\underset{CH_3}{N}-$ -CH=CH- | 2-Cℓ | 0 | |
| III-1168 | $=NCH_2\overset{O}{\overset{\|}{P}}(OEt)_2$ | | i-Pr | $-\underset{CH_3}{N}-$ $-CH_2CH_2CH_2-$ | 2-Cℓ | 0 | |
| III-1169 | $=NCH_2\overset{O}{\overset{\|}{P}}(OEt)_2$ | | i-Pr | $-\underset{CH_2OEt}{N}-$ $-CH_2CH_2-$ | 2-Cℓ | 0 | |
| III-1170 | $=NCH_2\overset{O}{\overset{\|}{P}}(OEt)_2$ | | i-Pr | $-\underset{CH_2OEt}{N}-$ -CH=CH- | 2-Cℓ | 0 | |
| III-1171 | $=NCH_2\overset{O}{\overset{\|}{P}}(OEt)_2$ | | i-Pr | $-\underset{CH_2OEt}{N}-$ $CH_2CH_2CH_2-$ | 2-Cℓ | 0 | |
| III-1172 | $=NCH_2\overset{O}{\overset{\|}{P}}(OEt)_2$ | | Et | O $-CH_2CH_2-$ | 2-Cℓ | 0 | |
| III-1173 | $=NCH_2\overset{O}{\overset{\|}{P}}(OEt)_2$ | | Et | O -CH=CH- | 2-Cℓ | 0 | |
| III-1174 | $=NCH_2\overset{O}{\overset{\|}{P}}(OEt)_2$ | | Et | O $-CH_2CH_2CH_2-$ | 2-Cℓ | 0 | |
| III-1175 | $=NCH_2\overset{O}{\overset{\|}{P}}(OEt)_2$ | | Et | S $-CH_2CH_2-$ | 2-Cℓ | 0 | |
| III-1176 | $=NCH_2\overset{O}{\overset{\|}{P}}(OEt)_2$ | | Et | S -CH=CH- | 2-Cℓ | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| Ⅲ-1177 | O<br>‖<br>$=NCH_2P(OEt)_2$ | | Et | S $-CH_2CH_2CH_2-$ | 2-Cℓ | 0 | |
| Ⅲ-1178 | O<br>‖<br>$=NCH_2P(OEt)_2$ | | Et | -NH- $-CH_2CH_2-$ | 2-Cℓ | 0 | |
| Ⅲ-1179 | O<br>‖<br>$=NCH_2P(OEt)_2$ | | Et | -NH- -CH=CH- | 2-Cℓ | 0 | |
| Ⅲ-1180 | O<br>‖<br>$=NCH_2P(OEt)_2$ | | Et | -NH- $-CH_2CH_2CH_2-$ | 2-Cℓ | 0 | |
| Ⅲ-1181 | O<br>‖<br>$=NCH_2P(OEt)_2$ | | Et | -N- $-CH_2CH_2-$<br>\|<br>$CH_3$ | 2-Cℓ | 0 | |
| Ⅲ-1182 | O<br>‖<br>$=NCH_2P(OEt)_2$ | | Et | -N- -CH=CH-<br>\|<br>$CH_3$ | 2-Cℓ | 0 | |
| Ⅲ-1183 | O<br>‖<br>$=NCH_2P(OEt)_2$ | | Et | -N- $-CH_2CH_2CH_3-$<br>\|<br>$CH_3$ | 2-Cℓ | 0 | |
| Ⅲ-1184 | O<br>‖<br>$=NCH_2P(OEt)_2$ | | Et | -N- $-CH_2CH_2-$<br>\|<br>$CH_2OEt$ | 2-Cℓ | 0 | |
| Ⅲ-1185 | O<br>‖<br>$=NCH_2P(OEt)_2$ | | Et | -N- -CH=CH-<br>\|<br>$CH_2OEt$ | 2-Cℓ | 0 | |
| Ⅲ-1186 | O<br>‖<br>$=NCH_2P(OEt)_2$ | | Et | -N- $-CH_2CH_2CH_2-$<br>\|<br>$CH_2OEt$ | 2-Cℓ | 0 | |
| Ⅲ-1187 | O<br>‖<br>$=NCH_2P(OEt)_2$ | | $CF_3$ | S -CH=CH- | 2-Cℓ, 6-F | 0 | |
| Ⅲ-1188 | O<br>‖<br>$=NCH_2P(OEt)_2$ | | $CF_3$ | O $-CHCH_2-$<br>\|<br>$CH_3$ | 2-Cℓ, 6-F | 0 | |
| Ⅲ-1189 | O<br>‖<br>$=NCH_2P(OEt)_2$ | | $CF_3$ | O -CH=CH- | 2-Cℓ, 6-F | 0 | |

| No. | $R^1$ | $R^2$ | $R^3$ | A-B-D-(E)- | | $R^6, R^7, R^8$ | n | Physical Constants |
|---|---|---|---|---|---|---|---|---|
| III-1190 | | O<br>‖<br>$=NCH_2P(OEt)_2$ | Et | O | $-CH_2CH_2-$ | 2-Cℓ, 6-F | 0 | |
| III-1191 | | O<br>‖<br>$=NCH_2P(OEt)_2$ | Et | O | -CH=CH- | 2-Cℓ, 6-F | 0 | |
| III-1192 | | O<br>‖<br>$=NCH_2P(OEt)_2$ | Et | O | $-CH_2CH_2CH_2-$ | 2-Cℓ, 6-F | 0 | |
| III-1193 | | O<br>‖<br>$=NCH_2P(OEt)_2$ | i-Pr | O | $-CH_2CH_2-$ | 2-Cℓ, 6-F | 0 | |
| III-1194 | | O<br>‖<br>$=NCH_2P(OEt)_2$ | i-Pr | O | -CH=CH- | 2-Cℓ, 6-F | 0 | |
| III-1195 | | O<br>‖<br>$=NCH_2P(OEt)_2$ | i-Pr | O | $-CH_2CH_2CH_2-$ | 2-Cℓ, 6-F | 0 | |
| III-1196 | | O<br>‖<br>$=NCH_2P(OEt)_2$ | $CF_3$ | S | $-CH_2CH_2-$ | 2-Cℓ, 6-F | 0 | |
| III-1197 | | O<br>‖<br>$=NCH_2P(OEt)_2$ | $CF_3$ | S | -CH=CH- | 2-Cℓ, 6-F | 0 | |
| III-1198 | | O<br>‖<br>$=NCH_2P(OEt)_2$ | $CF_3$ | S | $-CH_2CH_2CH_3-$ | 2-Cℓ, 6-F | 0 | |
| III-1199 | | O<br>‖<br>$=NCH_2P(OEt)_2$ | Et | S | $-CH_2CH_2-$ | 2-Cℓ, 6-F | 0 | |
| III-1200 | | O<br>‖<br>$=NCH_2P(OEt)_2$ | Et | S | -CH=CH- | 2-Cℓ, 6-F | 0 | |
| III-1201 | | O<br>‖<br>$=NCH_2P(OEt)_2$ | Et | S | $-CH_2CH_2CH_2-$ | 2-Cℓ, 6-F | 0 | |
| III-1202 | | O<br>‖<br>$=NCH_2P(OEt)_2$ | i-Pr | S | $-CH_2CH_2-$ | 2-Cℓ, 6-F | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| Ⅲ-1203 | | $=NCH_2P(O)(OEt)_2$ | i-Pr | S -CH=CH- | 2-Cℓ, 6-F | 0 | |
| Ⅲ-1204 | | $=NCH_2P(O)(OEt)_2$ | i-Pr | S $-CH_2CH_2CH_2-$ | 2-Cℓ, 6-F | 0 | |
| Ⅲ-1205 | | $=NCH_2P(O)(OEt)_2$ | $CF_3$ | -NH- $-CH_2CH_2-$ | 2-Cℓ, 6-F | 0 | |
| Ⅲ-1206 | | $=NCH_2P(O)(OEt)_2$ | $CF_3$ | -NH- -CH=CH- | 2-Cℓ, 6-F | 0 | |
| Ⅲ-1207 | | $=NCH_2P(O)(OEt)_2$ | $CF_3$ | -NH- $-CH_2CH_2CH_2-$ | 2-Cℓ, 6-F | 0 | |
| Ⅲ-1208 | | $=NCH_2P(O)(OEt)_2$ | Et | -NH- $-CH_2CH_2-$ | 2-Cℓ, 6-F | 0 | |
| Ⅲ-1209 | | $=NCH_2P(O)(OEt)_2$ | Et | -NH- -CH=CH- | 2-Cℓ, 6-F | 0 | |
| Ⅲ-1210 | | $=NCH_2P(O)(OEt)_2$ | Et | -NH- $-CH_2CH_2CH_2-$ | 2-Cℓ, 6-F | 0 | |
| Ⅲ-1211 | | $=NCH_2P(O)(OEt)_2$ | i-Pr | -NH- $-CH_2CH_2-$ | 2-Cℓ, 6-F | 0 | |
| Ⅲ-1212 | | $=NCH_2P(O)(OEt)_2$ | i-Pr | -NH- -CH=CH- | 2-Cℓ, 6-F | 0 | |
| Ⅲ-1213 | | $=NCH_2P(O)(OEt)_2$ | i-Pr | -NH- $-CH_2CH_2CH_2-$ | 2-Cℓ, 6-F | 0 | |
| Ⅲ-1214 | | $=NCH_2P(O)(OEt)_2$ | $CF_3$ | $-N(CH_3)-$ $-CH_2CH_2-$ | 2-Cℓ, 6-F | 0 | |
| Ⅲ-1215 | | $=NCH_2P(O)(OEt)_2$ | $CF_3$ | $-N(CH_3)-$ -CH=CH- | 2-Cℓ, 6-F | 0 | |

| No. | $R^1$ | $R^2$ | $R^3$ | A-B-D-(E)- | $R^6, R^7, R^8$ | n | Physical Constants |
|---|---|---|---|---|---|---|---|
| III-1216 | | $=NCH_2P(O)(OEt)_2$ | $CF_3$ | $-N(CH_3)-$ $-CH_2CH_2CH_2-$ | 2-Cℓ, 6-F | 0 | |
| III-1217 | | $=NCH_2P(O)(OEt)_2$ | Et | $-N(CH_3)-$ $-CH_2CH_2-$ | 2-Cℓ, 6-F | 0 | |
| III-1218 | | $=NCH_2P(O)(OEt)_2$ | Et | $-N(CH_3)-$ $-CH=CH-$ | 2-Cℓ, 6-F | 0 | |
| III-1219 | | $=NCH_2P(O)(OEt)_2$ | Et | $-N(CH_3)-$ $-CH_2CH_2CH_2-$ | 2-Cℓ, 6-F | 0 | |
| III-1220 | | $=NCH_2P(O)(OEt)_2$ | i-Pr | $-N(CH_3)-$ $-CH_2CH_2-$ | 2-Cℓ, 6-F | 0 | |
| III-1221 | | $=NCH_2P(O)(OEt)_2$ | i-Pr | $-N(CH_3)-$ $-CH=CH-$ | 2-Cℓ, 6-F | 0 | |
| III-1222 | | $=NCH_2P(O)(OEt)_2$ | i-Pr | $-N(CH_3)-$ $-CH_2CH_2CH_2-$ | 2-Cℓ, 6-F | 0 | |
| III-1223 | | $=NCH_2P(O)(OEt)_2$ | $CF_3$ | $-N(CH_2OEt)-$ $-CH_2CH_2-$ | 2-Cℓ, 6-F | 0 | |
| III-1224 | | $=NCH_2P(O)(OEt)_2$ | $CF_3$ | $-N(CH_2OEt)-$ $-CH=CH-$ | 2-Cℓ, 6-F | 0 | |
| III-1225 | | $=NCH_2P(O)(OEt)_2$ | $CF_3$ | $-N(CH_2OEt)-$ $-CH_2CH_2CH_2-$ | 2-Cℓ, 6-F | 0 | |
| III-1226 | | $=NCH_2P(O)(OEt)_2$ | Et | $-N(CH_2OEt)-$ $-CH_2CH_2-$ | 2-Cℓ, 6-F | 0 | |
| III-1227 | | $=NCH_2P(O)(OEt)_2$ | Et | $-N(CH_2OEt)-$ $-CH=CH-$ | 2-Cℓ, 6-F | 0 | |
| III-1228 | | $=NCH_2P(O)(OEt)_2$ | Et | $-N(CH_2OEt)-$ $-CH_2CH_2CH_2-$ | 2-Cℓ, 6-F | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| III-1229 | $=NCH_2P(=O)(OEt)_2$ | | i-Pr | $-N(CH_2OEt)-$ $-CH_2CH_2-$ | 2-Cℓ, 6-F | 0 | |
| III-1230 | $=NCH_2P(=O)(OEt)_2$ | | i-Pr | $-N(CH_2OEt)-$ $-CH=CH-$ | 2-Cℓ, 6-F | 0 | |
| III-1231 | $=NCH_2P(=O)(OEt)_2$ | | i-Pr | $-N(CH_2OEt)-$ $-CH_2CH_2CH_2-$ | 2-Cℓ, 6-F | 0 | |
| III-1232 | $=NCH_2P(=O)(OEt)_2$ | | $CF_3$ | O $-CH_2CH_2-$ | 2,6-$Cℓ_2$ | 0 | |
| III-1233 | $=NCH_2P(=O)(OEt)_2$ | | $CF_3$ | O $-CH=CH-$ | 2,6-$Cℓ_2$ | 0 | |
| III-1234 | $=NCH_2P(=O)(OEt)_2$ | | $CF_3$ | O $-CH_2CH_2CH_2-$ | 2,6-$Cℓ_2$ | 0 | |
| III-1235 | $=NCH_2P(=O)(OEt)_2$ | | Et | O $-CH_2CH_2-$ | 2,6-$Cℓ_2$ | 0 | |
| III-1236 | $=NCH_2P(=O)(OEt)_2$ | | Et | O $-CH=CH-$ | 2,6-$Cℓ_2$ | 0 | |
| III-1237 | $=NCH_2P(=O)(OEt)_2$ | | Et | O $-CH_2CH_2CH_2-$ | 2,6-$Cℓ_2$ | 0 | |
| III-1238 | $=NCH_2P(=O)(OEt)_2$ | | i-Pr | O $-CH_2CH_2-$ | 2,6-$Cℓ_2$ | 0 | |
| III-1239 | $=NCH_2P(=O)(OEt)_2$ | | i-Pr | O $-CH=CH-$ | 2,6-$Cℓ_2$ | 0 | |
| III-1240 | $=NCH_2P(=O)(OEt)_2$ | | i-Pr | O $-CH_2CH_2CH_2-$ | 2,6-$Cℓ_2$ | 0 | |
| III-1241 | $=NCH_2P(=O)(OEt)_2$ | | $CF_3$ | S $-CH_2CH_2-$ | 2,6-$Cℓ_2$ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-1242 | O<br>‖<br>$=NCH_2P(OEt)_2$ | | $CF_3$ | S -CH=CH- | 2-Cℓ, 6-F | 0 | |
| III-1243 | O<br>‖<br>$=NCH_2P(OEt)_2$ | | $CF_3$ | S $-CH_2CH_2CH_2-$ | 2-Cℓ, 6-F | 0 | |
| III-1244 | O<br>‖<br>$=NCH_2P(OEt)_2$ | | Et | S $-CH_2CH_2-$ | 2-Cℓ, 6-F | 0 | |
| III-1245 | O<br>‖<br>$=NCH_2P(OEt)_2$ | | Et | S -CH=CH- | 2,6-$Cℓ_2$ | 0 | |
| III-1246 | O<br>‖<br>$=NCH_2P(OEt)_2$ | | Et | S $-CH_2CH_2CH_2-$ | 2,6-$Cℓ_2$ | 0 | |
| III-1247 | O<br>‖<br>$=NCH_2P(OEt)_2$ | | i-Pr | S $-CH_2CH_2-$ | 2,6-$Cℓ_2$ | 0 | |
| III-1248 | O<br>‖<br>$=NCH_2P(OEt)_2$ | | i-Pr | S -CH=CH- | 2,6-$Cℓ_2$ | 0 | |
| III-1249 | O<br>‖<br>$=NCH_2P(OEt)_2$ | | i-Pr | S $-CH_2CH_2CH_2-$ | 2,6-$Cℓ_2$ | 0 | |
| III-1250 | O<br>‖<br>$=NCH_2P(OEt)_2$ | | $CF_3$ | -NH- $-CH_2CH_2-$ | 2,6-$Cℓ_2$ | 0 | |
| III-1251 | O<br>‖<br>$=NCH_2P(OEt)_2$ | | $CF_3$ | -NH- -CH=CH- | 2,6-$Cℓ_2$ | 0 | |
| III-1252 | O<br>‖<br>$=NCH_2P(OEt)_2$ | | $CF_3$ | -NH- $-CH_2CH_2CH_2-$ | 2,6-$Cℓ_2$ | 0 | |
| III-1253 | O<br>‖<br>$=NCH_2P(OEt)_2$ | | Et | -NH- $-CH_2CH_2-$ | 2,6-$Cℓ_2$ | 0 | |
| III-1254 | O<br>‖<br>$=NCH_2P(OEt)_2$ | | Et | -NH- -CH=CH- | 2,6-$Cℓ_2$ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-1255 | | $=NCH_2\overset{O}{\overset{\|}{P}}(OEt)_2$ | Et | $-NH-$ $-CH_2CH_2CH_2-$ | 2-Cℓ, 6-F | 0 | |
| III-1256 | | $=NCH_2\overset{O}{\overset{\|}{P}}(OEt)_2$ | i-Pr | $-NH-$ $-CH_2CH_2-$ | 2-Cℓ, 6-F | 0 | |
| III-1257 | | $=NCH_2\overset{O}{\overset{\|}{P}}(OEt)_2$ | i-Pr | $-NH-$ $-CH=CH-$ | 2-Cℓ, 6-F | 0 | |
| III-1258 | | $=NCH_2\overset{O}{\overset{\|}{P}}(OEt)_2$ | i-Pr | $-NH-$ $-CH_2CH_2CH_2-$ | $2,6\text{-}C\ell_2$ | 0 | |
| III-1259 | | $=NCH_2\overset{O}{\overset{\|}{P}}(OEt)_2$ | $CF_3$ | $-\underset{CH_3}{\underset{\|}{N}}-$ $-CH_2CH_2-$ | $2,6\text{-}C\ell_2$ | 0 | |
| III-1260 | | $=NCH_2\overset{O}{\overset{\|}{P}}(OEt)_2$ | $CF_3$ | $-\underset{CH_3}{\underset{\|}{N}}-$ $-CH=CH-$ | $2,6\text{-}C\ell_2$ | 0 | |
| III-1261 | | $=NCH_2\overset{O}{\overset{\|}{P}}(OEt)_2$ | $CF_3$ | $-\underset{CH_3}{\underset{\|}{N}}-$ $-CH_2CH_2CH_2-$ | $2,6\text{-}C\ell_2$ | 0 | |
| III-1262 | | $=NCH_2\overset{O}{\overset{\|}{P}}(OEt)_2$ | Et | $-\underset{CH_3}{\underset{\|}{N}}-$ $-CH_2CH_2-$ | $2,6\text{-}C\ell_2$ | 0 | |
| III-1263 | | $=NCH_2\overset{O}{\overset{\|}{P}}(OEt)_2$ | Et | $-\underset{CH_3}{\underset{\|}{N}}-$ $-CH=CH-$ | $2,6\text{-}C\ell_2$ | 0 | |
| III-1264 | | $=NCH_2\overset{O}{\overset{\|}{P}}(OEt)_2$ | Et | $-\underset{CH_3}{\underset{\|}{N}}-$ $-CH_2CH_2CH_2-$ | $2,6\text{-}C\ell_2$ | 0 | |
| III-1265 | | $=NCH_2\overset{O}{\overset{\|}{P}}(OEt)_2$ | i-Pr | $-\underset{CH_3}{\underset{\|}{N}}-$ $-CH_2CH_2-$ | $2,6\text{-}C\ell_2$ | 0 | |
| III-1266 | | $=NCH_2\overset{O}{\overset{\|}{P}}(OEt)_2$ | i-Pr | $-\underset{CH_3}{\underset{\|}{N}}-$ $-CH=CH-$ | $2,6\text{-}C\ell_2$ | 0 | |
| III-1267 | | $=NCH_2\overset{O}{\overset{\|}{P}}(OEt)_2$ | i-Pr | $-\underset{CH_3}{\underset{\|}{N}}-$ $-CH_2CH_2CH_2-$ | $2,6\text{-}C\ell_2$ | 0 | |

| No. | $R^1$ | $R^2$ | $R^3$ | A-B-D-(E)- | $R^6, R^7, R^8$ | n | Physical Constants |
|---|---|---|---|---|---|---|---|
| III-1268 | $=NCH_2P(O)(OEt)_2$ | | $CF_3$ | $-N(CH_2OEt)-$ $-CH_2CH_2-$ | 2,6-$Cl_2$ | 0 | |
| III-1269 | $=NCH_2P(O)(OEt)_2$ | | $CF_3$ | $-N(CH_2OEt)-$ $-CH=CH-$ | 2,6-$Cl_2$ | 0 | |
| III-1270 | $=NCH_2P(O)(OEt)_2$ | | $CF_3$ | $-N(CH_2OEt)-$ $-CH_2CH_2CH_2-$ | 2,6-$Cl_2$ | 0 | |
| III-1271 | $=NCH_2P(O)(OEt)_2$ | | Et | $-N(CH_2OEt)-$ $-CH_2CH_2-$ | 2,6-$Cl_2$ | 0 | |
| III-1272 | $=NCH_2P(O)(OEt)_2$ | | Et | $-N(CH_2OEt)-$ $-CH=CH-$ | 2,6-$Cl_2$ | 0 | |
| III-1273 | $=NCH_2P(O)(OEt)_2$ | | Et | $-N(CH_2OEt)-$ $-CH_2CH_2CH_2-$ | 2,6-$Cl_2$ | 0 | |
| III-1274 | $=NCH_2P(O)(OEt)_2$ | | i-Pr | $-N(CH_2OEt)-$ $-CH_2CH_2-$ | 2,6-$Cl_2$ | 0 | |
| III-1275 | $=NCH_2P(O)(OEt)_2$ | | i-Pr | $-N(CH_2OEt)-$ $-CH=CH-$ | 2,6-$Cl_2$ | 0 | |
| III-1276 | $=NCH_2P(O)(OEt)_2$ | | i-Pr | $-N(CH_2OEt)-$ $-CH_2CH_2CH_2-$ | 2,6-$Cl_2$ | 0 | |
| III-1277 | OH, H | | $CF_3$ | O $-CH(CH_3)CH_2-$ | 2-Cl | 0 | |
| III-1278 | OH, H | | $CF_3$ | O $-CH=CH-$ | 2-Cl | 0 | |
| III-1279 | OH, H | | $CF_3$ | O $-CH_2CH_2CH_2-$ | 2-Cl | 0 | |
| III-1280 | OH, H | | $CF_3$ | S $-CH_2CH_2-$ | 2-Cl | 0 | |
| III-1281 | OH, H | | $CF_3$ | S $-CH=CH-$ | 2-Cl | 0 | |
| III-1282 | OH, H | | $CF_3$ | S $-CH_2CH_2CH_2-$ | 2-Cl | 0 | |
| III-1283 | OH, H | | $CF_3$ | -NH- $-CH_2CH_2-$ | 2-Cl | 0 | |
| III-1284 | OH, H | | $CF_3$ | -NH- $-CH=CH-$ | 2-Cl | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| III-1285 | OH, | H | $CF_3$ | S $-CH_2CH_2CH_2-$ | 2-Cℓ | 0 | |
| III-1286 | OH, | H | $CF_3$ | -N($CH_3$)- $-CH_2CH_2-$ | 2-Cℓ | 0 | |
| III-1287 | OH, | H | $CF_3$ | -N($CH_3$)- -CH=CH- | 2-Cℓ | 0 | |
| III-1288 | OH, | H | $CF_3$ | -N($CH_3$)- $-CH_2CH_2CH_2-$ | 2-Cℓ | 0 | |
| III-1289 | OH, | H | $CF_3$ | -N($CH_2OEt$)- $-CH_2CH_2-$ | 2-Cℓ | 0 | |
| III-1290 | OH, | H | $CF_3$ | -N($CH_2OEt$)- -CH=CH- | 2-Cℓ | 0 | |
| III-1291 | OH, | H | $CF_3$ | -N($CH_2OEt$)- $-CH_2CH_2CH_2-$ | 2-Cℓ | 0 | |
| III-1292 | OH, | H | i-Pr | O $-CH_2CH_2-$ | 2-Cℓ | 0 | |
| III-1293 | OH, | H | i-Pr | O -CH=CH- | 2-Cℓ | 0 | |
| III-1294 | OH, | H | i-Pr | O $-CH_2CH_2CH_2-$ | 2-Cℓ | 0 | |
| III-1295 | OH, | H | i-Pr | S $-CH_2CH_2-$ | 2-Cℓ | 0 | |
| III-1296 | OH, | H | i-Pr | S -CH=CH- | 2-Cℓ | 0 | |
| III-1297 | OH, | H | i-Pr | S $-CH_2CH_2CH_2-$ | 2-Cℓ | 0 | |
| III-1298 | OH, | H | i-Pr | -NH- $-CH_2CH_2-$ | 2-Cℓ | 0 | |
| III-1299 | OH, | H | i-Pr | -NH- -CH=CH- | 2-Cℓ | 0 | |
| III-1300 | OH, | H | i-Pr | -NH- $CH_2CH_2CH_2-$ | 2-Cℓ | 0 | |
| III-1301 | OH, | H | i-Pr | -N($CH_3$)- $-CH_2CH_2-$ | 2-Cℓ | 0 | |
| III-1302 | OH, | H | i-Pr | -N($CH_3$)- -CH=CH- | 2-Cℓ | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| Ⅲ-1303 | OH, | H | i-Pr | -N- -$CH_2CH_2CH_2$-<br>\|<br>$CH_3$ | 2-Cℓ | 0 | |
| Ⅲ-1304 | OH, | H | i-Pr | -N- -$CH_2CH_2$-<br>\|<br>$CH_2OEt$ | 2-Cℓ | 0 | |
| Ⅲ-1305 | OH, | H | i-Pr | -N- -CH=CH-<br>\|<br>$CH_2OEt$ | 2-Cℓ | 0 | |
| Ⅲ-1306 | OH, | H | i-Pr | -N- -$CH_2CH_2CH_2$-<br>\|<br>$CH_2OEt$ | 2-Cℓ | 0 | |
| Ⅲ-1307 | OH, | H | Et | O -$CH_2CH_2$- | 2-Cℓ | 0 | |
| Ⅲ-1308 | OH, | H | Et | O -CH=CH- | 2-Cℓ | 0 | |
| Ⅲ-1309 | OH, | H | Et | O -$CH_2CH_2CH_2$- | 2-Cℓ | 0 | |
| Ⅲ-1310 | OH, | H | Et | S -$CH_2CH_2$- | 2-Cℓ | 0 | |
| Ⅲ-1311 | OH, | H | Et | S -CH=CH- | 2-Cℓ | 0 | |
| Ⅲ-1312 | OH, | H | Et | S -$CH_2CH_2CH_2$- | 2-Cℓ | 0 | |
| Ⅲ-1313 | OH, | H | Et | -NH- -$CH_2CH_2$- | 2-Cℓ | 0 | |
| Ⅲ-1314 | OH, | H | Et | -NH- -CH=CH- | 2-Cℓ | 0 | |
| Ⅲ-1315 | OH, | H | Et | -NH- $CH_2CH_2CH_2$- | 2-Cℓ | 0 | |
| Ⅲ-1316 | OH, | H | Et | -N- -$CH_2CH_2$-<br>\|<br>$CH_3$ | 2-Cℓ | 0 | |
| Ⅲ-1317 | OH, | H | Et | -N- -CH=CH-<br>\|<br>$CH_3$ | 2-Cℓ | 0 | |
| Ⅲ-1318 | OH, | H | Et | -N- -$CH_2CH_2CH_2$-<br>\|<br>$CH_3$ | 2-Cℓ | 0 | |
| Ⅲ-1319 | OH, | H | Et | -N- -$CH_2CH_2$-<br>\|<br>$CH_2OEt$ | 2-Cℓ | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| III-1320 | OH, | H | Et | -N- -CH=CH-<br>\|<br>$CH_2OEt$ | 2-Cℓ | 0 | |
| III-1321 | OH, | H | Et | -N- $-CH_2CH_2CH_2-$<br>\|<br>$CH_2OEt$ | 2-Cℓ | 0 | |
| III-1322 | OH, | H | $CF_3$ | O $-CH_2CH_2-$ | 2-Cℓ, 6-F | 0 | |
| III-1323 | OH, | H | $CF_3$ | O -CH=CH- | 2-Cℓ, 6-F | 0 | |
| III-1324 | OH, | H | $CF_3$ | O $-CH_2CH_2CH_2-$ | 2-Cℓ, 6-F | 0 | |
| III-1325 | OH, | H | Et | O $-CH_2CH_2-$ | 2-Cℓ, 6-F | 0 | |
| III-1326 | OH, | H | Et | O -CH=CH- | 2-Cℓ, 6-F | 0 | |
| III-1327 | OH, | H | Et | O $-CH_2CH_2CH_2-$ | 2-Cℓ, 6-F | 0 | |
| III-1328 | OH, | H | i-Pr | O $-CH_2CH_2-$ | 2-Cℓ, 6-F | 0 | |
| III-1329 | OH, | H | i-Pr | O -CH=CH- | 2-Cℓ, 6-F | 0 | |
| III-1330 | OH, | H | i-Pr | O $CH_2CH_2CH_2-$ | 2-Cℓ, 6-F | 0 | |
| III-1331 | OH, | H | $CF_3$ | S $-CH_2CH_2-$ | 2-Cℓ, 6-F | 0 | |
| III-1332 | OH, | H | $CF_3$ | S -CH=CH- | 2-Cℓ, 6-F | 0 | 〔86−88〕 |
| III-1333 | OH, | H | $CF_3$ | S $-CH_2CH_2CH_2-$ | 2-Cℓ, 6-F | 0 | |
| III-1334 | OH, | H | Et | S $-CH_2CH_2-$ | 2-Cℓ, 6-F | 0 | |
| III-1335 | OH, | H | Et | S -CH=CH- | 2-Cℓ, 6-F | 0 | |
| III-1336 | OH, | H | Et | S $-CH_2CH_2CH_2-$ | 2-Cℓ, 6-F | 0 | |
| III-1337 | OH, | H | i-Pr | S $-CH_2CH_2-$ | 2-Cℓ, 6-F | 0 | |
| III-1338 | OH, | H | i-Pr | S -CH=CH- | 2-Cℓ, 6-F | 0 | |
| III-1339 | OH, | H | i-Pr | S $CH_2CH_2CH_2-$ | 2-Cℓ, 6-F | 0 | |
| III-1340 | OH, | H | $CF_3$ | -NH- $-CH_2CH_2-$ | 2-Cℓ, 6-F | 0 | |
| III-1341 | OH, | H | $CF_3$ | -NH- -CH=CH- | 2-Cℓ, 6-F | 0 | |
| III-1342 | OH, | H | $CF_3$ | -NH- $-CH_2CH_2CH_2-$ | 2-Cℓ, 6-F | 0 | |
| III-1343 | OH, | H | Et | -NH- $-CH_2CH_2-$ | 2-Cℓ, 6-F | 0 | |

| No. | $R^1$ | $R^2$ | $R^3$ | A-B-D-(E)- | $R^6, R^7, R^8$ | n | Physical Constants |
|---|---|---|---|---|---|---|---|
| III-1344 | OH, | H | Et | -NH- -CH=CH- | 2-Cℓ, 6-F | 0 | |
| III-1345 | OH, | H | Et | -NH- $-CH_2CH_2CH_2-$ | 2-Cℓ, 6-F | 0 | |
| III-1346 | OH, | H | i-Pr | -NH- $-CH_2CH_2-$ | 2-Cℓ, 6-F | 0 | |
| III-1347 | OH, | H | i-Pr | -NH- -CH=CH- | 2-Cℓ, 6-F | 0 | |
| III-1348 | OH, | H | i-Pr | -NH- $-CH_2CH_2CH_2-$ | 2-Cℓ, 6-F | 0 | |
| III-1349 | OH, | H | $CF_3$ | -N($CH_3$)- $-CH_2CH_2-$ | 2-Cℓ, 6-F | 0 | |
| III-1350 | OH, | H | $CF_3$ | -N($CH_3$)- -CH=CH- | 2-Cℓ, 6-F | 0 | |
| III-1351 | OH, | H | $CF_3$ | -N($CH_3$)- $-CH_2CH_2CH_2-$ | 2-Cℓ, 6-F | 0 | |
| III-1352 | OH, | H | Et | -N($CH_3$)- $-CH_2CH_2-$ | 2-Cℓ, 6-F | 0 | |
| III-1353 | OH, | H | Et | -N($CH_3$)- -CH=CH- | 2-Cℓ, 6-F | 0 | |
| III-1354 | OH, | H | Et | -N($CH_3$)- $-CH_2CH_2CH_2-$ | 2-Cℓ, 6-F | 0 | |
| III-1355 | OH, | H | i-Pr | -N($CH_3$)- $-CH_2CH_2-$ | 2-Cℓ, 6-F | 0 | |
| III-1356 | OH, | H | i-Pr | -N($CH_3$)- -CH=CH- | 2-Cℓ, 6-F | 0 | |
| III-1357 | OH, | H | i-Pr | -N($CH_3$)- $-CH_2CH_2CH_2-$ | 2-Cℓ, 6-F | 0 | |
| III-1358 | OH, | H | $CF_3$ | -N($CH_2OEt$)- $-CH_2CH_2-$ | 2-Cℓ, 6-F | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| III-1359 | OH, | H | $CF_3$ | -N(CH₂OEt)- -CH=CH- | 2-Cℓ, 6-F | 0 | |
| III-1360 | OH, | H | $CF_3$ | -N(CH₂OEt)- $-CH_2CH_2CH_2-$ | 2-Cℓ, 6-F | 0 | |
| III-1361 | OH, | H | Et | -N(CH₂OEt)- $-CH_2CH_2-$ | 2-Cℓ, 6-F | 0 | |
| III-1362 | OH, | H | Et | -N(CH₂OEt)- -CH=CH- | 2-Cℓ, 6-F | 0 | |
| III-1363 | OH, | H | Et | -N(CH₂OEt)- $-CH_2CH_2CH_2-$ | 2-Cℓ, 6-F | 0 | |
| III-1364 | OH, | H | i-Pr | -N(CH₂OEt)- $-CH_2CH_2-$ | 2-Cℓ, 6-F | 0 | |
| III-1365 | OH, | H | i-Pr | -N(CH₂OEt)- -CH=CH- | 2-Cℓ, 6-F | 0 | |
| III-1366 | OH, | H | i-Pr | -N(CH₂OEt)- $-CH_2CH_2CH_2-$ | 2-Cℓ, 6-F | 0 | |
| III-1367 | OH, | H | $CF_3$ | O $-CH_2CH_2-$ | 2, 6-$Cℓ_2$ | 0 | |
| III-1368 | OH, | H | $CF_3$ | O -CH=CH- | 2, 6-$Cℓ_2$ | 0 | |
| III-1369 | OH, | H | $CF_3$ | O $-CH_2CH_2CH_2-$ | 2, 6-$Cℓ_2$ | 0 | **12 |
| III-1370 | OH, | H | Et | O $-CH_2CH_2-$ | 2, 6-$Cℓ_2$ | 0 | |
| III-1371 | OH, | H | Et | O -CH=CH- | 2, 6-$Cℓ_2$ | 0 | |
| III-1372 | OH, | H | Et | O $-CH_2CH_2CH_2-$ | 2, 6-$Cℓ_2$ | 0 | |
| III-1373 | OH, | H | i-Pr | O $-CH_2CH_2-$ | 2, 6-$Cℓ_2$ | 0 | |
| III-1374 | OH, | H | i-Pr | O -CH=CH- | 2, 6-$Cℓ_2$ | 0 | |
| III-1375 | OH, | H | i-Pr | O $-CH_2CH_2CH_2-$ | 2, 6-$Cℓ_2$ | 0 | |
| III-1376 | OH, | H | $CF_3$ | S $-CH_2CH_2-$ | 2, 6-$Cℓ_2$ | 0 | |

| No. | $R^1$ | $R^2$ | $R^{3'}$ | A-B-D-(E)- | $R^6, R^7, R^8$ | n | Physical Constants |
|---|---|---|---|---|---|---|---|
| III-1377 | OH, | H | $CF_3$ | S $-CH=CH-$ | $2,6-C\ell_2$ | 0 | |
| III-1378 | OH, | H | $CF_3$ | S $-CH_2CH_2CH_2-$ | $2,6-C\ell_2$ | 0 | |
| III-1379 | OH, | H | Et | S $-CH_2CH_2-$ | $2,6-C\ell_2$ | 0 | |
| III-1380 | OH, | H | Et | S $-CH=CH-$ | $2,6-C\ell_2$ | 0 | |
| III-1381 | OH, | H | Et | S $-CH_2CH_2CH_2-$ | $2,6-C\ell_2$ | 0 | |
| III-1382 | OH, | H | i-Pr | S $-CH_2CH_2-$ | $2,6-C\ell_2$ | 0 | |
| III-1383 | OH, | H | i-Pr | S $-CH=CH-$ | $2,6-C\ell_2$ | 0 | |
| III-1384 | OH, | H | i-Pr | S $-CH_2CH_2CH_2-$ | $2,6-C\ell_2$ | 0 | |
| III-1385 | OH, | H | $CF_3$ | -NH- $-CH_2CH_2-$ | $2,6-C\ell_2$ | 0 | |
| III-1386 | OH, | H | $CF_3$ | -NH- $-CH=CH-$ | $2,6-C\ell_2$ | 0 | |
| III-1387 | OH, | H | $CF_3$ | -NH- $-CH_2CH_2CH_2-$ | $2,6-C\ell_2$ | 0 | |
| III-1388 | OH, | H | Et | -NH- $-CH_2CH_2-$ | $2,6-C\ell_2$ | 0 | |
| III-1389 | OH, | H | Et | -NH- $-CH=CH-$ | $2,6-C\ell_2$ | 0 | |
| III-1390 | OH, | H | Et | -NH- $-CH_2CH_2CH_2-$ | $2,6-C\ell_2$ | 0 | |
| III-1391 | OH, | H | i-Pr | -NH- $-CH_2CH_2-$ | $2,6-C\ell_2$ | 0 | |
| III-1392 | OH, | H | i-Pr | -NH- $-CH=CH-$ | $2,6-C\ell_2$ | 0 | |
| III-1393 | OH, | H | i-Pr | -NH- $-CH_2CH_2CH_2-$ | $2,6-C\ell_2$ | 0 | |
| III-1394 | OH, | H | $CF_3$ | $-N(CH_3)-$ $-CH_2CH_2-$ | $2,6-C\ell_2$ | 0 | |
| III-1395 | OH, | H | $CF_3$ | $-N(CH_3)-$ $-CH=CH-$ | $2,6-C\ell_2$ | 0 | |
| III-1396 | OH, | H | $CF_3$ | $-N(CH_3)-$ $-CH_2CH_2CH_3-$ | $2,6-C\ell_2$ | 0 | |
| III-1397 | OH, | H | Et | $-N(CH_3)-$ $-CH_2CH_2-$ | $2,6-C\ell_2$ | 0 | |

| No. | $R^1$ | $R^2$ | $R^3$ | A-B-D-(E)- | $R^6, R^7, R^8$ | n | Physical Constants |
|---|---|---|---|---|---|---|---|
| Ⅲ-1398 | OH, | H | Et | $-N(CH_3)-$ $-CH=CH-$ | 2,6-$C\ell_2$ | 0 | |
| Ⅲ-1399 | OH, | H | Et | $-N(CH_3)-$ $-CH_2CH_2CH_2-$ | 2,6-$C\ell_2$ | 0 | |
| Ⅲ-1400 | OH, | H | i-Pr | $-N(CH_3)-$ $-CH_2CH_2-$ | 2,6-$C\ell_2$ | 0 | |
| Ⅲ-1401 | OH, | H | i-Pr | $-N(CH_3)-$ $-CH=CH-$ | 2,6-$C\ell_2$ | 0 | |
| Ⅲ-1402 | OH, | H | i-Pr | $-N(CH_3)-$ $-CH_2CH_2CH_2-$ | 2,6-$C\ell_2$ | 0 | |
| Ⅲ-1403 | OH, | H | $CF_3$ | $-N(CH_2OEt)-$ $-CH_2CH_2-$ | 2,6-$C\ell_2$ | 0 | |
| Ⅲ-1404 | OH, | H | $CF_3$ | $-N(CH_2OEt)-$ $-CH=CH-$ | 2,6-$C\ell_2$ | 0 | |
| Ⅲ-1405 | OH, | H | $CF_3$ | $-N(CH_2OEt)-$ $-CH_2CH_2CH_2-$ | 2,6-$C\ell_2$ | 0 | |
| Ⅲ-1406 | OH, | H | Et | $-N(CH_2OEt)-$ $-CH_2CH_2-$ | 2,6-$C\ell_2$ | 0 | |
| Ⅲ-1407 | OH, | H | Et | $-N(CH_2OEt)-$ $-CH=CH-$ | 2,6-$C\ell_2$ | 0 | |
| Ⅲ-1408 | OH, | H | Et | $-N(CH_2OEt)-$ $-CH_2CH_2CH_2-$ | 2,6-$C\ell_2$ | 0 | |
| Ⅲ-1409 | OH, | H | i-Pr | $-N(CH_2OEt)-$ $-CH_2CH_2-$ | 2,6-$C\ell_2$ | 0 | |
| Ⅲ-1410 | OH, | H | i-Pr | $-N(CH_2OEt)-$ $-CH=CH-$ | 2,6-$C\ell_2$ | 0 | |

| No. | $R^1$ | $R^2$ | $R^3$ | A-B-D-(E)- | $R^6$, $R^7$, $R^8$ | n | Physical Constants |
|---|---|---|---|---|---|---|---|
| III-1411 | OH, | H | i-Pr | -N- $-CH_2CH_2CH_2-$<br>|<br>$CH_2OEt$ | 2,6-$Cl_2$ | 0 | |
| III-1412 | Cl | H | $CF_3$ | -O $CH_2CH_2-$ | 2-Cl | 0 | |
| III-1413 | Br | H | $CF_3$ | -O $CH_2CH_2-$ | 2-Cl | 0 | |
| III-1414 | F | H | $CF_3$ | -O $CH_2CH_2-$ | 2-Cl | 0 | |
| III-1415 | F | F | $CF_3$ | -O $CH_2CH_2-$ | 2-Cl | 0 | |
| III-1416 | Cl | Cl | $CF_3$ | -O $CH_2CH_2-$ | 2-Cl | 0 | |
| III-1417 | Br | Br | $CF_3$ | -O $CH_2CH_2-$ | 2-Cl | 0 | |
| III-1418 | $OCH_3$ | H | $CF_3$ | -O $CH_2CH_2-$ | 2-Cl | 0 | |
| III-1419 | $SCH_3$ | H | $CF_3$ | -O $CH_2CH_2-$ | 2-Cl | 0 | |
| III-1420 | =O | | -COOEt | -O $CH_2CH_2-$ | 2-Cl | 0 | |
| III-1421 | =O | | $CH_3$<br>-CON<br>$CH_3$ | -O $CH_2CH_2-$ | 2-Cl | 0 | |
| III-1422 | =O | | 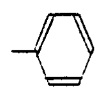 | -O $CH_2CH_2-$ | 2-Cl | 0 | |
| III-1423 | =O | | 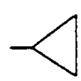 | -O $CH_2CH_2-$ | 2-Cl | 0 | |
| III-1424 | =O | | O<br>||<br>$-CCH_3$ | -O $CH_2CH_2-$ | 2-Cl | 0 | |
| III-1425 | =O | | $NO_2$ | -O $CH_2CH_2-$ | 2-Cl | 0 | |
| III-1426 | H, | H | $CF_3$ | -O $CH_2CH_2-$ | 2-Et | 0 | $n_D^{23.9}$ 1.5235 |
| III-1427 | H, | H | OH | $-CH_2SCH_2-$ | 2-Cl | 0 | [246-248] |
| III-1428 | H, | CN | $CF_3$ | $-O-CH_2CH_2-$ | 2-CN | 0 | [134-135] |
| III-1429 | H, | H | OH | $-CH_2-CH_2NCH_2-$<br>|<br>$CH_2Ph$ | 2-Cl | 0 | [119-204] |
| III-1430 | H, | H | Cl | $-CH_2-CH_2NCH_2-$<br>|<br>$CH_2Ph$ | 2-Cl | 0 | **13 |

| No. | $R^1$ $R^2$ | $R^3$ | A-B-D-(E)- | $R^6, R^7, R^8$ | n | Physical Constants |
|---|---|---|---|---|---|---|
| III-1431 | H, H | OMe | $-CH_2-CH_2NCH_2-$<br>\|<br>$CH_2Ph$ | 2-Cℓ | 0 | $n_D^{20.7}$ 1.5860 |
| III-1432 | H, H | $CF_3$ | $-O\ CH_2CH_2-$ | 2,4-$F_2$ | 1 | [77-78] |
| III-1433 | H, H | $CF_3$ | $-O\ CH_2CH_2-$ | 2,3,4,5,6-$F_5$ | 3 | [68-69] |
| III-1434 | =O | $CF_3$ | $-O\ CH_2CH_2-$ | 2,3,4,5,6-$F_5$ | 3 | [107-108] |
| III-1435 | H, F | $CF_3$ | $-O\ CH_2CH_2CH_2-$ | 2,6-$Cℓ_2$ | 0 | [108-110] |
| III-1436 | H, OMe | $CF_3$ | $-O\ CH_2CH_2CH_2-$ | 2,6-$Cℓ_2$ | 0 | [132-133] |
| III-1437 | H, H | i-Pr | $-O\ CH_2CH_2-$ | 2-Cℓ, 6-F | 0 | [ 99-111] |
| III-1438 | H, H | Et | $-O\ CH_2CH_2-$ | 2-Cℓ, 6-F | 0 | [140-141] |
| III-1439 | H, H | $CF_3$ | $-CH_2-CH_2SCH_2-$<br>‖<br>O | 2-Cℓ, 6-F | 0 | [136-137] |
| III-1440 | H, H | Et | $-CH_2-SCH_2-$ | 2-Cℓ, 6-F | 0 | [ 48- 50] |
| *3 | | | | | | [230-231] |
| III-1441 | H, H | $NMe_2$ | -CH=CHS- | 2-Cℓ | 0 | [134-136] |
| III-1442 | H, H | $CF_3$ | $-CH_2-CH_2OCH_2-$ | 2-Cℓ, 6-F | 0 | [ 93- 95] |
| III-1443 | H, COOEt | $CF_3$ | $-O\ CH_2CH_2-$ | 2-Cℓ | 0 | $n_D^{24.5}$ 1.5245 |
| III-1444 | H, COOH | $CF_3$ | $-O\ CH_2CH_2-$ | 2-Cℓ | 0 | [105-106, dec ] |
| III-1445 | H, H | $CF_3$ | $-CH_2-CH_2SCH_2-$ | 2-Cℓ, 6-F | 0 | [ 97- 98] |
| III-1446 | H, H | $CF_3$ | $-NH-CH_2CH_2-$ | 2-Cℓ | 0 | [166-168] |
| III-1447 | H, H | $CF_3$ | $S\ -CH_2CH_2CH_2-$ | 2,6-$Cℓ_2$ | 0 | [ 154 ] |
| III-1448 | =O | $CF_3$ | $S\ -CH_2CH_2CH_2-$ | 2,6-$Cℓ_2$ | 0 | [143-144] |
| III-1449 | =O | $CF_3$ | -NH-CH=CH- | 2-Cℓ | 0 | [230°Cup] |
| III-1450 | H, CN | $CF_3$ | $-O-CH_2CH_2-$ | 2-Cℓ | 0 | [ 94- 96] |
| III-1451 | H, H | OH | -CH=CHS- | 2-Cℓ | 0 | [223-224] |

| No. | $R^1$ | $R^2$ | $R^3$ | A-B-D-(E)- | $R^6$, $R^7$, $R^8$ | n | Physical Constants |
|---|---|---|---|---|---|---|---|
| III-1452 | H, | H | Cℓ | -CH=CHS- | 2-Cℓ | 0 | [ 93-94 ] |
| III-1453 | H, | H | SMe | -CH=CHS- | 2-Cℓ | 0 | [92.5-93.5] |
| III-1454 | H, | H | OMe | -CH=CHS- | 2-Cℓ | 0 | $n_D^{22.2}$ 1.6225 |
| III-1455 | =O | | OH | -CH=CHS- | 2-Cℓ | 0 | [220-221] |
| III-1456 | =O | | $CHF_2$ | $-O-CH_2CH_2-$ | 2-Cℓ, 6-F | 0 | [110-113] |
| III-1457 | H, | COOMe | $CF_3$ | $-O-CH_2CH_2-$ | 2-Cℓ | 0 | $n_D^{24.5}$ 1.5282 |
| III-1458 | =CHCOONa | | $CF_3$ | -S CH=CH- | 2-Cℓ, 6-F | 0 | [230℃dec] |
| III-1459 | $=CHCONMe_2$ | | $CF_3$ | -S CH=CH- | 2-Cℓ, 6-F | 0 | [135-136] |
| III-1460 | H, | H | Et | $-S-CH_2CH_2-$ | 2-Cℓ, 6-F | 0 | [136-137] |
| III-1461 | H, | H | Et | $-CH_2-CH_2OCH_2-$ | 2-Cℓ, 6-F | 0 | [ 78- 80] |
| III-1462 | =O | | $CF_3$ | $-N(CH_2OEt)-CH=CH-$ | 2-Cℓ | 0 | $n_D^{22.8}$ 1.5664 |
| III-1463 | H, | H | SMe | $-CH_2-CH_2N(CH_2Ph)CH_2-$ | 2-Cℓ | 0 | $n_D^{22.5}$ 1.6124 |

＊3

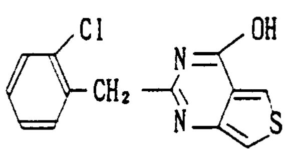

＊＊1 δ5.15(2H, d), 5.75(1H, m), 7.4-7.8(4H, m)

＊＊2 δ1.50(3H, d), 1.80(1H, m), 2.20(1H, m), 3.00(2H, m), 4.50(1H, m), 7.40(3H, m), 7.70(1H, m)

＊＊3 δ1.45(3H, d), 1.85(1H, m), 2.35(1H, m), 2.95(1H, m), 3.25(1H, m), 3.55(1H, m), 7.25-7.70(4H, m)

**4 δ0.78(3H, t), 1.0-1.25(4H, m), 3.10(2H, m), 3.45(2H, m), 4.75(2H, t), 5.45(1H, m), 7.25-7.50(4H, m), 7.60(1H, s)

**5 δ1.55(3H, d), 1.85(1H, m), 2.20(1H, m), 2.90-3.25(2H, m), 4.55(1H, m), 7.05-7.45(3H, m), 7.60(1H, s)

**6 δ1.3-1.4(6H, m), 4.00-4.40(6H, m), 7.05(1H, d), 7.3-7.5(4H, m), 7.95(1H, d)

**7 δ3.35(3H, d), 3.4(2H, m), 4.7(2H, t), 7.2-7.45(4H, m), 11.25(1H, br, d)

**8 δ1.2(3H, t), 3.35(2H, t), 3.55(2H, m), 4.7(2H, t), 4.8(1H, m), 7.2-7.55(4H, m)

**9 δ2.95(3H, s), 3.4(2H, t), 4.7(2H, t), 7.2-7.4(4H, m)

**10 δ1.00(3H, t), 1.30(3H, t), 3.45(2H, m), 4.05(2H, q), 4.30(2H, q), 4.75(2H, t), 7.30(4H, m)

**11 δ1.15(3H, t), 2.05(2H, m), 2.95(2H, t), 4.10(2H, q), 4.40(2H, t), 7.20-7.40(3H, m), 7.65(1H, s)

**12 δ2.10(2H, m), 3.00(2H, t), 4.45(2H, t), 4.85(1H, d), 6.55(1H, d), 7.10-7.30(3H, m)

**13 δ2.75(4H, s), 3.40(2H, s), 3.70(2H, s), 4.10(2H, s), 7.30(9H, m)

Herbicides and Fungicides

The compounds specified in the present invention show high herbicidal activity by either means of soil treatment or foliar application in the field of upland crops. The compounds of the present invention show high herbicidal activity particularly by soil treatment to various weeds in upland crop fields, such as crabgrass, giant foxtail and livid amaranth, wherein some compounds having selectivity to crops, such as maize, cereals and soybean, are also contained. In addition, several compounds which have excellent herbicidal activity against weeds in paddy rice fields, such as barnyardgrass, smallflower umbrellaplant and Sagittaris trifolia and selectivity to rice plants, are also included therein. Furthermore, the compounds of the present invention can be utilized for the control of weeds in orchards, lawns, railways, vacant land, etc.

On the other hand, the compounds of the present invention have superior fungicidal activity against wide range of pathogenic fungi, and therefore, it is possible to use the compounds of the present invention for the control of various fungus diseases outbreaks in agricultural and horticultural crop cultivation including ornamental flowers, tuff and meadows. For examples, the compounds of the present invention can be used for the control of many plant diseases, such as Cercospora leaf spot ( Cercospora beticola) on sugarbeets; Mycosphaerella leaf spot (Mycosphaerella arachidis) and leaf spot (Mycosphaerella berkeleyi) on groundnuts; powdery mildew ( Sphaerotheca fuliginea), gummy stem blight (Mycosphaerella melonis), sclerotinia rot (Sclerotinia sclerotiorum), gray-mold (Botrytis cinerea) and scab (Cladosporium cucumerinum) on cucumbers; gray-mold (Botrytis cinerea) and leaf mold (Cladosporium fulvum) on tomatoes; gray-mold (Botrytis cinerae), black rot (Corynespora melongenae) and powdery mildew ( Erysiphe cichoracearum) on eggplants; gray-mold ( Botrytis cinerea) and powdery mildew (Sphaerotheca humuli) on strawberries; gray-mold neck rot (Botrytis alli) and gray-mold (Botrytis cinerea) on onions; stem rot (Sclerotinia sclerotiorum) and gray-mold (Botrytis cinerea) on haricot beans; powdery mildew (Podosphaera leucotricha), scab (Venturia inaequalis) and blossom blight (Monilia mali) on apples; powdery mildew(Phyllactinia kakicola), anthracnose (Gloeosporium kaki) and angular leaf spot (Cercospora kaki) on persimmons; brown rot (Monilinia fructicola) on peaches and cherries; gray-mold (Botrytis cinerea), powdery mildew (Uncinula necator) and ripe rot (Glomerella cingulata) on grapes; scab (Venturia nashicola), rust (Gymnosporangium asiaticum) and black spot (Abternaria kikuchiana) on pears; gray blight (Pestalotia theae) and anthracnose (Colletotrichum theae-sinensis) on tea plants; scab (Elsinoe fawcetti), blue mold ( Penicillium italicum), common green mold (Penicillium digitatum) and gray mold (Botrytis cinerea) on citrus; powdery mildew (Erysiphe graminis f. sp. hordei) and loose smut (Ustilago nuda) on barley; Fusarium blight (Gibberella zeae), leaf rust (Puccinia recondita), spot blotch (Cochliobolus sativus), eye spot (Pseudocercosporella herpotrichoides), glume blotch (Leptosphaeria nodorum), powdery mildew (Erysiphe graminis f. sp. tritici) and Fusarium snow blight (Micronectriella nivalis) on wheat; blast (Pyricularia oryzae), sheath blight (Rhizoctonia solani), Bakanae-disease (Gibberella fujikuroi) and Helminthosporium leaf spot (Cochliobolus miyabeanus) on paddy rice; Sclerotinia rot (Sclerotinia sclerotiorum) and powdery mildew (Erysiphe cichoracearum) on tobacco; gray-mold (Botrytis cinerea) on tulips; Sclerotinia snow blight ( Sclerotinia borealis) on bentgrass; powdery mildew (Erysiphe graminis) on orchardgrass; purple stain (Cercospora kikuchii) on soybeans; late blight ( Phytophthora infestans) on potatoes and tomatoes; downy mildew (Pseudoperonospora cubensis) on cucumbers; and downy mildew (Plasmopara viticola) on grapes.

Furthermore, the compounds of the present invention are effective even against some strains of fungi which are resistant to benzimidazol-group fungicides, such as thiophanate methyl, benomyl and carbendazim, to the extent as much as effective against susceptible fungus strains to the fungicides in said group, and wherein resistant strains of pathogenic fungi causing diseases such as gray-mold (Botrytis cinerea), Cercospora leaf spot (Cercospora beticola) on sugarbeet, apple scab (Venturia inaequalis), and pear scab (Venturia nashicola) are included.

Moreover, the compounds of the present invention are also effective against the strains of fungus causing gray-mold (Botrytis cinerea) resistant to dicarboxyimide-group fungicides, such as vinclozolin, procymidone and iprodione to the extent as much as effective against the strains susceptible to the fungicides of said group.

The compounds of the present invention can be used not only as agricultural chemicals as described above, but also as an antiflouling agent for preventing adhesion of aquatic living organisms to underwater constructions, such as garboard, fish net, etc. and a general antiseptics for household and industrial uses.

When applying the compounds of the present invention obtained as described above practically, the compounds can be used in a normal form of formulation for common agricultural chemicals wherein one or more of the compounds represented by the general formula (I) described above are contained as the active ingredients. The active ingredient compounds can be formulated to a formulation, such as wettable powder, emulsifiable concentrate, granules, dusting powder, water soluble formulation, flowable etc., by mixing appropriate amounts of said active ingredient compounds with additives and carriers commonly used. For the additives and carriers, plant-origin powder such as soybean powder and wheat flour, mineral fine powders such as diatomaceous earth, apatite, gypsum, talc, pyrophilite, clay, white carbon (silica) and bentonite, mineral oils and vegetable oils can be used, when the objective formulation is solid type. On the other hand, kerosine, petroleum oil, solvent naphtha, benzene, xylene, cyclohexane, cyclohexanone, dimethylformamide, dimethylsulfoxide, alcohol, acetone, minerals oils, vegetable oils, water or the like can be used as the solvent, when the objective formulation is liquid type. In addition thereto, some surface active agents may be added to the formulation for improving the uniformity and stability thereof, when appropriate.

The content of the active ingredient in the formulation when using the compounds of the present invention as a herbicide or a fungicide can vary to various percentages depending upon the formulation types employed, however, the contents ranging from 5 to 70%, more preferably from 10 to 30% by weight for wettable powders, the one from 3 to 70%, more preferably from 5 to 20% by weight or in weight/volume for emulsifiable concentrates, and the one from 0.01 to 30%, more preferably from 0.05 to 10% by weight for granular formulations may be employed.

The wettable powders and emulsifiable concentrates obtained as described above can be applied to plants in a form of suspension or emulsion after diluting them with water up to a desired concentration, while the granular and dusting formulation can be applied without dilution. Particularly, in case of herbicides, which can be sprayed onto weeds or incorporated into soil either before or after the germination of weeds. For the practical use of a herbicide according to the present invention, an appropriate dose more than 0.1g of an active ingredient compound is applied per 10 are. On the other hand, in case of fungicides, it is used in a manner of direct spraying to plants.

The compounds of the present invention can be also used in combinations with any of known fungicides, acaricides, herbicide, plant growth regulators, or the like. The use of such combinations can contribute not only for labor saving but also for providing higher activity based on the synergistic action resulting between the combined products. In such case, it is also possible to combine the compound of the present invention with plurality of known compounds.

The representative examples of herbicides, fungicides, insecticides, acaricides and plant growth regulators which can be used in combination with the compounds of the present invention are shown hereinbelow.

[Herbicides]

Carbamate and thiocarbamate group herbicides, such as benthiocarb, molinate and dimepiperate[S-(2,2-dimethylbenzyl)1-piperidylcarbothiate]; acid amide group herbicides, such as butachlor, pretilachlor and mefenacet; diphenylether group herbicides, such as chlomethoxynil and bifenox; triazine group herbicides, such as atrazine and cyanazine; sulfonylurea group herbicides, such as bensulfuron methyl, pyrazosulfuron ethyl, chlorsulfuron and sulfometuron methyl; phenoxyalkane carboxylic acid group herbicides, such as MCP and MCPB; phenoxyphenoxy propionic acid group herbicides such as diclofop methyl; imidazolinone group herbicides such as imazaquin; pyridyloxyphenoxy propionic acid group herbicides such as fluazifopbutyl; benzoylamino propionic acid group herbicides, such as benzoylprop ethyl and flamprop ethyl; and other herbicides, such as piperophos, dymron, bentazone, difenzoqinat, naproanilide, HW-52 (4-ethoxymethoxybenz-2,3-dichloroanilide),KNW-242 [1-(3-methylphenyl)-5-phenyl-1H-1,2,4-triazol-3-carboxyamide], quinclorac (3,7-dichloro-8-quinoline carboxylic acid) and cyclohexanedion group herbicides, such as sethoxydim and alloxidim-sodium.

[Fungicides]

Captan, folpet, thiram, zineb, maneb, mancozeb, propineb, polycarbamate, chlorothalonil, quintozene, captafol, iprodione, procymidone, vinclozolin, fluoromide, cymoxanil, mepronil, flutolanil, pencycuron, oxycarboxin, fosetyl aluminium, propamocarb, triadimefon, triadimenol, propiconazole, dichlobutorazol, bitertanol, hexaconazol, myclobutanil, flusilazole, etaconazole, fluotrimazole, flutriafol, penconazole, diniconazole, cyproconazole, fenarimol, triflumizole, prochloraz, imazalil, pefurazoate, tridemorph, fenpropimorph, triforine, butyobate, pyrifenox, anilazine, polyoxins, metalaxyl, oxadixyl, furalaxyl, isoprothiolane probenazole, pyrrolenitrine, blasticidin-S, kasugamycin, validamycin, dihydrostreptomycin sulfate, benomyl, carbendazim, thiophanate-methyl, hymexazol, basic copper chloride, basic copper sulfate, fentin acetate, triphenyltin hydroxide, diethofencarb, methasulfocarb, quinomethionate, binapacryl, lecithin, sodium hydrogencarbonate, dithianon, dinocap, fenaminosulf, diclomezine, guazatine, dodine, IBP, edifenphos, mepanipyrim, ferimzone, triclamide, fluazinam, ethoquinolac, dimethomorph, pyroquilon, tecloftalam, and phthalide.

[Insecticides and Acaricides ]

Chlorobenzilate, chloropropylate, prochlonol, phenisobromolate, dicofol, dinobuton, chlorphenamidine, amitraz, BPPS, PPPS, benzomate, hexythiazox, fenbutatin oxide, polynactins complex, thioquinox, CPCBS, tetradifon, isoxathion, avermectin, polysulfide lime, chlofentezin, flubenzimine, flufenoxuron, BCBE, cyhexatin, pyridaben, fenpyroximate, fenthion, fenitrothion, diazinon, chlorpyrifos, ESP, vamidothion, phenthoate, dimethoate, formothion, malathion, dipterex, thiometon, phosmet, menazon, dichlorvos, acephate, EPBP, dialifor, methyl parathion, oxydemeton-methyl, ethion pyraclofos, monocrotophos, methomyl, aldicarb, propoxur, BPMC, MTMC, NAC, cartap, carbosulfan, benfuracarb, pirimicarb, ethiofencarb, fenoxycarb, permethrin, cypermethrin, decamethrin, fenvalerate, fenpropathrin, pyrethrin, allethrin, tetramethrin resmethrin, dimethrin propathrin, bifenthrin, prothrin, fluvalinate, cyfluthrin cyhalothrin, flucythrinate, etofenprox, cycloprothrin, tralomethrin, silafluofen, diflubenzuron, chlorfluazuron, triflumuron, teflubenzuron, buprofezin, and machine oil.

[Plant growth regulators ]

Gibberellic acids such as Gibberellin $A_3$, Gibberellin $A_4$ and Gibberellin $A_7$, IAA, NAA, inabenfide, ethephon, chlormequat, uniconazole, and paclobutrazol.

Now, several examples of the formulation composition according to the present invention are shown below. However, it should be noted that the types and the adding ratio of additives shall not be limited to the scope defined by the description in these examples and can be replaced by wide range of other additives. Parts in the examples are indicated by weight.

Example 13 : Wettable Powder

| | |
|---|---|
| The compound of the present invention | 40 parts |
| Diatomaceous earth | 53 parts |
| Higher alcohol sulfate ester | 4 parts |
| Alkylnaphthalene sulfonate | 3 parts |

All components described above are mixed and pulverized into fine powder to obtain a wettable powder containing 40% by weight of the compound as the active ingredient.

Example 14 : Wettable Powder

| | |
|---|---|
| The compound of the present invention | 20 parts |
| White carbon | 20 parts |
| Diatomaceous earth | 52 parts |
| Alkyl sodium sulfate | 8 parts |

All components described above are mixed and pulverized into fine powder to obtain a wettable powder containing 20% by weight of the compound as the active ingredient.

Example 15 : Emulsifiable Concentrate

| | |
|---|---|
| The compound of the present invention | 30 parts |
| Xylene | 33 parts |
| Dimethylformamide | 30 parts |
| Polyoxyethylenealkylallylether | 7 parts |

All components described above are mixed and dissolved to obtain an emulsifiable concentrate containing 30% in weight per volume of the compound as the active ingredient.

Example 16 : Emulsifiable Concentrate

| | |
|---|---|
| The compound of the present invention | 20 parts |
| Xylene | 55 parts |
| Dimethylformamide | 15 parts |
| Polyoxyethylenephenylether | 10 parts |

All components described above are mixed and dissolved to obtain an emulsifiable concentrate containing 20% in weight per volume of the compound as the active ingredient.

Example 17 : Granular Formulation

| | |
|---|---|
| The compound of the present invention | 5 parts |
| Talc | 40 parts |
| Clay | 38 parts |
| Bentonite | 10 parts |
| Sodium alkylsulfate | 7 parts |

All components described above are mixed, pulverized into fine powder, and granulated into the granules having a diameter of from 0.5 to 1.0 mm to obtain a granular formulation containing 5% by weight of the compound as the active ingredient.

Example 18 : Granular Formulation

| | |
|---|---|
| The compound of the present invention | 5 parts |
| Clay | 73 parts |
| Bentonite | 20 parts |
| Sodium salt of dioctylsulfosuccinate | 1 part |
| Sodium phosphate | 1 part |

All components described above are mixed, pulverized, kneaded by adding water, granulated, and then dried to obtain a granular formulation containing 5% by weight of the compound as the active ingredient.

Example 19 : Dusting Powder Formulation

| | |
|---|---|
| The compound of the present invention | 10 parts |
| Talc | 89 parts |
| Polyoxyethylenealkylallyl ether | 1 part |

All components described above are mixed homogeneously and pulverized into fine powder to obtain a dusting powder formulation containing 10% by weight of the compound as the active ingredient.

Example 20 : Suspension Concentrate

| | |
|---|---|
| The compound of the present invention | 10 parts |
| Sodium lignin sulfonate | 4 parts |
| Sodium dodecylbenzenesulfonate | 1 part |
| Xanthane gum | 0.2 part |
| Water | 84.8 parts |

All components described above are mixed and subjected to wet grinding process until the diameter of the particles reaching to 1 $\mu$ to obtain a suspension concentrate containing 10% by weight of the compound as the active ingredient.

Industrial Applicability

Now, test examples of the herbicides according to the present invention are shown below in respect of the herbicidal activity on some weeds.

Test Example 1 : Test on Soil Application Using Soil Collected from Upland Crop Fields

The soil collected from upland crop field was filled into a plastic pot having a surface area of 250 $cm^2$, then the seeds of weeds including crabgrass, giant foxtail and livid amaranth, were respectively sowed therein and covered with soil up to a thickness of 0.5 cm. On the next day, the diluted solution of a herbicide wettable powder as prepared in the Example 13 was uniformly sprayed over the surface of the soil at a rate of 200 g/10 are in respect of the active ingredient, then the herbicidal activities was assessed on 20th day after the application in accordance with a criteria for assessment as indicated below. The results were shown in Table 3.

A criteria for assessment

| % of Weeds Killed | Index for Herbicidal Activity |
|---|---|
| 0 % | 0 |
| 20 - 29 % | 2 |
| 40 - 49 % | 4 |
| 60 - 69 % | 6 |
| 80 - 89 % | 8 |
| 100 % | 10 |

In this criteria, the values of 1, 3, 5, 7 and 9 indicate the activity between 0 and 2, 2 and 4, 4 and 6, 6 and 8, and 8 and 10, respectively.

Weeds killed (%) = [(Total fresh weight of over-ground weeds in Untreated pot - Total fresh weight of under-ground weeds in Treated pot) ÷ Total fresh weight of over-ground weeds in Untreated pot ] × 100

Table. 3

| No. | active ingredient g／10a | Index for Herbicidal Activity | | |
|---|---|---|---|---|
| | | barnyard -grass | giant foxtail | livid amaranth |
| I − 10 | 200 | 10 | 10 | 10 |
| I − 11 | 200 | 10 | 10 | 10 |
| I − 15 | 200 | 10 | 10 | 10 |
| I − 21 | 200 | 10 | 10 | 10 |
| I − 22 | 200 | 10 | 10 | 10 |
| I − 23 | 200 | 10 | 10 | 10 |
| I − 24 | 200 | 10 | 10 | 10 |
| I − 48 | 200 | 10 | 10 | 10 |
| I − 49 | 200 | 10 | 10 | 10 |
| I − 53 | 200 | 10 | 10 | 10 |
| I − 92 | 200 | 10 | 10 | 10 |
| I − 93 | 200 | 10 | 10 | 10 |
| I − 94 | 200 | 10 | 10 | 10 |
| I − 96 | 200 | 10 | 10 | 10 |
| I −172 | 200 | 10 | 10 | 10 |
| I −180 | 200 | 10 | 10 | 10 |
| I −183 | 200 | 10 | 10 | 10 |
| I −185 | 200 | 10 | 10 | 10 |
| I −201 | 200 | 10 | 10 | 10 |
| I −276 | 200 | 10 | 10 | 10 |
| I −281 | 200 | 10 | 10 | 10 |
| I −297 | 200 | 10 | 10 | 10 |
| I −449 | 200 | 10 | 10 | 10 |
| I −450 | 200 | 10 | 10 | 10 |
| I −460 | 200 | 10 | 10 | 10 |
| I −462 | 200 | 10 | 10 | 10 |
| I −466 | 200 | 10 | 10 | 10 |
| I −467 | 200 | 10 | 10 | 10 |
| I −472 | 200 | 10 | 10 | 10 |
| I −496 | 200 | 10 | 10 | 10 |
| I −511 | 200 | 10 | 10 | 10 |
| I −518 | 200 | 10 | 10 | 10 |
| I −522 | 200 | 10 | 10 | 10 |
| I −527 | 200 | 10 | 10 | 10 |
| II − 59 | 200 | 10 | 10 | 10 |
| II − 68 | 200 | 10 | 10 | 10 |

Table. 3 (Continued)

| Compound No. | Active Ingredient g／10a | Index for Herbicidal Activity | | |
|---|---|---|---|---|
| | | barnyard-grass | giant foxtail | livid amaranth |
| Ⅲ－ 3 | 200 | 10 | 10 | 10 |
| Ⅲ－ 4 | 200 | 10 | 10 | 10 |
| Ⅲ－ 10 | 200 | 10 | 10 | 10 |
| Ⅲ－ 12 | 200 | 10 | 10 | 10 |
| Ⅲ－ 13 | 200 | 10 | 10 | 10 |
| Ⅲ－ 14 | 200 | 10 | 10 | 10 |
| Ⅲ－ 15 | 200 | 10 | 10 | 10 |
| Ⅲ－ 16 | 200 | 10 | 10 | 10 |
| Ⅲ－ 17 | 200 | 10 | 10 | 10 |
| Ⅲ－ 18 | 200 | 10 | 10 | 10 |
| Ⅲ－ 19 | 200 | 10 | 10 | 10 |
| Ⅲ－ 20 | 200 | 10 | 10 | 10 |
| Ⅲ－ 21 | 200 | 10 | 10 | 10 |
| Ⅲ－ 22 | 200 | 10 | 10 | 10 |
| Ⅲ－ 23 | 200 | 10 | 10 | 10 |
| Ⅲ－ 24 | 200 | 10 | 10 | 10 |
| Ⅲ－ 26 | 200 | 10 | 10 | 10 |
| Ⅲ－ 27 | 200 | 10 | 10 | 10 |
| Ⅲ－ 28 | 200 | 10 | 10 | 10 |
| Ⅲ－ 29 | 200 | 10 | 10 | 10 |
| Ⅲ－ 30 | 200 | 10 | 10 | 10 |
| Ⅲ－ 31 | 200 | 10 | 10 | 10 |
| Ⅲ－ 32 | 200 | 10 | 10 | 10 |
| Ⅲ－ 33 | 200 | 10 | 10 | 10 |
| Ⅲ－ 34 | 200 | 10 | 10 | 10 |
| Ⅲ－ 37 | 200 | 10 | 10 | 10 |
| Ⅲ－ 38 | 200 | 10 | 10 | 10 |
| Ⅲ－ 39 | 200 | 10 | 10 | 10 |
| Ⅲ－ 40 | 200 | 10 | 10 | 10 |
| Ⅲ－ 41 | 200 | 10 | 10 | 10 |
| Ⅲ－ 43 | 200 | 10 | 10 | 10 |
| Ⅲ－ 45 | 200 | 10 | 10 | 10 |
| Ⅲ－ 46 | 200 | 10 | 10 | 10 |
| Ⅲ－ 47 | 200 | 10 | 10 | 10 |
| Ⅲ－ 50 | 200 | 10 | 10 | 10 |
| Ⅲ－ 58 | 200 | 10 | 10 | 10 |
| Ⅲ－ 74 | 200 | 10 | 10 | 10 |
| Ⅲ－ 82 | 200 | 10 | 9 | 7 |
| Ⅲ－ 91 | 200 | 10 | 10 | 10 |
| Ⅲ－ 94 | 200 | 10 | 10 | 10 |
| Ⅲ－ 95 | 200 | 10 | 10 | 10 |
| Ⅲ－ 97 | 200 | 10 | 10 | 10 |
| Ⅲ－ 98 | 200 | 10 | 10 | 10 |
| Ⅲ－ 99 | 200 | 10 | 10 | 10 |

Table. 3 (Continued)

| Compound No. | Active Ingredient g/10a | Index for Herbicidal Activity | | |
|---|---|---|---|---|
| | | barnyard-grass | giant foxtail | livid amaranth |
| III-100 | 200 | 10 | 10 | 10 |
| III-103 | 200 | 10 | 10 | 10 |
| III-104 | 200 | 10 | 10 | 10 |
| III-112 | 200 | 10 | 10 | 10 |
| III-114 | 200 | 10 | 10 | 10 |
| III-115 | 200 | 10 | 10 | 10 |
| III-117 | 200 | 10 | 10 | 10 |
| III-118 | 200 | 10 | 10 | 10 |
| III-119 | 200 | 10 | 10 | 10 |
| III-126 | 200 | 10 | 10 | 10 |
| III-129 | 200 | 10 | 10 | 10 |
| III-151 | 200 | 10 | 10 | 10 |
| III-236 | 200 | 10 | 10 | 10 |
| III-256 | 200 | 10 | 10 | 10 |
| III-919 | 200 | 10 | 10 | 10 |
| III-1369 | 200 | 10 | 10 | 10 |
| III-1448 | 200 | 10 | 10 | 10 |
| III-1459 | 200 | 10 | 10 | 10 |

Test Example 2 : Test on Soil Application Using Soil Collected from Paddy Rice Field

The soil collected from paddy rice field was filled into a pot having open area of 150 $cm^2$, then the seeds of barnyardgrass, smallflower umbrellaplant and Sagittaria trifolia were respectively sowed therein in together with transplanting of rice seedlings at second leaf stage. On the next day, watering into the pot was carried out up to the height of 3 cm from the soil surface, then a prescribed dose of each granular formulations of the compounds was applied thereto and maintained in a greenhouse for germination. After 3 weeks from the application, the herbicidal activity onto each species of weeds were assessed in accordance with the criteria for assessment described above.

The results were shown in Table 4.

Table. 4

| Compound No. | Active Ingredient g/10a | phyto-toxicity | Index for Herbicidal Activity | | |
|---|---|---|---|---|---|
| | | rice plant | barnyard-grass | smallflower-umbrella-plant | sagitta-ria-trifolia |
| I - 11 | 100 | 0 | 10 | 10 | 10 |
| I - 23 | 100 | 0 | 10 | 10 | 10 |
| I - 29 | 100 | 0 | 10 | 10 | 10 |
| I - 48 | 100 | 0 | 10 | 10 | 10 |
| I - 49 | 100 | 0 | 10 | 10 | 10 |
| I - 53 | 100 | 0 | 10 | 10 | 10 |
| I - 54 | 100 | 0 | 10 | 10 | 10 |
| I - 88 | 100 | 0 | 10 | 10 | 10 |
| I - 93 | 100 | 0 | 10 | 10 | 10 |
| I - 94 | 100 | 0 | 10 | 5 | 3 |
| I - 96 | 100 | 2 | 10 | 10 | 10 |
| I - 172 | 100 | 0 | 10 | 4 | 0 |
| I - 180 | 100 | 0 | 10 | 10 | 10 |
| I - 183 | 100 | 0 | 10 | 6 | 7 |
| I - 185 | 100 | 2 | 10 | 10 | 10 |
| I - 201 | 100 | 0 | 10 | 10 | 10 |
| I - 281 | 100 | 0 | 10 | 10 | 10 |
| I - 292 | 100 | 1 | 10 | 3 | 6 |
| I - 297 | 100 | 0 | 10 | 7 | 6 |
| I - 438 | 100 | 2 | 10 | 10 | 10 |
| I - 449 | 100 | 0 | 10 | 10 | 10 |
| I - 450 | 100 | 0 | 10 | 10 | 10 |
| I - 460 | 100 | 0 | 10 | 10 | 10 |
| I - 466 | 100 | 2 | 10 | 10 | 10 |
| I - 467 | 100 | 2 | 10 | 5 | 2 |
| I - 470 | 100 | 0 | 10 | 9 | 4 |
| I - 473 | 100 | 0 | 10 | 10 | 10 |
| I - 480 | 100 | 1 | 10 | 8 | 7 |
| I - 498 | 100 | 0 | 10 | 10 | 10 |
| I - 500 | 100 | 0 | 10 | 10 | 10 |
| I - 503 | 100 | 2 | 9 | 6 | 2 |
| I - 509 | 100 | 0 | 10 | 10 | 10 |
| I - 514 | 100 | 2 | 10 | 10 | 10 |
| I - 516 | 100 | 0 | 10 | 10 | 10 |
| I - 517 | 100 | 2 | 10 | 10 | 10 |
| I - 520 | 100 | 2 | 10 | 10 | 10 |
| I - 527 | 100 | 4 | 10 | 9 | 6 |
| I - 568 | 100 | 0 | 8 | 10 | 5 |
| I - 584 | 100 | 0 | 9 | 8 | 6 |

Table. 4 (Continued)

| Compound No. | Active Ingredient g／10a | phyto-toxicity | Index for Herbicidal Activity | | |
|---|---|---|---|---|---|
| | | rice plant | barnyard -grass | smallflower- -umbrella- -plant | sagitta- -ria- trifolia |
| Ⅱ－55 | 100 | 0 | 10 | 10 | 10 |
| Ⅱ－56 | 100 | 0 | 10 | 10 | 10 |
| Ⅱ－57 | 100 | 0 | 10 | 10 | 10 |
| Ⅱ－59 | 100 | 2 | 10 | 10 | 10 |
| Ⅱ－61 | 100 | 3 | 10 | 7 | 3 |
| Ⅱ－142 | 100 | 0 | 10 | 10 | 7 |
| Ⅱ－143 | 100 | 3 | 10 | 8 | 5 |
| Ⅲ－3 | 100 | 0 | 10 | 6 | 4 |
| Ⅲ－4 | 100 | 0 | 10 | 4 | 4 |
| Ⅲ－10 | 100 | 2 | 10 | 9 | 9 |
| Ⅲ－12 | 100 | 0 | 10 | 9 | 9 |
| Ⅲ－13 | 100 | 0 | 10 | 9 | 9 |
| Ⅲ－14 | 100 | 2 | 10 | 10 | 10 |
| Ⅲ－15 | 100 | 0 | 10 | 9 | 6 |
| Ⅲ－16 | 100 | 2 | 10 | 10 | 10 |
| Ⅲ－17 | 100 | 0 | 10 | 10 | 10 |
| Ⅲ－18 | 100 | 2 | 10 | 10 | 10 |
| Ⅲ－19 | 100 | 2 | 10 | 10 | 10 |
| Ⅲ－20 | 100 | 2 | 10 | 10 | 10 |
| Ⅲ－21 | 100 | 2 | 10 | 8 | 8 |
| Ⅲ－22 | 100 | 0 | 8 | 4 | 4 |
| Ⅲ－23 | 100 | 4 | 10 | 10 | 8 |
| Ⅲ－26 | 100 | 0 | 10 | 10 | 8 |
| Ⅲ－27 | 100 | 2 | 10 | 10 | 8 |
| Ⅲ－28 | 100 | 4 | 10 | 10 | 9 |
| Ⅲ－29 | 100 | 4 | 10 | 10 | 9 |
| Ⅲ－30 | 100 | 3 | 10 | 8 | 8 |
| Ⅲ－31 | 100 | 4 | 10 | 10 | 10 |
| Ⅲ－32 | 100 | 4 | 10 | 10 | 9 |
| Ⅲ－33 | 100 | 4 | 10 | 10 | 10 |
| Ⅲ－34 | 100 | 0 | 8 | 7 | 3 |
| Ⅲ－37 | 100 | 2 | 9 | 8 | 8 |
| Ⅲ－38 | 100 | 1 | 8 | 5 | 8 |
| Ⅲ－39 | 100 | 4 | 10 | 10 | 9 |
| Ⅲ－40 | 100 | 0 | 10 | 2 | 10 |
| Ⅲ－41 | 100 | 4 | 10 | 10 | 8 |
| Ⅲ－43 | 100 | 5 | 10 | 10 | 9 |
| Ⅲ－45 | 100 | 0 | 9 | 6 | 4 |
| Ⅲ－46 | 100 | 5 | 10 | 10 | 8 |
| Ⅲ－47 | 100 | 4 | 9 | 10 | 9 |

Table. 4 (Continued)

| Compound No. | Active Ingredient g/10a | phyto-toxicity | Index for Herbicidal Activity | | |
|---|---|---|---|---|---|
| | | rice plant | barnyard-grass | smallflower-umbrella-plant | sagitta-ria-trifolia |
| III - 50 | 100 | 4 | 10 | 10 | 10 |
| III - 55 | 100 | 0 | 10 | 10 | 6 |
| III - 58 | 100 | 0 | 6 | 10 | 6 |
| III - 59 | 100 | 0 | 10 | 9 | 0 |
| III - 74 | 100 | 4 | 10 | 10 | 9 |
| III - 76 | 100 | 0 | 10 | 10 | 7 |
| III - 82 | 100 | 4 | 8 | 8 | 8 |
| III - 84 | 100 | 0 | 4 | 10 | 10 |
| III - 94 | 100 | 4 | 10 | 10 | 7 |
| III - 95 | 100 | 4 | 9 | 10 | 6 |
| III - 97 | 100 | 5 | 10 | 10 | 9 |
| III - 98 | 100 | 4 | 9 | 4 | 7 |
| III - 126 | 100 | 0 | 10 | 10 | 6 |
| III - 129 | 100 | 4 | 10 | 10 | 8 |
| III - 256 | 100 | 5 | 10 | 10 | 8 |
| III - 919 | 100 | 0 | 8 | 10 | 10 |
| III - 1062 | 100 | 0 | 8 | 10 | 10 |
| III - 1448 | 100 | 5 | 10 | 10 | 8 |
| III - 1458 | 100 | 0 | 8 | 10 | 10 |
| III - 1459 | 100 | 0 | 8 | 10 | 6 |

Now, it is shown that the compound of the present invention are useful as an active ingredient for fungicides for controlling various plant diseases with referring to Test Examples. The control activity of the fungicide of the present invention was evaluated according to a criteria as indicated below based on the observation in naked-eyes on the development of diseases on the test plants at the time of assessment, that is, the degrees of developing mycelium and the numbers of spots appeared on leaves, stems, etc. The criteria is expressed in indexes for indicating the degree of the diseases as the followings.

Criteria for the assessment of plant disease

5 : Neither mycelia nor spots were observed on the plant.

4 : The degree of disease on treated-plants are 90% less than the degree observed on untreated plants.

3 : The degree of disease on treated-plants are 75% less than the degree observed on untreated plants.

2 : The degree of disease on treated-plants are 50% less than the degree observed on untreated plants.

1 : The degree of disease on treated-plants are 25% less than the degree observed on untreated plants.

0 : Substantially no difference in the degree of disease between treated-plants and untreated plants.

Test Example 3 : Test on Apple Scab Control (Preventive Test)

Young apple seedlings (variety: Kokko, at 3 to 4 leaved stage) growing in an unglazed pottery pot were provided for this test. A wettable powder comprising the compound of the present invention was diluted to a prescribed concentration, then sprayed to the apple seedlings and dried. Then conidia of fungus ( Venturia

inaequalis) was inoculated to the seedlings. After leaving the seedlings in a humid room maintained at 20 °C under illumination of light and dark cycle for 2 weeks, the preventive activity of the compounds were assessed. The results were shown in Table 5.

Table 5

| Compound No. | Active ingredient (ppm) | Index | Phytotoxicity |
|---|---|---|---|
| I - 63 | 200 | 4 | None |
| I - 82 | 200 | 4 | None |
| I - 84 | 200 | 4 | None |
| I - 169 | 200 | 4 | None |
| Reference* Fungicide 1 | | 4 | None |
| Reference Fungicide 1 * : Captan 80% Wettable powder | | | |

Test Example 4 : Test on Grape Downy Mildew Control

Several leaves of grape (variety: Kaiji, at 3 years old) grown in open field were collected, then discs of the leaf in diameter of 30 mm were prepared from those leaves. The discs were then placed on the surface of the solution of wettable powder of the compound of the present invention prepared to a prescribed concentration. Then, the discs were sprayed with zoospore suspension of Plasmopara viticola for inoculation, maintained in a greenhouse at 20 °C under illumination. The degree of the disease was assessed on 10th day after the inoculation. The results are shown in Table 6:

Table. 6

| No. | Active Ingredient | Index | phytotoxicity |
|---|---|---|---|
| I－2 | 200 ppm | 4 | None |
| I－9 | 〃 | 4 | 〃 |
| I－10 | 〃 | 5 | 〃 |
| I－11 | 〃 | 5 | 〃 |
| I－14 | 〃 | 4 | 〃 |
| I－15 | 〃 | 5 | 〃 |
| I－16 | 〃 | 5 | 〃 |
| I－17 | 〃 | 4 | 〃 |
| I－18 | 〃 | 4 | 〃 |
| I－19 | 〃 | 4 | 〃 |
| I－20 | 〃 | 4 | 〃 |
| I－21 | 〃 | 5 | 〃 |
| I－22 | 〃 | 5 | 〃 |
| I－23 | 〃 | 5 | 〃 |
| I－24 | 〃 | 4 | 〃 |
| I－25 | 〃 | 5 | 〃 |
| I－26 | 〃 | 4 | 〃 |
| I－28 | 〃 | 5 | 〃 |
| I－29 | 〃 | 5 | 〃 |
| I－38 | 〃 | 4 | 〃 |
| I－43 | 〃 | 4 | 〃 |
| I－44 | 〃 | 5 | 〃 |
| I－55 | 〃 | 4 | 〃 |
| I－59 | 〃 | 4 | 〃 |
| I－60 | 〃 | 5 | 〃 |
| I－61 | 〃 | 5 | 〃 |
| I－62 | 〃 | 4 | 〃 |
| I－63 | 〃 | 4 | 〃 |
| I－69 | 〃 | 4 | 〃 |
| I－70 | 〃 | 4 | 〃 |
| I－74 | 〃 | 5 | 〃 |
| I－82 | 〃 | 4 | 〃 |
| I－83 | 〃 | 4 | 〃 |
| I－84 | 〃 | 4 | 〃 |
| I－88 | 〃 | 4 | 〃 |
| I－91 | 〃 | 4 | 〃 |
| I－92 | 〃 | 5 | 〃 |
| I－93 | 〃 | 5 | 〃 |
| I－94 | 〃 | 4 | 〃 |
| I－95 | 〃 | 4 | 〃 |
| I－96 | 〃 | 5 | 〃 |
| I－97 | 〃 | 4 | 〃 |

Table. 6 (Continued)

| No. | Active Ingredient | Index | phytotoxicity |
|---|---|---|---|
| I－101 | 200 | 4 | None |
| I－167 | 〃 | 4 | 〃 |
| I－168 | 〃 | 4 | 〃 |
| I－169 | 〃 | 4 | 〃 |
| I－172 | 〃 | 5 | 〃 |
| I－180 | 〃 | 5 | 〃 |
| I－183 | 〃 | 5 | 〃 |
| I－184 | 〃 | 5 | 〃 |
| I－185 | 〃 | 5 | 〃 |
| I－281 | 〃 | 5 | 〃 |
| I－297 | 〃 | 5 | 〃 |
| I－436 | 〃 | 5 | 〃 |
| I－438 | 〃 | 4 | 〃 |
| I－448 | 〃 | 5 | 〃 |
| I－449 | 〃 | 5 | 〃 |
| I－450 | 〃 | 4 | 〃 |
| I－466 | 〃 | 5 | 〃 |
| I－467 | 〃 | 5 | 〃 |
| I－470 | 〃 | 4 | 〃 |
| I－472 | 〃 | 4 | 〃 |
| I－473 | 〃 | 4 | 〃 |
| I－475 | 〃 | 4 | 〃 |
| I－476 | 〃 | 4 | 〃 |
| I－480 | 〃 | 5 | 〃 |
| I－481 | 〃 | 5 | 〃 |
| I－485 | 〃 | 4 | 〃 |
| I－486 | 〃 | 4 | 〃 |
| I－487 | 〃 | 4 | 〃 |
| I－488 | 〃 | 4 | 〃 |
| I－489 | 〃 | 4 | 〃 |
| I－490 | 〃 | 5 | 〃 |
| I－491 | 〃 | 4 | 〃 |
| I－496 | 〃 | 5 | 〃 |
| I－497 | 〃 | 5 | 〃 |
| I－498 | 〃 | 5 | 〃 |
| I－499 | 〃 | 5 | 〃 |
| I－500 | 〃 | 5 | 〃 |
| I－566 | 〃 | 5 | 〃 |
| I－568 | 〃 | 5 | 〃 |
| I－576 | 〃 | 4 | 〃 |
| I－577 | 〃 | 5 | 〃 |

Table. 6 (Continued)

| No. | Active Ingredient | Index | phytotoxicity |
|---|---|---|---|
| II－52 | 200 | 4 | None |
| II－54 | 〃 | 5 | 〃 |
| II－55 | 〃 | 5 | 〃 |
| II－56 | 〃 | 5 | 〃 |
| II－57 | 〃 | 5 | 〃 |
| II－58 | 〃 | 4 | 〃 |
| II－59 | 〃 | 5 | 〃 |
| II－60 | 〃 | 5 | 〃 |
| II－62 | 〃 | 4 | 〃 |
| II－142 | 〃 | 5 | 〃 |
| II－143 | 〃 | 5 | 〃 |
| II－144 | 〃 | 5 | 〃 |
| II－147 | 〃 | 4 | 〃 |
| II－151 | 〃 | 5 | 〃 |
| III－10 | 〃 | 5 | 〃 |
| III－11 | 〃 | 5 | 〃 |
| III－12 | 〃 | 5 | 〃 |
| III－13 | 〃 | 5 | 〃 |
| III－14 | 〃 | 5 | 〃 |
| III－15 | 〃 | 5 | 〃 |
| III－16 | 〃 | 5 | 〃 |
| III－17 | 〃 | 5 | 〃 |
| III－18 | 〃 | 5 | 〃 |
| III－19 | 〃 | 5 | 〃 |
| III－20 | 〃 | 5 | 〃 |
| III－21 | 〃 | 5 | 〃 |
| III－22 | 〃 | 5 | 〃 |
| III－27 | 〃 | 5 | 〃 |
| III－29 | 〃 | 5 | 〃 |
| III－30 | 〃 | 5 | 〃 |
| III－31 | 〃 | 5 | 〃 |
| III－32 | 〃 | 5 | 〃 |
| III－33 | 〃 | 5 | 〃 |
| III－34 | 〃 | 5 | 〃 |
| III－37 | 〃 | 5 | 〃 |
| III－38 | 〃 | 5 | 〃 |
| III－39 | 〃 | 5 | 〃 |
| III－40 | 〃 | 4 | 〃 |
| III－41 | 〃 | 5 | 〃 |
| III－45 | 〃 | 5 | 〃 |
| III－46 | 〃 | 5 | 〃 |
| III－47 | 〃 | 5 | 〃 |
| III－48 | 〃 | 5 | 〃 |
| III－49 | 〃 | 4 | 〃 |

Table. 6 (Continued)

| No. | Active Ingredient | Index | phytotoxicity |
|---|---|---|---|
| Ⅲ-50 | 200 | 5 | None |
| Ⅲ-53 | 〃 | 5 | 〃 |
| Ⅲ-56 | 〃 | 4 | 〃 |
| Ⅲ-58 | 〃 | 5 | 〃 |
| Ⅲ-59 | 〃 | 5 | 〃 |
| Ⅲ-60 | 〃 | 4 | 〃 |
| Ⅲ-61 | 〃 | 5 | 〃 |
| Ⅲ-64 | 〃 | 5 | 〃 |
| Ⅲ-65 | 〃 | 4 | 〃 |
| Ⅲ-69 | 〃 | 5 | 〃 |
| Ⅲ-71 | 〃 | 5 | 〃 |
| Ⅲ-73 | 〃 | 5 | 〃 |
| Ⅲ-74 | 〃 | 5 | 〃 |
| Ⅲ-75 | 〃 | 5 | 〃 |
| Ⅲ-76 | 〃 | 5 | 〃 |
| Comparative 2 | 〃 | 3 | 〃 |

Comparative 2: Mancozeb 75% wettable powder

Test Example 5 : Test on Cucumber Downy Mildew Control (Preventive test)

To the back side of leaves of young cucumber seedlings (Variety: Sagami-hanjiro) grown in a greenhouse for about 3 weeks, a wettable powder comprising the compound of the present invention was sprayed after diluting it to a solution at a prescribed concentration, then dried. The zoosporangial suspension of fungus, Pseudoperonospora cubensis, which was collected from the leaves infected with this disease, was sprayed to the seedlings, then the seedlings were placed in a inoculation box maintained at 20°C and R.H. 100%. After 2 days, the seedlings were transferred to greenhouse maintained at 23 to 26°C and a relative humidity of more than 70%, then preventive activity of the compounds were assessed on another 2 days later. The results were shown in Table 7.

Table. 7

| No. | Active Ingredient | Index | phytotoxicity |
|---|---|---|---|
| I－11 | 200 | 5 | None |
| I－14 | 〃 | 5 | 〃 |
| I－21 | 〃 | 4 | 〃 |
| I－22 | 〃 | 4 | 〃 |
| I－23 | 〃 | 5 | 〃 |
| I－28 | 〃 | 4 | 〃 |
| I－29 | 〃 | 4 | 〃 |
| I－44 | 〃 | 4 | 〃 |
| I－61 | 〃 | 5 | 〃 |
| I－74 | 〃 | 4 | 〃 |
| I－88 | 〃 | 4 | 〃 |
| I－91 | 〃 | 4 | 〃 |
| I－93 | 〃 | 5 | 〃 |
| I－168 | 〃 | 4 | 〃 |
| I－185 | 〃 | 4 | 〃 |
| I－186 | 〃 | 4 | 〃 |
| I－201 | 〃 | 4 | 〃 |
| I－276 | 〃 | 4 | 〃 |
| I－281 | 〃 | 5 | 〃 |
| I－297 | 〃 | 4 | 〃 |
| I－454 | 〃 | 4 | 〃 |
| I－466 | 〃 | 4 | 〃 |
| I－467 | 〃 | 4 | 〃 |
| I－479 | 〃 | 4 | 〃 |
| I－480 | 〃 | 4 | 〃 |
| I－491 | 〃 | 4 | 〃 |
| I－496 | 〃 | 4 | 〃 |
| I－497 | 〃 | 4 | 〃 |
| I－498 | 〃 | 5 | 〃 |
| I－508 | 〃 | 4 | 〃 |
| I－514 | 〃 | 4 | 〃 |
| I－577 | 〃 | 5 | 〃 |
| II－55 | 〃 | 4 | 〃 |
| II－57 | 〃 | 4 | 〃 |
| II－59 | 〃 | 4 | 〃 |
| II－142 | 〃 | 5 | 〃 |
| II－143 | 〃 | 5 | 〃 |
| II－144 | 〃 | 5 | 〃 |

Table. 7 (Continued)

| No. | Active Ingredient | Index | phytotoxicity |
|---|---|---|---|
| III-10 | 200 | 5 | None |
| III-11 | 〃 | 4 | 〃 |
| III-12 | 〃 | 5 | 〃 |
| III-13 | 〃 | 5 | 〃 |
| III-14 | 〃 | 5 | 〃 |
| III-15 | 〃 | 5 | 〃 |
| III-16 | 〃 | 5 | 〃 |
| III-18 | 〃 | 4 | 〃 |
| III-19 | 〃 | 5 | 〃 |
| III-20 | 〃 | 5 | 〃 |
| III-21 | 〃 | 5 | 〃 |
| III-22 | 〃 | 5 | 〃 |
| III-27 | 〃 | 4 | 〃 |
| III-28 | 〃 | 5 | 〃 |
| III-29 | 〃 | 5 | 〃 |
| III-30 | 〃 | 5 | 〃 |
| III-31 | 〃 | 5 | 〃 |
| III-32 | 〃 | 5 | 〃 |
| III-33 | 〃 | 5 | 〃 |
| III-34 | 〃 | 4 | 〃 |
| III-37 | 〃 | 5 | 〃 |
| III-38 | 〃 | 5 | 〃 |
| III-39 | 〃 | 5 | 〃 |
| III-41 | 〃 | 5 | 〃 |
| III-46 | 〃 | 5 | 〃 |
| III-47 | 〃 | 5 | 〃 |
| III-48 | 〃 | 5 | 〃 |
| III-50 | 〃 | 5 | 〃 |
| III-56 | 〃 | 5 | 〃 |
| III-64 | 〃 | 5 | 〃 |
| III-71 | 〃 | 4 | 〃 |
| III-73 | 〃 | 5 | 〃 |
| III-74 | 〃 | 5 | 〃 |
| III-75 | 〃 | 5 | 〃 |
| III-76 | 〃 | 4 | 〃 |
| Comparative 3 | 〃 | 3 | 〃 |

Comparative 3: Zineb 72% wettable powder

Test Example 6 : Test on Cucumber Downy Mildew Control (Curative test)

To young cucumber seedlings (Variety: Sagami-hanjiro, at 1.1 leaf stage) grown at room temperature in a greenhouse, the zoosporangial suspension of fungus, Pseudoperonospora cubensis, which was collected from the leaves infected to this disease, was sprayed. The seedlings were then placed in a humid inoculation box maintained at 20 °C. After 20 hours, a wettable powder comprising the compound of the present invention was sprayed after diluting it to a solution at a prescribed concentration, then dried, and the plants sprayed were transferred to a temperature-controlled room maintained at 24 to 26°C and a relative humidity of more than 70%. The degree of the disease on the plants were assessed on another 3 days later. The results are shown in Table 8.

Table 8

| No. | Active Ingredient | Index | phytotoxicity |
|---|---|---|---|
| III - 10 | 200 | 4 | None |
| III - 11 | 200 | 4 | " |
| III - 12 | 200 | 5 | " |
| III - 13 | 200 | 4 | " |
| III - 15 | 200 | 5 | " |
| III - 16 | 200 | 5 | " |
| III - 18 | 200 | 4 | " |
| III - 19 | 200 | 5 | " |
| III - 20 | 200 | 4 | " |
| III - 32 | 200 | 5 | " |
| III - 33 | 200 | 5 | " |
| III - 37 | 200 | 5 | " |
| III - 38 | 200 | 4 | " |
| III - 39 | 200 | 5 | " |
| III - 41 | 200 | 5 | " |
| III - 46 | 200 | 4 | " |
| III - 47 | 200 | 5 | " |
| III - 50 | 200 | 5 | " |
| Comparative 4 | 200 | 0 | " |
| Comparative 5 | 200 | 0 | " |

* Comparative 4 : Zineb 72% wettable powder
* Comparative 5 : Chlorothalonil 75% wettable powder

Test Example 7 : Test on Tamato Late Blight Control(Preventive test)

To young tomato seedlings (Variety: Ogata-fukuju, at 5 to 6 leaved stage) grown in unglazed pottery pots, a wettable powder comprising the compound of the present invention was sprayed after diluting it to a solution at a prescribed concentration and dried, then the zoosporangial suspension of fungus, Phytophthora infestans cultivated on potato tubers beforehand, was sprayed to the seedlings for inoculation. After placing the seedlings in a humid room maintained at 20 °C for 4 days, the degree of the disease was assessed to determine the effectiveness of the compounds. The results are shown in Table 9.

Table 9.

| No. | Active Ingredient | Index | Phytotoxicity |
|---|---|---|---|
| I－2 | 200( ppm) | 4 | None |
| I－3 | 200 | 4 | 〃 |
| I－10 | 200 | 4 | 〃 |
| I－11 | 200 | 4 | 〃 |
| I－14 | 200 | 4 | 〃 |
| I－20 | 200 | 4 | 〃 |
| I－21 | 200 | 4 | 〃 |
| I－22 | 200 | 4 | 〃 |
| I－23 | 200 | 4 | 〃 |
| I－28 | 200 | 4 | 〃 |
| I－29 | 200 | 4 | 〃 |
| I－69 | 200 | 4 | 〃 |
| I－462 | 200 | 4 | 〃 |
| I－480 | 200 | 4 | 〃 |

Table 9. (continued)

| No. | Active Ingredient | Index | Phytotoxicity |
|---|---|---|---|
| III-13 | 200 (ppm) | 5 | None |
| III-16 | 200 | 5 | 〃 |
| III-18 | 200 | 5 | 〃 |
| III-19 | 200 | 5 | 〃 |
| III-32 | 200 | 5 | 〃 |
| III-39 | 200 | 5 | 〃 |
| III-41 | 200 | 4 | 〃 |
| Comparative 6 | 200 | 4 | 〃 |
| Comparative 7 | 200 | 3 | 〃 |

* Comparative 6 : Chlorothalonil 75% wetter powder

* Comparative 7 : Zineb 72% wetter powder

## Claims

1. Pyrimidine derivatives and the salts thereof represented by a general formula (I),

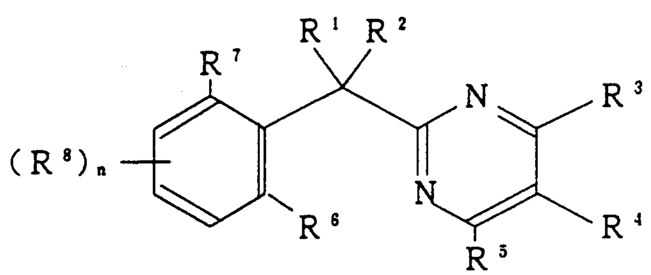

(I)

wherein $R^1$ and $R^2$ are each independently hydrogen; alkyl; alkenyl; alkynyl; phenyl; cyano; $COOR^9$ wherein $R^9$ is hydrogen or alkyl; halogen; $OR^{10}$ wherein $R^{10}$ is hydrogen, alkyl, alkenyl, alkynyl, phenyl or cycloalkyl; $SR^{11}$ wherein $R^{11}$ is hydrogen, alkyl, alkenyl, alkynyl, phenyl or cycloalkyl; $OCOR^{12}$ wherein $R^{12}$ is hydrogen, alkyl, alkenyl, alkynyl, phenyl or cycloalkyl; amino; $Z^1P(=Z^2)(Z^3R^{13})(Z^4R^{14})$ wherein $R^{13}$ and $R^{14}$ are each independently hydrogen, alkyl, alkenyl, alkynyl, phenyl or cycloalkyl, $Z^1$, $Z^2$, $Z^3$ and $Z^4$ are each independently O or S, or form in together oxo, thioxo, cyclic ketal, or cyclic thioketal; $=CR^{15}R^{16}$ wherein $R^{15}$ and $R^{16}$ are each independently hydrogen, nitro, cyano, alkyl, alkenyl, alkynyl, alkylcarbonyl, carbamoyl, carboxy, alkoxycarbonyl, alkenyloxycarbonyl, alkynyloxycarbonyl, phenyl, alkylsulfonyl or phenylsulfonyl; $=NR^{17}$ wherein $R^{17}$ is hydrogen, alkyl, alkenyl, alkynyl, phenyl or cycloalkyl; $=NNR^{18}R^{19}$ wherein $R^{18}$ and $R^{19}$ are each independently hydrogen, alkyl, alkenyl,

alkynyl, phenyl, cycloalkyl, alkoxycarbonyl or carboxy; $=NOR^{20}$ wherein $R^{20}$ is hydrogen, alkyl, alkenyl, alkynyl, phenyl or cycloalkyl; or any spiro ring selected from the group consisting of:

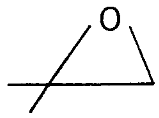 , 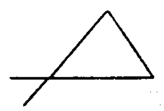 , 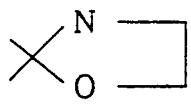 , 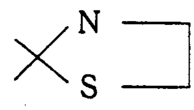 , and

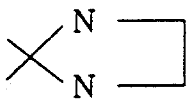

$R^3$ and $R^5$ are each independently hydrogen; halogen; alkyl; alkenyl; alkynyl; phenyl; cycloalkyl; $S(O)_m R^{21}$ wherein $R^{21}$ is hydrogen, alkyl, alkenyl, alkynyl, phenyl or cycloalkyl, and m denotes an integer 0, 1 or 2; $NR^{22}R^{23}$ wherein $R^{22}$ and $R^{23}$ are each independently hydrogen, alkyl, alkenyl, alkynyl, phenyl or cycloalkyl, or may form a ring in together; $NR^{24}NR^{25}R^{26}$ wherein $R^{24}$, $R^{25}$ and $R^{26}$ are each independently hydrogen, alkyl, alkoxycarbonyl or phenyl; $OR^{27}$ wherein $R^{27}$ is hydrogen, alkyl, alkenyl, alkynyl, phenyl or cycloalkyl; cyano; $COOR^{28}$ wherein $R^{28}$ is hydrogen, alkyl, alkenyl, alkynyl, phenyl or cycloalkyl; $CONR^{29}R^{30}$ wherein $R^{29}$ and $R^{30}$ are each independently hydrogen, alkyl, alkenyl, alkynyl, phenyl or cycloalkyl; $PZ^5(OR^{31})(OR^{32})$ wherein $Z^5$ is O or S, $R^{31}$ and $R^{32}$, which may be the same or different, represent alkyl or phenyl;

$R^4$ is hydrogen; halogen; amino; cyano; alkyl; alkenyl; alkynyl; phenyl; cycloalkyl; $COOR^{33}$ wherein $R^{33}$ is hydrogen, alkyl, alkenyl, alkynyl, phenyl or cycloalkyl; $COR^{34}$ wherein $R^{34}$ is hydrogen, alkyl, alkenyl, alkynyl, phenyl or cycloalkyl; $CONR^{35}R^{36}$ wherein $R^{35}$ and $R^{36}$ are each independently hydrogen, alkyl, alkenyl, alkynyl, phenyl or cycloalkyl; $OR^{37}$ wherein $R^{37}$ is hydrogen, alkyl, alkenyl, alkynyl, phenyl or cycloalkyl; $S(O)pR^{38}$ wherein $R^{38}$ is hydrogen, alkyl, alkenyl, alkynyl, phenyl or cycloalkyl, and p denotes an integer 0, 1 or 2, provided, however, when $R^4$ is hydrogen, $R^3$ and $R^5$ are different, and $R^4$ combines with $R^3$ or $R^5$ and a pyrimidine ring to form a condensed ring represented by one of the following formulas:

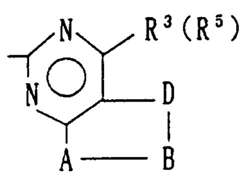 or 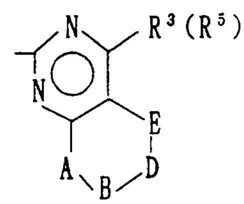

wherein one of A, B D and E represents:

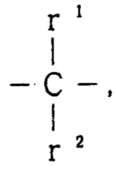

O, S(O)q or

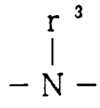

and the rest is

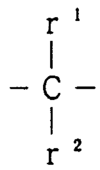

wherein herein $r^1$, $r^2$ and $r^3$ are each independently hydrogen, halogen, alkyl, phenyl, alkylcarbonyl, phenycarbony, alkoxycarbonyl, or they may form a double bond in combining adjacent $r^1$ or $r^3$, and q denotes an integar of 0, 1 or 2;

$R^6$ and $R^7$ are each independently hydrogen; halogen; alkyl; alkenyl; alkynyl; phenyl; cycloalkyl; S-(O)r$R^{39}$ wherein $R^{39}$ is alkyl, alkenyl, alkynyl, phenyl or cycloalkyl, and r denotes an integar of 0, 1 or 2; O$R^{40}$ where $R^{4\circ}$ is hydrogen, alkyl, alkenyl, alkynyl, phenyl or cycloalkyl; COO$R^{41}$ wherein $R^{41}$ is hydrogen, alkyl, alkenyl, alkynyl, phenyl or cycloalkyl; CON$R^{42}R^{43}$ wherein $R^{42}$ and $R^{43}$ are each independently hydrogen, alkyl, alkenyl, alkynyl, phenyl or cycloalkyl; amino; nitro; or cyano, provided, however, $R^6$ and $R^7$ are not hydrogen at the same time,

$R^8$ which may be the same or different, represents halogen; alkyl; alkenyl; alkynyl; phenyl; cycloalkyl; S(O)t$R^{44}$ wherein $R^{44}$ is alkyl, alkenyl, alkynyl, phenyl or cycloalkyl, and t denotes an integar of 0, 1 or 2; O$R^{45}$ wherein $R^{45}$ is hydrogen, alkyl, alkenyl, alkynyl, phenyl or cycloalkyl; COO$R^{46}$ wherein $R^{46}$ is hydrogen, alkyl, alkenyl, alkynyl, phenyl or cycloalkyl; CON$R^{47}R^{48}$ where in $R^{47}$ and $R^{48}$ are each independently hydrogen, alkyl, alkenyl, alkynyl, phenyl or cycloalkyl; amino; nitro; or cyano;

n denotes an integar from 0, 1, 2 or 3, provided, however, when both $R^1$ and $R^2$ are hydrogen, $R^4$ combines with $R^3$ or $R^5$ and a pyrimidine ring to form a condensed ring described above.

**2.** The pyrimidine derivatives according to Claim 1 wherein the condensed ring is:

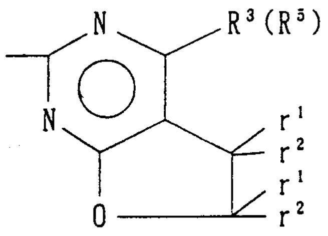
,
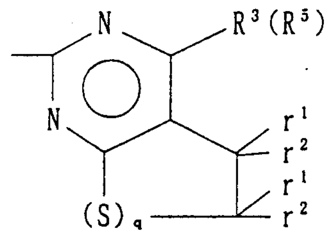
,
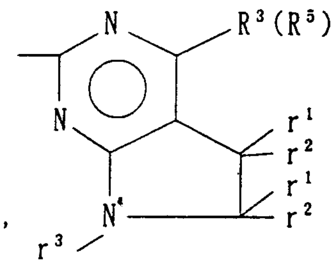

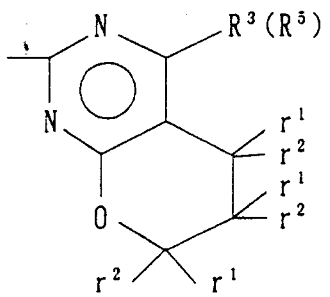
,
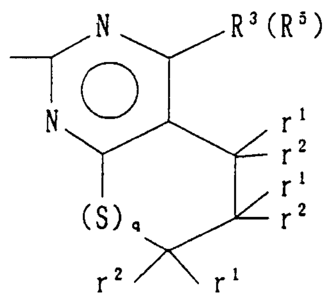

or

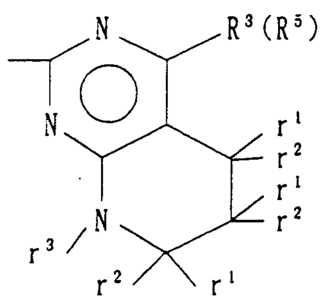

wherein $R^3$, $R^5$, $r^1$, $r^2$, $r^3$, and q are as defined above.

3. The pyrimidine derivatives according to Claims 1 or 2 wherein either of $R^1$ or $R^2$ is hydroxy or halogen, or $R^1$ and $R^2$ form in together a oxo-group.

4. The pyrimidine derivatives according to Claims 1 or 2 wherein either of $R^{15}$ or $R^{16}$ is carbamoyl or alkoxycarbonyl.

5. The pyrimidine derivatives according to Claims 1 or 2 wherein $R^{17}$ is alkyl substituted by

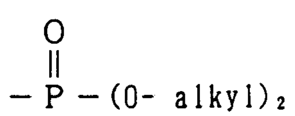
,
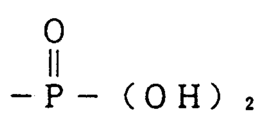
or
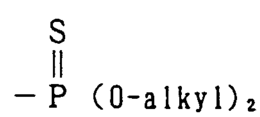
.

6. The pyrimidine derivatives according to Claims 1 or 2 wherein $R^3$ is haloalkyl or branched alkyl.

7. A herbicide comprising one or more of the pyrimidine derivatives represented by the following general formula (I) as the active ingredient:

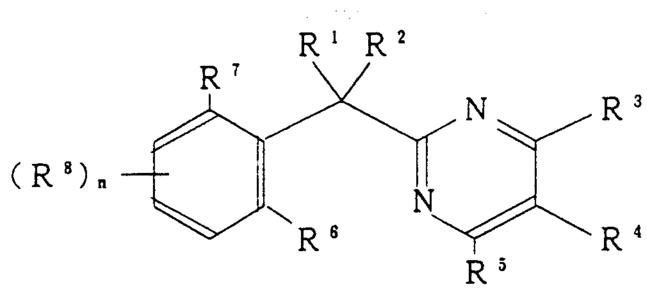

( I )

wherein $R^1$, $R^2$, $R^3$, $R^4$, $R^5$, $R^6$, $R^7$, $R^8$, and n are as defined above.

**8.** A fungicide for agricultural and horticultural use comprising one or more of the pyrimidine derivatives represented by the following general formula (I) as the active ingredient:

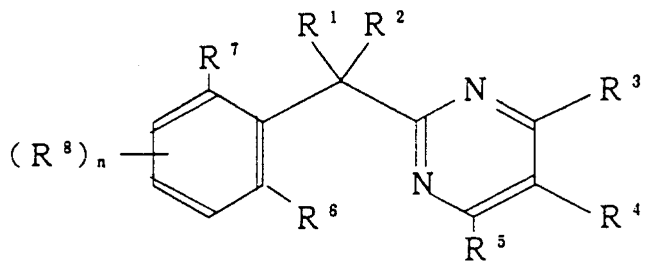

( I )

wherein $R^1$, $R^2$, $R^3$, $R^4$, $R^5$, $R^6$, $R^7$, $R^8$, and n are as defined as above.

# INTERNATIONAL SEARCH REPORT

International application No.
PCT/JP93/01478

**A. CLASSIFICATION OF SUBJECT MATTER**

Int. $Cl^5$ C07D239/26, C07D239/34, C07D239/38, C07D239/42, C07D401/04, C07D491/044, C07D495/04

According to International Patent Classification (IPC) or to both national classification and IPC

**B. FIELDS SEARCHED**

Minimum documentation searched (classification system followed by classification symbols)

Int. $Cl^5$ C07D239/26, C07D239/34, C07D239/38, C07D239/42, C07D401/04, C07D491/044, C07D495/04

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| X | JP, A, 3-58976 (Shering Agrochemicals Ltd.), March 14, 1991 (14. 03. 91), Claim & EP, A, 410590 | 1, 3, 6, 7, 8 |
| X | JP, A, 4-235967 (Sand AG.), August 25, 1992 (25. 08. 92), Claim & EP, A, 461079 | 1, 3, 6, 7, 8 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| December 15, 1993 (15. 12. 93) | January 11, 1994 (11. 01. 94) |

| Name and mailing address of the ISA/ | Authorized officer |
|---|---|
| Japanese Patent Office | |
| Facsimile No. | Telephone No. |